(19) Europäisches Patentamt
European Patent Office
Office européen des brevets

(11) EP 0 757 051 B1

(12) EUROPEAN PATENT SPECIFICATION

(45) Date of publication and mention of the grant of the patent:
**29.10.2003 Bulletin 2003/44**

(21) Application number: **96902477.7**

(22) Date of filing: **19.02.1996**

(51) Int Cl.⁷: **C07D 499/88**, A61K 31/43

(86) International application number:
**PCT/JP96/00366**

(87) International publication number:
**WO 96/025417 (22.08.1996 Gazette 1996/38)**

# (54) PENEM DERIVATIVES AND ANTIMICROBIAL AGENT CONTAINING THE SAME

PENEM-DERIVATE UND SIE ENTHALTENDE ANTI-MIKROBISCHE MITTEL

DERIVES DE PENEME ET AGENT ANTI-MICROBIEN LES CONTENANT

(84) Designated Contracting States:
**AT BE CH DE DK ES FR GB GR IE IT LI LU MC NL PT SE**

(30) Priority: **17.02.1995 JP 5205495**

(43) Date of publication of application:
**05.02.1997 Bulletin 1997/06**

(73) Proprietor: **Daiichi Suntory Pharma Co., Ltd.
Tokyo (JP)**

(72) Inventors:
- **ISHIGURO, Masaji
Takarazuka-shi, Hyogo 665 (JP)**
- **NAKATSUKA, Takashi
Mishima-gun, Osaka 618 (JP)**
- **TANAKA, Rie
Ibaraki-shi, Osaka 567 (JP)**
- **NAMIKAWA, Koshi
Hirakata-shi, Osaka 573 (JP)**
- **MATSUKI, Shinsuke
Ibaraki-shi, Osaka 567 (JP)**

(74) Representative: **Burford, Anthony Frederick
W.H. Beck, Greener & Co.
7 Stone Buildings
Lincoln's Inn
London WC2A 3SZ (GB)**

(56) References cited:
**EP-A- 0 038 869**
**GB-A- 2 109 379**
**JP-A- 58 008 085**
**JP-A- 60 034 970**
**JP-A- 60 222 486**

- **Chemistry and Biology of beta-lactam Antibiotics, vol.2, 1982, pages 311-361**
- **Ishiguro et al., J.Med.Chem., July 4, 1997; 40(14), pages 2126-2132**
- **Nomenclature of Organic Chemistry, Sections A,B,C,D,E,F and H, 1979 Edition, J. Rigaudy and S.P. Klesney, Pergamon Press, pages 5-7 and 16**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 757 051 B1

Printed by Jouve, 75001 PARIS (FR)

## Description

TECHNICAL FIELD

This invention relates to novel penem compounds, and more specifically to penem compounds equipped with antibacterial activities against various microorganisms, having effectiveness even against methicillin-resistant *Staphylococcus aureus* (MRSA) which has surfaced as a cause for nosocomial infection in recent years, and widely usable as drugs including animal drugs, and also to antibacterial agents containing same as effective ingredients.

BACKGROUND ART

A great deal of research has been conducted on penem antibiotics to date, as they have broad and strong antibacterial activities. From the results of such research, it has been ascertained that antibacterial activities of a penem compound significantly vary depending on the combination of steric configurations of three asymmetric carbons on the basal skeleton, namely, at the 1'-, 5- and 6 positions as numbered based on the below-described, commonly-employed penem skeleton, the kind of the substituent at the 2-position, and the like [for example, Chemistry and Biology of β-lactam Antibiotics, Vol. 2 (1982), pp.311-361, Eds. R.B. Morin and M. Gorman, Academic Press, New York].

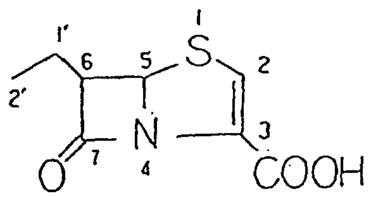

Those having the steric configuration of (1'R,5R, 6S) are considered to have the highest activities [for example, Yakugaku Zasshi, **107**, 175 (1987)]. Most of penem compounds which are known these days have this steric configuration.

Further, concerning those containing a hydroxyethyl group as a 6-substituent and having a steric configuration of (1'S,5R,6R), their activities have been reported (Tetrahedron Letters, 3485, 1981). They are however not sufficient in activities compared with those having the above-mentioned steric configuration of (1'R,5R,6S). Moreover, it is also known that, if the steric configuration of the 6-hydroxyalkyl group is (1'R,5R,6S), compounds containing a propyl or a higher alkyl group as the alkyl group are no longer equipped with any substantial activities [Japanese Patent Application Laid-Open (Kokai) No. SHO 60-222486 and Chemistry and Biology of β-lactam Antibiotics, vol. 2 (1982), p.357, Eds. R.B. Morin and M. Gorman, Academic Press, New York].

Accordingly, conversion of the 2-substituent alone has heretofore been considered to be effective for the improvement of activities of a penem compound.

In the meantime, it has become a serious problem that most of conventional antibiotics are ineffective against highly resistant MRSA (methicillin-resistant *Staphylococcus aureus)* which is recently increasing in number.

It is hence strongly desired to develop an antibiotic which is effective not only against many conventionally-known microorganisms but also against such MRSA.

GB-A-2042514 (published 24th September 1980) discloses that 2- substituted and 2,6-disubstituted penem compounds of the following formula are antibacterial agents:

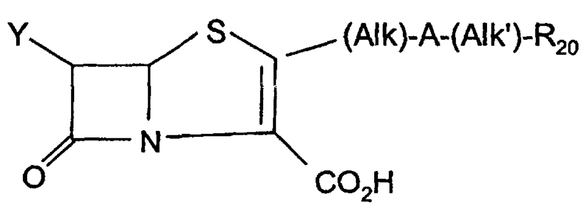

wherein:

Alk represents a $C_{1-2}$ alkylene group optionally substituted by a $C_{1-4}$ alkyl radical;

A is O, S, SO, $SO_2$ or $NR_{21}$ in which $R_{21}$ is hydrogen, $C_{1-6}$ alkyl, phenyl or phenyl $C_{1-6}$ alkyl;
Alk' is a $C_{2-4}$ alkylene group;
$R_{20}$ is a polar substituent selected from -NHOH, -N $R_{22}R_{23}$ (in which $R_{22}$ and $R_{23}$ are each independently hydrogen or $C_{1-6}$ alkyl) and $-NO_2$; and
Y is *inter alia* $C_{1-6}$ aliphatic substituted by inter alia hydroxy, preferably $C_{1-6}$ alkyl substituted (preferably at the α-carbon) by hydroxy.

The most preferred compounds are those wherein Y is α-hydroxyethyl and there is no exemplification of hydroxypropyl or higher hydroxyalkyl groups. There is no reference to treating methicillin-resistant Staphylococcus aureus (MRSA).

GB-A-2042515 (published 24th September 1980) discloses that 2- substituted and 2,6-disubstituted penem compounds of the following formula are antibacterial agents:

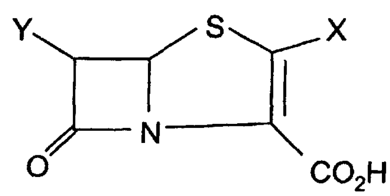

wherein:

X is *inter alia* a substituted $C_{1-6}$ aliphatic, $C_{3-8}$ cycloaliphatic or $C_{3-8}$ cycloaliphatic-$C_{1-6}$ aliphatic radical or a ring-substituted phenyl, phenyl-$C_{1-6}$ alkyl, heterocycle, heterocycle-$C_{1-6}$ alkyl or heterocyclothio-$C_{1-6}$ alkyl group, said substituents being selected from specified moieties; and
Y is *inter alia* $C_{1-6}$ aliphatic substituted by *inter alia* hydroxy, preferably $C_{1-6}$ alkyl substituted (preferably at the α-carbon) by hydroxy.

The most preferred compounds are those wherein Y is α-hydroxyethyl but specified higher hydroxyalkyl Y groups are α-hydroxypropyl, α-hydroxy-isopropyl, α-hydroxybutyl, and α-hydroxypentyl. There is no reference to treating methicillin-resistant Staphylococcus aureus (MRSA).

The only exemplified α-hydroxypropyl compounds in GB-A-2042515 are in a mixture of (1'S,5R,6S & 1'R,5S,6R)6-1'-hydroxy-1'-propyl)-2-methylpenem-3-carboxylic acid sodium salt (Example 48) or (1'R,5R,6S & 1'S,5S,6R) 6-1'-hydroxy-1'-propyl)-2-methylpenem-3-carboxylic acid mixed potassium and sodium salts (Example 49). The Minimum Inhibitory Concentration ("MIC") in mcg/ml of each these mixtures for each of the Staphylococcus aureus tested was as set forth in the following table together with data for the corresponding (1'R,5R,6R & 1'S,5S,6S)6-1'-hydroxy-1'-ethyl)-2-methylpenem-3-carboxylic acid mixed potassium and sodium salts (Example 34), (1'S,5R,6S & 1'R,5S,6R) 6-1'-hydroxy-1'-ethyl)-2-methylpenem-3-carboxylic acid (Example 35), (1'R,5R,6S & 1'S,5S,6R)6-1'-hydroxy-1'-ethyl)-2-methylpenem-3-carboxylic acid (Example 36), or (1'S,5R,6R & 1'R,5S,6S)6-1'-hydroxy-1'-ethyl)-2-methylpenem-3-carboxylic acid (Example 37):

MIC in mcg/ml for GB-A-2042515 Examples 34-37, 48 & 49

| Example | Configuration | S. aureus A9537 | S. aureus A9606 | S. aureus A15097 |
|---|---|---|---|---|
| 34 | 1'R,5R,6R & 1'S,5S,6S | 4 | 4 | 16 |
| 35 | 1'S,5R,6S & 1'R,5S,6R | >125 | >125 | >125 |
| 36 | 1'R,5R,6S & 1'S,5S,6R | 2 | 4 | 8 |
| 37 | 1'S,5R,6R & 1'R,5S,6S | 0.5 | 1 | 1 |
| 48 | 1'S,5R,6S & 1'R,5S,6R | >125 | >125 | >125 |
| 49 | 1'R,5R,6S & 1'S,5S,6R | 32 | 32 | >125 |

EP-A-0038869 (published 4th November 1981) relates to the preparation of antibiotic 2- substituted and

2,6-disubstituted carbapenem compounds of the following formula:

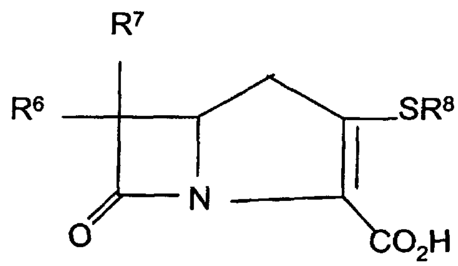

wherein $R^6$, $R^7$, and $R^8$ are independently selected from hydrogen, substituted and unsubstituted: alkyl, alkenyl, and alkynyl, having from 1-10 carbon atoms; cycloalkyl, cycloalkylalkyl, and alkylcycloalkyl, having 3-6 carbon atoms in the cycloalkyl ring and 1-6 carbon atoms in the alkyl moieties; aryl; aralkyl, aralkenyl, and aralkynyl wherein the aryl moiety is phenyl and the aliphatic portion has 1-6 carbon atoms; heteroaryl, heteroaralkyl, heterocyclyl and heterocyclylalkyl; the optional substituent (s) including *inter alia* hydroxy.

One specified class of compound are those in which $R^6$ is $\alpha$-hydroxyalkyl, preferably $\alpha$-hydroxyethyl, and exemplified compounds include the compound in which $R^6$ is $\alpha$-hydroxypropyl, $R^7$ is hydrogen, & $R^8$ is (o-aminomethyl)-phenyl (Table III, Compound 16). There is no reference to treating methicillin-resistant Staphylococcus aureus (MRSA).

GB-A-2104509 (published 9th March 1983) relates to antibacterial *trans*-6-substituted 5(R) 2-penem-3-carboxylic acid derivatives of the following formula:

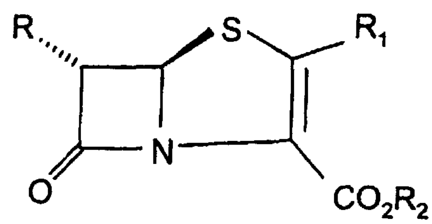

wherein:

R and $R_1$ independently are *inter alia* a group of formula $-(CH_2)Q-T$ (in which x is 0 or an integer of 1 to 10; Q is a bond; a saturated or unsaturated aliphatic chain, -O-, -S-, -SO-, $-SO_2-$, -N=; or $-NR_a-$ (wherein $R_a$ is hydrogen or $C_{1-12}$ alkyl); and T is a saturated or unsaturated aliphatic hydrocarbon, an aromatic, a cycloaliphatic or a heterocyclic group, optionally substituted by *inter alia* hydroxy); and
$R_2$ is hydrogen or a cation.

There is a reference to 8(R), 6(S)-hydroxyethyl-5(R)-penems of the above formula being obtained as single diastereoisomers but no 6- hydroxyalkyl compounds are specified. There is no reference to treating methicillin-resistant Staphylococcus aureus (MRSA).

GB-A-2109379 (published 2nd June 1983; divided from GB-A-2042515) discloses a process for preparing the antibacterial 2- substituted and 2,6-disubstituted penem compounds of GB-A-2042515 from corresponding 1-(carbonylmethylphosphoranyl)-2-azetidinone derivatives. Exemplified processes include the preparation of the mixture of (1'R,5R,6S & 1'S,5S,6R)6-1'-hydroxy-1'-propyl)-2-methylpenem-3-carboxylic acid mixed potassium and sodium salts (Example 39). There is no reference to treating methicillin-resistant Staphylococcus aureus (MRSA).

EP-A-0125207 (published 14th November 1984) discloses that 2-heterocyclyl-lower alkyl-6-hydroxy-lower alkyl penem compounds of the following formula are antibacterial agents:

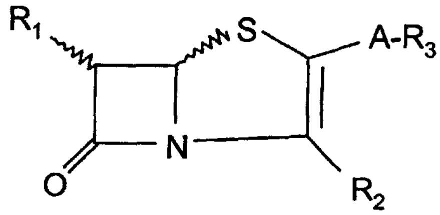

wherein:

$R_1$ is hydroxy-substituted lower alkyl;
$R_2$ is carboxy or functionally modified carboxy;
$R_3$ is an unsaturated monocyclic azaheterocyclyl radical bonded via a tertiary ring nitrogen atom to the radical -A-; and
A represents a lower alkylene radical.

The preferred compounds are those wherein $R_1$ is hydroxyethyl or α-hydroxyethyl but specified $R_1$ groups include α-hydroxypropyl, α-hydroxy-isopropyl, α-hydroxy-butyl, and α-hydroxy-s- butyl. In the exemplified compounds, $R_1$ is hydroxymethyl, α-hydroxyethyl or α-hydroxy-isopropyl. There is no reference to treating methicillin-resistant Staphylococcus aureus (MRSA).

The present inventors focused on penem compounds, and with a view to finding a compound having still broader and high antibacterial activities, they synthesized numerous penem derivatives by changing the kind and steric configuration of the 6-substituent group, the steric configuration on the β-lactam ring, the 2-substituent and the like, and investigated their pharmacological effects.

As a result, it has been found that penem derivatives containing a specific substituent and a particular steric configuration have broad and high antibacterial activities and especially are effective even against MRSA, leading to the completion of the present invention.

DISCLOSURE OF THE INVENTION

The present invention provides (5R,6R)-6-((S)-1-Hydroxypropyl)-penem derivatives represented by the following formula (I):

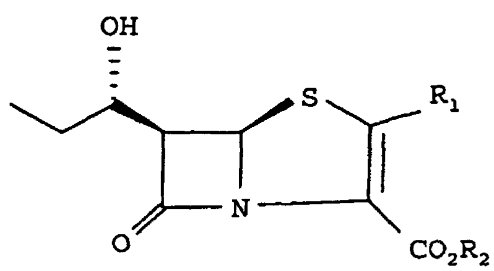

(I)

wherein:

$R_1$ represents an alkyl, alkenyl, aralkyl, aryl, alkylthio, alkenylthio, $C_{7-14}$ aralkylthio, $C_{6-10}$ arylthio, heterocyclic, heterocyclic thio, acylthio, or mercapto group or a hydrogen atom, any of said groups being optionally substituted with one or more selected from halogen; carboxyl; thiocarboxyl; formyl; nitro; cyano; hydroxyl; amino; imino; ($C_{1-6}$ alkylene)acetal; $C_{1-6}$ alkyl; monocyclic or polycyclic alkyl (which may be in the form of a fused ring with an aromatic hydrocarbon and may contain one or more carbonyl groups in its chain or ring); $C_{2-6}$ alkenyl; $C_{6-10}$ aryl; $C_{7-24}$ aralkyl; $C_{1-6}$ alkylthio; $C_{2-6}$ alkenylthio; $C_{7-24}$ aralkylthio; $C_{6-10}$ arylthio; $C_{1-6}$ alkyloxy; $C_{2-6}$ alkenyloxy; $C_{7-24}$ aralkyloxy; $C_{6-10}$ aryloxy; $C_{1-6}$ alkylsulfinyl; $C_{1-6}$ alkylsulfonyl; $C_{7-24}$ aralkylsulfinyl; $C_{7-24}$ aralkylsulfonyl; $C_{6-10}$ arylsulfinyl; $C_{6-10}$ arylsulfonyl; aminosulfonyl; carbamoyl; carbamoyloxy; carbamoyl $C_{1-6}$ alkyl; imino($C_{1-6}$ alkyl); imino($C_{1-6}$ alkyl)amino; imino(amino)-($C_{1-6}$ alkyl); acyloxy; acylalkyl; silyloxy; heterocyclic; heterocyclic thio; heterocyclic oxy; acyl; esterified carboxyl; esterified thiocarboxyl, or, only when $R_1$ is heterocyclic or heterocyclic thio, unsaturated

$C_{5-7}$ cycloalkyl or $C_{9-11}$ fused rings optionally containing one or more carbonyl groups in their rings; and said heterocyclic group or moiety being selected from 3-8 membered saturated or unsaturated, heteromonocyclic groups containing 1 to 4 nitrogen atoms; 1 or 2 oxygen atoms and 1 to 3 nitrogen atoms; 1 or 2 sulfur atoms and 1 to 3 nitrogen atoms; 1 or 2 oxygen atoms; or 1 sulfur atom; and 7-12 membered, unsaturated, heteropolycyclic groups containing 1 to 5 nitrogen atoms; 1 or 2 oxygen atoms and 1 to 3 nitrogen atoms; or 1 or 2 sulfur atoms and 1 to 3 nitrogen atoms; wherein alkyl or alkyl as part of an alkylthio, aralkyl or aralkylthio group includes monocyclic or polycyclic alkyl which may be in the form of a fused ring with an aromatic hydrocarbon and may contain one or more carbonyl groups in its chain or ring, and

$R_2$ represents a hydrogen atom or a carboxyl-protecting group which is removable *in vivo* to form the free acid; or a pharmacologically acceptable salt thereof.

The present invention also provides a medicament or antibacterial agent which comprises, as an active ingredient, a penem derivative represented by the formula (I) or a pharmacologically acceptable salt thereof.

The present invention also provides the (5R,6R)-6-((S)-1-hydroxypropyl)-penem derivatives represented by formula (I) for use in therapy and their use in the manufacture of an antibacterial medicament, especially for the treatment of infection with methicillin-resistant Staphylococcus aureus.

The present invention further provides compounds useful as a synthesis intermediate for the penem derivative represented by the formula (I), which is represented by the following formula (II):

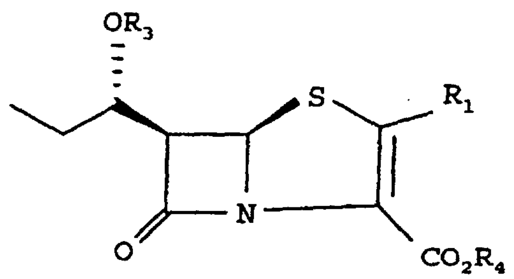

(II)

wherein $R_1$ is as defined above in connection with formula I, $OR_3$ represents a protected hydroxyl group, and $R_4$ represents a carboxyl-protecting group; or by the following formula (III) :

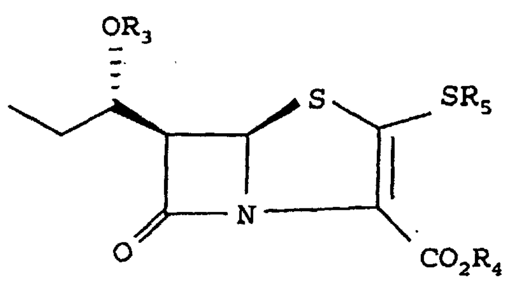

(III)

wherein:

$R_5$ represents an alkyl, alkenyl, $C_{7-14}$ aralkyl, $C_{6-10}$ aryl,heterocyclic or acyl group, of said groups being optionally substituted with one or more selected from halogen; carboxyl; thiocarboxyl; formyl; nitro; cyano; hydroxyl; amino; imino; ($C_{1-6}$ alkylene)acetal; $C_{1-6}$ alkyl; monocyclic or polycyclic alkyl (which may be in the form of a fused ring with an aromatic hydrocarbon and may contain one or more carbonyl groups in its chain or ring); $C_{2-6}$ alkenyl; $C_{6-10}$ aryl; $C_{7-24}$ aralkyl; $C_{1-6}$ alkylthio; $C_{2-6}$ alkenylthio; $C_{7-24}$ aralkylthio; $C_{6-10}$ arylthio; $C_{1-6}$ alkyloxy; $C_{2-6}$ alkenyloxy; $C_{7-24}$ aralkyloxy; $C_{6-10}$ aryloxy; $C_{1-6}$ alkylsulfinyl; $C_{1-6}$ alkylsulfonyl; $C_{7-24}$ aralkylsulfinyl; $C_{7-24}$ aralkylsulfonyl; $C_{6-10}$ arylsulfinyl; $C_{6-10}$ arylsulfonyl; aminosulfonyl; carbamoyl; carbamoyloxy; carbamoyl $C_{1-6}$ alkyl; imino($C_{1-6}$ alkyl) ; imino($C_{1-6}$ alkyl)amino; imino(amino)($C_{1-6}$ alkyl); acyloxy; acylalkyl; silyloxy; heterocyclic; heterocyclic thio; heterocyclic oxy; acyl; esterified carboxyl; esterified thiocarboxyl, or, only when $R_1$ is heterocyclic, unsaturated $C_{5-7}$ cycloalkyl or $C_{9-11}$ fused rings optionally containing one or more carbonyl groups in their rings; and said

heterocyclic group being selected from 3-8 membered saturated or unsaturated, heteromonocyclic groups containing 1 to 4 nitrogen atoms; 1 or 2 oxygen atoms and 1 to 3 nitrogen atoms; 1 or 2 sulfur atoms and 1 to 3 nitrogen atoms; 1 or 2 oxygen atoms; or 1 sulfur atom; and 7-12 membered, unsaturated, heteropolycyclic groups containing 1 to 5 nitrogen atoms; 1 or 2 oxygen atoms and 1 to 3 nitrogen atoms; or 1 or 2 sulfur atoms and 1 to 3 nitrogen atoms; wherein alkyl or alkyl as part of an alkylthio, aralkyl or aralkylthio group includes monocyclic or polycyclic alkyl which may be in the form of a fused ring with an aromatic hydrocarbon and may contain one or more carbonyl groups in its chain or ring,

$OR_3$ represents a protected hydroxyl group, and $R_4$ represents a carboxyl-protecting group, or by the following formula (IV):

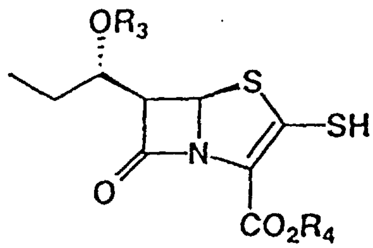

(IV)

wherein $OR_3$ represents a protected hydroxyl group and $R_4$ represents a carboxyl-protecting group.

## BEST MODE FOR CARRYING OUT THE INVENTION

In the penem derivatives (I) and (II) according to the present invention, preferred examples of $R_1$ include a hydrogen atom, a mercapto group, alkyl groups, alkenyl groups, aralkyl groups, aryl groups, alkylthio groups, alkenylthio groups, aralkylthio groups and arylthio groups. Specific examples will be described below. Further, in the compound (III), preferred examples of $R_5$ include alkyl groups, alkenyl groups, aralkyl groups and aryl groups which will be similarly exemplified below. Incidentally, throughout the present description, the term "lower" means preferably a carbon number of from 1 to 6, particularly preferably a carbon number of from 1 to 4 unless specifically indicated.

Namely, examples of the alkyl group and the alkyl group in the alkylthio group include linear or branched lower alkyl groups such as methyl, ethyl, n-propyl, isopropyl, cyclopropylmethyl, n-butyl, tert-butyl and hexyl; and monocyclic or polycyclic alkyl groups which may be in the form of a fused ring with an aromatic hydrocarbon, such as cyclopropyl, cyclopentyl, cyclohexyl, menthyl, fenchyl, bornyl and indanyl. They may contain one or more carbonyl groups in their chains or rings. Further, examples of the alkenyl group and the alkenyl group in the alkenylthio group include linear or branched lower alkenyl groups, such as vinyl, allyl, 1-propenyl, 2-butenyl and 2-methyl-2-propenyl.

In addition, examples of the aralkyl group and the aralkyl group in the aralkylthio group include aralkyl groups containing 7 to 24 carbon atoms, such as benzyl, phenethyl, 3-phenylpropyl, 2-naphthylmethyl, 2-(1-naphthyl)ethyl, trityl, benzhydryl and 1-phenyl-cyclopropan-1-yl. Examples of the aryl group and the aryl group in the arylthio group include aryl groups containing 6 to 10 carbon atoms, such as phenyl and naphthyl.

These alkyl, alkenyl, aralkyl, aryl, alkylthio, alkenylthio, aralkylthio and arylthio groups may each be substituted by one or more substituents.

Illustrative of these substituents are halogen atoms such as fluorine atom, chlorine atom and bromine atom; carboxyl group; thiocarboxyl group; formyl group; nitro group; cyano group; hydroxyl group; amino group; imino group; lower alkylene acetal groups; linear or branched lower alkyl groups such as methyl, ethyl, n-propyl, isopropyl, cyclopropylmethyl, n-butyl, tert-butyl and hexyl; monocyclic or polycyclic alkyl groups such as cyclopropyl, cyclopentyl, cyclohexyl, menthyl, fenchyl, bornyl and indanyl, each of which may be in the form of a fused ring with an aromatic hydrocarbon and may contain one or more carbonyl groups in its chain or ring; linear or branched lower alkenyl groups such as vinyl, allyl, 1-propenyl, 2-butenyl and 2-methyl-2-propenyl; aryl groups containing 6 to 10 carbon atoms, such as phenyl and naphthyl; and aralkyl groups containing 7 to 24 carbon atoms, such as benzyl, phenethyl, 3-phenylpropyl, 2-naphthylmethyl, 2-(1-naphthyl)ethyl, trityl, benzhydryl and 1-phenyl-cyclopropan-1-yl.

Exemplary substituents include alkylthio, alkenylthio, aralkylthio, arylthio, alkyloxy, alkenyloxy, aralkyloxy and aryloxy groups, which correspond to the above-described alkyl, alkenyl, aralkyl and aryl groups, respectively; alkylsulfinyl and alkylsulfonyl groups corresponding to the above-described alkyl groups; aralkylsulfinyl and aralkylsulfonyl groups corresponding to the above-described aralkyl groups; arylsulfinyl and arylsulfonyl groups corresponding to the above-described aryl groups; aminosulfonyl groups; carbamoyl groups; carbamoyloxy groups; carbamoylalkyl groups;

imino(lower alkyl) groups; imino(lower alkyl)amino groups; imino(amino)(lower alkyl) groups; acyloxy and acylalkyl groups corresponding to the below-described acyl groups; and silyloxy, heterocyclic, heterocyclic thio, heterocyclic oxy, acyl, esterified carboxyl and esterified thiocarboxyl groups, which will be described subsequently herein.

The above-described substituents may each be substituted further by one or more substituents, for example, one or more of the above-described substituents. Illustrative of further substituents for the above-described alkyl substituents (which are also equally applicable to the alkylthio, alkyloxy, alkylsulfinyl and alkylsulfonyl substituents) are halogen atoms, and carboxyl, thiocarboxyl, formyl, nitro, cyano, hydroxyl, amino, (lower alkylene)acetal, alkenyl, aryl, aralkyl, alkylthio, alkenylthio, aralkylthio, arylthio, alkyloxy, alkenyloxy, aralkyloxy, aryloxy, alkylsulfinyl, alkylsulfonyl, aralkylsulfinyl, aralkylsulfonyl, arylsulfinyl, arylsulfonyl, aminosulfonyl, carbamoyl, carbamoyloxy, imino, imino(lower alkyl)amino, imino-(amino) lower alkyl , acyloxy, silyloxy, heterocyclic, heterocyclic thio, heterocyclic oxy, acyl, esterified carboxyl and esterified thiocarboxyl groups.

Illustrative of further substituents for the above-described alkenyl substituents (which are also equally applicable to the alkenylthio and alkenyloxy substituents) are halogen atoms, and carboxyl, thiocarboxyl, formyl, nitro, cyano, hydroxyl, amino, imino, lower alkylene acetal, alkyl, aryl, aralkyl, alkylthio, alkenylthio, aralkylthio, arylthio, alkyloxy, alkenyloxy, aralkyloxy, aryloxy, alkylsulfinyl, alkylsulfonyl, aralkylsulfinyl, aralkylsulfonyl, arylsulfinyl, arylsulfonyl, aminosulfonyl, carbamoyl, carbamoyloxy, carbamoylalkyl, imino lower alkyl, imino-lower alkyl amino, imino(amino) lower alkyl, acyloxy, acylalkyl, silyloxy, heterocyclic, heterocyclic thio, heterocyclic oxy, acyl, esterified carboxyl and esterified thiocarboxyl groups.

Illustrative of further substituents for the above-described aralkyl substituents (which are also equally applicable to the aralkylthio, aralkyloxy, aralkylsulfinyl and aralkylsulfonyl substituents) are halogen atoms, and carboxyl, thiocarboxyl, formyl, nitro, cyano, hydroxyl, amino, imino, lower alkylene-acetal, alkyl, alkenyl, aryl, aralkyl, alkylthio, alkenylthio, aralkylthio, arylthio, alkyloxy, alkenyloxy, aralkyloxy, aryloxy, alkylsulfinyl, alkylsulfonyl, aralkylsulfinyl, aralkylsulfonyl, arylsulfinyl, arylsulfonyl, aminosulfonyl, carbamoyl, carbamoyloxy, carbamoylalkyl, imino lower alkyl , imino-lower alkyl amino, imino(amino) lower alkyl , acyloxy, acylalkyl, silyloxy, heterocyclic, heterocyclic thio, heterocyclic oxy, acyl, esterified carboxyl and esterified thiocarboxyl groups.

In addition, illustrative of further substituents for the above-described aryl substituents (which are also equally applicable to the arylthio, aryloxy, arylsulfinyl and arylsulfonyl substituents) are halogen atoms, and carboxyl, thiocarboxyl, formyl, nitro, cyano, hydroxyl, amino, imino, lower alkylene acetal, alkyl, alkenyl, aryl, aralkyl, alkylthio, alkenylthio, aralkylthio, arylthio, alkyloxy, alkenyloxy, aralkyloxy, aryloxy, alkylsulfinyl, alkylsulfonyl, aralkylsulfinyl, aralkylsulfonyl, arylsulfinyl, arylsulfonyl, aminosulfonyl, carbamoyl, carbamoyloxy, carbamoylalkyl, imino lower alkyl , imino lower alkyl - amino, imino(amino) lower alkyl , acyloxy, acylalkyl, silyloxy, heterocyclic, heterocyclic thio, heterocyclic oxy, acyl, esterified carboxyl and esterified thiocarboxyl groups.

On the other hand, illustrative of further substituents for the amino, imino, aminosulfonyl, carbamoyl, carbamoyloxy, carbamoylalkyl, imino lower alkyl , imino lower alkyl amino and imino(amino) lower alkyl substituents out of the substituents are halogen atoms, and carboxyl, thiocarboxyl, formyl, nitro, cyano, hydroxyl, amino, imino, lower alkylene acetal, alkyl, alkenyl, aryl, aralkyl, alkylthio, alkenylthio, aralkylthio, arylthio, alkyloxy, alkenyloxy, aralkyloxy, aryloxy, alkylsulfinyl, alkylsulfonyl, aralkylsulfinyl, aralkylsulfonyl, arylsulfinyl, arylsulfonyl, aminosulfonyl, carbamoyl, carbamoyloxy, carbamoylalkyl, imino lower alkyl , imino lower alkyl - amino, imino(amino) lower alkyl , acyloxy, acylalkyl, silyloxy, heterocyclic, heterocyclic thio, heterocyclic oxy, acyl, esterified carboxyl and esterified thiocarboxyl groups.

Other preferred examples of $R_1$ in the penem derivatives (I) and (II) according to the present invention include heterocyclic groups and heterocyclic thio groups. Specifically, those to be described next can be exemplified. Further, other preferred examples of $R_5$ in the compound (III) similarly include heterocyclic groups to be exemplified next.

Namely, the heterocyclic group and the heterocyclic group of the heterocyclic thio group (and also the heterocyclic group of the heterocyclic oxy group described above as a substituent) individually mean a saturated or unsaturated, single-ring or multiple-ring, heterocyclic group containing at least one hetero atom such as an oxygen atom, sulfur atom or nitrogen atom. Preferred examples of such heterocyclic groups include 3-8 membered, particularly preferably 5- or 6-membered, unsaturated, heteromonocyclic groups containing 1 to 4 nitrogen atoms; 3-8 membered, particularly preferably 5- or 6-membered, saturated, heteromonocyclic groups containing 1 to 4 nitrogen atoms; 7-12 membered, unsaturated, heteropolycyclic groups containing 1 to 5 nitrogen atoms; 3-8 membered, particularly preferably 5- or 6-membered, unsaturated, heteromonocyclic groups containing 1 or 2 oxygen atoms and 1 to 3 nitrogen atoms; 3-8 membered, particularly preferably 5- or 6-membered, saturated, single-ring heterocyclic groups containing 1 or 2 oxygen atoms and 1 to 3 nitrogen atoms; 7-12 membered, unsaturated, heteropolycyclic groups containing 1 or 2 oxygen atoms and 1 to 3 nitrogen atoms; 3-8 membered, particularly preferably 5- or 6-membered, unsaturated, heteromonocyclic groups containing 1 or 2 sulfur atoms and 1 to 3 nitrogen atoms; 3-8 membered, particularly preferably 5- or 6-membered, saturated, heteromonocyclic groups containing 1 or 2 sulfur atoms and 1 to 3 nitrogen atoms; 7-12 membered, unsaturated, heteropolycyclic groups containing 1 or 2 sulfur atoms and 1 to 3 nitrogen atoms; 3-8 membered, particularly preferably 5- or 6-membered, unsaturated, heteromonocyclic groups containing 1 or 2 oxygen atoms; 3-8 membered, particularly preferably 5-or 6-membered, saturated, heteromonocyclic groups containing 1 or

2 oxygen atoms; 3-8 membered, particularly preferably 5- or 6-membered, unsaturated, heteromonocyclic groups containing one sulfur atom; and 3-8 membered, particularly preferably 5- or 6-membered, saturated, heteromonocyclic groups containing one sulfur atom.

Specific examples of the above-described heterocyclic groups include, as 3-8 membered, unsaturated, heteromonocyclic groups containing 1 to 4 nitrogen atoms, pyrrolyl, pyrrolinyl, imidazolyl, pyrazolyl, pyridyl, pyrimidyl, pyrazinyl, pyridazinyl, triazolyl (for example, 4H-1,2,4-triazolyl, 1H-1,2,3-triazolyl and 2H-1,2,3-triazolyl), tetrazolyl (for example, 1H-tetrazolyl and 2H-tetrazolyl), and dihydrotriazinyl (for example, 4,5-dihydro-1,2,4-triazinyl and 2,5-dihydro-1,2,4-triazinyl) groups; as 3-8 membered, saturated, heteromonocyclic groups containing 1 to 4 nitrogen atoms, azetidinyl, pyrrolidinyl, imidazolidinyl, piperidinyl, pyrazolidinyl and piperazinyl groups; and as 7-12 membered, unsaturated, heteropolycyclic groups containing 1 to 5 nitrogen atoms, indolyl, isoindolyl, indolizinyl, benzimidazolyl, quinolyl, isoquinolyl, indazolyl, benzotriazolyl, tetrazolopyridyl, tetrazolopyridazinyl (for example, tetrazolo[1,5-b]pyridazinyl), dihydrotriazolopyridazinyl, and 6,7-dihydro-5H-cyclopenta[b]pyridin-7-yl groups.

Illustrative of the 3-8 membered, unsaturated, heteromonocyclic groups containing 1 or 2 oxygen atoms and 1 to 3 nitrogen atoms are oxazolyl, isooxazolyl, oxadiazolyl (for example, 1,2,4-oxadiazolyl, 1,3,4-oxadiazolyl and 1,2,5-oxadiazolyl) groups; illustrative of the 3-8 membered, saturated, heteromonocyclic groups containing 1 or 2 oxygen atoms and 1 to 3 nitrogen atoms is a morpholinyl group; and illustrative of the 7-12 membered, unsaturated, heteropolycyclic groups containing 1 or 2 oxygen atom and 1-3 nitrogen atoms are benzoxazolyl and benzoxadiazolyl groups.

Further, examples of the 3-8 membered, unsaturated, heteromonocyclic groups containing 1 or 2 sulfur atoms and 1 to 3 nitrogen atoms include 1,3-thiazolyl, 1,2-thiazolyl, thiazolinyl, and thiadiazolyl (for example, 1,2,4-thiadiazolyl, 1,3,4-thiadiazolyl, 1,2,5-thiadiazolyl and 1,2,3-thiadiazolyl) groups; examples of 3-8 membered, saturated, heteromonocyclic groups containing 1 or 2 sulfur atoms and 1-3 nitrogen atoms include a thiazolidinyl group; and examples of the 7-12 membered, unsaturated, heteropolycyclic groups containing 1 or 2 sulfur atoms and 1 to 3 nitrogen atoms include benzothiazolyl and benzothiadiazolyl groups.

In addition, illustrative of the 3-8 membered, unsaturated, heteromonocyclic groups containing 1 or 2 oxygen atoms include furanyl and pyranyl groups; illustrative of the 3-8 membered, saturated, heteromonocyclic groups containing 1 or 2 oxygen atoms include tetrahydrofuranyl and tetrahydropyranyl groups; illustrative of the 3-8 membered, unsaturated, heteromonocyclic groups containing one sulfur atom is a thienyl group; and illustrative of the 3-8 membered, saturated, heteromonocyclic groups containing one sulfur atom is a tetrahydrothienyl group.

The heterocyclic groups may include, in addition to those exemplified above, their N-oxides and S-oxides and those containing one or more carbonyl groups in their rings. In the case of heterocyclic groups containing a tertiary nitrogen atom, the nitrogen atom may be bonded to an appropriate substituent [for example, a lower alkyl group, a hydroxy lower alkyl group or the like] to form an intramolecular quaternary salt, for example, an N-methylpyridinium group or the like.

These heterocyclic groups may each be substituted by one or more substituents. Examples of such substituents include halogen atoms such as fluorine atom, chlorine atom and bromine atom; carboxyl group; thiocarboxyl group; formyl group; nitro group; cyano group; hydroxyl group; amino group; imino group; lower alkylene acetal groups; linear or branched lower alkyl groups such as methyl, ethyl, n-propyl, isopropyl, cyclopropylmethyl, n-butyl, tert-butyl and hexyl; monocyclic or polycyclic alkyl groups such as cyclopropyl, cyclopentyl, cyclohexyl, menthyl, fenchyl, bornyl and indanyl, each of which may be in the form of a fused ring with an aromatic hydrocarbon and may contain one or more carbonyl groups in its chain or ring; linear or branched lower alkenyl groups such as vinyl, allyl, 1-propenyl, 2-butenyl and 2-methyl-2-propenyl; aryl groups containing 6 to 10 carbon atoms, such as phenyl and naphthyl; and aralkyl groups containing 7 to 24 carbon atoms, such as benzyl, phenethyl, 3-phenyl-propyl, 2-naphthylmethyl, 2-(1-naphthyl)ethyl, trityl, benzhydryl and l-phenyl-cyclopropan-1-yl.

Further, exemplary substituents include alkylthio, alkenylthio, aralkylthio, arylthio, alkyloxy, alkenyloxy, aralkyloxy and aryloxy groups, which correspond to the above-described alkyl, alkenyl, aralkyl and aryl groups, respectively; alkylsulfinyl and alkylsulfonyl groups corresponding to the above-described alkyl groups; aralkylsulfinyl and aralkylsulfonyl groups corresponding to the above-described aralkyl groups; arylsulfinyl and arylsulfonyl groups corresponding to the above-described aryl groups; aminosulfonyl groups; carbamoyl groups; carbamoyloxy groups; carbamoylalkyl groups; imino(lower alkyl) groups; imino(lower alkyl)amino groups; imino(amino) (lower alkyl) groups; unsaturated cyclic compound groups containing 5 to 7 carbon atoms, such as cyclohexenyl and cycloheptatrienyl, and those containing one or more carbonyl groups in their rings; fused ring groups containing 9 to 11 carbon atoms, such as indanonyl, tetralonyl and benzosuberonyl, and those containing one or more carbonyl groups in their rings; acyloxy and acylalkyl groups corresponding to the below-described acyl groups; the below-described silyloxy groups; the above-described heterocyclic groups, heterocyclic thio groups and heterocyclic oxy groups; and the below-described acyl, esterified carboxyl and esterified thiocarboxyl groups.

The above-described substituents may each be substituted further by one or more substituents, for example, one or more of the above-described substituents. Illustrative of further substituents for the above-described alkyl substituents (which are also equally applicable to the alkylthio, alkyloxy, alkylsulfinyl and alkylsulfonyl substituents) are halogen atoms, and carboxyl, thiocarboxyl, formyl, nitro, cyano, hydroxyl, amino, lower alkylene acetal, alkenyl, aryl, aralkyl, alkylthio, alkenylthio, aralkylthio, arylthio, alkyloxy, alkenyloxy, aralkyloxy, aryloxy, alkylsulfinyl, alkylsulfonyl, aralkylsulfinyl, aralkylsulfonyl, arylsulfinyl, arylsulfonyl, aminosulfonyl, carbamoyl, carbamoyloxy, imino, imino lower alkyl amino, imino-(amino) lower alkyl , acyloxy, silyloxy, heterocyclic, heterocyclic thio, heterocyclic oxy, acyl, esterified carboxyl and esterified thiocarboxyl groups.

Illustrative of further substituents for the alkenyl substituents (which are also equally applicable to the alkenylthio and alkenyloxy substituents) are halogen atoms, and carboxyl, thiocarboxyl, formyl, nitro, cyano, hydroxyl, amino, imino, lower alkylene-acetal, alkyl, aryl, aralkyl, alkylthio, alkenylthio, aralkylthio, arylthio, alkyloxy, alkenyloxy, aralkyloxy, aryloxy, alkylsulfinyl, alkylsulfonyl, aralkylsulfinyl, aralkylsulfonyl, arylsulfinyl, arylsulfonyl, aminosulfonyl, carbamoyl, carbamoyloxy, carbamoylalkyl, imino lower alkyl, imino lower alkyl - amino, imino(amino) lower alkyl, acyloxy, acylalkyl, silyloxy, heterocyclic, heterocyclic thio, heterocyclic oxy, acyl, esterified carboxyl and esterified thiocarboxyl groups.

Illustrative of further substituents for the aralkyl substituents (which are also equally applicable to the aralkylthio, aralkyloxy, aralkylsulfinyl and aralkylsulfonyl substituents) are halogen atoms, and carboxyl, thiocarboxyl, formyl, nitro, cyano, hydroxyl, amino, imino, lower alkylene acetal, alkyl, alkenyl, aryl, aralkyl, alkylthio, alkenylthio, aralkylthio, arylthio, alkyloxy, alkenyloxy, aralkyloxy, aryloxy, alkylsulfinyl, alkylsulfonyl, aralkylsulfinyl, aralkylsulfonyl, arylsulfinyl, arylsulfonyl, aminosulfonyl, carbamoyl, carbamoyloxy, carbamoylalkyl, imino lower alkyl , imino lower alkyl amino, imino (amino) lower alkyl , acyloxy, acylalkyl, silyloxy, heterocyclic, heterocyclic thio, heterocyclic oxy, acyl, esterified carboxyl and esterified thiocarboxyl groups.

In addition, illustrative of further substituents for the aryl substituents (which are also equally applicable to the arylthio, aryloxy, arylsulfinyl and arylsulfonyl substituents) are halogen atoms, and carboxyl, thiocarboxyl, formyl, nitro, cyano, hydroxyl, amino, imino, lower alkylene acetal, alkyl, alkenyl, aryl, aralkyl, alkylthio, alkenylthio, aralkylthio, arylthio, alkyloxy, alkenyloxy, aralkyloxy, aryloxy, alkylsulfinyl, alkylsulfonyl, aralkylsulfinyl, aralkylsulfonyl, arylsulfinyl, arylsulfonyl, aminosulfonyl, carbamoyl, carbamoyloxy, carbamoylalkyl, imino lower alkyl , imino lower alkyl amino, imino (amino) lower alkyl , acyloxy, acylalkyl, silyloxy, heterocyclic, heterocyclic thio, heterocyclic oxy, acyl, esterified carboxyl and esterified thiocarboxyl groups.

On the other hand, illustrative of further substituents for the amino, imino, aminosulfonyl, carbamoyl, carbamoyloxy, carbamoylalkyl, imino lower alkyl , imino lower alkyl amino and imino(amino) lower alkyl, unsaturated cyclic compound and fused ring substituents out of the substituents are halogen atoms, and carboxyl, thiocarboxyl, formyl, nitro, cyano, hydroxyl, amino, imino, lower alkylene acetal, alkyl, alkenyl, aryl, aralkyl, alkylthio, alkenylthio, aralkylthio, arylthio, alkyloxy, alkenyloxy, aralkyloxy, aryloxy, alkylsulfinyl, alkylsulfonyl, aralkylsulfinyl, aralkylsulfonyl, arylsulfinyl, arylsulfonyl, aminosulfonyl, carbamoyl, carbamoyloxy, carbamoylalkyl, imino lower alkyl , imino lower alkyl - amino, imino(amino) lower alkyl , acyloxy, acylalkyl, silyloxy, heterocyclic, heterocyclic thio, heterocyclic oxy, acyl, esterified carboxyl and esterified thiocarboxyl groups.

Other preferred examples of $R_1$ in the penem derivatives (I) and (II) according to the present invention include an acylthio group. In the compound (III), other preferred examples of $R_5$ similarly include acyl groups to be exemplified next. Illustrative of the acyl group in the acylthio group (which are also equally applicable to a simple acyl group and also to acyloxy and acylalkyl groups) are alkylcarbonyl, alkenylcarbonyl, aralkylcarbonyl, arylcarbonyl, heterocyclic carbonyl and imino lower alkyl carbonyl groups corresponding to the above-described substituted or unsubstituted, alkyl, alkenyl, aralkyl, aryl, heterocyclic and imino lower alkyl groups, respectively.

Examples of the silyloxy group described above as a substituent include tri-substituted silyloxy groups, specifically trialkylsilyloxy, aryl(alkyl)alkoxysilyloxy, alkoxydiarylsilyloxy, triarylsilyloxy, alkyldiarylsilyloxy, aryldialkylsilyloxy and triaralkylsilyloxy groups.

More specific examples of the silyloxy group include trimethylsilyloxy, triethylsilyloxy, tri-isopropylsilyloxy, dimethylhexylsilyloxy, tert-butyl-dimethylsilyloxy, methyldiisopropylsilyloxy, isopropyl-dimethylsilyloxy, tert-butylmethoxyphenylsilyloxy, tert-butoxydiphenylsilyloxy, triphenylsilyloxy, tert-butyldiphenylsilyloxy, dimethylcumylsilyloxy and tribenzylsilyloxy groups.

Illustrative of the esterified carboxyl and esterified thiocarboxyl groups are carboxyl and thiocarboxyl groups esterified by the above-described alkyl, alkylthio, alkyloxy, alkenyl, alkenylthio, alkenyloxy, aralkyl, aralkylthio, aralkyloxy, aryl, arylthio, aryloxy, carbamoylalkyl, imino lower alkyl , acylalkyl, silyl (which is the same as the silyl group in the above-described silyloxy group), heterocyclic, heterocyclic thio and heterocyclic oxy groups.

On the other hand, no particular limitation is imposed on the carboxyl-protecting group represented by $R_2$ or $R_4$, insofar as it is generally used in the technical field of β-lactam compounds. Usable examples include those capable of forming ester moieties together with the carboxyl group and being removable by hydrolysis, photodecomposition, oxidation or reduction or removable enzymatically; and those capable of forming, together with the carboxyl group, ester moieties which liberate in the living body to form free carboxylic acids.

Preferred examples of the carboxyl-protecting group include groups capable of forming esters which are to

be described next.

Namely, examples of the carboxyl-protecting group first include tri-substituted silyl esters such as trialkylsilyl esters, aryl(alkyl)alkoxysilyl esters, alkoxydiarylsilyl esters, triarylsilyl esters, alkyl-diarylsilyl esters, aryldialkylsilyl esters and triaralkylsilyl esters (for example, trimethylsilyl esters, triethylsilyl esters, triisopropylsilyl esters, dimethylhexylsilyl esters, tert-butyldimethylsilyl esters, methyldiisopropylsilyl esters, isopropyldimethylsilyl esters, tert-butylmethoxyphenylsilyl esters, tert-butoxydiphenylsilyl esters, triphenylsilyl esters, tert-butyldiphenylsilyl esters, dimethylcumylsilyl esters and tribenzylsilyl esters); and trisubstituted silyl lower alkyl esters, for example, trialkylsilyl lower alkyl esters, aryl(alkyl)alkoxysilyllower alkyl esters, alkoxydiarylsilyl lower alkyl esters, triarylsilyl lower alkyl esters, alkyldiarylsilyl lower alkyl esters, aryldialkylsilyl lower alkyl esters, triaralkylsilyl lower alkyl esters [for instance, those formed by substituting the above-exemplified tri-substituted silyl groups to lower alkyl groups (e.g., linear or branched lower alkyl groups such as methyl, ethyl, n-propyl, isopropyl, n-butyl, tert-butyl and hexyl)].

Examples of the carboxyl-protecting group also include aromatic heterocyclic esters; lower alkyl esters, for example, lower alkyl esters which may contain one or more suitable substituents, such as lower alkanoyloxy(lower) alkyl esters, lower alkanesulfonyl(lower)alkyl esters, mono(or di or tri)halo-(lower)alkyl esters, lower alkoxycarbonyloxy (lower)-alkyl esters, phthalidylidene(lower)alkyl esters, (5-lower alkyl(or aryl)-2-oxo-1,3-dioxolen-4-yl)(lower)-alkyl esters; lower alkenyl esters (for example, vinyl esters and allyl esters); and lower alkynyl esters (for example, ethynyl esters and propynyl esters).

Among the above-described carboxyl-protecting groups, specific examples of the aromatic heterocyclic esters include pyridyl ester, pyrimidinyl ester, pyrazinyl ester and pyridazinyl ester, and those of the lower alkyl esters include methyl ester, ethyl ester, n-propyl ester, isopropyl ester, n-butyl ester, isobutyl ester, tert-butyl ester, pentyl ester and hexyl ester.

Out of the lower alkyl esters which may contain one or more suitable substituents, examples of the lower alkanoyloxy(lower)alkyl esters include acetoxymethyl ester, propionyloxymethyl ester, butyryloxymethyl ester, valeryloxymethyl ester, pivaloyloxymethyl ester, hexanoyloxymethyl ester, 1-(or 2-)acetoxyethyl ester, 1-(or 2- or 3-)acetoxypropyl ester, 1-(or 2-, 3-or 4-)acetoxybutyl ester, 1-(or 2-)propionyloxyethyl ester, 1-(or 2- or 3-)propionyloxypropyl ester, 1-(or 2-)butyryloxyethyl ester, 1-(or 2-)isobutyryloxyethyl ester, 1-(or 2-)pivaloyloxyethyl ester, 1-(or 2-) hexanoyloxyethyl ester, isobutyryloxymethyl ester, 2-ethylbutyryloxymethyl ester, 3,3-dimethylbutyryloxy-methyl ester, and 1-(or 2-)pentanoyloxyethyl ester.

Further, out of the lower alkyl esters which may contain one or more suitable substituents, illustrative of the lower alkanesulfonyl(lower)alkyl esters is 2-mesylethyl ester; illustrative of the mono(or di or tri)halo(lower)alkyl ester are 2-iodoethyl ester, 2,2-dichloroethyl ester and 2,2,2-trichloroethyl ester; illustrative of lower alkoxycarbonyloxy(lower)alkyl ester are methoxycarbonyloxymethyl ester, ethoxycarbonyloxymethyl ester, propoxycarbonyloxymethyl ester, tert-butoxycarbonyloxymethyl ester, 1-(or 2-)methoxy-carbonyloxyethyl ester, 1-(or 2-)ethoxycarbonyloxyethyl ester and 1-(or 2-)isopropoxycarbonyloxyethyl ester; and illustrative of the 5-lower alkyl(or aryl)-2-oxo-1,3-dioxolen-4-yl)(lower)alkyl esters are (5-methyl(or phenyl)-2-oxo-1,3-dioxolen-4-yl)methyl ester, (5-ethyl-2-oxo-1,3-dioxolen-4-yl)methyl ester and (5-propyl(or phenyl)-2-oxo-1,3-dioxolen-4-yl)ethyl ester.

In addition, examples of the carboxyl-protecting groups also include ar(lower)alkyl esters which may contain one or more suitable substitutes (for example, benzyl ester, 4-methoxybenzyl ester, 4-nitrobenzyl ester, 2-nitrobenzyl ester, phenethyl ester, trityl ester, benzhydryl ester, bis(methoxyphenyl)methyl ester, 3,4-dimethoxybenzyl ester and 4-hydroxy-3,5-di-tert-butylbenzyl ester); aryl esters which may contain one or more suitable substituents (for example, phenyl ester, 4-chlorophenyl ester, tolyl ester, tert-butyl-phenyl ester, xylyl ester, mesityl ester and cumenyl ester); and phthalidyl esters.

On the other hand, no particular limitation is imposed on the protected hydroxyl group represented by $OR_3$ in the compounds (II), (III) and (IV). Examples of the protected hydroxyl group therefore include hydroxyl groups protected by commonly-employed hydroxyl-protecting groups.

Examples of the protected hydroxyl group include trisubstituted silyloxy groups such as trialkylsilyloxy groups, aryl(alkyl)alkoxysilyloxy groups, alkoxydiarylsilyloxy groups, triarylsilyloxy groups, alkyldiarylsilyloxy groups, aryldialkylsilyloxy groups and triaralkylsilyloxy groups; lower alkoxy groups which may contain one or more suitable substituents; lower alkanoyloxy groups which may contain one or more suitable substituents; lower alkoxycarbonyloxy groups which may contain one or more suitable substituents; lower alkenyloxycarbonyloxy groups which may contain one or more suitable substituents; arylcarbonyloxy groups which may contain one or more suitable substituents; aralkyloxycarbonyloxy groups which may contain one or more suitable substituents; aryloxycarbonyloxy groups which may contain one or more suitable substituents; aralkyloxy groups which may contain one or more suitable substituents; and heterocyclic oxy groups which may contain one or more suitable substituents.

Among the protected hydroxyl groups, specific examples of the trisubstituted silyloxy groups include trimethylsilyloxy, triethylsilyloxy, triisopropylsilyloxy, dimethylhexylsilyloxy, tert-butyldimethylsilyloxy, methyldiisopropylsilyloxy, isopropyldimethylsilyloxy, tert-butylmethoxyphenylsilyloxy, tert-butoxydiphenylsilyloxy, triphenylsilyloxy, tert-butyldiphenylsilyloxy, dimethylcumylsilyloxy, and tribenzylsilyloxy.

Further, specific examples of the lower alkoxy groups which may contain one or more suitable substituents include methoxymethoxy, methoxyethoxymethoxy and triphenylmethoxy; and specific examples of the lower alkanoyloxy groups which may contain one or more suitable substituents include acetoxy, chloroacetoxy, methoxyacetoxy, propionyloxy, butyryloxy, isobutyryloxy, valeryloxy, pivaloyloxy, hexanoyloxy, 2-ethylbutyryloxy, 3,3-dimethylbutyryloxy and pentanoyloxy.

Specific examples of the lower alkoxycarbonyloxy groups which may contain one or more suitable substituents include methoxycarbonyloxy, ethoxycarbonyloxy, propoxycarbonyloxy, isopropoxycarbonyloxy, tert-butoxycarbonyloxy, 2-iodoethoxycarbonyloxy, 2,2-dichloroethoxycarbonyloxy and 2,2,2-trichloroethoxycarbonyloxy; specific examples of lower alkenyloxycarbonyloxy groups which may contain one or more suitable substituents include vinyloxycarbonyloxy, allyloxycarbonyloxy and 2-chloroallyloxycarbonyloxy; and specific examples of the arylcarbonyloxy groups which may contain one or more suitable substituents include benzoyloxy.

Specific examples of the aralkyloxycarbonyloxy groups which may contain one or more suitable substituents include benzyloxycarbonyloxy, p-nitro-benzyloxycarbonyloxy, p-methoxybenzyloxycarbonyloxy, phenethyloxycarbonyloxy, trityloxycarbonyloxy, benzhydryloxycarbonyloxy, bis(methoxyphenyl)methyloxycarbonyloxy, 3,4-dimethoxybenzyloxycarbonyloxy and 4-hydroxy-3,5-di-tert-butylbenzyloxycarbonyloxy; and specific examples of the aryloxycarbonyloxy groups which may contain one or more suitable substituents include phenyloxycarbonyloxy, 4-chlorophenyloxycarbonyloxy, tolyloxycarbonyloxy, tert-butylphenyloxycarbonyloxy, xylyloxycarbonyloxy, mesityloxycarbonyloxy and cumenyloxycarbonyloxy.

Finally, specific examples of the aralkyloxy groups which may contain one or more suitable substituents include benzyloxy, p-nitrobenzyloxy, p-methoxybenzyloxy, p-tert-butylbenzyloxy, 3,4-dimethylbenzyloxy, 2,4-dimethoxybenzyloxy, benzhydryloxy and trityloxy; and specific examples of the heterocyclic oxy groups which may contain one or more suitable substituents include tetrahydropyranyloxy.

Preferred specific examples of the penem derivative (I) according to the present invention include penem derivatives, in which $R_1$ is one of the following groups (i) and (ii), and pharmacologically acceptable salts thereof:

(i) a group represented by the following formula:

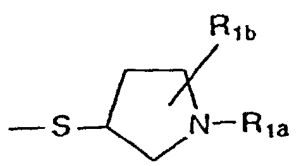

wherein $R_{1a}$ and $R_{1b}$ may be the same or different and represent a hydrogen atom, an alkyl group, an alkenyl group, an aralkyl group containing 7 to 24 carbon atoms, an aryl group containing 6 to 10 carbon atoms, an imino lower alkyl group, an imino lower alkyl amino group, an imino(amino) lower alkyl group, a carbamonyl group, a carbamoyl lower alkyl group, an acyl group, an acyl lower alkyl group, carboxyl group, a heterocyclic group or a heterocyclic lower alkyl group; one or more hydrogen atoms of said alkyl, alkenyl, aralkyl, aryl, imino lower alkyl , imino lower alkyl amino, imino(amino) lower alkyl, carbamoyl, carbamoyl lower alkyl heterocyclic or heterocyclic lower alkyl group may each be substituted by a halogen atom, a carboxyl group, a thiocarboxyl group, a formyl group, a nitro group, a cyano group, a hydroxyl group, an amino group, an imino group, a lower alkylene acetal group, an alkyl group, an alkoxyl group, an alkenyl group, an aralkyl group containing 7 to 24 carbon atoms, an aryl group containing 6 to 10 carbon atoms, an aryloxy group containing 6 to 10 carbon atoms, an imino lower alkyl group, an imino lower alkyl amino group, an imino-(amino) lower alkyl group, a carbamoyl group, a carbamonyloxy group, a carbamoyl lower alkyl group, a heterocyclic group, a heterocyclic lower alkyl group, an acyl group or an acylalkyl group; said acyl groups and the acyl group of said acyl lower alkyl groups represent an alkyl carbonyl, alkenylcarbonyl, aralkylcarbonyl, arylcarbonyl, heterocyclic carbonyl or heterocyclic lower alkyl carbonyl group containing said substituted or unsubstituted alkyl, alkenyl, aralkyl, aryl, heterocyclic or heterocyclic lower alkyl group; said carboxyl group may be esterified by said substituted or unsubstituted alkyl, alkenyl, aralkyl, aryl, heterocyclic or heterocyclic lower alkyl group; said heterocyclic groups and the heterocyclic group of said heterocyclic lower alkyl group may each contain one or more carbonyl group in the rings thereof and the tertiary nitrogen atom thereof may form an intramolecular quaternary salt by the introduction of said substituent; and

(ii) a group represented by the following formula:

$$-S-(CH_2)_n-R_{1c}$$

wherein n stands for 1 to 3; $R_{1c}$ represents a hydrogen atom, an aryl group containing 6 to 10 carbon atoms, an amino group, an imino lower alkyl amino group, an aminosulfonyl group, carbamoyl group, acyl group, a carboxyl group or a heterocyclic group; one or more hydrogen atoms of said aryl, amino, imino lower alkyl amino, aminosulfonyl, carbamoyl or heterocyclic group may each be substituted by a halogen atom, a carboxyl group, a thiocarboxyl group, a formyl group, a nitro group, a cyano group, a hydroxyl group, an amino group, an imino group, an alkyl group, an alkoxy group, an alkenyl group, an aralkyl group containing 7 to 24 carbon atoms, an aryl group containing 6 to 10 carbon atoms, an aryloxy group containing 6 to 10 carbon atoms, an imino lower alkyl group, an imino lower alkyl amino group, an imino(amino) lower alkyl group, a carbamoyl group, a carbamoyloxy group, a carbamoyl lower alkyl group, a heterocyclic group, a heterocyclic lower alkyl group, an acyl group or a acylalkyl group; said acyl groups and the acyl group of said acylalkyl groups recited as a substituent represent an alkylcarbonyl, alkenylcarbonyl, aralkylcarbonyl, arylcarbonyl, heterocyclic carbonyl or heterocyclic lower alkyl carbonyl group containing one or more alkyl, alkenyl, aralkyl, aryl, heterocyclic or heterocyclic (lower alkyl) groups; one or more hydrogen atoms of these acyl groups may each be substituted by a halogen atom, a carboxyl group, a thiocarboxyl group, a formyl group, a nitro group, a cyano group, a hydroxyl group, an amino group, an imino group, a lower alkylene - acetal group, an alkyl group, an alkoxy group, an alkenyl group, an aralkyl group containing 7 to 24 carbon atoms, an aryl group containing 6 to 10 carbon atoms, an aryloxy group containing 6 to 10 carbon atoms, an imino lower alkyl group, an imino lower alkyl amino group, an imino(amino) lower alkyl group, carbamoyl group, a carbamoyloxy group, a carbamoyl lower alkyl group, a heterocyclic group, a heterocyclic lower alkyl group, an acyl group or an acylalkyl group; said carboxyl group may be esterified by a substituted or unsubstituted alkyl, alkenyl, aralkyl, aryl, heterocyclic or heterocyclic lower alkyl group; said heterocyclic group and the heterocyclic group of said heterocyclic lower alkyl groups, the latter heterocyclic group being recited as a substituent, may each contain one or more carbonyl groups in the ring thereof and the tertiary nitrogen atom thereof may form an intramolecular quaternary salt by the introduction of said substituent.

Preferred examples of the penem derivative which is represented by (I) and is available in accordance with the present invention include compounds in which groups represented as $R_1$, said groups being 2-substituents on the penem ring in the formula (I), are represented by a group SR'5 and illustrative of R'5 are hydrogen atom, methyl, ethyl, n-propyl, isopropyl, n-butyl, isobutyl, tert-butyl, n-pentyl, neopentyl, 2-hydroxyethyl, 2-hydroxypropyl, 3-hydroxypropyl, 2-aminoethyl, 2-amino-2-iminoethyl, 2-aminopropyl, 3-aminopropyl, fluoromethyl, 2-fluoroethyl, 2-fluoropropyl, 3-fluoropropyl, 2-phenoxyethyl, 3-phenyloxypropyl, 2-[(1-iminoethyl)amino]ethyl, 3-[(1-iminoethyl)amino]propyl, 2-[(1-imino-1-phenylmethyl)amino] ethyl, 2-[N-methyl-N-(2-oxo-2-phenylethyl)-amino]ethyl, 2-[N-methyl-N-(2-oxo-2-pyridylethyl) amino]-ethyl, 2-(pyrrolidin-1-yl)ethyl, 2-(piperidin-1-yl)ethyl, 2-(piperazin-1-yl)ethyl, 2-(pyrrol-1-yl)ethyl, N-methylcarbamoylmethyl, N-benzyl-carbamoylmethyl, N-phenylcarbamoylmethyl, N-methyl-carbamoylethyl, N-benzylcarbamoylethyl, N-phenyl-carbamoylethyl, 2-morpholino-2-oxoethyl, [o-(N-methylcarbamoyl)phenyl]methyl, [o-(N-benzylcarbamoyl)phenyl]methyl, cyclopropyl, cyclopentyl, cyclohexyl, 1-indanyl, 2-indanyl, 1-indanon-2-yl, 1-indanon-3-yl, 6,7-dihydro-5H-cyclopenta[b]pyridin-5-yl, 6,7-dihydro-5H-cyclopenta[b]pyridin-6-yl, 6,7-dihydro-5H-cyclopenta[b]-pyridin-7-yl,

vinyl, allyl,

benzyl, 3,4-dichlorophenylmethyl, 3-cyanophenylmethyl, 4-cyanophenylmethyl, diphenylmethyl, trityl, (1-pyridinio)methyl, (2-pyridyl)methyl, (1-methyl-2-pyridinio)methyl, (1-carbamoylmethyl-2-pyridinio) - methyl, (3-pyridyl)methyl, (1-methyl-3-pyridinio)-methyl, (1-carbamoylmethyl-3-pyridinio)methyl, (4-pyridyl)methyl, (1-methyl-4-pyridinio)methyl, (1-carbamoylmethyl-4-pyridinio)methyl, (2-pyrimidyl) - methyl, (imidazol-2-yl)methyl, (1-methylimidazol-2-yl)methyl, (1-methylimidazolium-3-yl)methyl, (1-benzylimidazol-2-yl)methyl, (thiazol-2-yl)methyl, phenethyl, 2,2-diphenylethyl, (1-pyridinio)ethyl, 2-(2-pyridyl)ethyl, 2-(1-methyl-2-pyridinio)ethyl, 2-(1-carbamoylmethyl-2-pyridinio)ethyl, 2-(3-pyridyl)ethyl, 2-(1-methyl-3-pyridinio)ethyl, 2-(1-carbamoylmethyl-3-pyridinio)ethyl, 2-(4-pyridyl)ethyl, 2-(1-methyl-4-pyridinio)ethyl, 2-(1-carbamoylmethyl-4-pyridinio)-ethyl, 2-(2-pyrimidyl)ethyl, 2-(imidazol-2-yl)ethyl, 2-(1-methylimidazolium-3-yl)ethyl, 2-(thiazol-2-yl)ethyl, 3-phenylpropyl, 3,3-diphenylpropyl, (1-pyridinio)-propyl, 3-(2-pyridyl)propyl, 3-(1-methyl-2-pyridinio)-propyl, 3-(1-carbamoylmethyl-2-pyridinio)propyl, 3-(3-pyridyl)propyl, 3-(1-methyl-3-pyridinio) propyl, 3-(1-carbamoylmethyl-3-pyridinio)propyl, 3-(4-pyridyl)-propyl, 3-(l-methyl-4-pyridinio)propyl, 3-(1-carbamoylmethyl-4-pyridinio)propyl, 3-(2-pyrimidyl)propyl, 3-(imidazol-2-yl)propyl, 3-(1-methylimidazolium-3-yl)-propyl, 3-(thiazol-2-yl)propyl, 1-naphthylmethyl, 2-naphtylmethyl, 2-(1-naphthyl)ethyl, 2-(2-naphthyl)-ethyl, (o-hydroxymethyl)benzyl, [o-(1-methylimidazolium-3-yl)methyl]benzyl, (m-hydroxymethyl)-benzyl, [m-(1-methylimidazolium-3-yl)methyl]benzyl, (p-hydroxymethyl)benzyl, [p-(1-methylimidazolium-3-yl)-methyl]benzyl,

2-amino-2-phenylethyl, 2-amino-3-phenylpropyl, 2-oxo-2-phenylethyl, 2-oxo-2-(2-pyridyl)ethyl, 2-(1-methyl-2-pyridinio)-2-oxoethyl, 2-oxo-2-(3-pyridyl)-ethyl, 2-(1-methyl-3-pyridinio)-2-oxoethyl, 2-oxo-2-(4-pyridyl)ethyl, 2-(1-methyl-4-pyridinio)-2-oxoethyl, 2-(imidazol-2-yl)-2-oxoethyl, 2-oxo-2-(thiazol-2-yl)ethyl,

phenyl, 1-naphthyl, 2-naphthyl, 2-pyridyl, 1-methyl-2-pyridinio, 3-pyridyl, 1-methyl-3-pyridinio, 4-pyridyl, 1-methyl-4-pyridinio, 2-pyrimidyl, imidazol-2-yl, thiazol-2-yl, 4-phenylthiazol-2-yl, benzothiazol-2-yl,

azetidin-3-yl, 1-allylazetidin-3-yl, 1-benzylazetidin-3-yl, 1-phenylazetidin-3-yl, 1-(1-iminoethyl)-azetidin-3-yl, 1-(2-oxo-2-phenylethyl)azetidin-3-yl,

pyrrolidin-3-yl, 2-iminopyrrolidin-3-yl, 2-iminopyrrolidin-4-yl, 1-allylpyrrolidin-3-yl, 1-benzylpyrrolidin-3-yl, 1-phenethylpyrrolidin-3-yl, 1-cyclopropylpyrrolidin-3-yl, 1-cyclopentylpyrrolidin-3-yl, 1-cyclopropylmethylpyrrolidin-3-yl, 1-(3-phenylpropyl)-pyrrolidin-3-yl, 1-phenylpyrrolidin-3-yl, 1-(2-pyridyl)pyrrolidin-3-yl, 1-(1-methyl-2-pyridinio)-pyrrolidin-3-yl, 1-(3-pyridyl)pyrrolidin-3-yl, 1-(1-methyl-3-pyridinio)pyrrolidin-3-yl, 1-(4-pyridyl)-pyrrolidin-3-yl, 1-(1-methyl-4-pyridinio)pyrrolidin-3-yl, 1-(2-pyrimidyl)pyrrolidin-3-yl, 1-(thiazol-2-yl)-pyrrolidin-3-yl, 1-(o-aminophenyl)pyrrolidin-3-yl, 1-(m-aminophenyl)pyrrolidin-3-yl, 1-(p-aminophenyl)-pyrrolidin-3-yl, 1-(p-fluorophenyl)pyrrolidin-3-yl, 1-(p-hydroxyphenyl)pyrrolidin-3-yl, 1-(p-methylphenyl)-pyrrolidin-3-yl, 1-(p-methoxyphenyl)pyrrolidin-3-yl, 1-[p-(1-iminoethyl)aminophenyl]pyrrolidin-3-yl, 1-(2-hydroxyethyl)pyrrolidin-3-yl, 1-(2-hydroxy-2-phenylethyl)pyrrolidin-3-yl, 1-(2-fluoroethyl)pyrrolidin-3-yl, 1-(2-oxo-2-phenylethyl)pyrrolidin-3-yl, 1-[2-(o-hydroxy)phenyl-2-oxoethyl]pyrrolidin-3-yl, 1-[2-(m-hydroxy)phenyl-2-oxoethyl]pyrrolidin-3-yl, 1-[2-(p-hydroxy)phenyl-2-oxoethyl]pyrrolidin-3-yl, 1-[2-(m,p-dihydroxy)phenyl-2-oxoethyl]pyrrolidin-3-yl, 1-[2-(o,m-dihydroxy)phenyl-2-oxoethyl[pyrrolidin-3-yl, 1-[2-(p-fluoro)phenyl-2-oxoethyl]pyrrolidin-3-yl, 1-[2-(p-methyl)phenyl-2-oxoethyl]pyrrolidin-3-yl, 1-[2-(p-methoxy)phenyl-2-oxoethyl]pyrrolidin-3-yl, 1-[2-(p-amino)phenyl-2-oxoethyl]pyrrolidin-3-yl, 1-(1-methyl-2-oxo-2-phenylethyl)pyrrolidin-3-yl, 1-(3-oxo-3-phenylpropyl)pyrrolidin-3-yl, 1-(2-oxo-3-phenylpropyl)-pyrrolidin-3-yl, 1-(1-indanon-2-yl)pyrrolidin-3-yl, 1-(1-indanon-3-yl)pyrrolidin-3-yl, 1-[(1-pyridinio)-methyl]pyrrolidin-3-yl, 1-[(2-pyridyl)methyl]-pyrrolidin-3-yl, 1-[(1-methyl-2-pyridinio)methyl]-pyrrolidin-3-yl, 1-[(3-pyridyl)methyl)pyrrolidin-3-yl, 1-[(1-methyl-3-pyridinio)methyl]pyrrolidin-3-yl, 1-[(4-pyridyl)methyl]pyrrolidin-3-yl, 1-[(1-methyl-4-pyridinio)methyl]pyrrolidin-3-yl, 1-[(imidazol-2-yl)methyl]pyrrolidin-3-yl, 1-[(1-methylimidazolium-3-yl)methyl]pyrrolidin-3-yl, 1-[(4-carbamoylphenyl)-methyl]pyrrolidin-3-yl, 1-[(3-acetylphenyl)methyl]-pyrrolidin-3-yl, 1-[(2-oxo-2-piperazinyl)ethyl]-pyrrolidin-3-yl,

1-iminomethylpyrrolidin-3-yl, 1-(1-iminoethyl)-pyrrolidin-3-yl, 1-(1-imino-2-phenylethyl)pyrrolidin-3-yl, 1-iminopropylpyrrolidin-3-yl,

piperidin-2-ylmethyl, piperidin-3-yl, piperidin-4-yl, 1-allylpiperidin-4-yl, 1-benzylpiperidin-4-yl, 1-phenylpiperidin-4-yl, 1-(1-iminoethyl)piperidin-4-yl,

2-hydroxymethylpyrrolidin-4-yl, 2-(1-pyridinio)-methylpyrrolidin-4-yl, 2-(1-methylimidazolium-3-yl) - methylpyrrolidin-4-yl, 2-hydroxymethyl-1-(1-imino-ethyl)pyrrolidin-4-yl, 2-phenoxymethylpyrrolidin-4-yl, 2-phenylmethylpyrrolidin-4-yl,

pyrazolidin-4-yl, and indan-3-on-1-yl; and pharmacologically acceptable salts thereof.

It is to be noted that many of the penem compounds (I) according to the present invention have isomers and the present invention embraces all possible isomers other than the (1'S,5R,6R) isomers, being the characteristic feature of the penem derivatives according to the present invention, and mixtures thereof. For example, preferred examples of those containing a pyrrolidinyl group or a substituted pyrrolidinyl group as the group represented by $R_1$ among the penem derivatives (I) according to the present invention are those containing an (S)-pyrrolidinyl-3-yl group as the group.

The penem derivative (I) according to the present invention can be prepared by various processes and can be synthesized by any one of the below-described processes. These processes will hereinafter be described one by one.

Process 1:

Each compound of the formula (I) in which $R_1$ is the above-described substituted or unsubstituted alkylthio group, substituted or unsubstituted alkenylthio group, substituted or unsubstituted aralkylthio group, substituted or unsubstituted arylthio group, substituted or unsubstituted heterocyclic thio group, or substituted or unsubstituted acylthio group (Compound (Ia)) can be prepared in accordance with the following reaction scheme, using as a starting material a brominated penem compound represented by the formula (V).

(a)

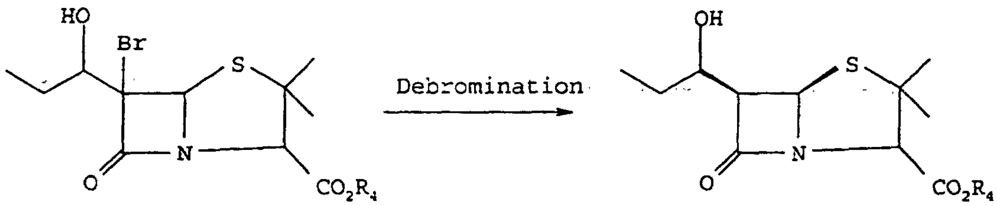


(V) (VI)

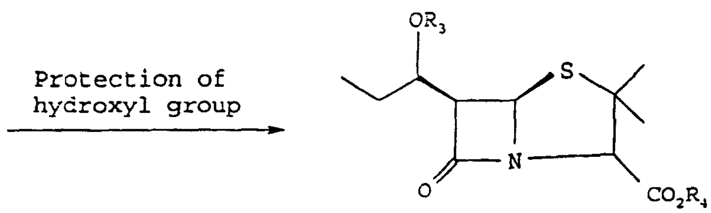


(VII)

(b)

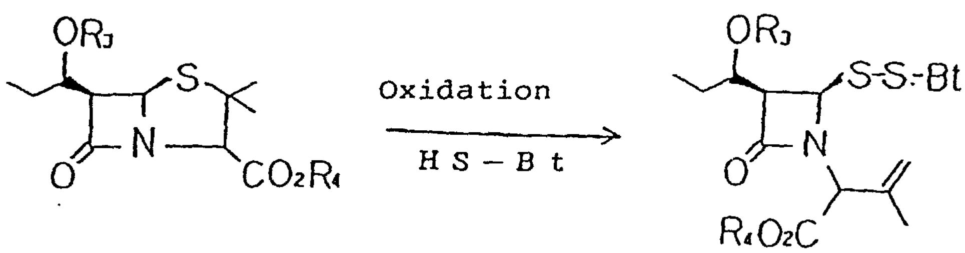


(VII) (VIII)

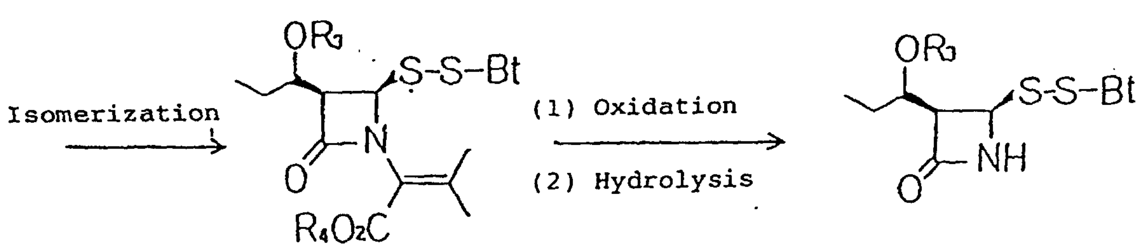


(VIII') (IX)

(c)

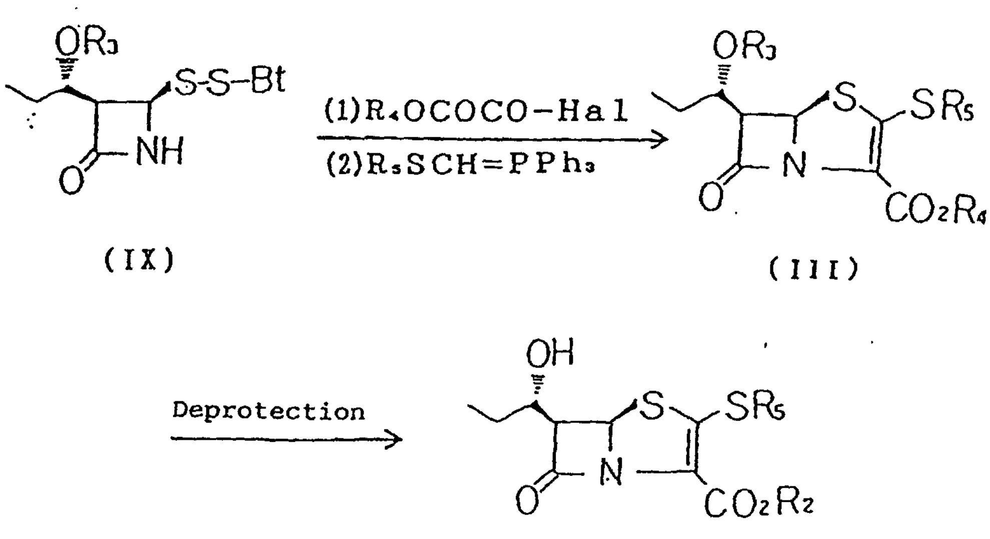


(IX) (III)

(Ia)

wherein $R_5$ represents a substituted or unsubstituted alkyl group, a substituted or unsubstituted alkenyl group, a substituted or unsubstituted aralkyl group, a substituted or unsubstituted aryl group, a substituted or unsubstituted heterocyclic group or a substituted or unsubstituted acyl group, Bt represents a benzothiazole group, and $R_2$, $OR_3$ and $R_4$ have the same meanings as defined above.

In the above process, step (a) is to protect the hydroxyl group subsequent to the removal of bromine from the compound (V) as a starting material.

The compound of the formula (V) as the starting material is a known compound which can be obtained by the process disclosed in J. Org. Chem., 42, 2966 (1977). Although it is obtained as a mixture of two types of isomers with respect to the asymmetric carbon to which the hydroxyl group is bonded, it can be reacted as the mixture up to the formula (IX) in step (b) which will be described subsequently herein.

The debromination can be conducted by reacting 1.0 to 5 equivalents of a reducing agent such as tributyltin hydride with 1 equivalent of the compound (V) under heat for 1 to 24 hours in a solvent, for example, an aromatic hydrocarbon such as benzene or toluene, or a saturated hydrocarbon such as hexane.

After completion of the bromination, the solvent is distilled off. Subsequent to dilution with acetonitrile, the solvent layer is washed with a saturated hydrocarbon such as hexane and the resulting acetonitrile layer is caused to evaporate to dryness, whereby the target compound (VI) can be obtained. It can be purified by chromatography or the like if necessary.

Then, the hydroxyl-protecting group is introduced into the compound (VI) so that the compound (VI) is converted into the hydroxyl-protected compound (VII). This reaction varies depending on the hydroxyl-protecting group to be introduced. For example, when a protecting group of the silyl type, such as a tert-butyldimethylsilyl group, is introduced, the introduction is conducted by reacting 1.0 to 5 equivalents of a corresponding silyl chloride and 1.0 to 1.5 equivalents of a tertiary amine, such as triethylamine, or imidazole with 1 equivalent of the compound (VI) for 1 to 24 hours at 0°C to 70°C, preferably room temperature in a solvent, for example, an aromatic hydrocarbon such as benzene or toluene, an amide such as N,N-dimethylformamide, a ketone such as acetone or methyl ethyl ketone, an ether such as tetrahydrofuran or diethyl ether, a saturated hydrocarbon such as hexane, a halogenated hydrocarbon such as methylene chloride or chloroform, or a mixture thereof in accordance with a known process (for example, Tetrahedron Lett., 99, 1979).

After the reaction, the reaction product is diluted with a water-immiscible organic solvent. The organic layer is successively washed with a saturated aqueous solution of potassium hydrogensulfate, a saturated aqueous solution of sodium hydrogencarbonate and a saturated aqueous solution of sodium chloride, and the solvent is distilled off, whereby the target compound (VII) is obtained. It can also be purified by chromatography or the like if necessary.

In Process 1, step (b) is to subject the hydroxyl-protected compound (VII) to ring-opening so that the compound (VII) is converted into the azetidinone derivative (IX).

According to this step, the sulfur atom on the penem ring of the compound (VII) is first oxidized into a sulfoxide, on which 2-mercaptobenzothiazole is caused to act so that the penem ring is opened to derive the compound represented by the formula (VIII).

The step in which the sulfur atom of the compound (VII) is oxidized into the sulfoxide can be conducted by causing 1.0 to 1.2 equivalents of an oxidizing agent led by a peroxy acid such as m-chloroperbenzoic acid to act on 1 equivalent of the compound (VII) at -20°C to room temperature, preferably 0°C for 10 minutes to 24 hours in a solvent, for example, a halogenated hydrocarbon such as dichloromethane, an aromatic hydrocarbon such as benzene or toluene, or a saturated hydrocarbon such as hexane.

After completion of the reaction, the reaction product is diluted with a water-immiscible organic solvent. The organic layer is successively washed with a saturated aqueous solution of sodium hydrogencarbonate and a saturated aqueous solution of sodium chloride, and the organic solvent is distilled off, whereby the sulfoxide is obtained. It can also be purified by chromatography or the like if necessary.

The thus-obtained sulfoxide is reacted with 2-mercaptobenzothiazole at a ratio of 1 equivalent to 1.0 to 5 equivalents at 50 to 150°C, preferably 110°C for 1 hour to 24 hours while using, as a solvent, an aromatic hydrocarbon such as benzene or toluene or a saturated hydrocarbon such as hexane. After the reaction, the solvent is distilled off to obtain the compound (VIII). It can also be purified by chromatography or the like if necessary.

The resultant compound (VIII) can be converted into the compound (IX) by isomerizing it with respect to the double bond to obtain an α,β-unsaturated ester represented by the formula (VIII'), subjecting the double bond to oxidative cleavage and then hydrolyzing the thus-obtained imide.

This isomerization can be conducted by reacting 0.01 to 0.5 equivalent, preferably 0.1 equivalent of a tertiary amine such as triethylamine with 1 equivalent of the compound (VIII) at 0°C to 50°C, preferably room temperature for 1 to 4 hours while using, as a solvent, a halogenated hydrocarbon such as dichloromethane, an aromatic hydrocarbon such as benzene or toluene, a ketone such as acetone or methyl ethyl ketone, or a saturated hydrocarbon such as hexane.

The target compound (VIII') can be obtained by distilling off the solvent after the isomerization. This compound can be purified by chromatography or the like if necessary.

Further, the oxidative cleavage of the double bond and the hydrolysis can be conducted by causing ozone to act on the compound (VIII') at -78°C to -50°C in a solvent such as an ester such as ethyl acetate or an alcohol such as methanol or ethanol, causing a reducing agent such as dimethyl sulfide, a phosphine such as triphenylphosphine or zinc to act, distilling off the solvent, and then causing water, methanol or a mixed solvent thereof to act for the hydrolysis of the imide.

The azetidinone derivative (IX) can be obtained by distilling off the solvent after completion of the reaction. By purification such as chromatography or recrystallization, it can be isolated into two types of isomers with respect to the asymmetric carbon to which the hydroxyl group is bonded.

A description will hereinafter be made assuming that the 1'S isomer out of the isolated isomers is the azetidinone derivative (IX).

Step (c) of Process 1 is to react the azetidinone derivative (IX) first with oxalyl halide monoester ($R_4OCOCO$-Hal) and then with thiomethylene triphenylphosphorane compound ($R_5SCH=PPh_3$) to close a ring and then to remove the hydroxyl-protecting group and, if necessary, the carboxyl-protecting group, whereby the penem compound (Ia) according to the present invention is obtained.

This reaction is conducted by first causing 1.0 to 1.2 equivalents of the oxalyl halide monoester to act on 1 equivalent of the azetidinone derivative (IX) at -20°C to 10°C in the presence of a tertiary amine such as triethylamine for 10 to 30 minutes in a solvent, for example, a halogenated hydrocarbon such as dichloromethane, an aromatic hydrocarbon such as benzene or toluene, or an ether such as tetrahydrofuran or diethyl ether.

After completion of the reaction, the reaction product is diluted with a water-immiscible organic solvent. The organic layer is successively washed with water, a saturated aqueous solution of sodium hydrogencarbonate and a saturated aqueous solution of sodium chloride, and the solvent is distilled off, whereby the imide is obtained. Without subjecting the imide to isolation or purification, the imide is reacted with thiomethylenetriphenylphosphorane compound at a ratio of 1 equivalent to 2 to 2.5 equivalents at -20°C to 0°C for 1 to 24 hours in a solvent, for example, an ether such as tetrahydrofuran or diethyl ether, an aromatic hydrocarbon such as toluene, or a saturated hydrocarbon such as hexane.

As the oxalyl monohalide monoester employed here, those represented by allyl oxalyl chloride, paranitrobenzyl oxalyl chloride and the like are usable. Further, usable examples of the thiomethylenetriphenylphosphorane compound include those containing, as $R_5$, a substituted or unsubstituted alkyl group, a substituted or unsubstituted alkenyl group, a substituted or unsubstituted aralkyl group, a substituted or unsubstituted aryl group, a substituted or unsubstituted heterocyclic group, or a substituted or unsubstituted acyl group. They are known, or can be prepared by processes similar to preparation processes of known compounds.

The target penem compound (Ia) can be obtained by diluting the reaction product with a water-immiscible organic solvent after completion of the reaction, washing the organic layer with water, distilling off the organic solvent and then subjecting the residue to a deprotecting reaction.

The removal of the hydroxyl-protecting group $R_3$ can be effected by suitably choosing a known method [for example, Japanese Patent Application Laid-Open (Kokai) No. SHO 61-207387 or Japanese Patent Application Laid-Open (Kokai) No. HEI 7-70126], although conditions vary depending on the nature of each protecting group. For example, when a protecting group of the silyl type, such as a tert-butyldimethylsilyl group, is used, a reaction is allowed to easily proceed by diluting the reaction product with a solvent and then bringing tetra-n-butylammonium fluoride, triethylamine trihydrogenfluoride or the like into contact with the reaction product. For this reaction, the preferred temperature ranges from room temperature to 50°C, and preferred usable exemplary solvents include ethers such as tetrahydrofuran and diethyl ether, aromatic hydrocarbons such as benzene and toluene, esters such as ethyl acetate, and ketones such as acetone and methyl ethyl ketone. The target compound (Ia) can be obtained by diluting the reaction product with a water-immiscible organic solvent after completion of the reaction, washing the organic layer successively with a saturated aqueous solution of potassium hydrogensulfate, water, a saturated aqueous solution of sodium hydrogencarbonate and a saturated aqueous solution of sodium chloride, and then distilling off the organic solvent.

On the other hand, the removal of the carboxyl-protecting group $R_4$ can be conducted if necessary. It can be effected by suitably choosing a known method [for example, Japanese Patent Application Laid-Open (Kokai) No. SHO 61-207387 or Japanese Patent Application Laid-Open (Kokai) No. HEI 6-321952], although conditions vary depending on the nature of each protecting group. For example, when an allyl group is used, a reaction is allowed to easily proceed by diluting the reaction product with a solvent and then causing tributyltin hydride, a carboxylic acid such as acetic acid or 2-ethylhexanoic acid, or an alkali metal salt such as the sodium salt of such a carboxylic acid to act in the presence of a palladium catalyst such as tetrakistriphenylphosphine palladium (0) or palladium (II) acetate. For this reaction, the preferred temperature ranges from room temperature to 50°C, and preferred usable exemplary solvents include halogenated hydrocarbons such as dichloromethane, ethers such as tetrahydrofuran and diethyl ether, aromatic hydrocarbons such as benzene and toluene, esters such as ethyl acetate, ketones such as acetone and methyl ethyl ketone, water, and mixed solvents thereof. The target compound (Ia) can be obtained by distilling off the solvent after completion of the reaction.

When the protecting group is an aralkyl group such as a paranitrobenzyl group, a deprotecting reaction can be carried out using a catalytic hydrogenating reaction which employs hydrogen in the presence of a palladium-carbon catalyst.

Incidentally, it is possible to simultaneously conduct conversion of the 2-substituent by using the method for the removal of the carboxyl-protecting group $R_4$ in the compound (III). Feasible examples of the conversion include reduction of a double bond or triple bond, deprotection of an amino group, and removal of a carboxyl-protecting group.

The target compound (Ia) which is obtained by removing the protecting groups for the hydroxyl group and/or the carboxyl group in the compound (III) can be purified by chromatography such as liquid chromatography, recrystallization or the like if necessary. Further, a mixture of isomers can be separated by chromatography such as column chromatography, recrystallization or the like if necessary.

Process 2:

Each compound of the formula (I) in which $R_1$ is the above-described substituted or unsubstituted alkylthio group, substituted or unsubstituted alkenylthio group, substituted or unsubstituted aralkylthio group, substituted or unsubstituted arylthio group, substituted or unsubstituted heterocyclic thio group, or substituted or unsubstituted acylthio group (Compound (Ia)) can be prepared in accordance with the following reaction scheme, by exchanging the 2-thio group of the compound (X) and then removing its hydroxyl-protecting group and, if necessary, its carboxyl-protecting group.

(a)

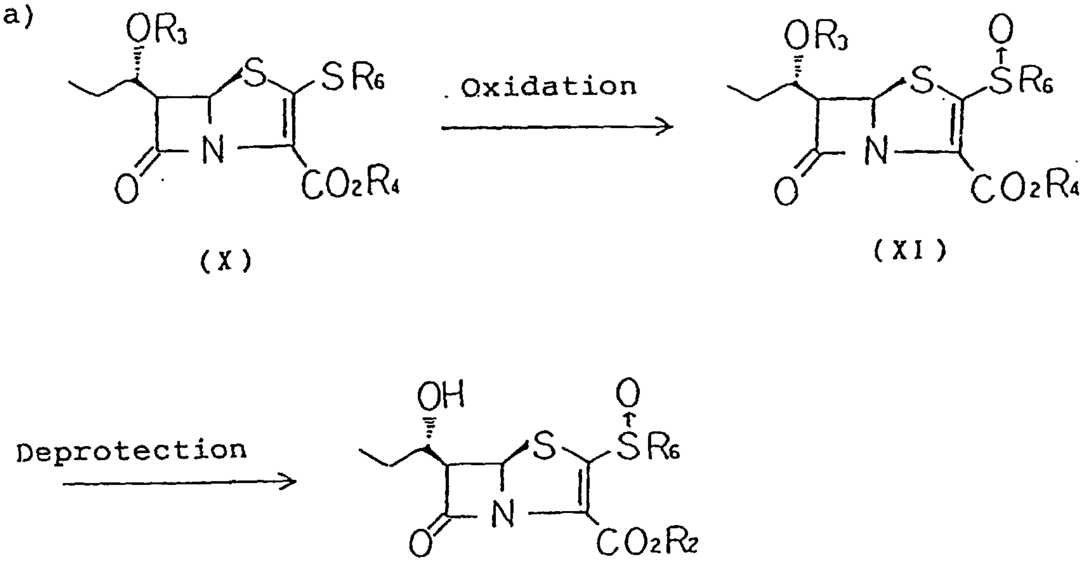


(X) (XI)

(XI')

(b)

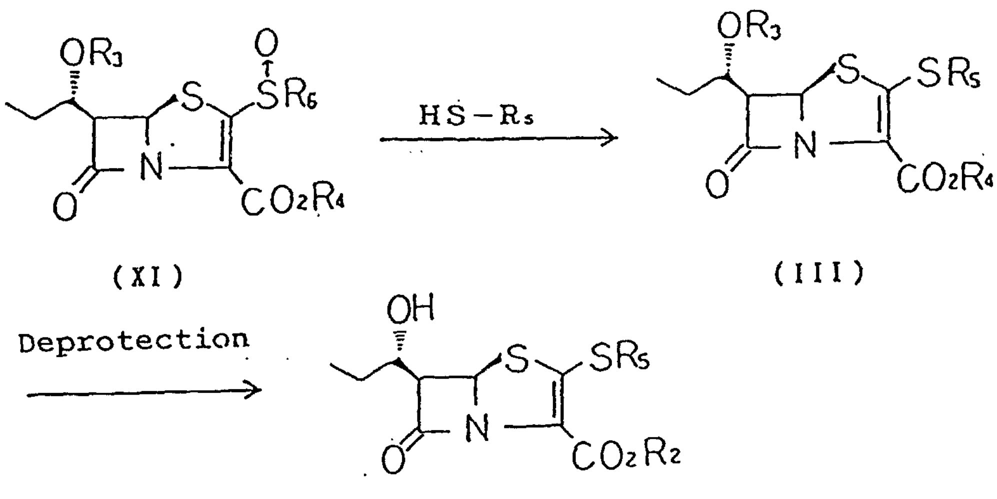


(XI) (III)

(Ia)

wherein $R_6$ represents a substituted or unsubstituted alkyl group, a substituted or unsubstituted alkenyl group, a substituted or unsubstituted aralkyl group or a substituted or unsubstituted aryl group, and $R_2$, $R_3$, $R_4$ and $R_5$ have the same meanings as defined above.

As preferred specific examples of the substituted or unsubstituted alkyl group, the substituted or unsubstituted alkenyl group, the substituted or unsubstituted aralkyl group or the substituted or unsubstituted aryl group of $R_6$, those described above in connection with $R_1$ can also be mentioned.

In the above process, step (a) is first to oxidize into a sulfoxide the sulfur atom of the substituted or unsubstituted alkylthio group, the substituted or unsubstituted alkenylthio group, the substituted or unsubstituted aralkylthio group or the substituted or unsubstituted arylthio group at the 2-position of the penem as the compound (X).

This oxidizing reaction can be carried out following a known process [for example, Japanese Patent Application Laid-Open (Kokai) No. SHO 57-77688]. For example, it can be effected by causing 1 to 1.2 equivalents of a peroxy acid such as m-chloroperbenzoic acid to act on 1 equivalent of the compound (X) at -78°C to 0°C for 30 minutes to 2 hours in a solvent, for example, a halogenated hydrocarbon such as dichloromethane, an aromatic hydrocarbon such as benzene or toluene, or a saturated hydrocarbon such as hexane.

The sulfoxide can be obtained by diluting the reaction product with a water-immiscible organic solvent after the reaction, washing the organic layer successively with a saturated aqueous solution of sodium hydrogencarbonate and a saturated aqueous solution of sodium chloride, and then distilling off the organic solvent.

It is also possible to obtain the compound (XI') by removing its hydroxyl-protecting group and, if necessary, its carboxyl-protecting group. The removal of these hydroxyl-protecting group and carboxyl-protecting groups can be effected as described above. Subsequent to the removal of these protecting groups, the compounds (XI) and (XI') can be purified further by chromatography or the like if necessary.

In the above process, the exchange reaction of the thio group in step (b) can be conducted by causing the thiol compound (HS-$R_5$) to act on the thus-obtained sulfoxide (XI). This can be practiced by a known method [for example, Japanese Patent Application Laid-Open (Kokai) No. SHO 56-156281). To react the thiol compound with the sulfoxide, it is preferred to react 1 equivalent of the sulfoxide with 1 to 2 equivalents of the thiol compound at -78°C to 0°C for 30 minutes to 4 hours in the presence of 1 to 1.5 equivalents of a tertiary amine such as triethylamine or diisopropylethylamine.

The compound (III) can be obtained by diluting the reaction product with a water-immiscible organic solvent after the reaction, washing the organic layer successively with a saturated aqueous solution of potassium hydrogensulfate, water, a saturated aqueous solution of sodium hydrogencarbonate and a saturated aqueous solution of sodium chloride, and then distilling off the organic solvent.

Illustrative of the thiol compound represented by the formula:

$$R_5\text{-SH}$$

are methanethiol, ethanethiol, n-propylmercaptan, isopropylmercaptan, n-butylmercaptan, isobutylmercaptan, tert-butylmercaptan, n-pentylmercaptan, neopentylmercaptan, 2-hydroxyethylmercaptan, 3-hydroxypropylmercaptan, 1-mercapto-2-(N-p-nitrobenzyloxycarbonylamino)ethane, 1-mercapto-3-(N-p-nitrobenzyloxycarbonylamino)propane, 2-fluoro-1-mercaptoethane, 3-fluoro-1-mercaptopropane, 1-mercapto-2-phenoxyethane, 1-mercapto-3-phenoxypropane, 1-mercapto-2-[N-methyl-N-(2-oxo-2-phenylethyl)amino]ethane, 1-(2-mercaptoethyl)-pyrrolidine, 1-(2-mercaptoethyl)piperidine, 1-(2-mercaptoethyl)pyrrole, 4-(2-mercaptoethyl)-1-(p-nitro-benzyloxycarbonyl)piperazine,

cyclopropanethiol, cyclopentanethiol, cyclohexanethiol, 1-mercaptoindane, 2-mercaptoindane, 2-mercapto-1-indanone, 3-mercapto-1-indanone, 6,7-dihydro-5-mercapto-5H-cyclopenta[b]pyridine, 6,7-dihydro-6-mercapto-5H-cyclopenta[b]pyridine, 6,7-dihydro-7-mercapto-5H-cyclopenta[b]pyridine,

allylmercaptan,

benzylmercaptan, diphenylmethylmercaptan, tritylmercaptan, (2-pyridyl)methylmercaptan, (3-pyridyl)-methylmercaptan, (4-pyridyl)methylmercaptan, (2-pyrimidyl)methylmercaptan, (imidazol-2-yl)methylmercaptan, (1-methyl-imidazol-2-yl)methylmercaptan, (1-benzyl-imidazol-2-yl)methylmercaptan, (thiazol-2-yl)-methylmercaptan, 2-phenylethanethiol, 2,2-diphenylethanethiol, 2-(2-mercaptoethyl)pyridine, 3-(2-mercaptoethyl)pyridine, 4-(2-mercaptoethyl)pyridine, 2-(2-mercaptoethyl)pyrimidine, 2-(2-mercaptoethyl)-imidazole, 1-mercapto-3-phenylpropane, 1-mercapto-3,3-diphenylpropane, 2-(3-mercaptopropyl)pyridine, 3-(3-mercaptopropyl)pyridine, 4-(3-mercaptopropyl)pyridine, 2-(3-mercaptopropyl)pyridine, 2-(3-mercaptopropyl)-imidazole, 2-(3-mercaptopropyl)thiazole, 1-naphthylmethanethiol, 2-naphthylmethanethiol, 2-(1-naphthyl)-ethanethiol, 2-(2-naphthyl)ethanethiol,

2- (p-nitrobenzyloxycarbonyl) amino-2-phenylethanethiol, 2-(p-nitrobenzyloxycarbonyl)amino-3-phenylpropanethiol, 1-mercapto-2-oxo-2-phenylethane, 1-mercapto-2-oxo-2-(2-pyridyl)ethane, 1-mercapto-2-oxo-2-(3-pyridyl) ethane, 1-mercapto-2-oxo-2-(4-pyridyl)-ethane, 1-mercapto-2-oxo-2-(imidazol-2-yl)ethane, 1-mercapto-2-oxo-2-(thiazol-2-yl)ethane, 3-mercapto-1-(p-nitrobenzyloxycarbonyl)azetidine, 1-allyloxycarbonyl-3-mercaptoazetidine, 1-benzyl-3-mercaptoazetidine, 3-mercapto-1-phenylazetidine, 3-mercapto-1-(2-oxo-2-phenylethyl)azetidine,

3-mercapto-1-(p-nitrobenzyloxycarbonyl)-pyrrolidine, 1-allyloxycarbonyl-3-mercaptopyrrolidine, 1-benzyl-3-mercaptopyrrolidine, 3-mercapto-1-phenethylpyrrolidine, 1-cyclopropyl-3-mercaptopyrrolidine, 1-cyclopentyl-3-mercaptopyrrolidine, 3-mercapto-1-(3-phenylpropyl)pyrrolidine, 3-mercapto-1-phenylpyrrolidine, 3-mercapto-1-(2-pyridyl)pyrrolidine, 3-mercapto-1-(3-pyridyl)pyrrolidine, 3-mercapto-1-(4-pyridyl)pyrrolidine, 3-mercapto-1-(2-pyrimidyl)-pyrrolidine, 1-(imidazol-2-yl)-3-mercaptopyrrolidine, 3-mercapto-1-(thiazol-2-yl)pyrrolidine, 3-mercapto-1-[4-(p-nitrobenzyloxycarbonyl)aminophenyl]pyrrolidine, 1-(2-hydroxyethyl)-3-mercaptopyrrolidine, 1-(2-hydroxy-2-phenylethyl)-3-mercaptopyrrolidine, 1-(2-fluoroethyl)-3-mercaptopyrrolidine, 3-mercapto-1-(2-oxo-2-phenylethyl)pyrrolidine, 3-mercapto-1-[2-(2-p-nitro-benzyloxy)phenyl-2-oxoethyl]pyrrolidine, 3-mercapto-1-[2-oxo-2-(3-p-nitrobenzyloxy)phenylethyl]pyrrolidine, 3-mercapto-1-[2-(4-p-nitrobenzyloxy)phenyl-2-oxoethyl]-pyrrolidine, 1-[2-(p-fluoro)phenyl-2-oxoethyl]-3-mercaptopyrrolidine, 3-mercapto-1-[2-(p-methyl)phenyl-2-oxoethyl]pyrrolidine, 3-mercapto-1-[2-(p-methoxy)-phenyl-2-oxoethyl]pyrrolidine, 3-mercapto-1-(1-methyl-2-oxo-2-phenylethyl)pyrrolidine, 3-mercapto-1-(3-oxo-3-phenylpropyl)pyrrolidine, 3-mercapto-1-(2-oxo-3-phenylpropyl)pyrrolidine, 1-(1-indanon-2-yl)-3-mercaptopyrrolidine, 1-(1-indanon-3-yl)-3-mercaptopyrrolidine, 3-mercapto-1-[(2-pyridyl)methyl]pyrrolidine, 3-mercapto-1-[(3-pyridyl)methyl]pyrrolidine, 3-mercapto-1-[(4-pyridyl)methyl]pyrrolidine,

2-mercaptomethyl-1-(p-nitrobenzyloxycarbonyl)-piperidine, 3-mercapto-1-(p-nitrobenzyloxycarbonyl)-piperidine, 4-mercapto-1-(p-nitrobenzyloxycarbonyl)-piperidine, 1-allyloxycarbonyl-4-mercaptopiperidine, 1-benzyl-3-mercaptopiperidine, 3-mercapto-1-phenylpiperidine,

5-hydroxymethyl-3-mercapto-1-(p-nitrobenzyloxycarbonyl)pyrrolidine, 3-mercapto-1-(p-nitrobenzyloxycarbonyl) -5-phenoxymethylpyrrolidine, 3-mercapto-1-(p-nitrobenzyloxycarbonyl) -5-phenylmethylpyrrolidine, and

4-mercapto-1-(1,2-di(p-nitrobenzyloxycarbonyl))-pyrazolidine.

The target compound (Ia) can then be obtained by removing the hydroxyl-protecting group and, if necessary, the carboxyl-protecting group by the above-described methods.

Incidentally, it is possible to simultaneously conduct conversion of the 2-substituent by using the method for the removal of the carboxyl-protecting group $R_4$ in the compound (III). Feasible examples of the conversion include reduction of a double bond or triple bond, deprotection of an amino group, and removal of a carboxyl-protecting group.

The target compound (Ia) which is obtained by removing the protecting groups for the hydroxyl group and/or the carboxyl group in the compound (III) can be purified by chromatography such as liquid chromatography, recrystallization or the like if necessary. Further, a mixture of isomers can be separated by chromatography such as column chromatography, recrystallization or the like if necessary.

Process 3:

Each compound of the formula (I) in which $R_1$ is the above-described substituted or unsubstituted alkyl group, substituted or unsubstituted alkenyl group, substituted or unsubstituted aralkyl group, substituted or unsubstituted aryl group, or substituted or unsubstituted heterocyclic group can be prepared in accordance with the following reaction scheme, using as a starting material the compound (VII') obtained in Process 1.

(a)

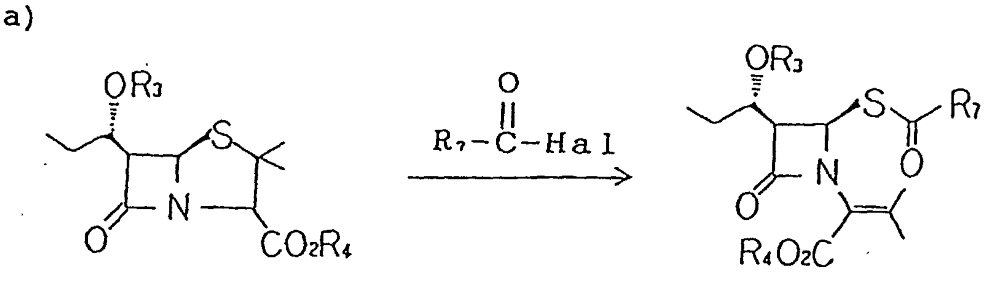


(VII') (XII)

(b)

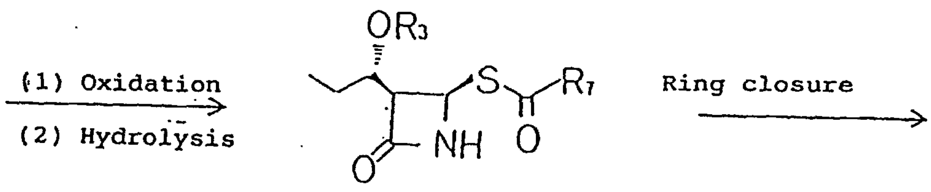


(XIII)

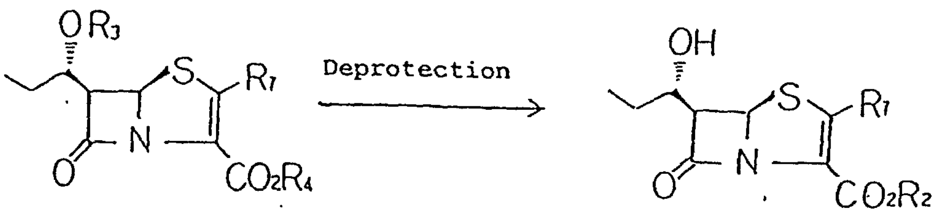


(XIV)

(Ib)

wherein $R_7$ represents a substituted or unsubstituted alkyl group, a substituted or unsubstituted alkenyl group, a substituted or unsubstituted aralkyl group, a substituted or unsubstituted aryl group or a substituted or unsubstituted heterocyclic group, and $R_2$, $R_3$ and $R_4$ have the same meanings as defined above.

As preferred specific examples of the substituted or unsubstituted alkyl group, the substituted or unsubstituted alkenyl group, the substituted or unsubstituted aralkyl group, the substituted or unsubstituted aryl group or the substituted or unsubstituted heterocyclic group of $R_7$, those described above in connection with $R_1$ can also be mentioned.

In the above process, step (a) is to open the ring of the compound (VII') to derive the compound (XII). This reaction is already known (Heterocycles, **31**, 617, 1990) or can be practiced following the known reaction. Incidentally, the compound (VII') can be obtained by chromatographic isolation of the compound (VII).

Namely, this reaction can be conducted as will be described next. Following the above-described publication, 1 to 1.2 equivalents of a silver salt such as silver chloride or silver nitrate are reacted with 1 equivalent of the compound (VII') at -20°C to 50°C, preferably room temperature in the presence of 1 to 2 equivalents of a strong base such as diazabicyclononene (DBN) or diazabicycloundecene (DBU) in a solvent, for example, acetonitrile, pyridine, an ether such as dioxane, or an amide such as N,N-dimethylformamide (DMF), whereby the compound (VII') is converted into the silver salt.

Next, the corresponding acid chloride ($R_7$-CO-Hal) is reacted with the resultant silver salt at room temperature, and the insoluble matter is filtered off. The reaction product can be purified by chromatography if necessary.

The thus-obtained compound (XII) is then subjected to oxidative cleavage at the double bond thereof, and the resulting imide is hydrolyzed into the azetidinone compound (XIII).

These oxidative cleavage and hydrolysis can be conducted by causing ozone to act on the compound (XII) at -78°C to -50°C in a solvent, for example, an ester such as ethyl acetate or an alcohol such as methanol or ethanol, causing a reducing agent, for example, dimethyl sulfide, a phosphine such as triphenylphosphine, or zinc to act, distilling off the organic solvent, and then causing water, methanol or a mixed solution thereof to act to hydrolyze the imide.

The azetidinone compound (XIII) can be obtained by distilling off the solvent after completion of the reaction. If necessary, it can be purified by chromatography or the like.

On the other hand, in the above process, step (b) is to subject the azetidinone derivative (XIII) to a ring-closing reaction to form the penem ring, that is, the compound (XIV) and then to remove its hydroxyl-protecting group and, if necessary, the carboxyl-protecting group.

To perform these reactions, an oxalyl halide monoester is first reacted with the azetidinone derivative (XIII)

at -20°C to 10°C for 10 to 30 minutes in the presence of a tertiary amine such as triethylamine while using, as a solvent, a halogenated hydrocarbon such as dichloromethane, an aromatic hydrocarbon such as benzene or toluene, or an ether such as tetrahydrofuran or diethyl ether. Next, the reaction product is diluted with a water-immiscible organic solvent, the organic layer is washed successively with a saturated aqueous solution of potassium hydrogensulfate, a saturated aqueous solution of sodium hydrogencarbonate and brine and, if necessary, the organic solvent is distilled off, whereby the imide is obtained.

Further, a phosphorous acid ester such as triethyl phosphite is caused to act on the thus-obtained residue at a ratio of 2 to 5 equivalents to 1 equivalent of the reaction product at 80°C to 150°C for 1 to 24 hours in a solvent, for example, an aromatic hydrocarbon such as benzene, toluene or xylene or a saturated hydrocarbon such as hexane. Finally, the organic solvent is distilled off so that the compound (XIV) is obtained. If necessary, it can be purified by chromatography, recrystallization or the like.

By the way, when the 2-substituent of the penem derivative (XIV) is partly substituted by one or more halogen-substituted alkyl groups such as bromine- or chlorine-substituted alkyl groups, these halogen atoms can be replaced by other substituents, for example, acetoxy groups, hydroxyl groups, or substituents capable of forming an intramolecular quaternary salt.

For example, when an aryl group such as a phenyl group contains a halogen-substituted alkyl group, this halogen atom can be changed to various functional groups, for example, acyloxy groups led by an acetoxy group, nitrogen-containing heterocyclic groups typified by a pyridinium group, and a hydroxyl group.

This reaction varies depending on the functional group to which the halogen is to be changed. For example, a change to a pyridinium group can be conducted by reacting 1 equivalent of the halogen-containing compound (XIV) with 1 to 5 equivalents of pyridine at 0°C to room temperature for 1 to 24 hours in a solvent, for example, an amide such as N,N-dimethylformamide. The target compound can be obtained by distilling off the organic solvent after completion of the reaction. It can also be purified by chromatography, such as HPLC, or the like if necessary.

The hydroxyl-protecting group and, if necessary, the carboxyl-protecting group are next removed from compound (XIV) to obtain the target penem compound (Ib) according to the present invention. This removal of the hydroxyl-protecting group and carboxyl-protecting group can be conducted in a manner similar to the above-described manner.

Incidentally, it is possible to simultaneously conduct conversion of the 2-substituent by using the method for the removal of the carboxyl-protecting group $R_4$ in the compound (XIV). Feasible examples of the conversion include reduction of a double bond or triple bond, deprotection of an amino group, and removal of a carboxyl-protecting group.

The target compound (Ib) which is obtained by removing the protecting groups for the hydroxyl group and/or the carboxyl group in the compound (XIV) can be purified by chromatography such as liquid chromatography, recrystallization or the like if necessary. Further, a mixture of isomers can be separated by chromatography such as column chromatography, recrystallization or the like if necessary.

Process 4:

Each compound of the formula (I) in which $R_1$ is the above-described substituted or unsubstituted alkyl group, substituted or unsubstituted alkenyl group, substituted or unsubstituted aralkyl group, substituted or unsubstituted aryl group, or substituted or unsubstituted heterocyclic group can also be prepared in accordance with the following reaction scheme.

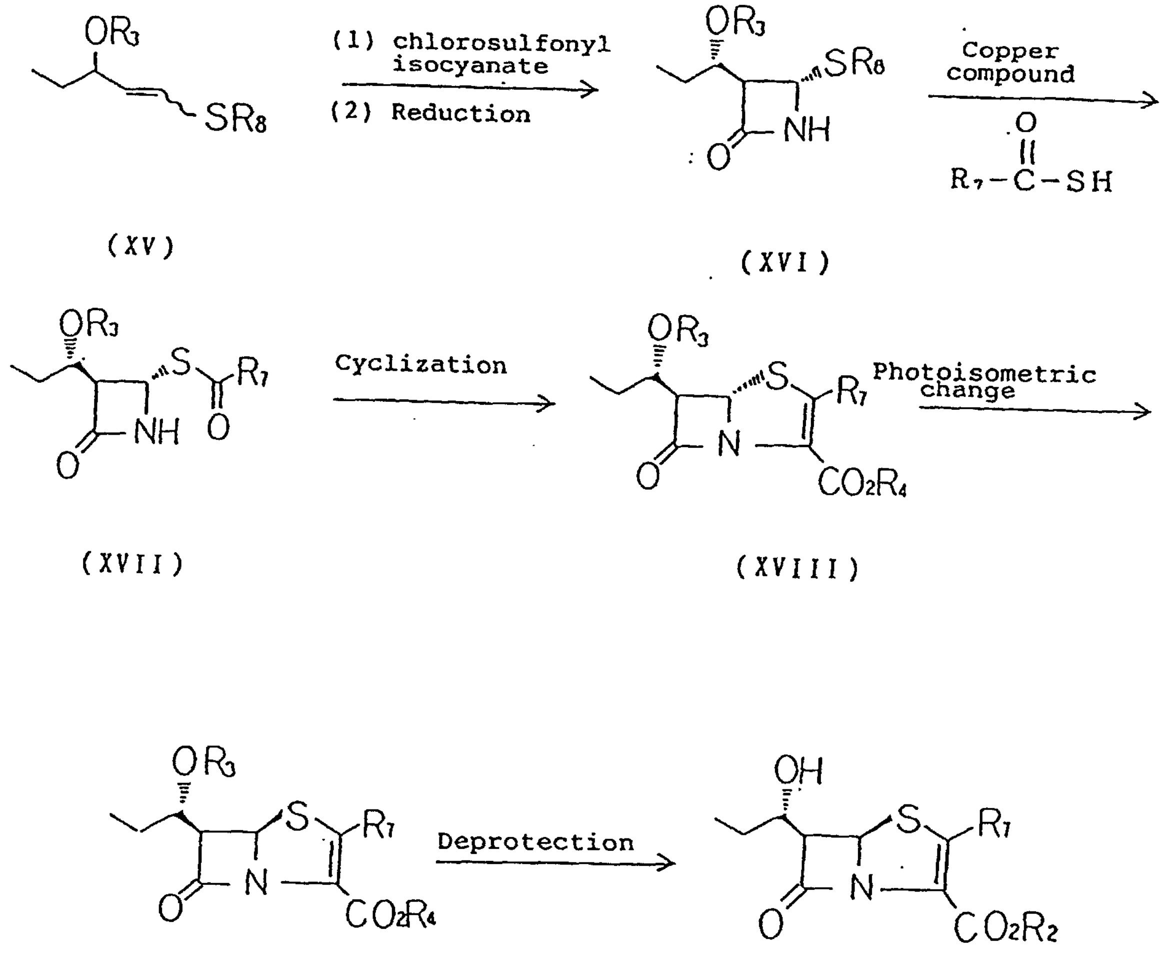


(XV) (XVI)

(XVII) (XVIII)

(XIX) (Ib)

wherein $R_8$ represents a substituted or unsubstituted alkyl group or a substituted or unsubstituted aryl group, and $R_2$, $R_3$, $R_4$ and $R_7$ have the same meanings as defined above.

As preferred specific examples of the substituted or unsubstituted alkyl group or the substituted or unsubstituted aryl group of $R_8$, those described above in connection with $R_1$ can also be mentioned.

The above process is to obtain the compound (Ib) by using the compound (XV). This process can be practiced following an already known process [see Japanese Patent Application Laid-Open (Kokai) No. SHO 61-207373, Japanese Patent Application Laid-Open (Kokai) No. HEI 3-127773 or Japanese Patent Application Laid-Open (Kokai) No. HEI 4-69387].

Namely, 1 to 1.5 equivalents of chlorosulfonyl isocyanate are reacted with 1 equivalent of vinyl sulfide (XV) at -20°C to room temperature while using, as a solvent, an ether such as diethyl ether, an aromatic hydrocarbon such as toluene, a saturated hydrocarbon such as hexane, or a halogenated hydrocarbon such as dichloromethane. After a cyclized product is obtained, a reducing agent such as pyridine-thiophenol, pyridine-thioacetic acid or sodium sulfite is caused to act to obtain the compound (XVI).

This compound (XVI) is converted into the azetidinone (XVII), followed by the cyclization to derive the compound (XVIII).

Specifically, 1 to 5 equivalents of acetic acid are reacted with 1 equivalent of the compound (XVI) at 50°C to 150°C for 1 to 24 hours in the presence of 1 to 5 equivalents of a copper compound, for example, a cuprous salt such as cuprous oxide or cuprous chloride or a cupric salt such as cupric oxide or cupric acetate while using, as a solvent, an amide such as N,N-dimethylformamide or an aromatic hydrocarbon such as toluene.

In a ketone such as acetone, acetonitrile, water or a mixed solvent thereof, the corresponding thiocarboxylic acid [$R_7C(O)SH$] is next caused to act at pH 10 to pH 7 and O°C to 60°C for 30 minutes to 12 hours, whereby the

compound (XVII) is obtained.

Further, the compound (XVII) is cyclized as in step (c) of Process 1 to obtain the compound (XVIII).

Next, this compound (XVIII) is exposed to light to conduct the isomerization of the steric configuration of the β-lactam ring. Specifically, the compound (XVIII) is dissolved in a solvent, for example, an ester such as ethyl acetate, a ketone such as acetone or an ether such as diethyl ether, and the resulting solution is exposed for 30 minutes to 12 hours to light such as radiation from a mercury lamp or sunlight. After the solvent is distilled off, the compound (XIX) is obtained. If necessary, the residue may be purified by chromatography, recrystallization or the like.

The thus-obtained compound (XIX) is subjected to the removal of the hydroxyl-protecting group and, if necessary, the carboxyl-protecting group by the above-described method, whereby the penem compound (Ib) according to the present invention is obtained.

Incidentally, it is possible to simultaneously conduct conversion of the 2-substituent by using the method for the removal of the carboxyl-protecting group $R_4$ in the compound (XIX). Feasible examples of the conversion include reduction of a double bond or triple bond, deprotection of an amino group, and removal of a carboxyl-protecting group.

The target compound (Ib) which is obtained by removing the carboxyl-protecting group in the compound (XIX) can be purified by chromatography such as liquid chromatography, recrystallization or the like if necessary. Further, a mixture of isomers can be separated by chromatography such as column chromatography, recrystallization or the like if necessary.

Process 5:

Further, the compound (Ia) according to the present invention can also be synthesized in accordance with the following reaction scheme.

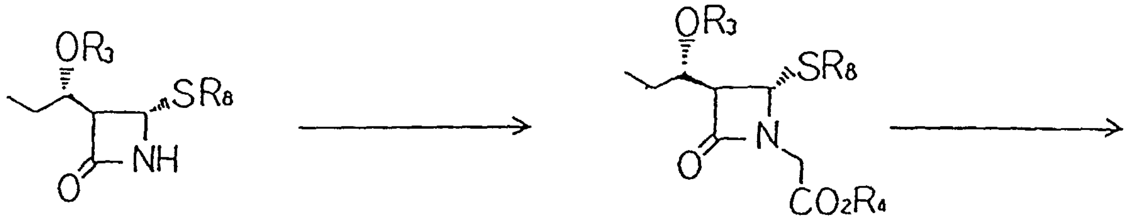

(XVI) (XX)

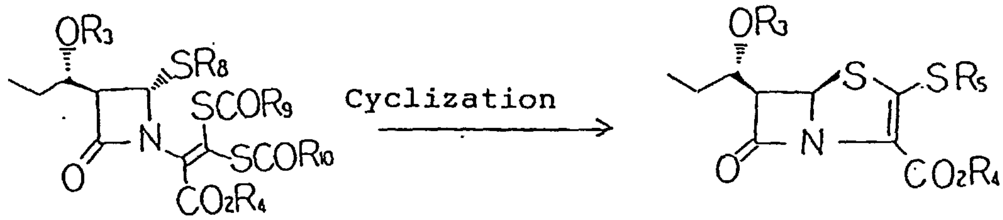


(XXI) (III)

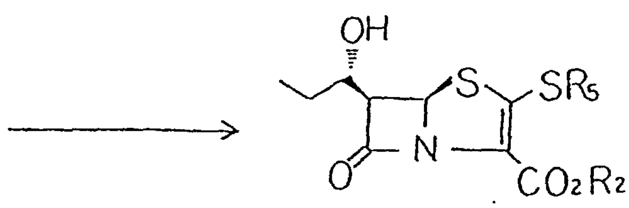

(Ia)

wherein $R_9$ and $R_{10}$ represent a substituted or unsubstituted alkyl group, a substituted or unsubstituted aryl group, a substituted or unsubstituted alkylthio group or a substituted or unsubstituted arylthio group, and $R_3$, $R_4$, $R_5$ and $R_8$ have the same meanings as defined above.

As preferred specific examples of the substituted or unsubstituted alkyl group, the substituted or unsubstituted aryl group, the substituted or unsubstituted alkylthio group or the substituted or unsubstituted arylthio group of $R_9$ and $R_{10}$, those described above in connection with $R_1$ can also be mentioned.

First, the compound (XX) is obtained from the compound (XVI) obtained in Process 4. This reaction can be conducted in accordance with a known process [see Japanese Patent Application Laid-Open (Kokai) No. HEI 5-25181]. Namely, a carbonate such as potassium carbonate or cesium carbonate or a tertiary amine such as triethylamine is caused to act on the α-haloacetic acid ester at room temperature to 50°C for 1 to 24 hours in an amide such as DMF, a ketone such as acetone or methyl ethyl ketone, an ether such as THF or diethyl ether, or a mixture thereof. The reaction product is diluted with a water-immiscible organic solvent, the resultant solution is washed with water and a weakly acidic solution such as an aqueous solution of potassium hydrogensulfate, and the solvent is then distilled off, whereby the compound (XX) is obtained. If necessary, it can be purified by recrystallization, chromatography or the like.

Also following a known process [see Japanese Patent Publication (Kokoku) No. HEI 1-34994], 1.5 to 2.5 equivalents of a base such as lithium hexamethyldisilazide (LHMDS) or lithium diisopropylamide (LDA) are caused to act on 1 equivalent of the compound (XX) at -78°C to -20°C in an ether solvent such as tetrahydrofuran (THF)or diethyl ether. Further, carbon disulfide and then 2.0 to 3.0 equivalents of an acid chloride were caused to act, whereby the compound (XXI) was obtained. As the acid chloride, it is possible to use phosgene, an aliphatic acid chloride such as

acetyl chloride or an aromatic acid chloride such as benzoyl chloride.

Chlorine gas, bromine gas, sulfuryl chloride or the like is caused to act on the thus-obtained compound (XXI) in a solvent, for example, a halogenated hydrocarbon such as methylene chloride or an aromatic hydrocarbon such as toluene or benzene, whereby halogenation of the 4-position of azetidinone is conducted. Next, a primary or secondary amine, namely, an aralkylamine such as benzylamine, an aliphatic amine such as methylamine or dimethylamine, or a nitrogen-containing heterocyclic compound such as morpholine is caused to act on the thus-obtained compound. In the presence of a base, a compound represented by the formula:

$$X\text{-}R_5$$

wherein $R_5$ has the same meaning as defined above, and X represents a halogen atom, an alkanesulfonyloxy group, a trihalogenomethanesulfonyloxy group or an arylsulfonyloxy group, for example, an alkyl halide such as methyl iodide or benzyl bromide is then caused to act, whereby the compound (III) can be obtained.

The target compound (Ia) can then be obtained by removing the hydroxyl-protecting group and, if necessary, the carboxyl-protecting group in the above-described manner.

Incidentally, it is possible to simultaneously conduct conversion of the 2-substituent by using the method for the removal of the carboxyl-protecting group $R_4$ in the compound (III). Feasible examples of the conversion include reduction of a double bond or triple bond, deprotection of an amino group, and removal of a carboxyl-protecting group.

The target compound (Ia) which is obtained by removing the carboxyl-protecting group in the compound (III) can be purified by chromatography such as liquid chromatography, recrystallization or the like if necessary. Further, a mixture of isomers can be separated by chromatography such as column chromatography, recrystallization or the like if necessary.

Process 6:

Further, the compound (Ic) according to the present invention can be obtained by the following process, from the compound (XX) obtained in Process 5.

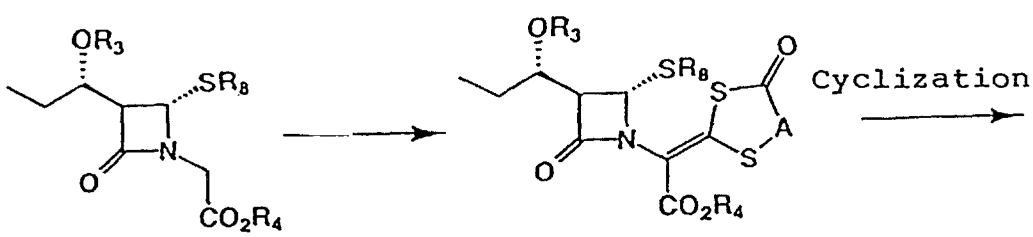


(XX) (XXII)

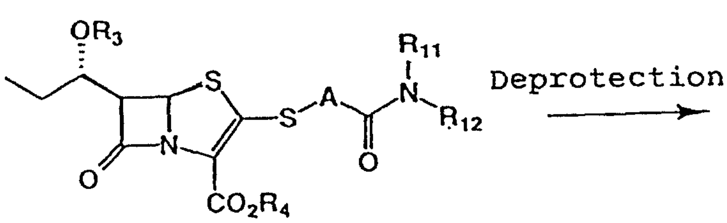


(XXIV)

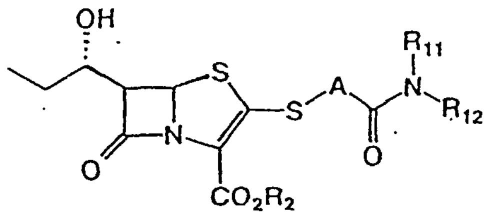

(Ic)

wherein $R_{11}$ and $R_{12}$ are the same or different and represent a substituted or unsubstituted alkyl group, a substituted or unsubstituted alkenyl group, a substituted or unsubstituted aralkyl group, a substituted or unsubstituted aryl group or a hydrogen atom, or $R_{11}$ and $R_{12}$ are coupled together to represent a substituted or unsubstituted, nitrogen-containing heterocyclic group, A represents a substituted or unsubstituted, linear or branched alkylene group, and $R_2$, $R_3$, $R_4$ and $R_8$ have the same meanings as defined above.

As preferred specific examples of the substituted or unsubstituted alkyl group, the substituted or unsubstituted alkenyl group, the substituted or unsubstituted aralkyl group or the substituted or unsubstituted aryl group of $R_{11}$ and $R_{12}$, those described above in connection with $R_1$ can also be mentioned. Further, as preferred specific examples of the nitrogen-containing heterocyclic group formed by $R_{11}$ and $R_{12}$, nitrogen-containing heterocyclic groups which are the same as the nitrogen-containing heterocyclic groups out of the heterocyclic groups described above in connection with $R_1$ can also be mentioned. In addition, preferred specific examples of the alkylene group represented by A include methylene, ethylene, trimethylene, ethylidene, propylene, propylidene, and the group represented by the following formula:

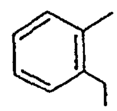

As a manner of substitution of the alkylene group represented by A out of the above-described groups, condensation of an aryl group to two carbon atoms of an alkylene group is included. As preferred substituents, the substituents for alkyl groups, said substituents having been described above in connection with $R_1$, can be mentioned. With respect to fused aryl groups, the same substituents as those described above in connection with aryl groups can be mentioned.

According to Process 6, carbon disulfide and an acid halide is caused to act on the compound (XX) obtained by a known process [for example, Japanese Patent Publication (Kokoku) No. HEI 1-34994], whereby the compound (XX) is converted into the compound (XXII). A halogen and an amine is then caused to act so that a ring is closed. The hydroxyl-protecting group and, if necessary, the carboxyl-protecting group are removed from the resultant compound (XXIV), whereby the compound (Ic) is obtained.

To obtain the compound (XXII) from the compound (XX), it is only necessary to cause 1.5 to 2.5 equivalents of a base such as LHMDS or LDA to act on 1 equivalent of the compound (XX) at a temperature of from - 78°C to -20°C in an ether solvent such as tetrahydrofuran or diethyl ether, followed by the further action of about 2 equivalents of carbon disulfide and the still further action of 1 to 2 equivalents, preferably about 1.5 equivalents of a reactive derivative of an acid halide, said reactive derivative being represented by the following formula (XXIII):

$$Z_1\text{-A-CO-}Z_2 \qquad \text{(XXIII)}$$

wherein $Z_1$ represents a halogen atom, $Z_2$ represents a halogen atom or a lower alkoxycarbonyloxy group, and A has the same meaning as defined above.

Incidentally, illustrative of the reactive derivative of the acid halide, said reactive derivative being represented by the formula (XXIII), are bromoacetyl bromide, bromoacetyl chloride, 2-bromopropionyl bromide, 2-bromopropionyl chloride, 3-bromopropionyl chloride, 2-bromobutyryl bromide, 3-bromobutyryl chloride, 4-bromobutyryl chloride, and

o-chloromethyl benzoyl chloride.

To obtain the compound (XXIV) from the compound (XXII), it is only necessary to cause 1 to 3 equivalents, preferably 1.5 equivalents of chlorine gas, sulfuryl chloride or bromine gas to act on 1 equivalent of the compound (XXII) in a solvent, for example, a halogenated hydrocarbon such as methylene chloride or an aromatic hydrocarbon such as toluene or benzene to conduct chlorination or bromination at the 4-position of azetidinone and then to cause a primary or secondary amine represented by the following formula:

$$HNR_{11}R_{12}$$

to act on the thus-obtained compound at a ratio of 1 to 5 equivalents, preferably 3 equivalents to 1 equivalent of the compound (XXII), and a tertiary amine such as triethylamine to act on the thus-obtained compound at a ratio of 1 to 5 equivalents, preferably 3 equivalents to 1 equivalent of the compound (XXII) at -40°C to room temperature in the same solvent.

Illustrative of the primary or secondary amine are aralkylamines such as benzylamines, aliphatic amines such as methylamine and dimethylamine, and secondary amines including a nitrogen-containing heterocycle, such as morpholine.

The thus-obtained compound (XXIV) can be purified by chromatography such as column chromatography, recrystallization or the like if necessary. The removal of the hydroxyl-protecting group and the carboxyl-protecting group from the compound (XXIV) can be conducted by the above-described methods, so that the target compound (Ic) can be obtained.

Incidentally, it is possible to simultaneously conduct conversion of the 2-substituent by using the method for the removal of the carboxyl-protecting group $R_4$ in the compound (XXIV). Feasible examples of the conversion include reduction of a double bond or triple bond, deprotection of an amino group, and removal of a carboxyl-protecting group.

The target compound (Ic) which is obtained by removing the hydroxyl-protecting group and/or the carboxyl-protecting group in the compound (XXIV) can be purified by chromatography such as liquid chromatography, recrystallization or the like if necessary. Further, a mixture of isomers can be separated by chromatography such as column chromatography, recrystallization or the like if necessary.

Process 7:

Among the compounds of the formula (I), the compound (Ia) according to the present invention can also be synthesized by the following reaction scheme.

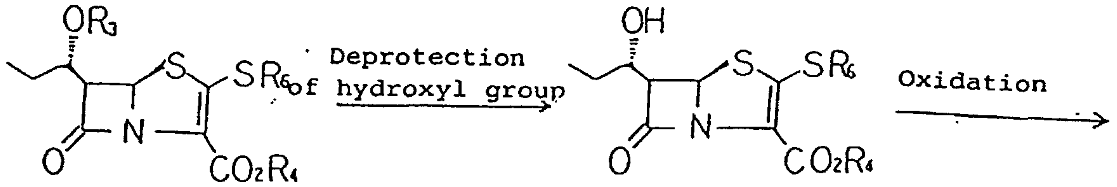


(X) (XXV)

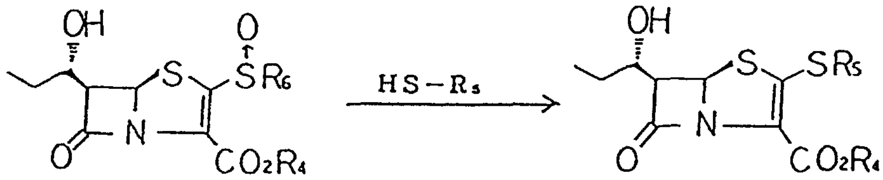


(XXVI) (XXVII)

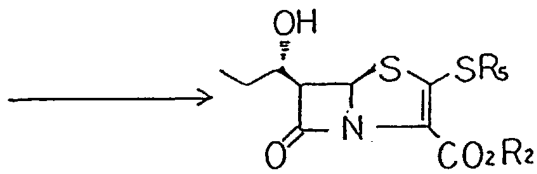

(Ia)

wherein $R_2$, $R_3$, $R_4$, $R_5$ and $R_6$ have the same meanings as defined above.

In this process, the hydroxyl-protecting group ($R_3$) on the propyl group at the 6-position of the compound (X) is first removed by the same method as described above to form the compound (XXV), which is then purified by chromatography or the like if necessary.

By a method similar to the reaction through which the compound (XI) is obtained from the compound (X) in step (a) of Process 2, the compound (XXV) is converted into the compound (XXVI), which is then purified by chromatography or the like if necessary. By a method similar to the reaction through which the compound (III) is obtained form the compound (XI) in step (b) of Process 2, the thiol compound ($R_5$-SH) is reacted with the compound (XXVI) and after completion of the reaction, post-treatment is conducted in a manner similar to step (b) of Process 2, whereby the compound (XXVII) is obtained. The target compound (Ia) can then be obtained by conducting chromatographic or like purification and the removal of the carboxyl-protecting group in the above-described manner as needed.

Incidentally, it is possible to simultaneously conduct conversion of the 2-substituent by using the method for the removal of the carboxyl-protecting group $R_4$ in the compound (XXVII). Feasible examples of the conversion include reduction of a double bond or triple bond, deprotection of an amino group, and removal of a carboxyl-protecting group.

The target compound (Ia) which is obtained by removing the hydroxyl-protecting group and/or the carboxyl-protecting group in the compound (XXVII) can be purified by chromatography such as liquid chromatography, recrystallization or the like if necessary. Further, a mixture of isomers can be separated by chromatography such as column chromatography, recrystallization or the like if necessary.

Process 8:

Among the compounds of the formula (I), the compound (Ia) according to the present invention can also be synthesized by the following reaction scheme.

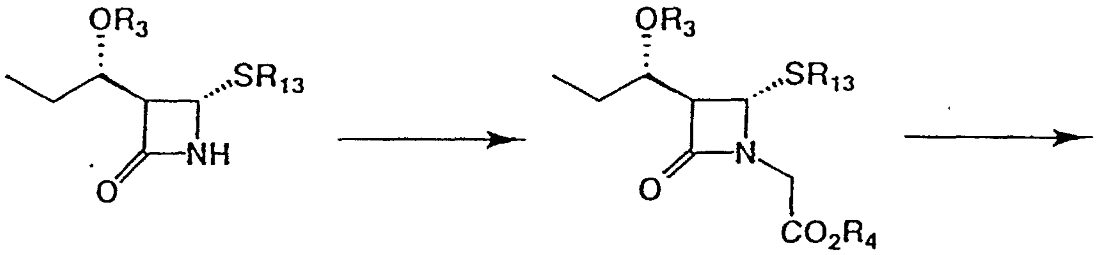

(XXVIII) (XXIX)

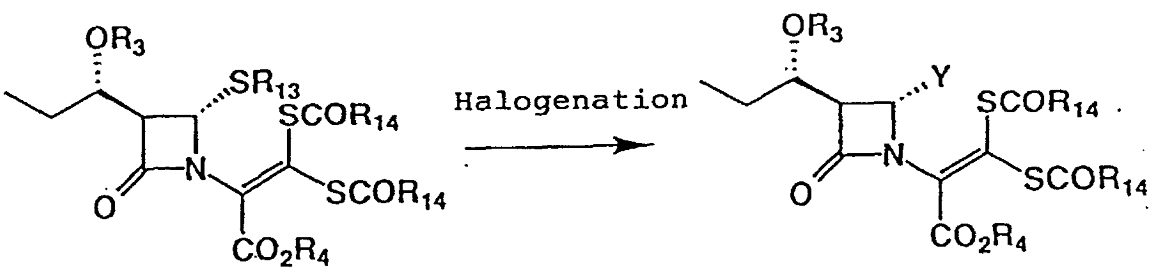


(XXX) (XXXI)

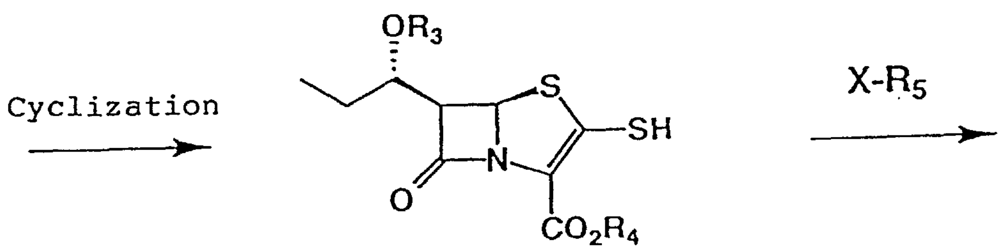


(IV)

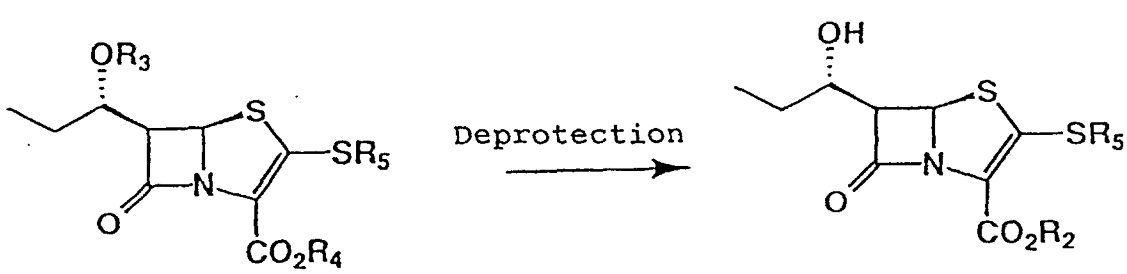


(III) (Ia)

wherein $R_2$, $R_3$, $R_4$ and $R_5$ have the same meanings as defined above, $R_{13}$ represents a substituted or unsubstituted alkyl group, a substituted or unsubstituted alkenyl group, a substituted or unsubstituted aralkyl group, a substituted or

unsubstituted aryl group, a substituted or unsubstituted heterocyclic group or a substituted or unsubstituted acyl group, $R_{14}$ either independently or in combination represents a substituted or unsubstituted alkyl group or a substituted or unsubstituted aryl group, X represents a halogen atom, an alkanesulfonyloxy group, a trihalogenomethanesulfonyloxy group or an arylsulfonyloxy group, and Y represents a chlorine atom or a bromine atom.

Among the above-described substituents, as preferred specific examples of the substituted or unsubstituted alkyl group, the substituted or unsubstituted alkenyl group, the substituted or unsubstituted aralkyl group, the substituted or unsubstituted aryl group, the substituted or unsubstituted heterocyclic group or the substituted or unsubstituted acyl group of $R_{13}$, those described above in connection with $R_1$ can also be mentioned. As preferred examples of the substituted or unsubstituted alkyl group or the substituted or unsubstituted aryl group of $R_{14}$, those described above in connection with $R_1$ can also be mentioned. Concerning X, examples of the halogen atom include chlorine, bromine and iodine, examples of the alkanesulfonyloxy group include methanesulfonyloxy and ethanesulfonyloxy, examples of the trihalogenomethanesulfonyloxy group include tri-fluoromethanesulfonyloxy, and examples of the arylsulfonyloxy group include benzenesulfonyloxy and p-toluenesulfonyloxy, respectively.

To practice this process, the compound (XXIX) is first obtained from the azetidinone compound (XXVIII). This reaction can be conducted in accordance with a known process [for example, Japanese Patent Application Laid-Open (Kokai) No. HEI 5-25181]. Specifically, the target compound (XXIX) can be obtained by causing 1 to 2 equivalents, preferably about 1.2 equivalent of an $\alpha$-substituted acetic acid ester, which is represented by the following formula (XXXII):

$$X\text{-}CH_2CO_2R_4 \qquad \text{(XXXII)}$$

wherein $R_4$ represents a carboxyl-protecting group and X has the same meaning as defined above, to act together with a carbonate such as potassium carbonate or cesium carbonate or a tertiary amine such as triethylamine on 1 equivalent of the azetidinone compound (XXVIII) at room temperature to 70°C for 1 to 24 hours in an amide such as N,N-dimethylformamide, a ketone such as methyl ethyl ketone, an ether such as tetrahydrofuran or diethyl ether, or a mixed solvent thereof, diluting the reaction product with a water-immiscible organic solvent, washing the resultant solution successively with a saturated aqueous solution of potassium hydrogensulfate, water, a saturated aqueous solution of sodium hydrogencarbonate and a saturated aqueous solution of sodium chloride, and then distilling off the solvent.

It can be purified by chromatography such as column chromatography, recrystallization or the like if necessary.

By the way, the azetidinone compound (XXVIII) is a known compound and can be obtained following a known process [for example, Japanese Patent Application Laid-Open (Kokai) No. SHO 61-207373].

The thus-obtained compound (XXIX) is then reacted with carbon disulfide and an acid halide to derive the compound (XXX). Following a known process [for example, Japanese Patent Publication (Kokoku) No. HEI 1-34994], this reaction can be conducted by causing 1.5 to 2.5 equivalents of a base such as lithium hexamethyl disilazide (LHMDS) or lithium diisopropylamide (LDA) to act on 1 equivalent of the compound (XXIX) at -78°C to -20°C in an ether solvent such as tetrahydrofuran or dimethyl ether and then causing about 2 equivalents of carbon disulfide and 2-4 equivalents of an acid halide to act successively.

Usable examples of the acid halide include phosgene, aliphatic acid halides such as pivaloyl chloride and acetyl chloride, aromatic acid halides such as benzoyl chloride, and aromatic acid dihalides such as phthaloyl chloride.

The compound (XXX) can be provided for the next reaction without isolation.

The thus-obtained compound (XXX) can be converted into the compound (XXXI) by further chlorinating or brominating it at the 4-position. The resulting compound (XXXI) is, either as is or after isolation, reacted with a primary or secondary amine at a ratio of 1 equivalent to 2 to 5 equivalents and with a tertiary amine such as triethyl amine at a ratio of 1 equivalent to 1 to 5 equivalents, whereby the compound (XXXI) is cyclized to obtain the compound (IV).

In these reactions, the chlorination or bromination is conducted by causing 1 to 3 equivalents, preferably 1.5 equivalents of chlorine gas, sulfuryl chloride or bromine gas to act on 1 equivalent of the compound (XXX) in a solvent, for example, a halogenated hydrocarbon such as methylene chloride or an aromatic hydrocarbon such as toluene or benzene. Further, usable examples of the primary or secondary amine employed for the cyclization include aralkylamines such as benzylamine, aliphatic amines such as methylamine, and secondary amines including a nitrogen-containing heterocycle, such as morpholine.

The thus-obtained compound (IV) can be provided for the next reaction without isolation. However, when isolated, it exists as a mixture with a thioxo isomer (IV') which is its tautomer and is represented by the below-described formula. Further, in the presence of a tertiary amine such as triethylamine or in the presence of tetraalkylammonium cations such as tetrabutylammonium cations, the compound (IV) can be isolated as its or their salt.

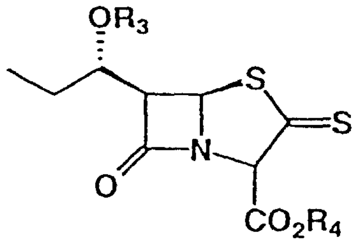

(IV')

To obtain the compound (Ia) from the compound (IV), it is only necessary to react the thus-obtained compound (IV), either as is or after isolation, with a compound, which is represented by the formula (XXXIII):

$$X\text{-}R_5 \qquad \text{(XXXIII)}$$

wherein $R_5$ and X have the same meanings as defined above, to form the compound (III) and then to conduct a deprotecting reaction as needed.

The reaction between the compound (IV) and the compound (XXXIII) is conducted by causing 1 to 5 equivalents of the compound (XXXIII) to act on 1 equivalent of the compound (IV) in the presence of a base such as triethylamine. Incidentally, usable examples of the compound represented by the formula (XXXIII) include alkyl halides such as methyl iodide, ethyl iodide, 2-fluoroethyl bromide, n-propyl iodide, isopropyl iodide, isobutyl chloride and neopentyl bromide; aralkyl halides such as benzyl bromide, 2-bromoethylbenzene, 3-bromopropylbenzene, p-dihydroxymethylbenzyl bromide, 1-benzyl-2-chloromethylimidazole, phenacyl bromide and 2-bromoacetylpyridine; aralkyl mesylates such as benzyl mesylate; aralkyl tosylate such as benzyl tosylate; and alkyl triflate such as trifluoromethanesulfonyloxymethylbenzene.

Further, the removal of the hydroxyl-protecting group $R_3$ and the removal of the carboxyl-protecting group $R_4$ for obtaining the target compound (Ia) from the compound (III) can be conducted by the above-described methods.

Incidentally, it is possible to simultaneously conduct conversion of the 2-substituent by using the method for the removal of the carboxyl-protecting group $R_4$ in the compound (III). Feasible examples of the conversion include reduction of a double bond or triple bond, deprotection of an amino group, and removal of a carboxyl-protecting group.

The target compound (Ia) which is obtained by removing the hydroxyl-protecting group and/or the carboxyl-protecting group in the compound (III) can be purified by chromatography such as liquid chromatography, recrystallization or the like if necessary. Further, a mixture of isomers can be separated by chromatography such as column chromatography, recrystallization or the like if necessary.

Process 9:

Among the compounds of the formula (I), the compound (Ia) according to the present invention can also be synthesized by the following reaction scheme.

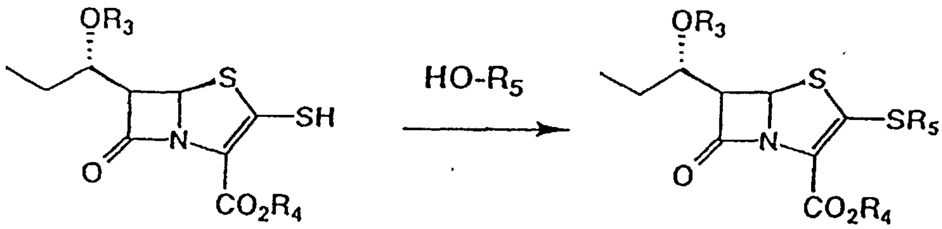


(IV) (III)

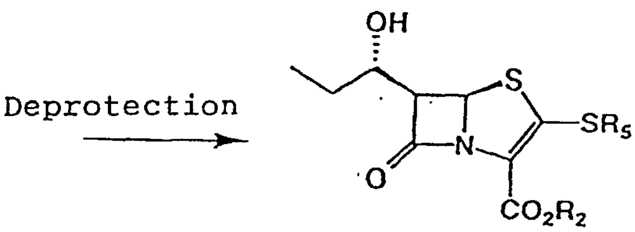


(Ia)

wherein $R_2$, $R_3$, $R_4$ and $R_5$ have the same meanings as defined above.

Namely, the compound (Ia) can be obtained by reacting the alcohol compound, which is represented by the following formula (XXXIV):

$$R_5\text{-OH}$$

wherein $R_5$ have the same meaning as defined above, with the compound (IV) in the presence of triphenylphosphine and a dialkyl azodicarboxylate such as diethyl azodicarboxylate in accordance with a known process (for example, J. Chem. Soc., Chem. Commun., 1982, 713) to obtain the compound (III) and then removing the protecting group from the compound (III) by the above-described method.

In the above reaction, 1 to 2 equivalents, preferably about 1.5 equivalents of triphenylphosphine, 1 to 2 equivalents, preferably about 1.5 equivalents of the alcohol compound (XXXIV) and 1 to 2 equivalents, preferably about 1.5 equivalents of the dialkyl azodicarboxylate are used per equivalent of the compound (IV).

As reaction conditions, -20°C to room temperature is suited, and preferred usable solvents include ethers such as tetrahydrofuran and diethyl ether, aromatic hydrocarbons such as toluene and benzene, esters such as ethyl acetate, and ketones such as acetone and methyl ethyl ketone. Further, after completion of the reaction, the reaction product is diluted with a water-immiscible organic solvent, the organic layer is washed successively with a saturated aqueous solution of sodium hydrogencarbonate and a saturated aqueous solution of sodium chloride, and the solvent is then distilled off, whereby the target compound (III) is obtained.

Illustrative of the alcohol compound represented by the formula (XXXIV) are:

methanol, ethanol, n-propyl alcohol, isopropyl alcohol, 2-fluoroethanol, 2-phenoxyethanol, 3-phenoxypropanol, 1-hydroxyethylpyrrolidine, 1-hydroxyethylpiperidine, 4-hydroxyethyl-1-(p-nitrobenzyloxycarbonyl)piperazine, 1-hydroxyethylpyrrole, cyclopentanol, cyclohexanol, 1-hydroxyindane, 2-hydroxyindane, 6,7-dihydro-5-hydroxy-5H-cyclopenta[b]pyridine, 6,7-dihydro-6-hydroxy-5H-cyclopenta[b]pyridine, 6,7-dihydro-7-hydroxy-5H-cyclopenta [b]pyridine, allyl alcohol, benzyl alcohol, 2-cyanophenylmethanol, 3-cyanophenylmethanol, 4-cyanophenylmethanol, 2-chlorophenylmethanol, 3-chlorophenylmethanol, 4-chlorophenylmethanol, 2,3-dichlorophenylmethanol, 3,4-dichlorophenylmethanol, diphenylmethanol, 2-hydroxymethylpyridine, 3-hydroxymethylpyridine, 4-hydroxymethylpyridine, 2-hydroxymethylpyrimidine, 2-hydroxymethylimidazole, 2-hydroxymethylthiazole, phenethyl alcohol, 2-(2-hydroxyethyl)pyridine, 3-(2-hydroxyethyl)pyridine, 4-(2-hydroxyethyl)pyridine, 2-(2-hydroxyethyl)-pyrimidine, 2-(2-hydroxyethyl)imidazole, 2-(2-hydroxyethyl)thiazole, 3-phenylpropanol, 2-(3-hydroxypropyl)-pyridine, 3-(3-hydroxypropyl)pyridine, 4-(3-hydroxypropyl)pyridine, 2-(3-hydroxypropyl)pyrimidine, 2-(3-hydroxypropyl)imidazole, 2-(3-hydroxypropyl)thiazole, 1-hydroxymethylnaphthalene, 2-hydroxymethylnaphthalene, 1-(2-hydroxyethyl)naphthalene, 2-(2-hydroxyethyl)-naphthalene,
2-(p-nitrobenzyloxycarbonyl)amino-2-phenylethanol, 2-(p-nitrobenzyloxycarbonyl)amino-3-phenylpropanol,
3-hydroxy-1-(p-nitrobenzyloxycarbonyl)azetidine, 1-allyloxycarbonyl-3-hydroxyazetidine, 1-benzyl-3-hydroxyazetidine, 3-hydroxy-1-phenylazetidine, 3-hydroxy-1-(2-oxo-2-phenylethyl)azetidine,
3-hydroxy-1-(p-nitrobenzyloxycarbonyl)-pyrrolidine, 1-allyloxycarbonyl-3-hydroxypyrrolidine, 1-benzyl-3-hydroxypyrrolidine, 3-hydroxy-1-phenethylpyrrolidine, l-cyclopropyl-3-hydroxypyrrolidine, 1-cyclopropylmethyl-3-hydroxy-1-pyrrolidine, 1-cyclopentyl-3-hydroxypyrrolidine, 3-hydroxy-1-phenylpyrrolidine, 3-hydroxy-1-(2-pyridyl) pyrrolidine, 3-hydroxy-1-(3-pyridyl)pyrrolidine, 3-hydroxy-1-(4-pyridyl)pyrrolidine, 3-hydroxy-1-(2-pyrimidyl)-pyrrolidine, 3-hydroxy-1-(imidazol-2-yl)pyrrolidine, 3-hydrdxy-1-(thiazol-2-yl)pyrrolidine, 1-[4-(p-nitro-benzyloxycarbonyl)aminophenyl]-3-hydroxypyrrolidine, 1-(2-fluoroethyl)-3-hydroxypyrrolidine, 3-hydroxy-1-(2-oxo-2-phenylethyl) pyrrolidine, 3-hydroxy-1-[2-(2-p-nitrobenzyloxy)phenyl-2-oxoethyl]pyrrolidine, 3-hydroxy-1-[2-(3-p-nitrobenzyloxy)phenyl-2-oxoethyl]-pyrrolidine, 3-hydroxy-1-[2-(4-p-nitrobenzyloxy)phenyl-2-oxoethyl]pyrrolidine, 1-[2-(p-fluoro)phenyl-2-oxoethyl]-3-hydroxypyrrolidine, 3-hydroxy-1-[2-(p-methyl)phenyl-2-oxoethyl]pyrrolidine, 3-hydroxy-1-[2-(p-methoxy)phenyl-2-oxoethyl]pyrrolidine, 3-hydroxy-1-(1-methyl-2-oxo-2-phenylethyl)pyrrolidine, 3-hydroxy-1-(3-oxo-3-phenylpropyl)pyrrolidine, 3-hydroxy-1-(2-oxo-3-phenylpropyl)pyrrolidine, 3-hydroxy-1-(1-indanon-2-yl)pyrrolidine, 3-hydroxy-1-(1-indanon-3-yl)pyrrolidine, 3-hydroxy-1-[(2-pyridyl)methyl]-pyrrolidine, 3-hydroxy-1-[(3-pyridyl)methyl]-pyrrolidine, 3-hydroxy-1-[(4-pyridyl)methyl]-pyrrolidine,
2-hydroxymethyl-1-(p-nitrobenzyloxycarbonyl)-piperidine 3-hydroxy-1-(p-nitrobenzyloxycarbonyl)-piperidine, 4-hydroxy-1-(p-nitrobenzyloxycarbonyl)-piperidine, 1-allyloxycarbonyl-4-hydroxypiperidine, 1-benzyl-4-hydroxypiperidine, 4-hydroxy-1-phenylpiperidine, and
4-hydroxy-(1,2-di(p-nitrobenzyloxycarbonyl))-pyrazolidine.

Incidentally, it is possible to simultaneously conduct conversion of the 2-substituent by using the method for the removal of the carboxyl-protecting group $R_4$ in the compound (III). Feasible examples of the conversion include reduction of a double bond or triple bond, deprotection of an amino group, and removal of a carboxyl-protecting group.

The target compound (Ia) which is obtained by removing the hydroxyl-protecting group and/or the carboxyl-protecting group in the compound (III) can be purified by chromatography such as liquid chromatography, recrystallization or the like if necessary. Further, a mixture of isomers can be separated by chromatography such as column chromatography, recrystallization or the like if necessary.

Process 10:

Among the compounds of the formula (I), the compound (Id) according to the present invention in which $R_1$ represents an N-substituted pyrrolidinyl group can be obtained by the below-described methods from the compound (XXVI) obtained in Process 7 in accordance with the following reaction scheme.

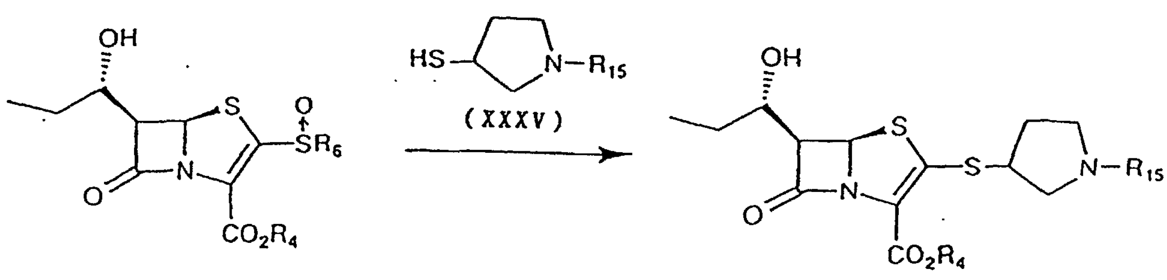

(XXVI) (XXXV) (XXXVI)

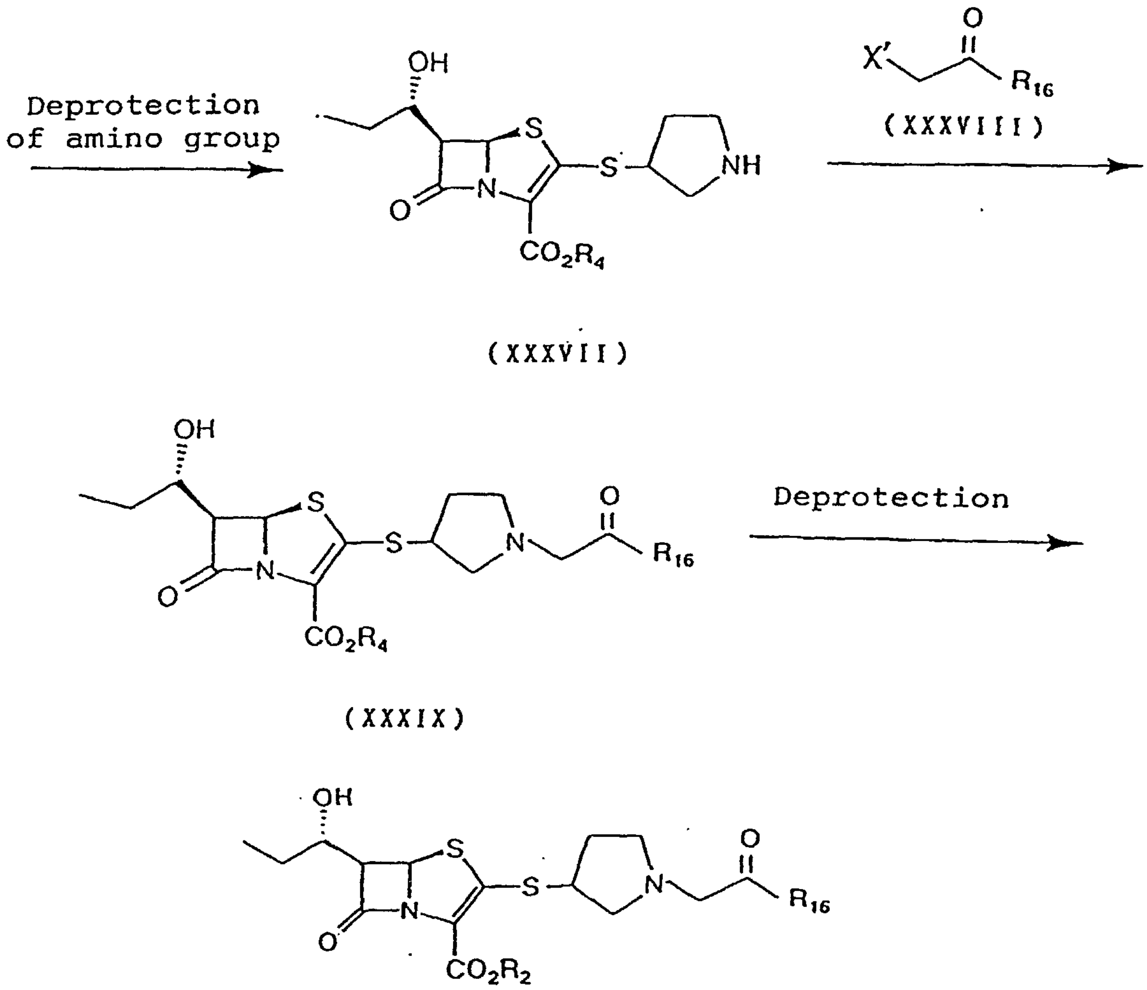


(XXXVIII)

(XXXVII)

(XXXIX)

(Id)

wherein $R_{15}$ has the same meaning as the substituent for an amino group defined above in connection with $R_1$, $R_{16}$ represents a substituted or unsubstituted alkyl group, a substituted or unsubstituted alkenyl group, a substituted or unsubstituted aralkyl group, a substituted or unsubstituted aryl group or a substituted or unsubstituted heterocyclic group, X' represents a halogen atom, and $R_2$, $R_4$ and $R_6$ have the same meanings as defined above.

As preferred specific examples of the substituted or unsubstituted alkyl group, the substituted or unsubstituted alkenyl group, the substituted or unsubstituted aralkyl group, the substituted or unsubstituted aryl group or the substituted or unsubstituted heterocyclic group of $R_{16}$, those described above in connection with $R_1$ can also be mentioned.

In the above process, 1.0 to 3.5 equivalents of the amino-protected pyrrolidinyl-3-thiol (XXXV) and 1.0 to 3.5 equivalents of a tertiary amine such as triethylamine are reacted with 1 equivalent of the compound (XXVI), which has been obtained in process 7, at 40°C to 30°C, preferably -20°C for 0.2 to 4 hours in an ether such as tetrahydrofuran or diethyl ether, an aromatic hydrocarbon such as toluene, a saturated hydrocarbon such as hexane, a halogenated hydrocarbon such as dichloromethane, or an amide such as dimethylformamide. After the reaction, the reaction product is diluted with a water-immiscible organic solvent, the organic layer is washed successively with a saturated aqueous solution of sodium hydrogencarbonate, a weak acid and water, and the organic solvent is distilled off, whereby the target compound (XXXVI) is obtained. If necessary, it can be purified by chromatography or the like.

From the thus-obtained compound (XXXVI), the amino-protecting group is selectively removed to obtain the compound (XXXVII). Any protecting group may be used insofar as it does not react to $R_4$ but reacts only with the amino-protecting group to afford an amine. When $R_4$ is, for example, a p-nitrobenzyl group, an allyloxycarbonyl group, a 4-pentenoyl group or the like can be mentioned. An allyloxycarbonyl group can be removed by causing dimedone, formic acid or the like to act thereon in the presence of a palladium catalyst such as tetrakistriphenylphosphine palladium (0) or palladium acetate (II). Where a 4-pentenoyl group is used, on the other hand, deprotection can be effected by

causing iodine to act. If necessary, the reaction product can be purified by chromatography or the like.

The α-haloketone (XXXVIII) is reacted with 1 equivalent of the compound (XXXVII) at -40°C to 30°C, preferably room temperature for 0.2 to 4 hours in the presence of 1.0 to 3.5 equivalents of a tertiary amine such as triethylamine in an ether such as tetrahydrofuran or diethyl ether, an aromatic hydrocarbon such as toluene, a saturated hydrocarbon such as hexane, a halogenated hydrocarbon such as dichloromethane, or an amide such as dimethylformamide, whereby the compound (XXXIX) is obtained. If necessary, this compound can be purified by chromatography or the like.

If necessary, the thus-obtained compound can be converted into the compound (Id) by removing the carboxyl-protecting group in the above-described manner.

Incidentally, it is possible to simultaneously conduct conversion of the 2-substituent by using the method for the removal of the carboxyl-protecting group $R_4$ in the compound (XXXIX). Feasible examples of the conversion include reduction of a double bond or triple bond, deprotection of an amino group, and removal of a carboxyl-protecting group.

The target compound (Id) which is obtained by removing the hydroxyl-protecting group and/or the carboxyl-protecting group in the compound (XXXIX) can be purified by chromatography such as liquid chromatography, recrystallization or the like if necessary. Further, a mixture of isomers can be separated by chromatography such as column chromatography, recrystallization or the like if necessary.

Each compound (I) of the present invention obtained as described above can be purified by a method such as recrystallization or column chromatography. Further, it can be obtained in the form of a pharmacologically acceptable salt as needed. Examples of such a salt include salts with inorganic metals, for example, alkali metals such as lithium, sodium and potassium, and alkaline earth metals such as calcium and magnesium; salts with basic amino acids such as lysine; and salts with organic amines such as ammonium salts. Preferred are salts with alkali metals such as sodium and potassium.

With respect to certain compounds (I) of the present invention, their general antibacterial activities and their antibacterial activities (MIC) against various methicillin-resistant strains of *Staphylococcus aureus* (methicillin-resistant *Staphylococcus aureus:* MRSA) were investigated. The results will be shown next.

Of the investigations, the general antibacterial activities were investigated by a standard *in vitro* dilution test.

As test bacteria, S*taphylococcus aureus* 209P JC-1, *Escherichia coli* NIHJ JC-2 and MRSAs were used. The results are summarized in Table 1 to Table 4.

Table 1

| Test comp'd (MIC: μg/mℓ) | Test bacterium strain ($10^5$ cfu/mℓ) | | | |
|---|---|---|---|---|
| | MRSA 31 high-resist. (IPM resist.) | MRSA 33 high-resist. (IPM resist.) | *Staphylococcus aureus* 209P JC-1 strain | *Escherichia coli* NIHJ JC-2 strain |
| Ex. 16 | 6.25 | 6.25 | 0.39 | 0.78 |
| Ex. 17 | 3.13 | 3.13 | 0.10 | 0.39 |
| Ex. 18 | 3.13 | 3.13 | ≤0.025 | 0.39 |
| Ex. 44 | 3.13 | 3.13 | 0.1 | 3.13 |
| Ex. 49*1 | 6.25 | 6.25 | 0.1 | 0.2 |
| Ex. 103 | 3.13 | 3.13 | 0.1 | 0.39 |
| Ex. 105 | 1.56 | 1.56 | 0.1 | >12.5 |
| Ex. 106 | 6.25 | 6.25 | 0.05 | 3.13 |
| Ex. 107 | 6.25 | 6.25 | 0.1 | 1.56 |
| Ex. 108 | 6.25 | 6.25 | 0.05 | 1.56 |
| Ex. 111 | 3.13 | 3.13 | 0.1 | 0.2 |
| Ex. 112 | 6.25 | 6.25 | 0.1 | 0.78 |
| Ex. 113 | 6.25 | 6.25 | 0.1 | 0.39 |
| Ex. 114 | 6.25 | 6.25 | 0.2 | 0.2 |
| Ex. 116 | 3.13 | 3.13 | 0.05 | 0.2 |
| Ex. 117 | 6.25 | 6.25 | 0.1 | 1.56 |

*1 (5R,6R)-2-((S)-pyrrolidin-3-yl)thio-6-((S)-1-hydroxypropyl)-penem-3-carboxylic acid.

Table 2

| Test comp'd (MIC: µg/mℓ) | Test bacterium strain ($10^5$ cfu/mℓ) | | | |
|---|---|---|---|---|
| | MRSA 31 high-resist. (IPM resist.) | MRSA 33 high-resist. (IPM resist.) | *Staphylococcus aureus* 209P JC-1 strain | *Escherichia coli* NIHJ JC-2 strain |
| Ex. 120*2 | 3.13 | 3.13 | 0.1 | 0.2 |
| Ex. 120*3 | 3.13 | 3.13 | 0.1 | 0.2 |
| Ex. 123 | 6.25 | 6.25 | 0.2 | 3.13 |
| Ex. 125*4 | 6.25 | 6.25 | 0.2 | 12.5 |
| Ex. 129 | 6.25 | 6.25 | 0.2 | 6.25 |
| Ex. 131 | 6.25 | 6.25 | 0.1 | 0.78 |
| Ex. 132 | 6.25 | 6.25 | 0.1 | 1.56 |
| Ex. 134 | 6.25 | 6.25 | 0.39 | 12.5 |
| Ex. 140 | 6.25 | 6.25 | 0.1 | 0.73 |

*2 Isomer A of (5R,6R)-6-((S)-1-hydroxypropyl)-2-((S)-1-(1-indanon-2-yl)pyrrolidin-3-yl)thio-penem-3-carboxylic acid.
*3 Isomer B of (5R,6R)-6-((S)-1-hydroxypropyl)-2-((S)-1-(1-indanon-2-yl)pyrrolidin-3-yl)thio-penem-3-carboxylic acid.
*4 Isomer A of (5R,6R)-6-((S)-1-hydroxypropyl)-2-(1-benzylpiperidin-3-yl)thio-penem-3-carboxylic acid.

Table 3

| Test comp'd (MIC: µg/mℓ) | Test bacterium strain ($10^5$ cfu/mℓ) | | | |
|---|---|---|---|---|
| | MRSA 31 high-resist. (IPM resist.) | MRSA 33 high-resist. (IPM resist.) | *Staphylococcus aureus* 209P JC-1 strain | *Escherichia coli* NIHJ JC-2 strain |
| Ex. 142 | 6.25 | 6.25 | 0.1 | 0.78 |
| Ex. 143 | 6.25 | 6.25 | 0.39 | 3.13 |
| Ex. 147 | 3.13 | 3.13 | 0.2 | 6.25 |
| Ex. 151 | 6.25 | 6.25 | 0.1 | 25 |
| Ex. 152 | 3.13 | 3.13 | 0.05 | 50 |
| Ex. 236 | 1.56 | 1.56 | 0.1 | 50 |
| Ex. 239 | 0.78 | 1.56 | 0.1 | 12.5 |
| Ex. 203 | 3.13 | 3.13 | 0.05 | 0.78 |
| Ex. 204 | 3.13 | 3.13 | 0.1 | 6.25 |
| Ex. 205 | 3.13 | 3.13 | 0.1 | 3.13 |
| Ex. 254 | 6.25 | 6.25 | 0.1 | 0.78 |
| Ex. 255 | 3.13 | 3.13 | 0.1 | 0.78 |
| Ex. 231 | 1.56 | 1.56 | 0.1 | 3.13 |
| Ex. 232 | 3.13 | 3.13 | 0.05 | 12.5 |
| Ex. 250 | 6.25 | 6.25 | 0.2 | 1.56 |
| Ex. 207 | 6.25 | 6.25 | 0.1 | 0.39 |
| Ex. 224 | 3.13 | 3.13 | 0.2 | 3.13 |

Table 4

| Test comp'd (MIC: µg/mℓ) | Test bacterium strain ($10^5$ cfu/mℓ) | | | |
|---|---|---|---|---|
| | MRSA 31 high-resist. (IPM resist.) | MRSA 33 high-resist. (IPM resist.) | *Staphylococcus aureus* 209P JC-1 strain | *Escherichia coli* NIHJ JC-2 strain |
| Ex. 225 | 1.56 | 1.56 | 0.05 | 12.5 |
| Ex. 257 | 3.13 | 1.56 | 0.05 | 12.5 |
| Ex. 209 | 6.25 | 6.25 | 0.39 | 25 |
| Ex. 218 | 6.25 | 6.25 | 0.2 | 25 |
| Ex. 243 | 6.25 | 6.25 | 0.2 | 1.56 |
| Ex. 230 | 6.25 | 6.25 | 0.2 | 0.78 |
| Ex. 210 | 6.25 | 6.25 | 0.1 | 12.5 |
| Ex. 215 | 6.25 | 6.25 | 0.1 | 3.13 |
| Ex. 226 | 6.25 | 6.25 | 0.2 | 0.39 |
| Ex. 214 | 6.25 | 3.13 | 0.1 | 6.25 |
| Ex. 227 | 6.25 | 6.25 | 0.1 | 0.78 |
| Ex. 228 | 3.13 | 3.13 | 0.05 | 12.5 |
| Ex. 253 | 6.25 | 6.25 | 0.1 | 0.78 |
| Ex. 235 | 6.25 | 6.25 | 0.1 | 0.78 |
| Ex. 229 | 3.13 | 3.13 | 0.1 | 12.5 |

As is evident from the above results, each penem compound (I) according to the present invention has been found to have broad antibacterial activities at test amounts of from 0.025 to 50 µg/mℓ and also to have antibacterial activities specific to MRSA.

The dosage of each penem compound (I) according to the present invention is dependent on many factors such as the object of administration and the age, body weight and conditions of the person to be administered. However, a typical daily dosage is from 50 mg to 5 g per standard adult in the case of oral administration, with administration of 100 mg to 4 g in portions being preferred. In general, its administration may be effected using a unit dosage form which comprises an appropriate amount of the active ingredient and a suitable, physiologically acceptable carrier, extender or diluent.

For oral administration, tablets or capsules can be used. They may contain, together with the active ingredient, an extender, for example, lactose, glucose, sucrose, mannitol, sorbitol or cellulose and a lubricant, for example, talc, or steric acid or a salt thereof. Tablets may further contain a binder, for example, magnesium silicate or starch.

For parenteral administration, namely, intravenous administration, intra-arterial administration, intramuscular administration and subcutaneous administration, isotonic aqueous solutions or suspensions are suited.

Further, the penem compounds (I) according to the present invention can be used not only for men but also as an antibacterial agent for animals.

The penem compounds (I) according to the present invention are novel compounds which are different from any conventionally-known penem compounds in view of their steric structure and 6-substituent. In addition, as is apparent from the above tests, the compounds (I) exhibit high antibacterial activities, especially high antibacterial activities against MRSA so that they are useful not only as general-purpose antibacterial agents but also as antibacterial agents for MRSA against which conventional antibacterial agents are not observed to be effective.

CAPABILITY OF EXPLOITATION IN INDUSTRY

As has been described above, the penem compounds (I) according to the present invention are compounds having excellent antibacterial activities against general pathogenic bacteria and MRSA and, like general penem derivatives, their toxicity in the living body is not high. They can therefore be used widely as antibacterial agents by oral administration, parenteral administration or external administration.

In particular, they have excellent effects for MRSA against which no effective antibacterial substances have heretofore existed, so that it is extremely valuable as an antibacterial agent against MRSA.

EXAMPLES

The present invention will next be described in further detail by the following Production Examples and Examples. It should however be borne in mind that the present invention is by no means limited by these Examples.

Production Example 1

Synthesis of (R)-1-benzyl-2-hydroxymethylpyrrolidine

After benzyl bromide (1.2 ml, 10.1 mmol) and triethylamine (1.4 ml, 10.0 mmol) were added to a solution of (R)-2-hydroxymethylpyrrolidine (1.01 g, 9.98 mmol) in dry N,N-dimethylformamide (5 ml) under ice cooling and an argon gas stream, the reaction mixture was maintained at room temperature.

Seventy minutes later, the reaction mixture was poured into methylene chloride (100 ml), followed by the successive washing with a saturated aqueous solution of sodium hydrogencarbonate (50 mℓ, twice) and then with a saturated aqueous solution of sodium chloride (100 mℓ). The organic layer was dried over anhydrous sodium sulfate, the solvent was distilled off under reduced pressure, and the residue was subjected to column chromatography on silica gel (50 g). From ethyl acetate-hexane (2:1, V/V), the title compound was obtained as a colorless oil (1.36 g, 71% yield).

Production Example 2

Synthesis of (S)-1-benzyl-2-hydroxymethylpyrrolidine

Following the procedures of Production Example 1 except that (S)-2-hydroxymethylpyrrolidine (1009 mg, 9.97 mmol) was used instead of (R)-2-hydroxymethylpyrrolidine, the title compound was obtained as a colorless oil (1.32 g, 69% yield).

Production Example 3

Synthesis of 1-benzyl-2-hydroxymethylpiperidine

Following the procedures of Production Example 1 except that 2-hydroxymethylpiperidine (1.02 g, 8.89 mmol) was used instead of (R)-2-hydroxymethylpyrrolidine, the title compound was obtained as a colorless oil (1.23 g, 67% yield).

Production Example 4

Synthesis of 1-benzyl-3-hydroxypiperidine

Following the procedures of Production Example 1 except that 3-hydroxypiperidine (1.03 g, 10.2 mmol) was used instead of (R)-2-hydroxymethylpyrrolidine, the title compound was obtained as a pale yellow oil (0.95 g, 41% yield).

Production Example 5

Synthesis of 1-benzyl-4-(2-hydroxyethyl)-piperazine

Following the procedures of Production Example 1 except that 1-(2-hydroxyethyl)piperazine (1.30 g, 10.0 mmol) was used instead of (R)-2-hydroxymethylpyrrolidine, the title compound was obtained as white needles (1.54 g, 70% yield).

Production Example 6

Synthesis of 1-(2-hydroxyethyl)-4-(2-pyrimidyl)-piperazine

After 2-bromoethanol (0.71 mℓ, 10.0 mmol) and triethylamine (4.2 mℓ, 30.1 mmol) were added to a solution of 1-(2-pyrimidyl)piperazine (2.36 g, 10.0 mmol) in dry N,N-dimethylformamide (5 mℓ) under ice cooling and an argon gas stream, the reaction mixture was maintained at room temperature.

Fifty-five hours later, the reaction mixture was poured into methylene chloride (100 mℓ), followed by the washing with a saturated aqueous solution of sodium hydrogencarbonate (100 mℓ, twice). The organic layer was dried over anhydrous sodium sulfate, the solvent was distilled off under reduced pressure, and the residue was subjected to column chromatography on silica gel (50 g). From ethyl acetate-methanol (5:1, V/V), the title compound was obtained as colorless needles (1.06 g, 51% yield).

Production Example 7

Synthesis of 1-(2-benzoylthioethyl)pyrrolidine

To a solution of 1-(2-hydroxyethyl)pyrrolidine (2.00 g, 17.4 mmol) in distilled tetrahydrofuran (35 mℓ), triphenylphosphine (9.12 g, 34.8 mmol) and diethyl azodicarboxylate (5.5 mℓ, 34.9 mmol) were added under ice cooling and an argon gas stream.

One hour later, thiobenzoic acid (4.1 mℓ, 34.8 mmol) was added to the reaction mixture. Two hours later, the solvent in the reaction mixture was distilled off under reduced pressure, followed by column chromatography on silica gel (100 g). From ethyl acetate-hexane (5:1, V/V), the title compound was obtained as a pale yellow oil (2.61 g, 64% yield).

Production Example 8

Synthesis of 1-(2-benzoylthioethyl)pyrrole

Following the procedures of Production Example 7 except that 1-(2-hydroxyethyl)pyrrole (983 mg, 8.8 mmol) was used instead of 1-(2-hydroxyethyl)-pyrrolidine, the title compound was obtained as a pale brown oil (1.69 g, 83% yield).

Production Example 9

Synthesis of 1-(2-benzoylthioethyl)pyrrolidin-2-one

Following the procedures of Production Example 7 except that 1-(2-hydroxyethyl)pyrrolidin-2-one (2.00 g, 15.5 mmol) was used instead of 1-(2-hydroxyethyl)-pyrrolidine, the title compound was obtained as a pale yellow oil (3.54 g, 92% yield).

Production Example 10

Synthesis of (R)-1-benzyl-2-benzoylthiomethylpyrrolidine

Following the procedures of Production Example 7 except that (R)-1-benzyl-2-hydroxymethylpyrrolidine (1.36 g, 7.1 mmol) was used instead of 1-(2-hydroxyethyl)pyrrolidine, the title compound was quantitatively obtained as a pale yellow oil (2.3 g).

Production Example 11

Synthesis of (S)-1-benzyl-2-benzoylthiomethylpyrrolidine

Following the procedures of Production Example 7 except that (S)-1-benzyl-2-hydroxymethylpyrrolidine (1.32 g, 6.9 mmol) was used instead of 1-(2-hydroxyethyl)pyrrolidine, the title compound was obtained as a pale yellow oil (2.08 g, 97% yield).

Production Example 12

Synthesis of 1-benzyl-2-benzoylthiomethylpiperidine

Following the procedures of Production Example 7 except that 1-benzyl-2-hydroxymethylpiperidine (1.23 g, 5.6 mmol) was used instead of 1-(2-hydroxyethyl)-pyrrolidine, the title compound was obtained as a pale yellow oil (1.86 g, 95% yield).

Production Example 13

Synthesis of 1-benzyl-3-benzoylthiopiperidine

Following the procedures of Production Example 7 except that 1-benzyl-3-hydroxypiperidine (1.42 g, 7.42 mmol) was used instead of 1-(2-hydroxyethyl)-pyrrolidine, the title compound was obtained as a pale yellow oil (0.95 g, 41% yield).

Production Example 14

Synthesis of 1-benzyl-4-(2-benzoylthioethyl)-piperazine

Following the procedures of Production Example 7 except that 1-benzyl-4-(2-hydroxyethyl)piperazine (1.48 g, 6.71 mmol) was used instead of 1-(2-hydroxyethyl)pyrrolidine, the title compound was obtained as a pale yellow oil (1.56 g, 68% yield).

Production Example 15

Synthesis of 1-(2-benzoylthioethyl)-4-(2-pyrimidyl)piperazine

Following the procedures of Production Example 7 except that 1-(2-hydroxyethyl)-4-(2-pyrimidyl)-piperazine (1.06 g, 5.1 mmol) was used instead of 1-(2-hydroxyethyl)pyrrolidine, the title compound was obtained as a pale yellow oil (1.36 g, 82% yield).

Production Example 16

Synthesis of (S)-3-benzoylthiopyrrolidine trifluoroacetate

To a solution of (S)-3-benzoylthio-N-(tert-butoxycarbonyl)pyrrolidine (5.56 g, 18.1 mmol) in anisole (20 mℓ), trifluoroacetic acid (8.36 mℓ, 109 mmol) was added under ice cooling, followed by stirring overnight at room temperature. The reaction mixture was concentrated under reduced pressure and the residue was crystallized from ethyl ether, whereby the title compound was obtained as white crystals (4.00 g, 68.7% yield).

Production Example 17

Synthesis of (S)-3-benzoylthio-N-fenacylpyrrolidine

To a solution of (S)-3-benzoylthiopyrrolidine trifluoroacetate (1.92 g, 6 mmol) in methylene chloride (20 mℓ), triethylamine (1.84 mℓ, 13.2 mmol) and phenacyl bromide (1.44 g, 7.2 mmol) were added under ice cooling. The temperature of the reaction mixture was allowed to rise to room temperature, at which the reaction mixture was stirred for 3.5 hours. The reaction product was diluted with ethyl acetate and then washed with a saturated aqueous solution of sodium hydrogencarbonate. Subsequent to drying over sodium sulfate, column chromatography was conducted on silica gel (40 mℓ, "Merck 9385"). From ethyl acetate-n-hexane (1:3, V/V), the title compound was obtained as an orange oil (1.53 g, 78% yield).

Production Example 18

Synthesis of (S)-N-(1-benzoylethyl)-3-benzoylthiopyrrolidine

Following the procedures of Production Example 17 except that 2-bromopropiophenone (1022 mg, 4.80 mmol) was used instead of phenacyl bromide, the title compound was obtained as a pale yellow oil (925 mg, 68% yield).

Production Example 19

Synthesis of (S)-N-acetonyl-3-benzoylthiopyrrolidine

Following the procedures of Production Example 17 except that chloroacetone (277 mg, 3.00 mmol) was used instead of phenacyl bromide, the title compound was obtained as a pale yellow oil (351 mg, 66% yield).

Production Example 20

Synthesis of (S)-3-benzoylthio-N-(2-benzoylethyl)pyrrolidine

Following the procedures of Production Example 17 except that β-chloropropiophenone (404 mg, 2.40 mmol) was used instead of phenacyl bromide, the title compound was obtained as a pale yellow oil (116 mg, 22% yield).

Production Example 21

Synthesis of (S)-3-benzoylthio-N-(1-indanon-2-yl)pyrrolidine

Following the procedures of Production Example 17 except that 2-bromo-1-indanone (464 mg, 2.2 mmol) was used instead of phenacyl bromide, the title compound was obtained as a pale yellow oil (419 mg, 62% yield).

Production Example 22

Synthesis of (S)-3-benzoylthio-N-(2-oxo-2-p-tolylethyl)pyrrolidine

Following the procedures of Production Example 17 except that 2-bromo-4'-methylacetophenone (213 mg, 1.00 mmol) was used instead of phenacyl bromide, the title compound was obtained as a colorless oil (298 mg, 88% yield).

Production Example 23

Synthesis of (S)-3-benzoylthio-N-(2-p-fluorophenyl-2-oxoethyl)pyrrolidine

Following the procedures of Production Example 17 except that 2-chloro-4'-fluoroacetophenone (173 mg, 1.00 mmol) was used instead of phenacyl bromide, the title compound was obtained as a pale yellow oil (181 mg, 53% yield).

Production Example 24

Synthesis of (S)-3-benzoylthio-N-(1-tetralon-2-yl)pyrrolidine

Following the procedures of Production Example 17 except that 2-bromo-1-tetralone (491 mg, 2.20 mmol) was used instead of phenacyl bromide, the title compound was obtained as a pale yellow oil (28 mg, 4.0% yield).

Production Example 25

Synthesis of (S)-3-benzoylthio-N-phenylaminocarbonylmethylpyrrolidine

Following the procedures of Production Example 17 except that N-bromoacetylaniline (428 mg, 2.00 mmol) was used instead of phenacyl bromide, the title compound was obtained as a pale yellow oil (592 mg, 87% yield).

Production Example 26

Synthesis of (S)-3-benzoylthio-N-phenethylpyrrolidine

Following the procedures of Production Example 17 except that phenethyl bromide (185 mg, 1.00 mmol) was used instead of phenacyl bromide, the title compound was obtained as a pale yellow oil (21 mg, 6.7% yield).

Production Example 27

Synthesis of (S)-3-benzoylthio-N-benzylaminocarbonylmethylpyrrolidine

Following the procedures of Production Example 17 except that N-benzyl-α-bromoacetamide (456 mg, 2.00 mmol) was used instead of phenacyl bromide, the title compound was obtained as colorless crystals (486 mg, 68% yield).

Production Example 28

Synthesis of (S)-3-benzoylthio-N-((R)-2-hydroxy-2-phenylethyl)pyrrolidine

Following the procedures of Production Example 17 except that (R)-styrenoxide (120 mg, 1.00 mmol) was used instead of phenacyl bromide, the title compound was obtained as a pale yellow oil (35 mg, 11% yield).

Production Example 29

Synthesis of (S)-3-benzoylthio-N-((S)-2-hydroxy-2-phenylethyl)pyrrolidine

Following the procedures of Production Example 17 except that (S)-styrenoxide (120 mg, 1.00 mmol) was used instead of phenacyl bromide, the title compound was obtained as a pale yellow oil (31 mg, 9.5% yield).

Production Example 30

Synthesis of (S)-3-benzoylthio-N-(2-p-methoxyphenyl-2-oxoethyl)pyrrolidine

Following the procedures of Production Example 17 except that 2-bromo-4'-methoxyacetophenone (229 mg, 1.00 mmol) was used instead of phenacyl bromide, the title compound was obtained as a pale yellow oil (216 mg, 61% yield).

Production Example 31

Synthesis of (S)-3-benzoylthio-N-(1-benzosuberon-2-yl)pyrrolidine

Following the procedures of Production Example 17 except that 2-bromo-1-benzosuberone (478 mg, 2.00 mmol) was used instead of phenacyl bromide, the title compound was obtained as a pale yellow oil (60 mg, 82% yield).

Production Example 32

Synthesis of (S)-3-benzoylthio-N-(2-p-phenylphenyl-2-oxoethyl)pyrrolidine

Following the procedures of Production Example 17 except that 2-bromo-4'-phenylacetophenone (275 mg, 1.00 mmol) was used instead of phenacyl bromide, the title compound was obtained as a pale yellow oil (306 mg, 76% yield).

Production Example 33

Synthesis of (S)-3-benzoylthio-N-benzoylpyrrolidine

Following the procedures of Production Example 17 except that benzoyl chloride (506 mg, 3.6 mmol) was used instead of phenacyl bromide, the title compound was obtained (570 mg, 61% yield).

Production Example 34

Synthesis of (S)-3-benzoylthio-N-(2-pyridylmethyl)pyrrolidine

Following the procedures of Production Example 17 except that α-picolyl chloride (300 mg, 1.8 mmol) was used instead of phenacyl bromide, the title compound was obtained as a yellow oil (100 mg, 22% yield).

Production Example 35

Synthesis of 3-hydroxy-1-phenylpyrrolidine

To a solution of 1,4-dibromobutan-2-ol (1.2 g, 5 mmol) in acetone (25 mℓ), aniline (0.45 mℓ, 1.5 mmol), sodium iodide (0.73 g, 5 mmol) and potassium carbonate (1.38 g, 10 mmol) were added, followed by stirring overnight at 55°C under heat.

Added further were 1,4-dibromobutan-2-ol (0.6 g, 2.5 mmol) and potassium carbonate (1.38 g, 10 mmol), followed by stirring at 55°C under heat for 4 days. The reaction mixture was poured into water and then extracted with ethyl acetate. The organic layer was washed with brine and dried over sodium sulfate. The solvent was distilled off, followed by purification by column chromatography, whereby the title compound was obtained as a brown solid (0.76 g, 93% yield).

Production Example 36

Synthesis of 1-phenyl-3-acetylthiopyrrolidine

To a solution of 3-hydroxy-1-phenylpyrrolidine (0.89 g, 5.5 mmol), which had been obtained in Production Example 35, in methylene chloride (25 mℓ), triethylamine (0.92 mℓ, 6.6 mmol) was added under ice cooling.

After a solution of methanesulfonyl chloride (0.51 mℓ, 6.6 mmol) in methylene chloride (7 mℓ) was added dropwise over 5 minutes, the resultant mixture was stirred for 2 hours. The reaction mixture was poured into ice water, followed by extraction twice with methylene chloride. The organic layers were combined together, washed with brine and dried over magnesium sulfate. The solvent was distilled off. The crude product was dissolved in DMF (18 mℓ), followed by the addition of potassium thioacetate (0.75 g, 6.6 mℓ) at room temperature. The resultant mixture was stirred at 65°C for 1 hour. After the reaction mixture was allowed to cool down to room temperature, it was poured into ice water and extracted twice with ethyl acetate. The organic layers were combined together, washed with brine, and dried over magnesium sulfate. The solvent was distilled off and the residue was purified by column chromatography, whereby the title compound was obtained as a reddish brown oil (0.64 g, 52% yield).

Production Example 37

Synthesis of 4-acetylthio-N-phenylbutyrylamide

After a suspension of 4-chloro-N-phenylbutyrylamide (400 mg), potassium thioacetate (400 mg) and potassium iodide (140 mg) in ethanol (4 mℓ) was heated under reflux for 1 hour, the reaction product was diluted with ethyl acetate. The organic layer was washed successively with water and a saturated aqueous solution of sodium hydrogencarbonate. After the organic layer was dried over anhydrous sodium sulfate, the solvent was distilled off under reduced pressure and the residue was purified by flash column chromatograpy, whereby the title compound was obtained (359 mg, 80% yield).

Production Example 38

Synthesis of 3-acetylthio-N-phenethylpropionylamide

Following the procedures of Production Example 37 except that 3-chloro-N-phenethylpropionylamide (400 mg, 2 mmol) was used instead of 4-chloro-N-phenylbutyrylamide, the title compound was obtained (359 mg, 81% yield).

Production Example 39

Synthesis of 3-acetylthio-N-benzyl-N-methylpropionylamide

Following the procedures of Production Example 37 except that 3-chloro-N-benzyl-N-methylpropionylamide (400 mg, 2 mmol) was used instead of 4-chloro-N-phenylbutyrylamide, the title compound was obtained (309 mg, 85% yield).

Production Example 40

Synthesis of 4-acetylthio-N-benzylbutyrylamide

Following the procedures of Production Example 37 except that 4-chloro-N-benzylbutyrylamide (100 mg, 0.5 mmol) was used instead of 4-chloro-N-phenylbutyrylamide, the title compound was obtained (78 mg, 62% yield).

Production Example 41

Synthesis of 3-acetylthio-N-methylpropionylamide

Following the procedures of Production Example 37 except that 3-chloro-N-methylpropionylamide (600 mg, 4.8 mmol) was used instead of 4-chloro-N-phenylbutyrylamide, the title compound was obtained (150 mg, 19% yield).

Production Example 42

Synthesis of 2-acetylthio-N-methyl-N-phenacylethylamine

Following the procedures of Production Example 7 except that 2-hydroxy-N-methyl-N-phenacylethylamine (500 mg, 2.58 mmol) was used instead of 1-(2-hydroxyethyl)pyrrolidine and thioacetic acid was employed in lieu of thiobenzoic acid, the title compound was obtained as colorless crystals (94 mg, 16% yield).

Production Example 43

Synthesis of 3-acetylthio-N-phenylpropanamide

Following the procedures of Production Example 37 except that 3-chloro-N-phenylpropionylamide (395 mg, 2.15 mmol) was used instead of 4-chloro-N-phenylbutyrylamide, the title compound was obtained as pale brown crystals (458 mg, 95% yield).

Production Example 44

Synthesis of 3-acetylthio-N-((S)-1-phenylethyl)-propionylamide

Following the procedures of Production Example 37 except that 3-chloro-N-((S)-1-phenylethyl)propionylamide (424 mg, 2.00 mmol) was used instead of 4-chloro-N-phenylbutyrylamide, the title compound was obtained as colorless crystals (440 mg, 87% yield).

Production Example 45

Synthesis of 3-acetylthio-N-((R)-1-phenylethyl)-propionylamide

Following the procedures of Production Example 37 except that 3-chloro-N-((R)-1-phenylethyl)propionylamide (424 mg, 2.00 mmol) was used instead of 4-chloro-N-phenylbutyrylamide, the title compound was obtained as pale yellow crystals (513 mg, 100% yield).

Production Example 46

Synthesis of (S)-3-mercapto-N-phenacylpyrrolidine

To a solution of (S)-3-benzoylthio-N-phenacylpyrrolidine (195 mg) in methanol (6 mℓ), 1 N sodium hydroxide (0.62 mℓ) was added at room temperature, followed by stirring at the same temperature for 25 minutes. After the reaction product was diluted with methylene chloride, the organic layer was washed with a saturated aqueous solution of sodium hydrogencarbonate. Further, the water layer was extracted twice with methylene chloride. The thus-obtained methylene chloride solution was dried over anhydrous sodium sulfate, and the solvent was distilled off under reduced pressure. The residue so obtained was provided for the next reaction without purification.

Production Example 47

Synthesis of (S)-3-mercapto-N-(p-nitrobenzyloxycarbonyl)pyrrolidine

Following the procedures of Production Example 46 except that (S)-3-benzoylthio-N-(p-nitrobenzyloxycarbonyl)pyrrolidine (194 mg, 0.6 mmol) was used instead of (S)-3-benzoylthio-N-phenacylpyrrolidine, the title compound was obtained and provided for the next reaction.

Production Example 48

Synthesis of N-(2-benzoylethyl)-3-mercaptopyrrolidine

Following the procedures of Production Example 46 except that N-(2-benzoylethyl)-3-benzoylthiopyrrolidine (102 mg, 0.30 mmol) was used instead of (S)-3-benzoylthio-N-phenacylpyrrolidine, the title compound was obtained and provided for the next reaction.

Production Example 49

Synthesis of 2-mercaptomethylpyridine

Following the procedures of Production Example 46 except that 2-benzoylmethylpyridine (184 mg, 1.2 mmol) was used instead of (S)-3-benzoylthio-N-phenacylpyrrolidine, the title compound was obtained and provided for the next reaction.

Production Example 50

Synthesis of (S)-3-mercapto-N-(2-pyridylmethyl)-pyrrolidine

Following the procedures of Production Example 46 except that (S)-3-benzoylthio-N-(2-pyridylmethyl)-pyrrolidine (188 mg, 0.63 mmol) was used instead of (S)-3-benzoylthio-N-phenacylpyrrolidine, the title compound was obtained and provided for the next reaction.

Production Example 51

Following the procedures of Production Example 46 except that the products of Production Examples 7 to 15, 18 to 19, 21 to 32 and 36 to 45 were used instead of (S)-3-benzoylthio-N-phenacylpyrrolidine, compounds with the benzoylthio group or acetylthio group changed to a mercapto group were obtained and provided for the next reactions.

Data which show physical properties of the compounds obtained in Production Examples 1 to 51 are shown in Tables 5 to 13.

In the subsequent tables of the present description, "s" represents a singlet, "d" a doublet, "t" a triplet, "q" a quartet, "quint" a quintet, "m" a multiplet, "bs" or "brs" a broad singlet.

Employed as an internal standard was TSP where $D_2O$ was used as a measuring solvent or TMS where another measuring solvent was used.

Table 5

| Production Example No. | N M R (δ ppm) |
|---|---|
| 1 | ($CDCl_3$)<br>1.6(1H,bs),1.64-1.78(2H,m),1.78-1.87(1H,m),1.87-1.96(1H,m),2.30(1H,dt,J=7.6Hz, 9.3Hz),2.71-2.76(1H, m),2.95-3.00(1H,m),3.37(1H,d,J=13.1Hz),3.42(1H,dd, J=2.1Hz, 10.7Hz),3.65(1H,dd,J=3.5Hz,10.7Hz),3.96 (1H,d,J=13.1Hz),7.2-7.4(5H,m) |
| 2 | ($CDCl_3$)<br>1.55(1H,bs),1.64-1.78(2H,m),1.78-1.87(1H,m),1.87-1.98(1H,m),2.30(1H,dt,J=7.6Hz, 9.3Hz),2.71-2.75(1H,m), 2.95-3.00(1H,m),3.37(1H,d,J=13.1Hz),3.42(1H,dd, J=2.1Hz, 10.7Hz),3.65(1H,dd,J=3.5Hz,10.7Hz),3.96(1H,d, J=13.1Hz),7.2-7.4(5H,m) |
| 3 | ($CDCl_3$)<br>1.34-1.41(2H,m),1.54-1.74(4H,m),2.12-2.18(1H,m),2.43-2.48(1H,m),2.7(1H,bs), 2.84-2.89(1H,m),3.31(1H,d,J=13.4Hz), 3.51(1H,dd,J=3.9Hz,10.8Hz),3.87(1H,dd, J=4.3Hz,10.8Hz), 4.06(1H,d,J=13.4Hz),7.2-7.35(5H,m) |
| 4 | ($CDCl_3$)<br>1.45-1.7(3H,m),1.75-1.85(1H,m),2.2-2.3(1H,m),2.35-2.6(4H,m) ,3.50(2H,s),3.81(1H, bs),7.2-7.35(5H,m) |

Table 5 (continued)

| Production Example No. | N M R (δ ppm) |
|---|---|
| 5 | ($CDCl_3$)<br>2.4-2.65(10H,m),3.51(2H,s),3.60(2H,t,J=5.4Hz),7.2-7.35(5H,m) |
| 6 | ($CDCl_3$)<br>2.58(4H,t,J=5.1Hz),2.59(2H,t,J=5.5Hz),2.6-2.8(1H,bs),3.67 (2H,t,J=5.5Hz),3.84(4H,t, J=5.1Hz),6.49(1H,t,J=4.7Hz),8.31 (2H,d,J=4.7Hz) |

Table 6

| Production Example No. | N M R (δ ppm) |
|---|---|
| 7 | ($CDCl_3$)<br>1.97(4H,t,J=6.8Hz),3.06-3.10(6H,m),3.40(2H,t,J=7.6Hz),7.3-8.2(5H,m) |
| 8 | ($CDCl_3$)<br>3.39(2H,t,J=7.0Hz),4.14(2H,t,J=7.0Hz),6.17(2H,t,J=2.0Hz), 6.73(2H,t,J=2.0Hz), 7.4-8.0(5H,m) |
| 9 | ($CDCl_3$)<br>2.03(2H,tt,J=7.9Hz,14.7Hz),2.38(2H,t,J=7.9Hz),3.25(2H,t, J=7.1Hz),3.54(2H,t, J=7.1Hz),3.54(2H,t,J=14.7Hz),7.4-8.0 (5H,m) |
| 10 | ($CDCl_3$)<br>1.6-1.85(3H,m),1.95-2.05(1H,m),2.2-2.3(1H,m),2.8-2.9(1H,m), 2.9-3.0(1H,m),3.11 (1H,dd,J=7.1Hz,13.4Hz),3.36(1H,d,J=13.1Hz), 3.51(1H,dd,J=3.3Hz,13.4Hz),4.16(1H, d,J=13.1Hz),7.2-8.05(10H,m) |
| 11 | ($CDCl_3$)<br>1.6-1.85(3H,m),1.95-2.1(1H,m),2.2-2.3(1H,m),2.8-2.9(1H,m),2.9-3.05(1H,m),3.11(1H, dd,J=7.1Hz,13.3Hz),3.36(1H,d,J=13.1Hz),3.51 (1H,dd,J=3.3Hz,13.3Hz),4.16(1H,d, J=13.1Hz),7.2-8.05(10H,m) |
| 12 | ($CDCl_3$)<br>1.25-1.8(6H,m),2.08(1H,ddd,J=3.7Hz,9.4Hz,11.7Hz),2.67(1H,dt, J=3.4Hz,6.0Hz),2.78 (1H,dt,J=4.1Hz,8.0Hz),3.32(1H,d,J=13.4Hz), 3.38(1H,dd,J=6.8Hz,13.6Hz),3.48(1H, dd,J=3.1Hz,13.6Hz), 4.10(1H,d,J=13.4Hz),7.2-8.05(10H,m) |

Table 7

| Production Example No. | N M R (δ ppm) |
|---|---|
| 13 | ($CDCl_3$)<br>1.6-1.85(2H,m),1.95-2.05(1H,m),2.22(1H,dt,J=7.6Hz,9.2Hz), 2.4-2.55(1H,m),2.84(1H, ddd,J=3.1Hz,7.0Hz,10.7Hz),2.96(1H, t,J=7.1Hz),3.12(1H,dd,J=7.1Hz,13.4Hz),3.36 (1H,d,J=13.0Hz), 3.53(1H,dd,J=3.1Hz,13.4Hz),4.17(1H,d,J=13.0Hz),7.2-8.1 (10H,m) |
| 14 | ($CDCl_3$)<br>1.56(4H,bs),1.57(4H,bs),2.65(2H,t,J=7.6Hz),3.21(2H,t, J=7.6Hz),3.52(2H,s),7.25-8.0 (10H,m) |
| 15 | ($CDCl_3$)<br>2.61(4H,t,J=5.1Hz),2.70(2H,t,J=7.3Hz),3.26(2H,t,J=7.3Hz), 3.85(4H,t,J=5.1Hz),6.48 (1H,t,J=4.7Hz),7.45(2H,t,J=7.6Hz), 7.58(1H,t,J=7.6Hz),7.98(2H,d,J=7.6Hz),8.31(2H, d,J=4.7Hz) |

Table 7 (continued)

| Production Example No. | N M R (δ ppm) |
|---|---|
| 16 | ($CDCl_3$) 2.17(1H,m),2.57(1H,m),3.31(1H,dd,J=6Hz,12Hz),3.41-3.51 (2H,m),3.88(1H,dd, J=8Hz,12Hz),4.25(1H,m),7.46(2H,t, J=8Hz),7.60(1H,t,J=7Hz),7.91(2H,d,J=8Hz), 10.14(2H,bs) |
| 17 | ($CDCl_3$) 7.99(1H,d,J=7.1Hz),7.94(1H,d,J=7.1Hz),7.51-7.61(1H,m), 7.47(2H,d,J=8.1Hz),7.43 (2H,d,J=7.8Hz),4.13-4.25(1H,m), 4.04(2H,d,J=4.2Hz),3.32(1H,dd,J=7.0Hz,10.0Hz), 2.90-3.00 (1H,m),2.79-2.90(2H,m),2.46-2.59(1H,m),1.89-2.02(1H,m) |
| 18 | ($CDCl_3$) 1.39(3H,m),1.90(1H,m),2.45(1H,m),2.76(2H,m),2.83(1H,m), 3.29(1H,m),4.10(1H,m), 4.12(1H,m),7.41-7.47(4H,m),7.55(2H, t,J=7Hz),7.92(2H,d,J=8Hz),8.10(2H,d,J=8Hz) |

Table 8

| Production Example No. | N M R (δ ppm) |
|---|---|
| 19 | ($CDCl_3$) 1.96(1H,m),2.16(3H,s),2.50(1H,m),2.66-2.78(2H,m),2.80-2.85(1H,m),3.18(1H,dd, J=3Hz,10Hz),3.39(2H,d,J=4Hz), 4.16(1H,m),7.44(2H,d,J=7Hz),7.55(1H,t,J=7Hz), 7.93 (2H,d,J=8Hz) |
| 20 | ($CDCl_3$) 1.89(1H,m),2.47(1H,m),2.63(1H,q,J=8Hz),2.70(1H,d,J=5Hz), 2.81(1H,m),2.90-3.02 (2H,m),3.13(1H,dd,J=7Hz,10Hz), 3.21(2H,t,J=7Hz),4.14(1H,m),7.44(4H,d,J=8Hz), 7.55 (2H,dd,J=7Hz,8Hz),7.95(4H,m) |
| 21 | ($CDCl_3$) 1.89(1H,m),2.44(1H,m),2.59(1H,m),2.67-2.88(4H,m), 3.08-3.23(1H,m),4.12(1H,m), 4.69(1H,m),7.44(3H,m), 7.56(1H,m),7.64(1H,m),7.70(1H,m),7.76(1H,d,J=8Hz), 7.92 (2H,dd,J=8Hz,2Hz) |
| 22 | ($CDCl_3$) 1.95(1H,m),2.41(3H,s),2.52(1H,m),2.83(2H,m),2.91(1H, m),3.29(1H,dd,J=7Hz,10Hz), 4.00(2H,d,J=6Hz),4.19(1H,m), 7.25(2H,d,J=7Hz),7.44(2H,d,J=8Hz),7.56(1H,d,J= 7Hz),7.89(2H,d,J=8Hz),7.94(2H,dd,J=1Hz,8Hz) |
| 23 | ($CDCl_3$) 1.96(1H,m),2.51(1H,m),2.77-2.85(2H,m),2.91(1H,m), 3.28(1H,dd,J=8Hz,10Hz),3.98 (2H,d,J=4Hz),4.19(1H,m), 7.13(2H,d,J=9Hz),7.44(2H,d,J=8Hz),7.57(1H, d,J=8Hz), 7.93(2H,d,J=8Hz),8.03(2H,m) |
| 24 | ($CDCl_3$) 1.79-1.89(1H,m),2.13-2.24(1H,m),2.26-2.37(1H,m),2.38-2.53(2H,m),2.55-2.63(1H,m), 2.66-2.71(1H,m),2.81-3.11(2H,m), 3.32(1H,dd,J=7Hz,10Hz),3.59(1H,m),4.13(1H,m), 7.36-7.60 (6H,m),7.89-7.95(2H,m),8.00-8.04(1H,m) |

Table 9

| Production Example No. | N M R (δ ppm) |
|---|---|
| 25 | ($CDCl_3$) 1.99(1H,m),2.53(1H,m),2.76(1H,m),2.89(1H,dd,J=4Hz,10Hz), 3.00(1H,m),3.18(1H, dd,J=7Hz,10Hz),3.34(2H,dd,J=17Hz,39Hz), 4.20(1H,m),7.09(1H,d,J=7Hz),7.30(2H, dd,J=7Hz,8Hz), 7.46(2H,dd,J=7Hz,8Hz),7.57(3H,m),7.94(2H,dd,J=1Hz,8Hz), 9.05 (1H,bs) |
| 26 | ($CDCl_3$) 1.87-1.97(1H,m),2.41-2.54(1H,m),2.58-2.68(1H,m), 2.70-2.76(3H,m),2.78-2.90(3H, m),3.13(1H,dd,J=7Hz, 10Hz),4.15(1H,m),7.18-7.32(5H,m),7.46(2H,d,J= 7Hz),7.57 (1H,m),7.94(2H,dd,J=2Hz,8Hz) |
| 27 | ($CDCl_3$) 1.90(1H,m),2.44(1H,m),2.66(1H,dd,J=9Hz,15Hz), 2.77(1H,dd,J=4Hz,10Hz),2.88(1H, m),3.10(1H,dd,J=7Hz,10Hz), 3.26(2H,dd,J=7Hz,28Hz),4.11(1H,m),4.48(2H,m), 7.21-7.28 (4H,m),7.44(3H,m),7.58(1H,d,J=8Hz),7.89(1H, dd,J=1Hz,8Hz) |
| 28 | ($CDCl_3$) 1.93(1H,m),2.48(1H,m),2.57(1H,dd,J=3Hz,12Hz),2.78-2.83(3H,m),2.91(1H,dd,J=5Hz, 10Hz),3.11(1H,dd,J=7Hz, 10Hz),4.16(1H,m),4.70(1H,dd,J=3Hz,11Hz),7.28-7.39 (5H, m),7.45(2H,d,J=8Hz),7.57(1H,d,J=7Hz), 7.94(2H,d,J=7Hz) |
| 29 | ($CDCl_3$) 1.87-1.98(1H,m),2.44-2.65(3H,m),2.71-2.84(2H,m), 3.00-3.08(1H,m),3.31(1H,dd, J=7Hz,10Hz),4.16(1H,m), 4.73(1H,dd,J=3Hz,9Hz),7.27-7.40(5H,m),7.46(2H,d, J=8Hz),7.57(1H,m),7.94(2H,dd,J=2Hz,8Hz) |
| 30 | ($CDCl_3$) 1.95(1H,m),2.52(1H,m),2.84(2H,m),2.91(1H,m),3.31 (1H,dd,J=7Hz,10Hz),3.87(3H,s), 3.99(2H,d,J=6Hz), 4.19(1H,m),6.93(2H,d,J=9Hz),7.44(2H,d,J=8Hz), 7.56(1H,d, J=7Hz),7.93(2H,d,J=9Hz),7.99(2H,m) |

Table 10

| Production Example No. | N M R (δ ppm) |
|---|---|
| 31 | ($CDCl_3$) 1.60-1.87(3H,m),2.11-2.24(1H,m),2.26-2.31(1H,m), 2.32-2.37(1H,m),2.42(1H,m),2.53 (1H,m),2.66(1H, m),2.80-3.10(2H,m),3.13(1H,m),3.46(1H,m),4.05 (1H,m),7.21-7.26 (1H,m),7.30-7.48(5H,m),7.54(1H, d,J=7Hz),7.92(2H,d,J=8Hz) |
| 32 | ($CDCl_3$) 1.97(1H,m),2.53(1H,m),2.86(2H,m),2.94(1H,m), 3.32(1H,dd,J=7Hz,10Hz),4.05(2H,d, J=5Hz), 4.20(1H,m),7.40-7.49(5H,m),7.56(1H,d,J= 8Hz),7.62(2H,dd,J=2Hz,9Hz),7.68 (2H,d,J=8Hz), 7.94(2H,dd,J=2Hz,8Hz),8.07(2H,d,J=8Hz) |
| 33 | ($CDCl_3$) 7.96(1H,d,J=7.4Hz),7.89(1H,d,J=7.5Hz),7.32-7.65 (8H,m),4.06-4.24(1H,m),3.40-3.72 (2H,m),3.72-3.92(2H,m),2.40-2.55(1H,m),2.00-2.20(1H,m) |
| 34 | ($CDCl_3$) 8.56(1H,d,J=4.8Hz),7.93(2H,d,J=8.1Hz),7.66(1H, t,J=7.6Hz),7.55(1H,t,J=7.7Hz),7.43 (3H,t,J=7.4Hz), 7.16(1H,t,J=5.0Hz),4.10-4.12(1H,m),3.78-3.91(2H,m), 3.15-3.25(1H, m),2.75-2.88(1H,m),2.63-2.75(2H,m), 2.43-2.57(1H,m),1.87-1.99(1H,m) |

Table 10 (continued)

| Production Example No. | N M R (δ ppm) |
|---|---|
| 35 | ($CDCl_3$) 7.20-7.35(2H,m),6.70(1H,t,J=7.1Hz),6.58(2H,d, J=8.4Hz),4.56-4.69(1H,m),3.45-3.65 (2H,m),3.20-3.45(2H,m),2.15-2.29(1H,m),2.00-2.15(1H,m) |
| 36 | ($CDCl_3$) 7.19-7.30(2H,m),6.70(1H,t,J=7.2Hz),6.55 (2H,d,J=7.9Hz),4.10-4.20(1H,m),3.76(1H, dd,J=6.8Hz, 10.0Hz),3.31-3.52(2H,m),3.25(1H,dd,J=5.1Hz,10.1Hz), 2.40-2.54(1H,m), 1.96-2.10(1H,m),2.34(3H,s) |

Table 11

| Production Example No. | N M R (δ ppm) |
|---|---|
| 37 | ($CDCl_3$) 2.03(quint,J=7Hz,2H),2.36(s,3H),2.42(t,J=7Hz,2H), 2.98(t,J=7Hz,3H),7.10(t,J=7Hz, 1H),7.32(t,2H), 7.55(d,J=7Hz,3H) |
| 38 | ($CDCl_3$) 2.30(s,3H),2.42(t,J=7Hz,2H),2.82(t,J=7Hz,2H), 3.12(t,J=7Hz,2H),3.51(q,J=7Hz,2H), 5.50(bs,1H), 7.15-7.35(m,5H) |
| 39 | ($CDCl_3$) 2.29,2.33(s,total 3H),2.70(t,J=7Hz,2H),2.89, 2.95(s,total 3H),3.15-3.25(m,2H), 4.51,4.59(s, total 2H),7.10-7.40(m,5H) |
| 40 | ($CDCl_3$) 1.96(quint,J=7Hz,2H),2.28(t,J=7Hz,2H),2.33(s,3H), 2.93(t,J=7Hz,2H),4.44(d,J=6Hz, 2H),5.93(bs,1H), 7.25-7.35(m,5H) |
| 41 | ($CDCl_3$) 2.33(s,3H),2.47(t,J=7Hz,2H),2.82(d,J=5Hz,3H),3.14 (t,J=7Hz,2H),5.55(bs,1H) |
| 42 | ($CDCl_3$) 2.32(3H,s),2.45(3H,s),2.78(2H,t,J=7Hz),3.05(2H, t,J=7Hz),3.90(2H,s),7.46(2H,d, J=7Hz), 7.57(1H,d,J=7Hz),7.97(2H,d,J=8Hz) |

Table 12

| Production Example No. | N M R (δ ppm) |
|---|---|
| 43 | ($CDCl_3$) 2.35(3H,s),2.67(2H,t,J=7Hz),3.22(2H,t,J=7Hz), 7.11(1H,d,J=7Hz),7.32(2H,d,J=8Hz), 7.51(2H,d,J=7Hz) |
| 44 | ($CDCl_3$) 1.48(3H,d,J=7Hz),2.30(3H,s),2.47(2H,t,J=7Hz), 3.12(2H,t,J=7Hz),5.11(1H,quint., J=Hz), 5.90(1H,bs),7.23-7.35(5H,m) |
| 45 | ($CDCl_3$) 1.47(3H,d,J=8Hz),2.30(3H,s),2.47(2H,t,J=7Hz), 3.11(2H,t,J=7Hz),5.11(1H,quint., J=7Hz),7.22-7.36(5H,m) |

Table 12 (continued)

| Production Example No. | N M R (δ ppm) |
|---|---|
| 46 | ($CDCl_3$) 7.98(2H,d,J=7.2Hz),7.56(1H,t,J=7.5Hz),7.45(2H,t, J=7.8Hz),4.00(2H,d,J=2.0Hz), 3.39-3.50(1H,m), 3.30(1H,dd,J=7.1Hz,9.4Hz),2.90-3.00(1H,m),2.72-2.80(1H,m),2.55 (1H,dd,J=6.6Hz,9.4Hz),2.38-2.50 (1H,m),1.74-1.89(2H,m) |
| 47 | ($CDCl_3$) 8.22(2H,d,J=8.6Hz),7.52(2H,d,J=8.3Hz),3.82(1H,dd, J=6.6Hz,10.7Hz),3.61-3.70(1H, m),3.39-3.55(2H,m), 3.27-3.39(1H,m),2.26-2.40(1H,m),1.79-1.87(1H,m), 1.70-1.75 (1H,bs) |
| 48 | ($CDCl_3$) 1.75(1H,m),1.84(1H,bs),2.38(1H,m),2.46(1H,dd, J=6Hz,9Hz),2.70(2H,t,J=6Hz),2.93 (2H,m),3.08(1H, dd,J=7Hz,9Hz),3.18(2H,t,J=7Hz),3.37(1H,bs), 7.47(2H,d,J=7Hz), 7.56(1H,d,J=7Hz), 7.96(2H,d,J=8Hz) |

Table 13

| Production Example No. | N M R (δ ppm) |
|---|---|
| 49 | ($CDCl_3$) 8.55(1H,d,J=4.6Hz),7.93(2H,d,J=8.1Hz),7.67(1H, t,J=7.6Hz),7.55(1H,t,J=7.1Hz), 7.11-7.23(1H,m), 3.86(2H,d,J=4.9Hz),2.00(1H,bs) |
| 50 | ($CDCl_3$) 8.55(1H,d,J=4.2Hz),7.66(1H,t,J=7.6Hz),7.55(1H, t,J=7.7Hz),7.17(1H,t,J=5.1Hz),3.83 (1H,d,J= 13.6Hz),3.78(1H,d,J=13.6Hz),3.32-3.49(1H,m), 3.08-3.20(1H,m),2.69-2.83 (2H,m),2.34-2.55(2H,m), 1.72-1.92(2H,m) |

Production Example 52

Synthesis of (R)-1-(2-cyclopropyl-2-benzoylethyl)-3-hydroxypyrrolidine

To a solution of 2-cyclopropyl-2-benzoylethane iodide (280 mg, 0.98 mmol) in DMF (6 mℓ), triethylamine (409 μℓ, 2.94 mmol) and (S)-pyrrolidinol hydrochloride (181 mg, 1.47 mmol) were added, followed by stirring at 50°C for 1 hour and further at 80°C for 20 minutes. After the reaction mixture was allowed to cool down, it was diluted with ethyl acetate, followed by washing with water. After the resulting mixture was dried over sodium sulfate, the solvent was distilled off and the residue was subjected to silica gel chromatography (15 cc, chloroform:methanol = 10:1), whereby the title compound was obtained as a colorless oil (144 mg, 60% yield).

Production Example 53

Synthesis of (S)-3-hydroxy-1-(2-phenoxyethyl)-pyrrolidine

A mixture of (S)-3-hydroxypyrrolidine hydrochloride (494 mg, 4 mmol), 1-methanesulfonyloxy-3-phenoxyethane (1.00 g, 4.6 mmol), sodium iodide (659 mg, 4.5 mmol) and potassium carbonate (1.1 g, 8 mmol) in DMF (20 mℓ) was heated at 60°C for 14 hours. The reaction mixture was dissolved in ethyl acetate-water and the resultant solution was allowed to separate into two layers. After the organic layer was dried over anhydrous sodium sulfate, the solvent was distilled off under reduced pressure. The residue was purified by silica gel chromatography, whereby the title compound was obtained (404 mg, 48% yield).

Production Examples 54 to 57

Following the procedures of Production Example 53 except that various halogenated compounds were used instead of 1-methanesulfonyloxy-3-phenoxyethane, the corresponding 3-hydroxypyrrolidine derivatives were obtained.

Data which show physical properties of the compounds obtained in Production Examples 52 to 57 are shown in Tables 14 to 15.

Table 14

| Chemical Structure | N M R |
| --- | --- |
| 52 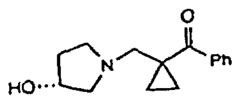 | ($CDCl_3$) 0.80-0.89(2H,m),1.23-1.36(2H,m), 1.96-2.07(1H,m),2.10-2.18(1H,m), 2.33-2.40(1H,m),2.50-2.57(1H,m), 2.69-2.97(4H,m),4.09-4.17(1H,m), 7.40-7.46(2H,m),7.48-7.54(1H,m), 7.79-7.85(1H,m) |
| 53 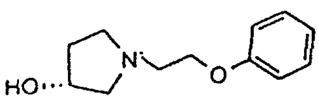 | ($CDCl_3$) 1.70-1.80(m,1H),2.15-2.25(m,1H), 2.38-2.45(m,1H),2.60-2.70(m,1H), 2.75-2.85(m,1H),2.89(t,2H,6Hz), 4.10(t,2H,6Hz),4.30-4.40(m,1H), 8.90-9.00(m,3H),7.25-7.30(m,2H) |
| 54 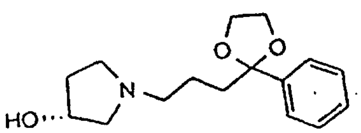 | ($CDCl_3$) 1.69-1.77(m,2H),1.85-1.95(m,3H), 2.18-2.30(m,1H),2.65-2.80(m,3H), 2.82-2.90(m,1H),2.93-3.15(m,2H), 3.75-3.80(m,2H), 4.00-4.05(m,2H), 4.42-4.50(m,1H),7.25-7.35(m,3H), 7.40-7.45(m,2H) |
| 55 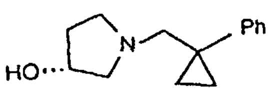 | ($CDCl_3$) 0.72(s,2H),0.80-0.93(m,2H),1.57-1.67(m,1H),2.00-2.10(m,1H),2.12-2.35(m,2H),2.38-2.45(m,1H),2.57 (s,1H,J=12Hz),2.65-2.75(m,2H), 2.80-2.90(m,2H),4.10-4.20(m,1H), 7.10-7.15(m,1H),7.25-7.30(m,2H), 7.30-7.40(m,2H) |

Table 15

| Chemical Structure | N M R |
|---|---|
| 56 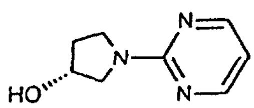 | ($CDCl_3$) 2.50-2.70(m,2H),4.10-4.30(m,4H), 5.05-5.15(m,1H),6.95(t,1H,J=4Hz), 8.84(d,2H,J=4Hz) |
| 57 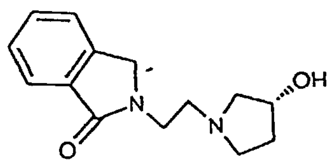 | ($CDCl_3$) 1.68-1.78(1H,m),2.10-2.20(1H,m), 2.35-2.45(1H,m),2.60-2.65(1H,m), 1.70-1.80(2H,m),2.90-2.98(1H,m), 3.45-3.65(1H,m),3.74(2H,t,J=7Hz), 4.30-4.38(1H,m),4.48(2H,s), 7.44(2H,t,J=7Hz),7.50-7.55(1H,m), 7.82(s,d,J=7Hz) |

Production Example 58

Synthesis of (S)-3-hydroxy-1-(4-pyridyl)-pyrrolidine

A solution of (R)-3-pyrrolidinol hydrochloride (1.0 g, 8.1 mmol), 4-chloropyrrolidine (1.50 g, 10 mmol) and triethylamine (5.5 mℓ) in ethanol/water (2.5 mℓ/7.5 mℓ) was heated at 150°C under agitation for 7 hours in a sealed tube. After cooling, the reaction product was diluted in ethanol (50 mℓ) and dried over potassium carbonate, and the solvent was distilled off under reduced pressure. The residue so obtained was purified by flash column chromatography (chloroform/methanol = 7/3), whereby the title compound was obtained (0.99 g, 85%).

Production Examples 59 to 60

Following the procedures of Production Example 58 except that various halogenated compounds were used instead of 4-chloropyrrolidine, the corresponding 3-hydroxypyrrolidine derivatives were obtained.

Physical data of the compounds obtained in Production Examples 58 to 60 are shown in Table 16.

Table 16

| Chemical Structure | N M R |
|---|---|
| 58 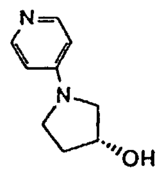 | ($CDCl_3$-DMSO,d-6) 2.08-2.15(m,2H),3.40-3.60(m,4H), 4.55-4.60(m,1H), 6.46(d,7Hz,2H),8.12(d,7Hz,2H) |
| 59 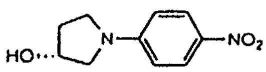 | ($CDCl_3$) 1.65-1.70(1H,m),2.10-2.26(2H,m), 3.39(1H,d,J=11.1Hz),3.51(1H,dt,J=3.2 and 9.7Hz),3.58-3.65(2H,m), 4.65-4.70(1H,brs),7.46-7.52(2H,m), 8.11-8.16(2H,m) |
| 60 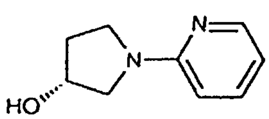 | ($CDCl_3$) 2.04-2.19(2H,m),3.49-3.65(4H,m), 4.57-4.61(1H,m),6.36(1H,d,J=9Hz), 6.53(1H,dd,J=5Hz,7Hz),7.40-7.44 (1H,m),8.13(1H,dd,J=1Hz,5Hz) |

Production Example 61

Synthesis of (S)-3-hydroxy-1-(2-thiazolyl)-pyrrolidine

(S)-pyrrolidinol hydrochloride (1.23 g, 10 mmol) and triethylamine (1.4 mℓ, 19 mmol) were dissolved in dimethylformamide (25 mℓ), to which 2-bromothiazole (1.1 mℓ, 12 mmol) was added at room temperature. The resultant mixture was stirred at room temperature for 5 hours, at 55°C for 4 hours, and then at room temperature for 3 days. Sodium iodide (1.46 g, 10 mmol) was added further, followed by stirring overnight. 2-Bromothiazole (1.1 mℓ, 12 mmol) was added, followed by stirring at 55°C for 4 days and further at room temperature for 3 days. The solvent was distilled off. The residue was purified by silica gel chromatography ("Merck 9385" 120mℓ, ethyl acetate:n-hexane = 1:3 to 1:0), whereby the title compound was obtained as a white solid (0.32 g, 19% yield).

NMR ($CDCl_3$):
2.05-2.30 (m,2H), 3.48-3.70 (m,4H), 4.60-4.70 (m,1H), 6.48 (d,4Hz,1H), 7.20 (d,4Hz,1H).

Production Example 62

Synthesis of (2S,4R)-2-(iminomethoxymethyl)-4-tert-butyldiphenysilyloxy-1-p-nitrobenzyloxycarbonylpyrrolidine

Under an argon atmosphere, $Me_3OBF_4$ (507 mg, 3.43 mmol) was added at room temperature to a solution of (2S,4R)-2-aminocarbonyl-4-tert-butyldimethylsilyloxy-1-p-nitrobenzyloxycarbonylpyrrolidine (1.88 g, 3.43 mmol) in methylene chloride (20 mℓ), followed by stirring for 4 hours. The reaction mixture was diluted with ethyl acetate. The resultant solution was washed successively with a saturated aqueous solution of sodium hydrogencarbonate, water and a saturated solution of sodium hydrogencarbonate. After the solution was dried over anhydrous sodium sulfate, the solvent was distilled off, whereby the title compound was obtained in a foamy form (1.84 g, 96% yield).
NMR ($CDCl_3$):
1.03 (9H,s), 1.82-1.92 (1H,m), 2.25-2.35 (1H,m), 3.31-3.63 (2H,m), 3.68 (3H,s), 4.35-4.58 (2H,m), 5.15-5.30 (2H, m), 7.32-7.49 (10H,m), 7.55-7.63 (2H,m), 8.16-8.22 (2H,m).

Production Example 63

Synthesis of (2S,4R)-2-amidino-4-(tert-butyldiphenysilyloxy)-1-(p-nitrobenzyloxycarbonyl) - pyrrolidine

Under an argon atmosphere, ammonium chloride (192 mg, 3.60 mmol) was added to a solution of (2S,4R)-2-(iminomethoxymethyl)-4-tert-butyldiphenylsilyloxy-1-p-nitrobenzyloxycarbonylpyrrolidine (1.84 g, 3.28 mmol) in methanol (20 mℓ), followed by heating under reflux for 11 hours. After the reaction mixture was allowed to cool down, it was diluted with ethyl acetate, washed successively with a saturated aqueous solution of sodium hydrogencarbonate and a saturated aqueous solution of sodium chloride. After the solution was dried over anhydrous sodium sulfate, the solvent was distilled off, whereby the title compound was obtained in a foamy form (1.8 g, quantitative).
NMR ($CDCl_3$):
1.03 (9H,s), 1.87-2.04 (9H,m), 2.25-2.38 (1H,m), 3.32-3.81 (2H,m), 4.40-4.54 (2H,m), 5.12-5.30 (2H,m), 7.35-7.52 (10H,m), 7.58-7.66 (2H,m), 8.17-8.25 (2H,m).

Production Example 64

Synthesis of (2S,4R)-2-amidino-4-hydroxy-1-(p-nitrobenzyloxycarbonyl)pyrrolidine

To a solution of (2S,4R)-2-amidino-4-(tert-butyldiphenylsilyloxy)-1-(p-nitrobenzyloxycarbonyl)-pyrrolidine (0.96 mg, 1.76 mmol) in THF (10 mℓ), tetra-n-butylammonium fluoride (1 M THF solution; 2.63 mℓ, 2.63 mmol) was added at room temperature, followed by stirring overnight. The resultant solution was diluted with ethyl acetate and washed successively with a saturated aqueous solution of sodium hydrogencarbonate, water and a saturated aqueous solution of sodium chloride. These water layers were combined together and extracted with ethyl acetate. The extract was combined with the former ethyl acetate layer. Then, the ethyl acetate solution was dried over anhydrous sodium sulfate and the solvent was distilled off, whereby the title compound was obtained as slightly yellow crystals (100 mg, 18% yield).
NMR (MeOH-$d_4$):
1.83-1.95 (1H,m), 2.02-2.20 (1H,m), 4.17-4.31 (3H,m), 5.00-5.25 (3H,m), 7.58-7.65 (2H,m), 8.17-8.25 (2H,m).

Production Example 65

Synthesis of (R)-3-hydroxy-1-(N-phenylamidino)-pyrrolidine

A solution of (R)-3-hydroxypyrrolidine hydrochloride (741 mg, 6.0 mmol), 2-methyl-1-phenylisothiourea hydroiodide (1.76 g, 6.0 mmol) and triethylamine (1.2 g, 12 mmol) in DMF (30 mℓ) was heated overnight at 60°C. After the solvent was distilled off under reduced pressure, the residue so obtained was purified by silica gel chromatography, whereby the title compound was obtained (609 mg, 50% yield).
NMR ($CDCl_3$):
2.0-2.2 (m,2H), 3.50-3.70 (m,4H), 4.51-4.6 (m,1H), 6.90-7.00 (m,3H), 7.20-7.30 (m,2H).

Production Example 66

Synthesis of 1-benzoyl-2-methylisothiourea hydroiodide

To a solution of N-benzoylthiourea (90 mg, 0.5 mmol) in methylene chloride (2 mℓ), methyl iodide (170 mg, 1.2 mmol) was added. The resultant mixture was left over at room temperature for 18 hours. The resulting crystals were collected by filtration, whereby the title compound was obtained (60 mg, 37% yield).

Production Example 67

Synthesis of (R)-3-hydroxy-1-(N-benzoylamidino)-pyrrolidine

A solution of (R)-1-hydroxypyrrolidine hydrochloride (25 mg, 0.2 mmol), 1-benzoyl-2-methylisothiourea hydroiodide (64 mg, 0.2 mmol) and triethylamine (101 mg, 1.0 mmol) in DMF (2.5 mℓ) was heated at 140°C for 8 hours. After cooling, ethyl acetate and a saturated aqueous solution of sodium hydrogencarbonate were added to extract the reaction product. The organic layer was dried over anhydrous sodium sulfate. The solvent was distilled off under reduced pressure and the residue so obtained was purified by silica gel chromatograpy, whereby the title compound was obtained (17 mg, 36% yield).
NMR ($CDCl_3$) :
2.05-2.20 (2H,m), 3.61-3.77 (4H,brs), 4.58-4.63 (1H,m), 7.37-7.48 (3H,m), 8.21-8.27 (2H,m).
IR (NaCl):
3316, 1664, 1560, 1458.

Production Example 68

Synthesis of 1-tert-butoxycarbonyl-4-tert-butyldimethylsilyloxy-2-(1-hydroxybenzyl)-pyrrolidine

To a solution of 1-tert-butoxycarbonyl-4-tert-butyldimethylsilyloxy-2-formylpyrrolidine (1 g, 3 mmol) in THF (5 mℓ), a 2 M THF solution (2 mℓ) of phenylmagnesium bromide was added at -78°C. After the temperature of the resultant mixture was raised to -40°C, a saturated solution of ammonium chloride was added. Subsequent to extraction with ethyl acetate, the organic layer was washed with brine. The organic layer was dried over anhydrous sodium sulfate. The solvent was distilled off under reduced pressure and the residue so obtained was purified by silica gel chromatography, whereby the title compound was obtained (1.04 g, 87% yield).

Production Example 69

Synthesis of 2-benzoyl-1-tert-butoxycarbonyl-4-tert-butyldimethylsilyloxypyrrolidine

To a solution of oxalyl chloride (280 mg, 3.2 mmol) in methylene chloride (8 mℓ), DMSO (0.33 mℓ) was added dropwise at -78°C. Ten minutes later, a solution of l-tert-butoxycarbonyl-4-tert-butyldimethylsilyloxy-2-(1-hydroxybenzyl)pyrrolidine (200 mg, 0.5 mmol) in methylene chloride (2 mℓ) was added. Ten minutes later, triethylamine (580 mg, 5.8 mmol) was added. The temperature of the resultant mixture was allowed to rise to 0°C over 1 hour. Ethyl acetate was added to the reaction mixture. The organic layer was washed with an aqueous solution of potassium hydrogensulfate, brine, a saturated aqueous solution of sodium hydrogencarbonate and brine, and was then dried over anhydrous sodium sulfate. The solvent was distilled off under reduced pressure and the residue so obtained was purified by silica gel chromatography, whereby the title compound was obtained (200 mg, 100% yield).

Production Example 70

Synthesis of 2-benzoyl-4-hydroxy-1-p-nitro-benzyloxycarbonylpyrrolidine

To 2-benzoyl-1-tert-butoxycarbonyl-4-tert-butyldimethylsilyloxypyrrolidine (200 mg, 0.5 mmol), 5%-HCl/methanol (2 mℓ) and 4 N-HCl/dioxane (2 mℓ) were added at room temperature. Thirty minutes later, the solvent was distilled off under reduced pressure. The residue so obtained was added with water (0.8 mℓ) and sodium hydrogencarbonate (84 mg, 1 mmol), followed by the addition of a solution of p-nitrobenzyloxycarbonyl chloride (130 mg, 0.5 mmol) in dioxane (0.8 mℓ) at room temperature. The resultant mixture was vigorously stirred for 1 hour. After extraction with ethyl acetate, the organic layer was washed with brine and then dried over anhydrous sodium sulfate. The solvent was distiled off under reduced pressure and the residue was purified by silica gel chromatography, whereby the title compound was obtained (100 mg, 100% yield).

Production Examples 71 to 84

Following the procedures of Production Example 7 except that various 3-hydroxypyrrolidine derivatives were used instead of 1-(2-hydroxyethyl)pyrrolidine, the corresponding 3-benzoylthiopyrrolidine derivatives were obtained. Also following the procedures of Production Example 7 except that various 3-hydroxypyrrolidine derivatives and thioacetic acid were used instead of 1-(2-hydroxyethyl)pyrrolidine and thiobenzoic acid, respectively, the corresponding 3-acetylthiopyrrolidine derivatives were obtained.

Physical data of the thus-obtained 3-benzoylthiopyrrolidine derivatives and 3-acetylthiopyrrolidine derivatives are shown in Table 17 to Table 20.

Table 17

| Chemical Structure | N M R |
|---|---|
| 7 1 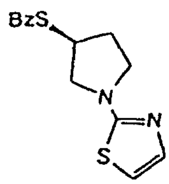 | ($CDCl_3$) 2.15-2.25(m,1H),2.55-2.65(m,1H), 3.50-3.70(m,3H),4.00-4.05(m,1H), 4.30-4.40(m,1H),6.50(d,4Hz,1H), 7.20(d,7Hz,1H),7.40(m,2H), 7.58(t,7Hz,1H),7.95(dd,8Hz,1Hz,2H) |
| 7 2 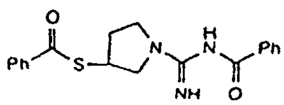 | ($CDCl_3$) 2.13-2.24(1H,m),2.50-2.61(1H,m), 3.56-3.86(4H,brs),4.29-4.36(1H,m), 6.30-6.45(2H,brs),7.35-7.52(5H,m), 7.59-7.65(1H,m),7.92-7.97(2H,m), 8.22-8.27(2H,m) |
| 7 3 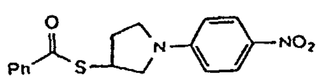 | ($CDCl_3$) 2.18-2.29(1H,m),2.55-2.66(1H,m), 3.46(1H,dd,J=5.1 and 10.9Hz), 3.51-3.72(2H,m),3.99(1H,dd,J= 6.8 and 10.9Hz),4.34-4.42(1H,m), 6.50(2H,d,J=9.3Hz),7.40-7.65(3H,m), 7.92-7.97(2H,m),8.13(2H,d,J=9.3Hz) |
| 7 4 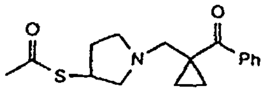 | ($CDCl_3$) 0.78-0.85(2H,m),1.23-1.32(2H,m), 2.15-2.28(1H,m),2.26(3H,s), 2.33-2.41(2H,m),2.48-2.56(1H,m), 2.70-2.86(3H,m),3.75-3.83(1H,m), 7.39-7.52(3H,m),7.78-7.85(2H,m) |

Table 18

| Chemical Structure | N M R |
|---|---|
| 75 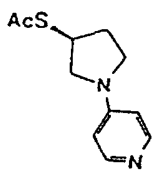 | ($CDCl_3$) 2.05-2.15(m,1H),2.36(s,3H), 2.45-2.55(m,1H), 3.31(dd,J=12Hz,6Hz,1H), 3.42-3.52(m,2H), 3.84(dd,J=9Hz,7Hz,1H), 4.10-4.20(m,1H), 6.41(d,J=6Hz,2H),8.28 (d,J=6Hz,2H) |
| 76 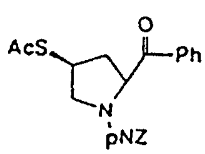 | ($CDCl_3$) 1.85-2.00(m,1H),2.85-3.00(m,1H), 3.50(t,J=8Hz,1H),3.9-4.1(m,2H),5.01 (d,J=12Hz,1/2H),5.20(d,J=12Hz,1/2H), 5.25(s,2/2H),5.35(t,J=8Hz,1/2H), 5.41(t,J=8Hz,1/2H),7.45-7.50(m,2H), 7.52(d,J=8Hz,2H),7.55-7.65(m,1H), 7.88(d,J=8Hz,2/2H), 8.02(d,J=8Hz,2/2H), 8.21(d,J=8Hz,2H) |
| 77 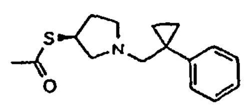 | ($CDCl_3$) 0.72(2H,m),0.84(2H,m),1.59-1.70 (1H,m),2.27(3H,s),2.46(1H,dd,J= 6Hz,10Hz),2.49-2.61(4H,m),2.69 (1H,d,J=13Hz),2.98(1H,dd,J=7Hz, 10Hz),3.81-3.83(1H,m),7.16(1H,m), 7.24-7.28(3H,m),7.31-7.34(1H,m) |
| 78 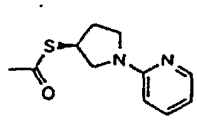 | ($CDCl_3$) 1.99-2.09(1H,m),2.33(3H,s), 2.40-2.49(1H,m),3.43(1H,dd, J=5Hz,11Hz),3.49-3.60(2H,m), 3.91(1H,dd,J=7Hz,11Hz), 4.10-4.16(1H,m), 6.33(1H,d,J=8Hz), 6.54(1H,dd,J=5Hz,7Hz), 7.42(1H,m), 8.14(1H,dd,J=1Hz,5Hz) |

Table 19

| Chemical Structure | N M R |
|---|---|
| 7 9 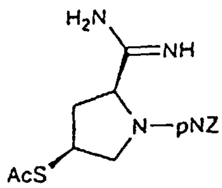 | ($CDCl_3$) 2.33(3H,s),2.30-2.61(2H,m), 3.35-3.63(1H,m),3.92-4.01(1H,m), 4.10-4.17(1H,m),4.37-4.42(1H,m), 5.18-5.32(2H,m),7.49-7.62(2H,m), 8.18-8.26(2H,m) |
| 8 0 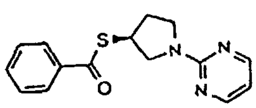 | ($CDCl_3$) 2.11-2.22(1H,m),2.49-5.52(1H,m), 3.65-3.80(3H,m),4.08-4.15(1H,m), 4.30-4.39(1H,m),6.51(1H,dd, J=5Hz,7Hz),7.42-7.49(2H,m), 7.53-7.61(1H,m),7.94(2H,dd, J=2Hz,9Hz),8.33(2H,d,J=5Hz) |
| 8 1 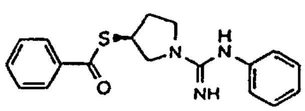 | ($CDCl_3$) 2.08-2.14(1H,m),2.46-2.56(1H,m), 3.51(1H,dd,J=5Hz,10Hz),3.55-3.64 (2H,s),4.01(1H,dd,J=7Hz,10Hz), 4.27(1H,m),6.92-6.98(3H,m), 7.24-7.28(2H,m),7.43-7.47(2H,m), 7.58(1H,m),7.94(2H,m) |
| 8 2 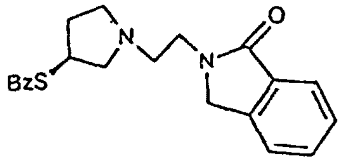 | ($CDCl_3$) 1.71-1.76(1H,m),2.28(3H,s), 2.29-2.39(1H,m),2.56-2.62(2H,m), 2.71-2.81(3H,m),3.02(1H,dd, J=7Hz,10Hz),3.72(2H,t,J=6Hz), 3.89-3.93(1H,m),4.50(2H,s),7.42-7.45(2H,m),7.52(1H,dd,J=5Hz,8Hz), 7.84(1H,d,J=8Hz) |

Table 20

| Chemical Structure | N M R |
|---|---|
| 8 3 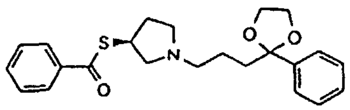 | ($CDCl_3$) 1.87-1.90(2H,m),1.92-1.98(2H,m), 2.17-2.21(1H,m),2.62-2.67(1H,m), 3.12(2H,m),3.24(1H,dd,J=6Hz,12Hz), 3.42(1H,m),3.73(2H,m),3.91(1H,m), 4.00(2H,m),4.27(1H,m),7.26-7.39 (3H,m),7.49-7.60(3H,m),7.90(2H,m), 8.10(2H,m) |
| 8 4 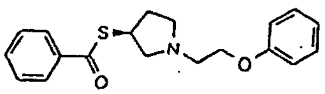 | ($CDCl_3$) 1.87-1.94(1H,m),2.43-2.50(1H,m), 2.69-2.76(2H,m),2.83-2.88(1H,m), 2.89-2.99(2H,m),3.22(1H,dd, J=7Hz,10Hz),4.09-4.19(3H,m), 6.90-6.95(3H,m),7.23-7.28(2H,m), 7.40-7.45(2H,m),7.55(1H,dd, J=7Hz,7Hz),7.93(2H,d,J=8Hz) |

Production Example 85

Synthesis of N-(4-pentenoyl)-3-benzoylthiopyrrolidine

To a solution of 3-benzoylthiopyrrolidinetrifluoroacetate (980 mg, 3.05 mmol), 1-ethyl-3-(3-dimethylaminopropyl)-carbodiimide hydrochloride (702 mg, 3.7 mmol) and 1-hydroxybenzotriazole (560 mg, 3.7 mmol) in methylene chloride (6 mℓ), 4-pentenoic acid (367 mg, 3.74 mmol) and triethylamine (320 mg, 3.2 mmol) were successively added under ice cooling, followed by stirring at room temperature for 2 hours. After the reaction mixture was diluted with ethyl acetate, the organic layer was washed successively with a saturated aqueous solution of potassium hydrogensulfate, brine, a saturated aqueous solution of sodium hydrogencarbonate and brine. The organic layer was dried over anhydrous sodium sulfate and the solvent was distilled off under reduced pressure. The residue so obtained was purified by silica gel chromatography, whereby the title compound was obtained (768 mg, 86% yield).
NMR ($CDCl_3$) :
2.00-2.08 (m,$\frac{1}{2}$H), 2.08-2.17 (m,$\frac{1}{2}$H), 2.32-2.48 (m,4$\frac{1}{2}$H), 2.48-2.58 (m,$\frac{1}{2}$H), 3.45-3.50 (dd,J=11Hz, 5Hz,$\frac{1}{2}$H), 3.55-3.75 (m,2$\frac{1}{2}$H), 3.95-4.05 (m,1H), 4.17-4.27 (m,1H), 4.95-5.10 (m,1H), 5.80-5.95 (m,1H), 7.40-7.50 (m,2H), 7.55-7.65 (m,1H), 7.90-7.95 (m,2H).

Production Examples 86 to 92

Following the procedures of Production Example 17 except that various halogenated compounds were used instead of phenacyl bromide, the corresponding 3-benzoylthiopyrrolidine derivatives were obtained.
Physical data of the thus-obtained compounds are shown in Table 21 and Table 22.

Table 21

| Chemical Structure | N M R |
|---|---|
| 86 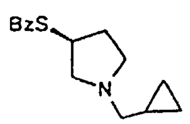 | ($CDCl_3$) 0.13(2H,m),0.50(2H,m),0.90-0.95 (1H,m),1.85-1.93(1H,m),2.31-2.53 (3H,m),2.62-2.70(2H,m),2.75-2.81 (1H,m),3.16(1H,dd,J=7Hz,10Hz), 4.10-4.17(1H,m),7.44(2H,dd, J=8Hz,8Hz),7.56(1H,m), 7.94(2H,dd,J=1Hz,7Hz) |
| 87 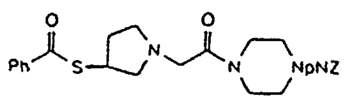 | ($CDCl_3$) 1.85-1.96(1H,m),2.42-2.53(1H,m), 2.61-2.69(1H,m),2.70-2.77(1H,m), 2.80-2.89(1H,m),3.12(1H,dd,J=7.0 and 9.9Hz),3.37(2H,s),3.48-3.69 (8H,m),4.10-4.18(1H,m),5.24(2H,s), 7.42-7.61(5H,m),7.90-7.95(2H,m), 8.20-8.25(2H,m) |
| 88 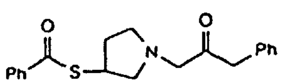 | ($CDCl_3$) 1.85-1.96(1H,m),2.41-2.53(1H,m), 2.61-2.69(1H,m),2.70-2.75(1H,m), 2.77-2.83(1H,m),3.13(1H,dd,J=7.2 and 10.0Hz),3.38(1H,dd,J=17.1Hz), 3.42(1H,dd,J=17.1Hz),3.75(2H,s), 4.09-4.18(1H,m),7.21-7.28(2H,m), 7.30-7.37(2H,m),7.42-7.47(2H,m), 7.54-7.60(1H,m),7.91-7.96(2H,m) |
| 89 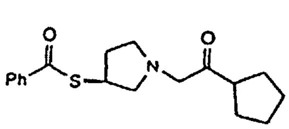 | ($CDCl_3$) 1.56-1.87(8H,m),1.88-1.98(1H,m), 2.43-2.54(1H,m),2.68-2.76(2H,m), 2.79-2.87(1H,m),2.91-3.00(1H,m), 3.19(1H,dd,J=7.3 and 9.9Hz), 3.43(1H,d,J=17.4Hz),3.48(1H,d, J=17.4Hz),4.10-4.19(1H,m),7.41-7.47(2H,m),7.53-7.59(1H,m),7.92-7.96(2H,m) |

Table 22

| Chemical Structure | N M R |
|---|---|
| 9 0<br>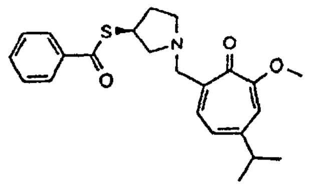 | ($CDCl_3$)<br>1.28(6H,d,J=7Hz),1.85-1.98(1H,m),<br>2.43-2.56(1H,m),2.66-2.75(2H,m),<br>2.82-2.91(2H,m),3.15(1H,dd,<br>J=7Hz,10Hz),3.81(2H,s),3.94(3H,s),<br>4.11-4.22(1H,m),6.71(1H,s),6.86<br>(1H,d,J=10Hz),7.45(2H,dd,J=7Hz,9Hz),<br>7.54-7.60(1H,m),7.72(1H,d,J=9Hz),<br>7.94(2H,d,J=9Hz) |
| 9 1<br>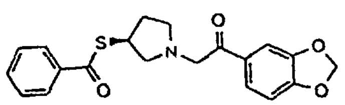 | ($CDCl_3$)<br>1.90-1.96(1H,m),2.47-2.53(1H,m),<br>2.76-2.82(2H,m),2.86-2.91(1H,m),<br>3.27(1H,dd,J=7Hz,10Hz),3.91<br>(2H,d,J=3Hz),4.16-4.20(1H,m),<br>6.02(2H,s),6.84(1H,d,J=8Hz),<br>7.43(2H,dd,J=7Hz,7Hz),7.48<br>(1H,d,J=2Hz),7.55(1H,dd,J=7Hz,7Hz),<br>7.62(1H,dd,J=2Hz,8Hz),<br>7.93(2H,d,J=8Hz) |
| 9 2<br>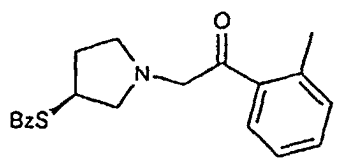 | ($CDCl_3$)<br>1.90-2.00(m,1H),2.50(s,3H),2.50-2.55<br>(m,1H),2.80-2.95(m,3H),3.28-3.35<br>(m,1H),3.90(d,2H,J=8Hz),4.15-4.25<br>(m,1H),7.20-7.30(m,2H),7.37<br>(t,1H,J=7Hz),7.40-7.50(m,2H),<br>7.56(t,1H,J=6Hz),7.63(d,1H,J=7Hz),<br>7.92(d,2H,J=7Hz) |

Production Example 93

Synthesis of (S)-3-benzoylthio-N-(4-oxo-4-phenylbutan-1-yl)pyrrolidine

After a mixed solution of (S)-2-[[3-(3-benzoylthiopyrrolidin-1-yl)propan]-1-yl]-2-phenyl-1,3-dioxolane (484 mg, 1.21 mmol) in methanol (10 ml) and a 10% aqueous solution of hydrochloric acid (10 ml) were stirred at room temperature for 3 hours, methylene chloride was added to extract the reaction product. The organic layer was dried over anhydrous sodium sulfate and the solvent was distilled off under reduced pressure, whereby the hydrochloride of the title compound was obtained as a yellow oil (424 mg, 90% yield).

NMR ($CDCl_3$) :
2.31-2.36 (3H,m), 2.73 (1H,m), 3.25-3.31 (4H,m), 3.31-3.71 (3H,m), 4.11 (1H,brs), 4.35 (1H,t,J=7Hz), 7.43-7.47 (3H,m), 7.52-7.62 (2H,m), 7.65-7.70 (1H,m), 7.90 (2H,d,J=7Hz), 7.95 (2H,d,J=8Hz).

Production Example 94

Synthesis of (S)-3-benzovlthio-N-cyclopentanylpyrrolidine

(S) -3-benzoylthiopyrrolidine trifluoroacetate (0.32 g, 1 mmol) was dissolved in methanol (4.7 ml), to which cyclopentanone (83µl, 0.94 mmol) and 95% $NaBH_3CN$ (43 mg, 0.065 mmol) were added under ice cooling. The resultant mixture was stirred overnight at room temperature. The reaction mixture was diluted with ethyl acetate and the resulting solution was poured into a saturated solution of sodium hydrogencarbonate. The organic layer was separated, dried over anhydrous sodium sulfate, and then purified by chromatography on a silica gel column ("Merck 9385", 15 mℓ; ethyl acetate:n-hexane = 1:2), whereby the title compound was obtained as a yellow oil (0.19 g, 69% yield).
NMR ($CDCl_3$):
1.45-1.80 (8H,m), 1.80-1.95 (1H,m), 2.40-2.80 (4H,m), 3.15-3.25 (1H,m), 4.10-4.20 (1H,m), 7.40-7.50 (2H,m), 7.52-7.60 (1H,m), 7.94 (2H,d,J=10Hz).

Production Example 95

Synthesis of (S)-3-benzoylthio-N-(indan-2-yl)-pyrrolidine

Following the procedures of Production Example 94 except that (S)-3-benzoylthiopyrrolidine trifluoroacetate was used in an amount of 643 mg (2 mmol) and 2-indanone (264 mg, 2 mmol) was used instead of cyclopentanone, the title compound was obtained (345 mg, 53% yield).
NMR ($CDCl_3$):
1.87-1.95 (1H,m), 2.45-2.53 (1H,m), 2.66-2.71 (2H,m), 2.78-2.82 (1H,m), 2.88-2.95 (2H,m), 2.98-3.18 (2H,m), 3.12-3.28 (2H,m), 4.11-4.18 (1H,m), 7.10-7.18 (4H,m), 7.43 (2H,dd,J=8Hz,8Hz), 7.55 (1H,dd,J=7Hz,9Hz), 7.94 (2H, d,J=7Hz).

Production Example 96

Synthesis of 1-cyclopropyl-3-hydroxypyrrolidine

Following the procedures of Production Example 35 except that 1,4-dibromobutan-2-ol was used in an amount of 2.3 g (10 mmol) and cyclopropylamine (0.68 g, 12 mmol) was used instead of aniline, the title compound was obtained (0.78 g, 60% yield).
NMR ($CDCl_3$):
0.50-0.60 (m,2H), 0.65-0.78 (m,2H), 1.80-2.00 (m,2H), 2.18-2.25 (m,1H), 2.75-3.00 (m,3H), 3.08-3.15 (m,1H), 4.40-4.45 (m,1H).

Production Example 97

Synthesis of 3-acetylthio-1-cyclopropylpyrrolidine

Following the procedures of Production Example 36 except that 1-cyclopropyl-3-hydroxypyrrolidine (139 mg, 1.0 mmol) was used instead of 3-hydroxy-1-phenylpyrrolidine, the title compound was obtained (29 mg, 58% yield).
NMR ($CDCl_3$):
0.35-0.45 (m,4H), 1.60-1.80 (m,2H), 2.28 (s,3H), 2.30-2.40 (m,1H), 2.57-2.63 (m,1H), 2.65-2.80 (m,1H), 3.12-3.18 (m,1H), 3.85-3.95 (m,1H).

Production Example 98

Synthesis of (S)-3-benzoylthio-1-(1-methoxyimino-1-phenylethyl)pyrrolidine

A solution of (R)-3-benzoylthio-1-phenacylpyrrolidine trifluoroacetate (325 mg, 1.0 mmol), methoxyamine (251 mg, 3.0 mmol) and potassium acetate (294 mg, 3.0 mmol) in methanol (30 mℓ) was stirred overnight. The reaction mixture was diluted with methylene chloride, followed by washing with water. The organic layer was dried over anhydrous sodium sulfate. The solvent was distilled off under reduced pressure and the residue so obtained was purified by silica gel chromatography, whereby the title compound was obtained (124 mg, 35% yield).
NMR (δppm, $CDCl_3$):

1.16-1.18 (1H,m), 2.27-2.32 (1H,m), 2.42-2.47 (1H,m), 2.62-2.75 (2H,m), 3.04 (1H,dd,J=7Hz, 10Hz), 3.76 (2H, m), 3.97 (3H,s), 4.02 (1H,m), 7.33-7.36 (3H,m), 7.42 (2H,dd,J=7Hz,10Hz), 7.52 (1H,m), 7.73-7.75 (2H,m), 7.90 (2H,m).

Production Examples 99 to 113

Following the procedures of Production Example 46 except that various 3-benzoylthiopyrrolidine derivatives and 3-acetylthiopyrrolidine derivatives were used instead of (S)-3-benzoylthio-N-phenacylpyrrolidine, the corresponding 3-mercaptopyrrolidine derivatives were obtained.
Physical data of the thus-obtained 3-mercaptopyrrolidine compounds are shown in Tables 23 to 26.

Table 23

| Chemical Structure | N M R |
| --- | --- |
| 99 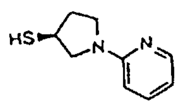 | ($CDCl_3$) 1.95-2.03(1H,m),2.39-2.47(1H,m), 3.39(1H,dd,J=6Hz,11Hz),3.46-3.60 (2H,m),3.64-3.69(1H,m),3.89 (1H,dd,J=7Hz,11Hz),6.34(1H,d,J= 8Hz),6.54(1H,dd,J=5Hz,7Hz), 7.41-7.46(1H,m),8.15(1H,dd,J= 1Hz,5Hz) |
| 100 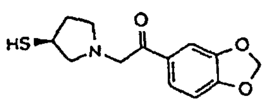 | ($CDCl_3$) 1.76-1.89(1H,m),2.37-2.43(1H,m), 2.53(1H,dd,J=7Hz,10Hz),2.76-2.80 (1H,m),2.85-2.92(1H,m),3.25 (1H,dd,J=7Hz,10Hz),3.44-3.57 (1H,m),3.88(2H,s),6.03(2H,s), 6.84(1H,d,J=8Hz),7.47(1H,d,J= 2Hz),7.61(1H,dd,J=2Hz,8Hz) |
| 101 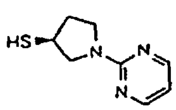 | ($CDCl_3$) 1.91-2.06(1H,m),2.38-2.48(1H,m), 3.46-3.68(3H,m),3.75-3.85(1H,m), 3.94-4.04(1H,m),6.50(1H,dd,J= 5Hz,5Hz),8.32(2H,d,J=5Hz) |
| 102 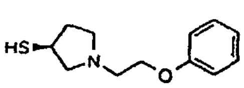 | ($CDCl_3$) 1.72-1.79(1H,m),2.35-2.40(1H,m), 2.49(1H,dd,J=7Hz,10Hz),2.70-2.95 (4H,m),3.19(1H,dd,J=7Hz,10Hz), 3.37(1H,brs),4.09(2H,t,J=6Hz), 6.92(3H,m),7.26(2H,m) |

Table 24

| Chemical Structure | N M R |
|---|---|
| 1 0 3 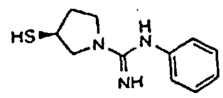 | ($CDCl_3$) 1.95-1.97(1H,m),2.38-2.42(1H,m), 3.35(1H,m),3.48-3.53(2H,m), 3.63-3.68(1H,m),3.86-3.90(1H,m), 6.90-6.97(3H,m),7.24-7.27(2H,m) |
| 1 0 4 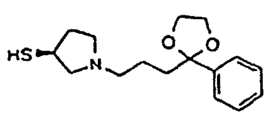 | ($CDCl_3$) 1.56(2H,m),1.81-1.91(1H,m), 1.92-1.95(2H,m),2.30-2.71 (5H,m),3.02(1H,m),3.33(1H,m), 3.76(2H,brs),4.00(2H,brs), 4.09(1H,m),7.55(5H,m) |
| 1 0 5 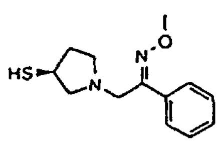 | ($CDCl_3$) 1.66-1.73(1H,m),2.27-2.33(1H,m), 2.44(1H,dd,J=7Hz,10Hz),2.63-2.69 (2H,m),3.04(1H,dd,J=7Hz,10Hz), 3.26(1H,m),3.76(2H,s), 3.97(3H,s),7.34(3H,m), 7.75 (2H,m) |
| 1 0 6 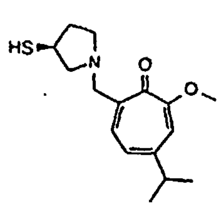 | ($CDCl_3$) 1.28(6H,d,J=7Hz),1.78(1H,m), 1.86(1H,m),2.39(1H,m), 2.56(1H,dd,J=6Hz,10Hz), 2.69-2.87(2H,m),3.05(1H,dd,J= 7Hz,10Hz),3.37(1H,m),3.77 (2H,d,J=3Hz),3.93(3H,s), 6.70(1H,s),6.84(1H,d,J=9Hz), 7.67(1H,d,J=10Hz) |

Table 25

| Chemical Structure | N M R |
|---|---|
| 107 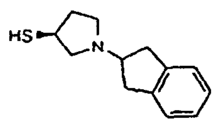 | (CDCl₃) 1.73-1.87(2H,m),2.38-2.49(2H,m), 2.65-3.20(7H,m),3.36-3.45(1H,m), 7.11-7.18(4H,m) |
| 108 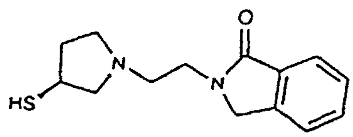 | (CDCl₃) 1.69-1.78(1H,m),2.30-2.39(1H,m), 2.49(1H,dd,J=6Hz,9Hz), 2.69-2.82(5H,m), 3.07(1H,dd,J=7Hz,10Hz), 3.31-3.37(1H,m), 3.68-3.78(2H,m), 4.50(2H,d,J=3Hz), 7.42-7.53(3H,m), 7.84(1H,d,J=7Hz) |
| 109 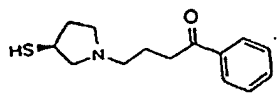 | (CDCl₃) 1.81(1H,m),1.92-1.96(3H,m), 2.41(1H,m),2.53-2.64(4H,m), 3.02(2H,d,J=7Hz),3.30(1H,m), 4.09(1H,m),7.45(2H,m), 7.55(1H,m),7.96(2H,m) |
| 110 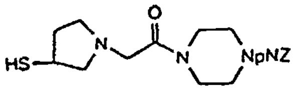 | (CDCl₃) 1.72-1.81(1H,m),1.83(1H,d,J= 7.1Hz),2.33-2.45(1H,m), 2.52-2.59(1H,m),2.63-2.71 (1H,m),2.75-2.82(1H,m), 3.00-3.08(1H,m),3.31-3.65(11H,m), 5.24(2H,s),7.51(2H,d,J=8.8Hz), 8.22(2H,d,J=8.8Hz) |

Table 26

| Chemical Structure | N M R |
|---|---|
| 1 1 1 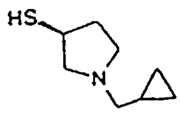 | ($CDCl_3$) 0.12(2H,t,J=5Hz),0.50(2H,t,J=5Hz), 0.89(1H,m),1.73(1H,m), 2.32-2.39(4H,m),2.61(1H,m), 2.79(1H,m),3.17(1H,dd,J=7Hz,10Hz), 3.40(1H,m) |
| 1 1 2 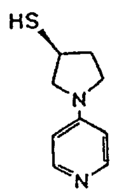 | ($CDCl_3$) 2.05-2.11(m,1H),2.45-2.55(m,1H), 3.27-3.33(m,1H),3.41-3.50(m,1H), 3.57-3.67(m,1H),3.75-3.82(m,1H), 4.15-4.25(m,1H),6.45(d,J=7Hz,2H), 8.31(d,J=7Hz,2H) |
| 1 1 3 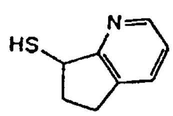 | ($CDCl_3$) 2.00-2.10(m,1H),2.19(d,J=5Hz,1H), 2.60-2.70(m,1H),2.86-2.96(m,1H), 3.05-3.15(m,1H),4.45-4.50(m,1H), 7.09(dd,J=7Hz,5Hz,1H), 7.52(d,J=7Hz,1H), 8.43(d,J=5Hz,1H) |

Example 1

Synthesis of 2,2,2-trichloroethyl 6,6-dibromopenicillanic acid

To a solution of 6,6-dibromopenicillanic acid (29.5 g, 0.082 mol) in dry ethyl acetate (200 mℓ), pyridine (16.6 mℓ, 0.205 mol) was added at -10°C under an argon gas stream, followed by the addition of 2,2,2-trichloroethyl chloroformate (22.6 mℓ, 0.164 mol) over 20 minutes.

Fifty minutes later, the reaction mixture was poured into ethyl acetate (200 mℓ) and the resultant mixture was washed successively with water (200 mℓ), a saturated aqueous solution of potassium hydrogensulfate (100 mℓ), a 5%

aqueous solution of potassium hydrogensulfite (100 mℓ), a saturated aqueous solution of sodium hydrogencarbonate (100 mℓ) and a saturated aqueous solution of sodium chloride (100 mℓ). The organic layer was dried over anhydrous sodium sulfate. After the solvent was distilled off under reduced pressure, column chromatography was conducted using silica gel (250 g). From ethyl acetate-hexane (1:15, V/V), the title compound was obtained as a yellow solid (30.7 g, 76% yield).

NMR δ ($CDCl_3$):

1.55 (3H,s), 1.67 (3H,s), 4.68 (1H,s), 4.80 (2H,s), 5.84 (1H,s).

Example 2

Synthesis of 2,2,2-trichloroethyl 6-bromo-6-((S,R)-1-hydroxypropyl)penicillanate and 2,2,2-trichloroethyl 6-bromo-6-((R)-1-hydroxypropyl)-penicillanate

To a solution of 2,2,2-trichloroethyl 6,6-dibromopenicillanate (30.1 g, 0.061 mol) in dry methylene chloride (100 mℓ), a 2.83 M ether solution of methyl magnesium bromide (22.0 mℓ, 0.062 mol) was added under an argon gas stream at -78°C over 8 minutes, followed by the addition of propionaldehyde (4.29 g, 0.074 mol) in methylene chloride (20 mℓ) over 7 minutes. Thirty minutes later, a 0.1 M phosphate buffer (pH 7.0, 100 mℓ) was added to the reaction mixture. The temperature of the resultant mixture was then allowed to rise to room temperature, followed by the addition of water (200 mℓ) and methylene chloride (400 mℓ). Insoluble matter was removed through "Celite".

The organic layer was collected and then dried over anhydrous sodium sulfate. After the solvent was distilled off under reduced pressure, column chromatography was conducted using silica gel (300 g). From fractions eluted with ethyl acetate-hexane (1:5, V/V) and having lower polarity, a mixture of aldol adducts of isomers including the target (S)-hydroxypropyl isomer (a 1:3 mixture of the (S)-hydroxylpropyl isomer and the (R)-hydroxypropyl isomer) was obtained as a pale yellow oil (16.75 g, 58% yield). From fractions of higher polarity, on the other hand, the (R)-hydroxypropyl isomer was obtained as a white solid (6.24 g, 22% yield).

NMR δ ($CDCl_3$) :

(as the isomer mixture of the (S)-hydroxypropyl isomer and the (R)-hydroxypropyl isomer)

[1.05 (t,J=7Hz), 1.07 (t,J=7Hz)](3H), 1.4-1.95 (2H,m), [1.55 (s), 1.57 (s)](3H), [1.70 (s), 1.71 (s)](3H), [2.23 (d, J=5Hz), 2.32 (d,J=5Hz)](1H), [3.86 (dt,J=3Hz,5Hz), 3.97 (ddd,J=4Hz,7Hz,9Hz)] (1H), [4.64 (s), 4.65 (s)](1H), 4.7-4.85 (2H,m), [5.52 (s), 5.63 (s)](1H).

Example 3

Synthesis of a mixture of 2,2,2-trichloroethyl (5R,6R)-6-(1-hydroxypropyl)penicillanate and 2,2-dichloroethyl (5R,6R)-6-(1-hydroxypropyl)-penicillanate

To a solution of a mixture (16.75 g, 0.036 mol) of 2,2,2-trichloroethyl 6-bromo-6-((S,R)-1-hydroxypropyl)penicillanate and 2,2,2-trichloroethyl 6-bromo-6-((R)-1-hydroxypropyl)penicillanate in dry benzene (150 mℓ), tributyltin hydride (19.9 mℓ, 0.074 mol) was added at room temperature. Under an argon gas stream, the resultant mixture was heated at 95-105°C for 2 hours and 30 minutes, and the reaction mixture was then allowed to cool down to room temperature.

Fourteen hours later, the solvent in the reaction mixture was distilled off under reduced pressure, the residue was dissolved in acetonitrile (200 mℓ), and the resultant solution was washed three times with hexane (200 mℓ). After the solvent of the acetonitrile layer was distilled off under reduced pressure, column chromatography was conducted using silica gel (100 g). From ethyl acetate-hexane (1:3, V/V), the mixture of the title compounds was obtained as a pale yellow oil (12.84 g, 97% yield).

NMR δ ($CDCl_3$):

(as the mixture of the title compounds)

0.9-1.1 (3H,m), 1.45-1.8 (2H,m), [1.57 (s), 1.58 (s)](3H), [1.72 (s), 1.74 (s)](3H), 2.65 (1H,d,J=2Hz), [3.59 (dd, J=4Hz,9Hz), 3.65 (dd,J=4Hz,9Hz)](1H), 4.0-4.2 (1H,m), 4.4-4.6 (m; $CO_2CH_2CHCl_2$), 4.53 (s), 4.57 (s) (1H), 4.72 (d, J=12Hz; $CO_2CH_2CCl_3$), 4.85 (d,J=12Hz; $CO_2CH_2CHCCl_3$), [5.42 (d,J=5Hz), 5.48 (d,J=5Hz)] (1H), 5.87 (t,J=5Hz; $CO_2CH_2CHCl_2$).

Example 4

Synthesis of a mixture of 2,2,2-trichloroethyl (5R,6R)-6-(1-tert-butyldimethylsilyloxypropyl)-penicillanate and 2,2-dichloroethyl (5R,6R)-6-(1-tert-butyldimethylsilyloxypropyl)penicillanate

To a solution of a mixture (8.18 g, 0.022 mol) of 2,2,2-trichloroethyl (5R,6R)-6-(1-hydroxypropyl)-penicillanate and 2,2-dichloroethyl (5R,6R)-6-(1-hydroxypropyl)penicillanate in dry dimethylformamide (50 mℓ), t-butyldimethylchlorosilane (5.17 g, 0.034 mol) was added under an argon gas stream at room temperature, followed by the addition of triethylamine (4.16 mℓ, 0.030 mol) and 4-dimethylaminopyridine (small amount).

Fourteen hours later, the reaction mixture was poured into diethyl ether (250 mℓ) and then washed successively with a saturated aqueous solution of potassium hydrogensulfate (250 mℓ), a saturated aqueous solution of sodium hydrogencarbonate (250 mℓ) and a saturated aqueous solution of sodium chloride (250 mℓ). The organic layer was dried over anhydrous sodium sulfate, the solvent was distilled off under reduced pressure, and column chromatography was conducted using silica gel (125 g). From ethyl acetate-hexane (1:20, V/V), the mixture of the title compounds was obtained as a pale yellow oil (9.94 g, 93% yield).

NMR δ ($CDCl_3$):

(As the mixture of the title compounds)

0.06 (3H,s), 0.13 (3H,s), 0.8-1.0 (3H,m), 0.90 (9H,s), 1.4-1.65 (2H,m), [1.50 (s), 1.57 (s)](3H), [1.69 (s), 1.73 (s)] (3H), 3.65-3.8 (1H,m), 4.05-4.3 (1H,m), 4.4-4.6 (m; $CO_2$-$CH_2CHCl_2$), [4.48 (s), 4.52 (s)](1H), 4.71 (d,J=12Hz; $CO_2CH_2CCl_3$), 4.84 (d,J=12Hz; $CO_2CH_2CCl_3$), 5.25-5.45 (1H,m), 5.86 (t,J=5Hz; $CO_2CH_2CHCl_2$).

Example 5

Synthesis of a mixture of 2,2,2-trichloroethyl 2-[(3R,4R)-4-(benzothiazol-2-yldithio)-3-(1-tert-butyldimethylsilyloxypropyl)-2-oxoazetidin-1-yl]-3-methyl-3-butenoate and 2,2-dichloroethyl 2-[(3R,4R)-4-(benzothiazol-2-yldithio)-3-(1-tert-butyldimethylsilyloxypropyl)-2-oxoazetidin-1-yl]-3-methyl-3-butenoate

To a solution of a mixture (13.98 g, 0.029 mol) of 2,2,2-trichloroethyl (5R,6R)-6-(1-tert-butyl-dimethylsilyloxypropyl)penicillanate and 2,2-dichloroethyl (5R,6R)-6-(1-tert-butyldimethylsilyloxypropyl)-penicillanate in dry methylene chloride (55 mℓ), m-chloroperbenzoic acid (5.06 g, 0.029 mol) was added under an argon gas stream at 0°C.

Twenty minutes later, the reaction mixture was diluted with 250 mℓ of ethyl acetate and then washed successively twice with a saturated aqueous solution of sodium hydrogencarbonate (250 mℓ) and once with a saturated aqueous solution of sodium chloride (150 mℓ). The organic layer was dried over anhydrous sodium sulfate and the solvent was distilled off under reduced pressure, whereby a colorless oil was obtained. A solution of the thus-obtained residue in toluene (250 mℓ) was added at room temperature with 2-mercaptobenzothiazole (4.91 g, 0.029 mol), followed by heating under reflux for 2 hours and 30 minutes under an argon gas stream. The solvent in the reaction mixture was distilled off under reduced pressure and the residue was subjected to column chromatography by using silica gel (250 g), whereby a mixture of the title compounds was obtained (18.64 g, 99% yield).

NMR δ ($CDCl_3$):

(As the mixture of the title compounds)

0.05-0.25 (6H,m), 0.8-1.05 (12H,m), 1.75-2.2 (5H,m), 3.8-3.95 (1H,m), 4.2-4.3 (1H,m), 4.4-4.7 (m; $CO_2CH_2CHCl_2$), 4.7-4.9(m; $CO_2CH_2CCl_3$), 4.95-5.3 (3H,m), 5.4-5.55 (1H,m), 5.8-5.9 (m; $CO_2CH_2CHCl_2$), 7.3-7.5 (2H, m), 7.8-8.0 (2H,m).

Example 6

Synthesis of a mixture of 2,2,2-trichloroethyl 2-[(3R,4R)-4-(benzothiazol-2-yldithio)-3-(1-tert-butyldimethylsilyloxypropyl)-2-oxoazetidin-1-yl]-3-methyl-2-butenoate and 2,2-dichloroethyl 2-[(3R,4R)-4-(benzothiazol-2-yldithio)-3-(1-tert-butyldimethylsilyloxypropyl)-2-oxoazetidin-1-yl]-3-methyl-3-butenoate

To a solution of a mixture (19.29 g, 0.030 mol) of 2,2,2-trichloroethyl 2-[(3R,4R)-4-(benzothiazol-2-yldithio)-3-(1-tert-butyldimethylsilyloxypropyl)-2-oxoazetidin-1-yl]-3-methyl-3-butenoate and 2,2-dichloroethyl 2-[(3R,4R)-4-(benzothiazol-2-yldithio)-3-(1-tert-butyldimethylsilyloxypropyl)-2-oxoazetidin-1-yl]-3-methyl-3-butenoate in dry methylene chloride (60 mℓ), triethylamine (0.49 mℓ, 3.5 mmol) was added at room temperature.

One hour and 30 minutes later, the solvent in the reaction mixture was distilled off under reduced pressure and the residue was subjected to column chromatography by using silica gel (250 g). From ethyl acetate-hexane (1: 5, V/V), a mixture of the 2,2,2-trichloroethyl ester and 2,2-dichloroethyl ester as isomers with respect to the double bond was obtained as a dark yellow oil (16.96 g, 88% yield).

NMR δ ($CDCl_3$):

(As the mixture of the title compounds)

0.05-0.25 (6H,m), 0.8-1.1 (12H,m), 1.75-2.2 (8H,m), 3.85-3.9 (1H,m), 4.15-4.3 (1H,m), 4.45-4.9 (m; $CO_2CH_2CHCl_2$, $CO_2CH_2CCl_3$), 5.55-5.7 (1H,m), 5.7-5.8 (m; $CO_2CH_2CHCl_2$), 7.3-7.5 (2H,m), 7.75-7.95 (2H,m).

Example 7

Synthesis of 2-[(3R,4R)-3-(1-tert-butyldimethylsilyloxypropyl)-2-oxoazetidin-4-yldithio]benzothiazole

Through a solution of a mixture (2.95 g, 4.54 mmol) of 2,2,2-trichloroethyl 2-[(3R,4R)-4-(benzothiazol-2-yldithio)-3-(1-tert-butyldimethylsilyloxypropyl)-2-oxoazetidin-1-yl]-3-methyl-2-butenoate and 2,2-dichloroethyl 2-[(3R,4R)-4-(benzothiazol-2-yl-dithio)-3-(1-tert-butyldimethylsilyloxypropyl)-2-oxoazetidin-1-yl]-3-methyl-2-butenoate in ethyl acetate (120 mℓ), ozone was caused to bubble at -78°C. One hour and forty minutes later, the bubbling was finished, followed by the addition of dimethyl sulfide (10 mℓ, 0.136 mol). The solvent in the reaction mixture was distilled off under reduced pressure, whereby the imide derivatives were obtained as a yellow oil.

Silica gel (20 g) and water (7.5 mℓ) were added at room temperature to a solution of the imide derivatives in methanol (75 mℓ). One hour and thirty minutes later, the silica gel in the reaction mixture was filtered off, the solvent in the reaction mixture was distilled off under reduced pressure, and the residue was subjected to column chromatography by using silica gel (100 g). From fractions eluted with ethyl acetate-hexane (1:2, V/V), a mixture of isomers containing the target (S)-silyloxypropyl isomer was obtained as a white solid [0.80g, 40% yield; (S)-silyloxypropyl isomer:(R)-silyloxypropyl isomer = 1:2].

NMR δ ($CDCl_3$):

(As the mixture of the 1'S isomer and 1'R isomers))

[0.14 (s), 0.16 (s)](3H,s), [0.16 (s), 0.19 (s)](3H), 0.95 (9H,s), [0.98 (t,J=7Hz), 0.99 (t,J=7Hz)](3H), 1.7-2.1 (2H, m), 3.7-3.85 (1H,m), 4.1-4.25 (1H,m), [5.12 (d,J=5Hz), 5.18 (d,J=5Hz)](1H), [6.54 (bs), 6.70 (bs)](1H), 7.3-7.5 (2H,m), 7.75-7.95 (2H,m).

Example 8

Synthesis of allyl (5R,6R)-6-((S)-1-tertbutyldimethylsilyloxypropyl)-2-methylthiopenem-3-carboxylate

To a solution of 2-[(3R,4R)-3-(1-tert-butyl-dimethylsilyloxypropyl)-2-oxoazetidin-4-yldithio]benzothiazole of Example 7 [0.80 g, 1.82 mmol; (S)-silyloxypropyl isomer: (R)-silyloxypropyl isomer = 1:2] in dry methylene chloride (8.7 mℓ), allyl oxalyl chloride (315 μℓ, 2.63 mmol) and triethylamine (330 μℓ, 2.36 mmol) were added under an argon gas stream at 0°C.

Fifteen minutes later, the reaction mixture was diluted with ethyl acetate, followed by successive washing with water, a saturated aqueous solution of sodium hydrogencarbonate and a saturated aqueous solution of sodium chloride. The organic layer was dried over anhydrous sodium sulfate and the solvent was distilled off under reduced pressure, whereby the imide derivative was obtained as a bluish brown oil.

To a suspension of methylthiomethylenetriphenylphosphonium chloride (1.61 g, 4.5 mmol) in dry tetrahydrofuran (79 mℓ), a 1.6 N hexane solution of n-butyllithium (2.28 mℓ, 3.65 mmol) was added under an argon gas stream at room temperature. Twenty-five minutes later, the resultant solution was cooled to -25°C, to which a solution of the above-obtained imide derivative in distilled tetrahydrofuran (40 mℓ) was added. Three hours later, the solvent in the reaction mixture was distilled off under reduced pressure and the resulting residue was dissolved in ethyl acetate. The solution so obtained was then washed with water.

The organic layer was dried over anhydrous sodium sulfate, the solvent was distilled off under reduced pressure, and column chromatography was then conducted using silica gel. Obtained were a mixture of allyl (5R,6R)-6-((S)-1-tert-butyldimethylsilyloxypropyl)-2-methylthiopenem-3-carboxylate and allyl (5R,6R)-6-((R)-1-tert-butyldimethylsilyloxypropyl)-2-methylthiopenem-3-carboxylate as a pale yellow oil (416.3 mg, 53% yield; (S)-silyloxypropyl isomer:(R)-silyloxypropyl isomer = 1:1) and allyl (5R,6R)-6-((R)-1-tert-butyldimethylsilyloxypropyl)-2-methylthiopenem-3-carboxylate as a pale yellow oil (189.0 mg, 24% yield).

NMR δ ($CDCl_3$):

((S)-silyloxypropyl isomer)

0.11 (6H,s), 0.88 (9H,s), 0.98 (3H,t,J=7Hz), 1.7-1.9 (2H,m), 2.55 (3H,s), 4.06 (1H,dd,J=4Hz,9Hz), 4.24 (1H,dt, J=4Hz,9Hz), 4.3-4.4 (1H,m), 4.6-4.85 (2H,m), 5.24 (1H,dd,J=1Hz,11Hz), 5.42 (1H,dd,J=1Hz,17Hz), 5.71 (1H,d,J=4Hz), 5.85-6.05 (1H,m).

Example 9

Synthesis of allyl (5R,6R)-6-((S)-1-tert-butyldimethylsilyloxypropyl)-2-methylsulfinylpenem-3-carboxylate

To a solution of allyl (5R,6R)-6-((S)-1-tertbutyldimethylsilyloxypropyl)-2-methylthiopenem-3-carboxylate (203 mg, 0.47 mmol) in dry methylene chloride (14 mℓ), m-chloroperbenzoic acid (91 mg, 0.53 mmol) was added under an argon gas stream at -45°C.

One hour later, the reaction mixture was poured into ethyl acetate (100 mℓ), followed by successive washing with a saturated aqueous solution of sodium hydrogencarbonate (50 mℓ) and a saturated aqueous solution of sodium chloride (50 mℓ). The organic layer was dried over anhydrous sodium sulfate, the solvent was distilled off under reduced pressure, and column chromatography was conducted using silica gel (10 g), whereby the title compound was obtained as a pale yellow oil (148 mg, 70% yield).

NMR δ ($CDCl_3$):

(As a mixture of isomers with respect to the sulfoxide)

0.13 (6H,s), 0.87 (9H,s), 0.99 (3H,t,J=7Hz), 1.7-1.9 (2H,m), [2.95 (s), 2.97 (s)](3H), 4.1-4.2 (1H,m), 4.3-4.45 (1H, m), 4.6-4.85 (2H,m), 5.30 (1H,dd,J=1Hz,11Hz), 5.42 (1H,dd,J=1Hz,17Hz), [5.76 (d,J=4Hz), 5.91 (d,J=4Hz)](1H), 5.8-6.0 (1H,m).

Example 10

Synthesis of allyl (5R,6R)-2-(1-allyloxycarbonyl-3-pyrrolidine)thio-6-((S)-1-tert-butyldimethylsilyloxypropyl)penem-3-carboxylate

To a solution of allyl (5R,6R)-6-((S)-1-tert-butyldimethylsilyloxypropyl)-2-methylsulfinylpenem-3-carboxylate (148 mg, 0.33 mmol) in dry dimethylformamide (10 mℓ), a solution of diisopropylethylamine (65 μℓ, 0.37 mmol) in dry dimethylforamide (5 mℓ) and a solution of 1-allyloxycarbonyl 3-mercaptopyrrolidine (103 mg, 0.55 mmol) in dry dimethylformamide (5 mℓ) were added under an argon gas stream at -45°C.

Fifteen minutes later, the reaction mixture was diluted in ethyl acetate (200 mℓ), followed by successive washing with a saturated aqueous solution of potassium hydrogensulfate (100 mℓ), water (100 mℓ), a saturated aqueous solution of sodium hydrogencarbonate (100 mℓ) and a saturated aqueous solution of sodium chloride (100 mℓ). The organic layer was dried over anhydrous sodium sulfate, the solvent was distilled off under reduced pressure, and column chromatography was conducted using silica gel (10 g). Using ethyl acetate-hexane (1:4, V/V), one (A) of the isomers of allyl (5R,6R)-2-(1-allyloxycarbonyl-3-pyrrolidine)thio-6-((S)-1-tert-butyldimethylsilyloxypropyl)penem-3-carboxylate was obtained as a pale yellow oil from fractions of higher polarity (95.1 mg, 50% yield).

From fractions of lower polarity, the other isomer (B) of allyl (5R,6R)-2-(1-allyloxycarbonyl-3-pyrrolidine)thio-6-((S)-1-tert-butyldimethylsilyloxypropyl)penem-3-carboxylate was obtained as a pale yellow oil (49.5 mg, 26% yield).

NMR δ ($CDCl_3$):

(Isomer A)

0.11 (3H,s), 0.12 (3H,s), 0.88 (9H,s), 0.98 (3H,t,J=7Hz), 1.7-1.9 (1H,m), 1.9-2.15 (1H,m), 2.15-2.3 (1H,m), 2.3-2.5 (1H,m), 3.4-3.7 (3H,m), 3.7-3.8 (1H,m), 3.8-3.95 (1H,m), 4.05-4.2 (1H,m), 4.3-4.4 (1H,m), 4.5-4.9 (4H,m), 5.15-5.5 (4H,m), 5.73 (1H,d,J=9Hz), 5.85-6.05 (2H,m).

NMR δ ($CDCl_3$):

(Isomer B)

0.12 (6H,s), 0.88 (9H,s), 0.98 (3H,t,J=7Hz), 1.7-1.95 (2H,m), 1.95-2.2 (1H,m), 2.2-2.5 (1H,m), 3.4-3.7 (3H,m), 3.8-3.95 (2H,m), 4.05-4.15 (1H,m), 4.3-4.4 (1H,m), 4.55-4.85 (4H,m), 5.2-5.5 (4H,m), 5.72 (1H,d,J=5Hz), 5.85-6.05 (2H,m).

Example 11

Synthesis of allyl (5R,6R)-2-(1-allyloxycarbonyl-4-piperidine) thio-6-((S)-1-tert-butyldimethylsilyloxypropyl)penem-3-carboxylate

From a mixture (146 mg, 0.33 mmol) of allyl (5R,6R)-2-methylsulfinyl-6-((S)-1-tert-butyldimethylsilyloxypropyl) penem-3-carboxylate and allyl (5R,6R)-2-methylsulfinyl-6-((R)-1-tert-butyldimethylsilyloxypropyl)penem-3-carboxylate [(S)-silyloxypropyl isomer: (R)-silyloxypropyl isomer = 4:3], the penem(2-piperidinethio derivative) of the (S)-silyloxypropyl isomer and the penem(2-piperidinethio derivative) of the (R)-silyloxypropyl isomer were obtained as a pale yellow oil (102.8 mg, 54% yield) and as a pale yellow oil (49.6 mg, 26% yield), respectively, by causing 1-allyloxycarbonyl 4-mercaptopiperidine to act in a similar manner as in Example 10.

NMR δ ($CDCl_3$):

((S)-silyloxypropyl isomer)

0.11 (3H,s), 0.11 (3H,s), 0.88 (9H,s), 0.97 (3H,t,J=7Hz), 1.45-1.75 (2H,m), 1.75-1.9 (1H,m), 1.9-2.2 (2H,m), 2.85-3.15 (2H,m), 3.3-3.45 (1H,m), 4.0-4.2 (3H,m), 4.3-4.4 (1H,m), 4.55-4.85 (4H,m), 5.15-5.45 (4H,m), 5.69 (1H,d, J=4Hz), 5.85-6.05 (2H,m).

Example 12

Synthesis of allyl (5R,6R)-6-((S)-1-hydroxypropyl)-2-methylthiopenem-3-carboxylate

To a solution of allyl (5R,6R)-6-((S)-1-tert-buthyldimethylsilyloxypropyl)-2-methylthiopenem-3-carboxylate in dry tetrahydrofuran (60 µl), acetic acid (31 µl, 0.54 mmol) and a 1 M tetrahydrofuran solution of tetra-n-butylammonium fluoride (0.27 ml, 0.27 mmol) were added under an argon gas stream at room temperature.

Twenty-one hours later, ethyl acetate (50 ml) was poured into the reaction mixture, followed by successive washing with a saturated aqueous solution of potassium hydrogensulfate (25 ml), water (25 ml), a saturated aqueous solution of sodium hydrogencarbonate (25 ml) and a saturated aqueous solution of sodium chloride (25 ml). The organic layer was dried over anhydrous sodium sulfate, the solvent was distilled off under reduced pressure, and column chromatography was conducted using silica gel (10 g). From ethyl acetate-hexane (1:2, V/V), the title compound was obtained as a colorless oil (21.4 mg, 74% yield).

NMR δ ($CDCl_3$) :

1.04 (3H,t,J=7Hz), 1.5-1.7 (2H,m), 1.79 (1H,d,J=5Hz), 1.9-2.1 (1H,m), 2.55 (3H,s), 3.90 (1H,dd,J=4Hz,11Hz), 4.0-4.2 (1H,m), 4.6-4.9 (2H,m), 5.24 (1H,dd,J=1Hz,11Hz), 5.41 (1H,dd,J=1Hz,17Hz), 5.73 (1H,d,J=4Hz), 5.85-6.05 (1H,m).

Example 13

Synthesis of allyl (5R,6R)-2-(1-allyloxycarbonyl-3-pyrrolidine)thio-6-((S)-1-hydroxypropyl)penem-3-carboxylate (isomer A)

From allyl (5R,6R)-2-(1-allyloxycarbonyl-3-pyrrolidine)thio-6-((S)-1-tert-butyldimethylsilyloxy-propyl)penem-3-carboxylate (the isomer A obtained in Example 10) (95.1 mg, 0.17 mmol), the title compound was obtained as a colorless oil in a similar manner as in Example 12 (44.9 mg, 59% yield).

NMR δ ($CDCl_3$):

1.04 (3H,t,J=7Hz), 1.5-1.7 (2H,m), 1.80 (1H,d,J=2Hz), 1.9-2.2 (1H,m), 2.3-2.5 (1H,m), 3.4-3.7 (3H,m), 3.92 (1H, dd,J=4Hz,10Hz), 3.8-4.0 (2H,m), 4.0-4.2 (1H,m), 4.5-4.85 (4H,m), 5.15-5.5 (4H,m), 5.76 (1H,d,J=4Hz), 5.85-6.05 (2H, m).

Example 14

Synthesis of allyl (5R,6R)-2-(1-allyloxycarbonyl-3-pyrrolidine)thio-6-((S)-1-hydroxypropyl)penem-3-carboxylate (isomer B)

From allyl (5R,6R)-2-(1-allyloxycarbonyl-3-pyrrolidine)thio-6-((S)-1-tert-butyldimethylsilyloxy-propyl)penem-3-carboxylate (the isomer B obtained in Example 10) (49.5 mg, 0.09 mmol), the title compound was obtained as a colorless oil (34.4 mg, 87% yield) in a similar manner as in Example 12.

NMR δ ($CDCl_3$):

1.06 (3H,t,J=7Hz), 1.45-1.65 (2H,m), 1.65-1.8 (1H,bs), 1.9-2.2 (1H,m), 2.3-2.5 (1H,m), 3.4-3.7 (3H,m), 3.8-4.0 (3H,m), 3.8-4.0 (3H,m), 4.0-4.2 (1H,m), 4.5-4.85 (4H,m), 5.15-5.5 (4H,m), 5.76 (1H,d,J=5Hz), 5.85-6.05 (2H,m).

Example 15

Synthesis of allyl (5R,6R)-2-(1-allyloxycarbonyl-4-pyridine)thio-6-((S)-1-hydroxypropyl)penem-3-carboxylate

From allyl (5R,6R)-2-(1-allyloxycarbonyl-4-piperidine)thio-6-((S)-1-tert-butyldimethylsilyloxy-propyl)penem-3-carboxylate (101 mg, 0.17 mmol), the title compound was obtained as a pale yellow oil (36.9 mg, 45% yield) in a similar manner as in Example 12.

NMR δ ($CDCl_3$) :

1.05 (3H,t,J=7Hz), 1.5-1.8 (3H,m), 1.82 (1H,d,J=6Hz), 1.9-2.25 (3H,m), 2.9-3.2 (2H,m), 3.3-3.45 (1H,m), 3.92

(1H,dd,J=11Hz), 3.95-4.2 (3H,m), 4.5-4.85 (4H,m), 5.15-5.5 (4H,m), 5.73 (1H,d,J=4Hz), 5.85-6.05 (2H,m).

Example 16

Synthesis of (5R,6R)-6-((S)-1-hydroxypropyl)-2-methylthiopenem-3-carboxylic acid

To a solution of allyl (5R,6R)-6-((S)-1-hydroxypropyl)-2-methylthiopenem-3-carboxylate (33.4 mg, 0.11 mmol) in a mixed solvent of dry methylene chloride (150 μℓ) and dry ethyl acetate (450 μℓ), sodium 2-ethylhexanoate (20.4 mg, 0.12 mmol), tetrakistriphenylphosphine palladium (11.2 mg, 0.01 mmol) and triphenylphosphine (12.8 mg, 0.05 mmol) were added under an argon gas stream at room temperature.

Twenty-five minutes later, the solvent in the reaction mixture was distilled off under reduced pressure and the residue was dissolved in water (3 mℓ). The solution was then washed with diethyl ether (4 mℓ). The water layer was filtered and then purified by HPLC, whereby the title compound was obtained as a white solid (8.9 mg, 30% yield).

NMR δ ($CDCl_3$):

1.06 (3H,t,J=7Hz), 1.5-1.7 (1H,m), 1.9-2.1 (1H,m), 2.56 (3H,s), 3.92 (1H,dd,J=4Hz,6Hz), 4.05-4.2 (1H,m), 5.75 (1H,d,J=4Hz).

IR $\nu_{max}$(NaCl):

1770, 1683 $cm^{-1}$.

Example 17

Synthesis of (5R,6R)-2-(1-allyl-3-pyrrolidine)-thio-6-((S)-1-hydroxypropyl)penem-3-carboxylic acid (isomer A)

To a solution of allyl (5R,6R)-2-(1-allyloxycarbonyl-3-pyrrolidine)thio-6-((S)-1-hydroxypropyl)penem-3-carboxylate (the isomer A obtained in Example 13) (44.9 mg, 0.10 mmol) in a mixed solvent of dry methylene chloride (350 μℓ) and dry tetrahydrofuran (350 μℓ), acetic acid (31 μℓ, 0.54 mmol), tetrakistriphenylphosphine palladium (26.4 mg, 0.02 mmol) and triphenylphosphine (14.8 mg, 0.06 mmol) were added under an argon gas stream at room temperature. Thirty-five minutes later, the solvent in the reaction mixture was distilled off under reduced pressure and the residue was dissolved in water (3 mℓ). The solution was then washed with diethyl ether (5 mℓ).

The water layer was filtered and then purified by ODS-HPLC, whereby the title compound was obtained as a white solid (22.4 mg, 61% yield).

NMR δ ($CD_3OD$) :

1.03 (3H,t,J=7Hz), 1.4-1.6 (1H,m), 1.8-2.1 (2H,m), 2.45-2.65 (1H,m), 3.1-3.5 (3H,m), 3.5-3.6 (1H,m), 3.71 (2H, d,J=7Hz), 3.85-4.1 (3H,m), 5.4-5.6 (2H,m), 5.70 (1H,d,J=3Hz), 5.85-6.05 (1H,m).

IR $\nu_{max}$(NaCl) :

1770, 1590, 1379 $cm^{-1}$.

Example 18

Synthesis of (5R,6R)-2-(1-allyl-3-pyrrolidine)-thio-6-((S)-1-hydroxypropyl)penem-3-carboxylic acid (isomer B)

From allyl (5R,6R)-2-(1-allyloxycarbonyl-3-pyrrolidine)thio-6-((S)-1-hydroxypropyl)penem-3-carboxylate (the isomer B obtained in Example 14) (34.4 mg, 0.08 mmol), the title compound was obtained as a white solid (17.2 mg, 61% yield) in a similar manner as in Example 17.

NMR δ ($CD_3OD$) :

1.03 (3H,t,J=7Hz), 1.4-1.6 (1H,m), 1.8-2.15 (2H,m), 2.45-2.7 (1H,m), 3.1-3.4 (2H,m), 3.4-3.6 (2H,m), 3.6-3.85 (2H,m), 3.85-4.1 (3H,m), 5.4-5.6 (2H,m), 5.77 (1H,d,J=4Hz), 5.85-6.05 (1H,m).

IR $\nu_{max}$(NaCl):

1768, 1590, 1376 $cm^{-1}$.

Example 19

Synthesis of (5R,6R)-2-(1-allyl-4-piperidine)-thio-6-((S)-1-hydroxypropyl)penem-3-carboxylic acid

From allyl (5R,6R)-2-(1-allyloxycarbonyl-4-piperidine)thio-6-((S)-1-hydroxypropyl)penem-3-carboxylate (36.9 mg, 0.08 mmol), the title compound was obtained as a white solid (20.0 mg, 66% yield) in a similar manner as in Example 17.

NMR δ ($CD_3OD$):

1.03 (3H,t,J=7Hz), 1.4-1.6 (1H,m), 1.8-2.15 (3H,m), 2.15-2.3 (1H,m), 2.3-2.45 (1H,m), 2.8-2.95 (2H,m), 3.3-3.5 (3H,m), 3.5-3.7 (2H,m), 3.8-4.0 (2H,m), 5.48 (1H,s), 5.53 (1H,d,J=6Hz), 5.70 (1H,d,J=3Hz), 5.95-6.15 (1H,m).
IR $\nu_{max}$(NaCl):
1766, 1585, 1378 $cm^{-1}$.

Example 20

Synthesis of 2,2-dichloroethyl 2-((3R,4R)-4-benzoylthio-3-(1-tert-butyldimethylsilyloxy-propyl)-2-oxoazetidin-1-yl)-3-methyl-2-butenoate

To a solution of 2,2-dichloroethyl (5R,6R)-6-(1-tert-butyldimethylsilyloxypropyl)penicillanate (467 mg, 1 mmol) in acetonitrile (4 mℓ), AgCl (160 mg) and diazabicycloundecene (180 μℓ) were successively added at room temperature, followed by stirring for 80 minutes. The resultant solution was added with benzoyl chloride (260 μℓ), immediately followed by dilution with ethyl ether. The organic layer was washed successively with a saturated aqueous solution of sodium hydrogencarbonate and brine.

The organic layer was dried over anhydrous sodium sulfate and the solvent was then distilled off under reduced pressure. The residue so obtained was purified by flash chromatography, whereby a mixture of the (1'R) isomer and the (1'S) isomer was obtained (482 mg).
NMR δ ($CDCl_3$):
(As the mixture of the two isomers)
[0.112 (s), 0.15 (s), 0.16 (s), 0.22 (s)](6H), 0.9-1.0 (m,3H), [0.94 (s), 0.97 (s)](9H), 2.02 (s,3H), [2.19 (s), 2.20 (s)](3H), 3.90-3.97 (m,1H), [4.0-4.08 (m), 4.2-4.3 (m)](1H), 4.45-4.55 (m,1H), 4.75-4.85 (m,1H), 6.08 (t,J=6Hz,1H), 6.17 (d,J=6Hz,1H), 7.4-7.5 (m,2H), 7.55-7.65 (m,1H), 7.90-7.93 (m,2H).

Example 21

Synthesis of (3R,4R)-4-benzoylthio-3-(1-tert-butyldimethylsilyloxy)propyl-azetidin-2-one

Into a solution of the compound obtained in Example 20 (482 mg, 0.84 mmol) in ethyl acetate (20 mℓ), ozone was blown at -78°C for 20 minutes. After dimethyl sulfide (2 mℓ) was added at the same temperature, the temperature of the resultant mixture was allowed to rise to room temperature. After the solvent was distilled off under reduced pressure, methanol (12 mℓ), silica gel (3 g) and water (1.2 g) were added, followed by stirring for 30 minutes. Insoluble matter was filtered off and the solvent was then distilled off. The residue was purified by flash column chromatography, whereby the title compound was obtained (293 mg, 93% yield).
NMR δ ($CDCl_3$):
(As a mixture of two isomers)
0.25-0.20 (m,6H), [0.94 (s), 0.95 (s)](1H), 0.95-0.98 (t,3H), 7.45-7.5 (m,2H), 7.55-7.65 (m,1H), 7.90-7.95 (m,2H).

Example 22

Synthesis of allyl (5R,6R)-6-(1-tert-butyl-dimethylsilyloxy)propyl-2-phenylpenem-3-carboxylate

To a solution of the compound obtained in Example 21 (293 mg, 0.77 mmol) in methylene chloride (3 mℓ), allyloxalyl chloride (171 mg) and triethylamine (160. μℓ) were successively added dropwise. After the resultant mixture was stirred for 10 minutes, the organic layer was washed successively with a saturated aqueous solution of potassium hydrogensulfate, a saturated aqueous solution of sodium hydrogencarbonate and brine.

After the solvent was distilled off under reduced pressure, the residue so obtained was dissolved in xylene (1.2 mℓ), to which triethyl phosphite (396 μℓ) was added. The resultant mixture was heated at 80°C for 2 hours. Subsequent to dilution with xylene (54 mℓ), the thus-obtained mixture was heated under reflux for 2 hours and 30 minutes. After the solvent was distilled off, the residue was purified by flash column chromatography, whereby the title compound was obtained (187 mg, 53%).
NMR δ ($CDCl_3$):
(As a mixture of two isomers)
0.08-0.15 (m,6H), 0.15-1.05 (m,12H), 1.55-1.90 (m,2H), 3.85-4.15 (m,1H), 4.35-4.7 (m,3H), 5.1-5.2 (2H), [5.66 (d,J=5Hz), 5.80 (d,J=4Hz)](1H), 5.7-5.9 (m,1H), 7.35-7.52 (m,5H).

Example 23

Synthesis of allyl (5R,6R)-6-((S)-1-hydroxy-propyl)-2-phenylpenem-3-carboxylate

To a solution of the compound obtained in Example 22 (187 mg, 0.41 mmol) in THF (0.5 mℓ), acetic acid (100 μℓ) and tetra-n-butylammonium fluoride (1 M THF solution) (0.66 mℓ) were successively added dropwise at room temperature. After the resultant mixture was stirred for 20 hours, the reaction mixture was washed successively with a saturated aqueous solution of potassium hydrogensulfate, a saturated aqueous solution of sodium hydrogencarbonate and brine.

After the solvent was distilled off under reduced pressure, the residue was purified by flash column chromatography, whereby a fraction composed primarily of the title compound was obtained (15 mg, 10%).
NMR δ ($CDCl_3$):
1.05 (t,J=7Hz,3H), 1.5-1.7 (m,1H), 1.95-2.1 (m,1H), 3.95 (dd,J=10Hz,4Hz,1H), 4.21 (br.t,1H), 4.5-4.7 (m,2H), 5.1-5.25 (m,2H), 5.7-5.85 (m,1H), 5.80 (d,J=5Hz,1H), 7.35-7.5 (m,5H).

Example 24

Synthesis of (5R,6R)-6-((S)-1-hydroxypropyl)-2-phenylpenem-3-carboxylic acid

To a solution of allyl (5R,6R)-6-((S)-1-hydroxy-propyl)-2-phenylpenem-3-carboxylate (15 mg) in a mixed solvent of ethyl acetate (210 μℓ) and methylene chloride (70 μℓ) , triphenylphosphine (2 mg), sodium 2-ethylhexanoate (7 mg) and tetrakistriphenylphosphine palladium (3 mg) were added, followed by stirring for 3 hours. After the reaction mixture was diluted with water (4 mℓ), the water layer was washed twice with ethyl ether and the resulting water layer was purified by HPLC, whereby the title compound was obtained (5 mg, 33% yield).
NMR δ ($CD_3OD$):
1.00 (t,J=7Hz,3H), 1.52-1.63 (m,1H), 1.85-1.95 (m,1H), 4.05-4.15 (m,2H), 5.88 (d,J=4Hz,1H), 7.35-7.55 (m,5H).
IR(KBr):
1774 $cm^{-1}$.

Example 25

Synthesis of 2,2-dichloroethyl 2-((3R,4R)-4-(p-bromomethylbenzoylthio)-3-(1-tert-butyldimethylsilyloxypropyl-2-oxoazetidin-1-yl)-3-methyl-2-butenoate

Using 2,2-dichloroethyl (5R,6R)-6-(1-tert-butyldimethylsilyloxypropyl)penicillanate (1868 mg, 4 mmol) and, p-bromomethylbenzoic acid bromide as an acid chloride, a reaction was conducted in a similar manner as in Example 20, whereby the title compound was obtained (1.78 g, 80%).
NMR δ ($CDCl_3$):
(As a mixture of two isomers)
[0.11 (s), 0.15 (s), 0.16 (s), 0.22 (s)](6H), 0.85-1.0 (m,12H), 1.65-1.9 (m,2H), 2.0 (s,3H), 2.2 (s,3H), 3.9-4.05 (m, 1H), 4.5 (s,2H), 4.75-4.85 (m,1H), 6.07 (t,J=6Hz,1H), 6.15 (d,J=6Hz,1H), 7.48 (d,J=6Hz,2H), 7.90 (d,J=6Hz,2H).

Example 26

Synthesis of (3R,4R)-4-(p-bromomethyl)benzoyl-thio-3-(1-tert-butyldimethylsilyloxy)propylazetidin-2-one

Using the compound obtained in Example 25 (1780 mg, 2.69 mmol), a reaction was conducted in a similar manner as in Example 21, whereby the title compound was obtained (0.977 g, 76% yield).
NMR δ ($CDCl_3$):
(As a mixture of two isomers)
[0.83 (s), 0.88 (s), 0.14 (s)](6H), 0.85-0.93 (m,12H), 1.6-1.9 (m,2H), [3.75-3.95 (m), 3.92-3.97 (m)](1H), 4.45 (s, 2H), 5.62-5.65 (m,1H), [6.1 (br.s), 6.15 (br.s)](1H), 7.45 (d,J=7Hz,2H), 7.86 (d,J=7Hz,2H).

Example 27

Synthesis of allyl (5R,6R)-2-(p-bromomethylphenyl)-6-((S)-1-tert-butyldimethylsilyloxypropyl)penem-3-carboxylate

Using the compound obtained in Example 26 (646 mg, 1.35 mmol), a reaction was conducted in a similar

manner as in Example 22, whereby a fraction composed primarily of the title compound was obtained (154 mg, 19% yield).

NMR δ ($CDCl_3$):

0.99-0.11 (m,6H), 0.91 (s,9H), 0.99 (t,J=6Hz,3H), 1.6-1.9 (m,2H), 4.12 (d,J=7Hz,4Hz), 4.5 (s,2H), 4.55-4.7 (m, 1H), 4.76-4.9 (m,2H), 5.1-5.25 (m,2H), 5.6-5.9 (m,1H), 5.78 (d,J=4Hz,1H), 7.35-7.5 (m,4H).

Example 28

Synthesis of allyl (5R,6R)-2-(p-acetoxymethylphenyl)-6-((S)-1-hydroxypropyl)penem-3-carboxylate

Using a fraction composed primarily of allyl (5R,6R)-2-(p-bromomethylphenyl)-6-((S)-1-tert-butyldimethylsilyloxypropyl)penem-3-carboxylate (35 mg, 0.06 mmol), a reaction was conducted in a similar manner as in Example 23, whereby the title compound was obtained (10 mg, 38% yield).

NMR δ ($CDCl_3$):

1.06 (t,J=7Hz,3H), 1.65-1.8 (m,1H), 1.9-2.05 (m,1H), 2.1 (s,3H), 3.95 (dd,J=7Hz,4Hz,1H), 4.15-4.3 (br.t,1H), 4.5-4.7 (m,2H), 5.1 (s,2H), 5.15-5.3 (m,2H), 5.7-5.9 (m,1H), 5.80 (d,J=4Hz,1H), 7.35-7.5 (m,4H).

Example 29

Synthesis of (5R,6R)-2-(p-acetoxymethylphenyl)-6-((S)-1-hydroxypropyl)penem-3-carboxylic acid

Using the compound obtained in Example 28 (10 mg, 0.02 mmol), a reaction was conducted in a similar manner as in Example 24, whereby the title compound was obtained (2.5 mg, 25% yield).

NMR d ($CD_3OD$) :

1.02 (t,J=7Hz,3H), 1.5-1.65 (m,1H), 1.8-2.0 (m,1H), 2.12 (s,3H), 4.0-4.15 (m,2H), 5.13 (s,2H), 5.86 (d,J=4Hz, 1H), 7.43 (dd,J=27Hz,8Hz,4H).

Example 30

Synthesis of (5R,6R)-6-((S)-1-hydroxypropyl)-2-(p-pyridiniummethylphenyl)penem-3-carboxylic acid

To a solution of allyl (5R,6R)-2-(p-bromomethylphenyl)-6-((S)-1-tert-butyldimethylsilyloxypropyl)penem-3-carboxylate (320 mg, 0.54 mmol) in DMF (500 μl), pyridine (160 μl) was added at room temperature, followed by stirring for 2 hours at the same temperature. After the solvent was distilled off under reduced pressure, the residue was dissolved in THF (0.5 μl), followed by the successive dropwise addition of tetrabutylammonium fluoride (1 M THF solution, 0.88 ml) and acetic acid (130 μl) at room temperature.

After the resultant mixture was stirred for 20 hours, the reaction mixture was diluted with water (3 ml) and the water layer was washed twice with ethyl ether. The water layer so obtained was purified by HPLC.

After lyophilization, a THF solution of the resultant residue was added with triphenylphosphine (25 mg), acetic acid (250 μℓ) and tetrakistriphenylphosphine palladium (30 mg), followed by stirring for 30 minutes. After the solvent was distilled off, the residue was diluted with water (3 mℓ) and the water layer was washed twice with ethyl ether. The thus-obtained water layer was then purified by HPLC, whereby the title compound was obtained (48 mg, 22% yield).

NMR δ ($D_2O$) :

1.01 (t,J=7Hz,3H), 1.5-1.7 (m,1H), 1.85-2.0 (m,1H), 4.04 (dd,J=10Hz,4Hz,1H), 4.15-4.25 (m,1H), 5.84 (s,2H), 5.89 (d,J=4Hz,1H), 7.48 (q,J=9Hz,4H), 8.08 (t,J=6Hz,2H), 8.57 (t,J=6Hz,1H), 8.9 (d,J=6Hz,2H).

IR (KBr):

1768, 1604 $cm^{-1}$.

Example 31

Synthesis of (1'R,3S,4R and 1'S,3R,4S)-3-(1'-tert-butyldimethylsilyloxypropyl)-4-phenyl-thioazetidinones

To a solution of chlorosulfonylisocyanate (9.87 mℓ) in diethyl ether (174 mℓ), a solution of 3-tert-butyldimethylsilyloxy-1-phenylthio-1-pentene (23.45 g) in diethyl ether (46 mℓ) was added under an argon gas atmosphere at room temperature, followed by stirring at the same temperature for 4 hours. The reaction mixture was cooled to -50°C, followed by the addition of thiophenol (19.3 mℓ) and then of pyridine (15.2 mℓ). The resultant mixture was stirred at -20°C for 30 minutes. The reaction mixture was diluted with ethyl acetate. The thus-obtained solution was washed with a saturated aqueous solution of potassium hydrogensulfate, a saturated aqueous solution of sodium hydrogencarbonate and a saturated aqueous solution of sodium sulfate, and was then dried over anhydrous sodium sulfate.

The solvent was distilled off under reduced pressure and the residue was purified by silica gel chromatography, whereby a mixture (5:2) of (1'R,3S,4R and 1'S,3R,4S)-3-(1'-tert-butyldimethylsilyloxypropyl)-4-phenylthioazetidinone and (1'S,3S,4R and 1'R,3R,4S)-3-(1'-tert-butyldimethylsilyloxypropyl)-4-phenylthioazetidinone was obtained (7.67 g, 29% yield). From this mixture, the title compounds were obtained by recrystallization.

IR (KBr):

3158, 1762 $cm^{-1}$.

NMR δ ($CDCl_3$):

7.42-7.52 (2H,m), 7.33-7.42 (3H,m), 6.03 (1H,s) 5.09 (1H,d,J=2.6Hz), 4.00-4.08 (1H,m), 3.14 (1H,t,J=2.6Hz), 1.45-1.67 (2H,m), 0.87 (9H,s), 0.06 (3H,s), 0.05 (3H,s).

Example 32

Synthesis of (1'R,3R,4R and 1'S,3S,4S)-3-(1'-tert-butyldimethylsilyloxypropyl)-4-acetoxyazetidinone

(1'R,3S,4R and 1'S,3R,4S)-3-(1'-tert-butyldimethylsilyloxypropyl)-4-phenylthioazetidinone (4.92 g, 14 mmol) was dissolved in acetic acid (30 mℓ), to which cupric acetate monohydrate (2.00 g, 10 mmol) was added at room temperature. The resultant mixture was heated at 100°C for 75 minutes. The reaction mixture was filtered through "Celite" and the solvent was then distilled off.

The residue was diluted with ethyl acetate. The resultant solution was washed with water, a saturated aqueous solution of sodium hydrogencarbonate and a saturated aqueous solution of sodium sulfate, and was then dried over anhydrous magnesium sulfate. The solvent was distilled off under reduced pressure and the residue was purified by silica gel chromatography, whereby the title compound was obtained as colorless crystals (3.37 g, 80% yield).

IR (KBr):

3170, 1780, 1748$cm^{-1}$.

NMR δ ($CDCl_3$):

6.46 (1H,bs), 5.84 (1H,s), 4.01-4.10 (1H,m), 3.31 (1H,t,J=4.3Hz), 2.11 (3H,s), 1.53-1.68 (2H,m), 0.87 (9H,s), 0.07 (3H,s), 0.06 (3H,s).

Example 33

Synthesis of (3S,4R)-3-((R)-1-tert-butyldimethylsilyloxypropyl)-4-[((R)-tetrahydrofuran-2-yl)-carbonylthio]azetidin-2-one and (3R,4S)-3-((S)-1-tert-butyldimethylsilyloxypropyl)-4-[((R)-tetrahydrofuran-2-yl)carbonylthio]azetidin-2-one

1 N sodium hydroxide (about 9 mℓ) was added to (R)-tetrahydro-2-furylthiocarboxylic acid (1.19 g, 9 mmol) to adjust the pH to 9 to 10. The resultant mixture was added with a solution of the compound obtained in Example 32 (1.81 g, 6 mmol) in acetone (6 mℓ), followed by stirring at 50°C.

About 10 minutes later, the reaction mixture was adjusted again to pH 8 to 9, followed by further stirring under heat for 2 hours. The reaction mixture was diluted with ethyl acetate. The resultant solution was washed with a saturated aqueous solution of sodium hydrogencarbonate and a saturated aqueous solution of sodium sulfate, and was then dried over anhydrous magnesium sulfate. The solvent was distilled off under reduced pressure and the residue was purified by silica gel chromatography, whereby the title compounds were obtained as a colorless oil (1.79 g, 80% yield).

IR (KBr):

3158, 1770, 1698 $cm^{-1}$.

NMR δ ($CDCl_3$):

(As the mixture of the title compounds)

6.28, 6.27 (1H,bs), 5.24, 5.21 (1H,d,J=2.6Hz), 4.43-4.52 (1H,m), 3.91-4.16 (3H,m), 3.30 (1H,dd,J=2.6Hz,2.6Hz), 2.18-2.37 (1H,m), 1.85-2.18 (3H,m), 1.42-1.68 (2H,m), 0.89 (9H,s), 0.08 (6H,s).

Example 34

Synthesis of allyl (5S,6R)-6-((S)-1-tert-butyldimethylsilyloxypropyl)-2-((R)-tetrahydro-2-furanyl)penem-3-carboxylate

The compound obtained in Example 33 (1.49 g, 4 mmol) was dissolved in methylene chloride (2 mℓ). Under cooling at -20°C, a solution of allyl oxalyl chloride (0.99 g, 6.7 mℓ) in methylene chloride (1.6 mℓ) was added, followed by the addition of a solution of triethylamine (0.69 g, 6.8 mmol) in methylene chloride (1.6 mℓ). The resultant mixture was stirred at the same temperature for 1.5 hours. The reaction mixture was diluted with methylene chloride. The solution was washed with water and then with a saturated aqueous solution of sodium hydrogencarbonate and a

saturated aqueous solution of sodium sulfate, and was thereafter dried over anhydrous sodium sulfate.

Toluene was added and, after the solvent was distilled off under reduced pressure, xylene (20 mℓ) was added, followed by heating under reflux for 3 hours. The reaction mixture was diluted with hexane. The organic layer so obtained was washed with water and then dried over anhydrous magnesium sulfate. The solvent was distilled off under reduced pressure and the residue was purified by silica gel chromatography, whereby a mixture of allyl (5S,6R)-6-((S)-1-tert-butyldimethylsilyloxypropyl)-2-((R)-tetrahydro-2-furanyl)-penem-3-carboxylate and (5R,6S)-6-((R)-1-tert-butyldimethylsilyloxypropyl)-2-((R)-tetrahydro-2-furanyl)-penem-3-carboxylate was obtained as a yellow oil (1.04 g, 58% yield).

The mixture was then carefully purified by silica gel chromatography, whereby the title compound, allyl (5S, 6R)-6-((S)-1-tert-butyldimethylsilyloxypropyl)-2-((R)-tetrahydro-2-furanyl)penem-3-carboxylate was obtained (0.48 g).

IR (film):

2955, 1790, 1707 $cm^{-1}$.

NMR δ ($CDCl_3$):

5.84-6.02 (1H,m), 5.58 (1H,d,J=1.3Hz), 5.32-5.49 (2H,m), 5.24 (1H,d,J=10.6Hz), 4.59-4.82 (2H,m), 4.00-4.11 (1H,m), 3.89-4.00 (1H,m), 3.73-3.89 (2H,m), 2.33-2.50 (1H,m), 1.87-2.05 (2H,m), 1.69-1.87 (1H,m), 1.45-1.69 (2H,m), 0.89 (9H,s), 0.08 (3H,s), 0.07 (3H,s).

Example 35

Synthesis of allyl (5R,6R)-6-((S)-1-tert-butyldimethylsilyloxypropyl)-2-((R)-tetrahydro-2-furanyl)penem-3-carboxylate

Allyl (5S,6R)-6-((S)-1-tert-butyldimethylsilyloxypropyl)-2-((R)-tetrahydro-2-furanyl)penem-3-carboxylate (343 mg, 1 mmol) was dissolved in deaerated ethyl acetate (200 mℓ). The resultant solution was placed in a Pyrex container and through a Pyrex filter, was exposed for 1 hour to light under a 200 W highpressure mercury lamp (manufactured by Ishii Rika K.K.). After the solvent was distilled off, the residue was purified by silica gel chromatography, whereby the title compound was obtained (106 mg, 32% yield). In addition, the raw material was recovered (191 mg, 51% recovery rate).

IR (KBr):

3500, 1790, 1704 $cm^{-1}$.

NMR δ ($CDCl_3$):

5.85-6.02 (1H,m), 5.55 (1H,d,J=4.0Hz), 5.20-5.48 (3H,m), 4.58-5.82 (2H,m), 4.31-4.42 (1H,m), 3.79-4.09 (3H, m), 2.34-2.53 (1H,m), 1.71-2.10 (5H,m), 0.97 (3H,t,J=7.9Hz), 0.87 (9H,s), 0.11 (3H,s), 0.12 (3H,s).

Example 36

Synthesis of allyl (5R,6R)-6-((S)-1-hydroxypropyl)-2-((R)-tetrahydro-2-furanyl)penem-3-carboxylate

Allyl (5R,6R)-6-((S)-1-tert-butyldimethylsilyloxypropyl)-2-((R)-tetrahydro-2-furanyl)penem-3-carboxylate (138 mg, 0.30 mmol) was dissolved in tetrahydrofuran (0.61 mℓ), followed by the addition of acetic acid (0.069 mℓ) and then of 1.0 M tetrahydrofuran solution of tetra-n-butylammonium fluoride (0.89 mℓ, 0.89 mmol) at room temperature. The resultant mixture was stirred at 50°C for 6 hours.

The reaction mixture was diluted with ethyl acetate. The solution was washed with water, a saturated aqueous solution of potassium hydrogensulfate, a saturated aqueous solution of sodium hydrogencarbonate and a saturated aqueous solution of sodium sulfate, and was then dried over anhydrous sodium sulfate.

The solvent was distilled off under reduced pressure and the residue was purified by silica gel chromatography, whereby the title compound was obtained (79 mg, 77% yield).

IR (film):

2955, 1790, 1704 $cm^{-1}$.

NMR δ ($CDCl_3$):

5.86-6.03 (1H,m), 5.58 (1H,d,J=4.0Hz), 5.22-5.47 (3H,m), 4.77 (1H,dd,J=5.3Hz,13.9Hz), 4.63 (1H,dd,J=5.3Hz, 13.9Hz), 4.12-4.27 (1H,m), 3.95-4.06 (1H,m), 3.82-3.95 (2H,m), 2.39-2.55 (1H,m), 1.76-2.10 (5H,m), 1.03 (3H,t, J=7.3Hz).

Example 37

Synthesis of (5R,6R)-6-((S)-1-hydroxypropyl)-2-((R)-tetrahydro-2-furanyl)penem-3-carboxylic acid

Using allyl (5R,6R)-6-((S)-1-hydroxypropyl)-2-((R)-tetrahydro-2-furanyl)penem-3-carboxylate (20 mg), a reaction was conducted in a similar manner as in Example 24, whereby the title compound was obtained (9 mg, 50% yield).

IR (KBr):

3401, 1771 $cm^{-1}$.

NMR δ ($CDCl_3$):

5.60 (1H,d,J=4.0Hz), 5.34 (1H,t,J=7.3Hz), 4.12-4.25 (1H,m), 3.93-4.07 (1H,m), 3.81-3.93 (2H,m), 2.40-2.59 (1H, m), 1.78-2.12 (4H,m), 1.48-1.63 (1H,m), 1.04 (3H,t,J=7.3Hz).

Example 38

Synthesis of p-nitrobenzyl [(3R,4S)-3-(1-(S)-(tert-butyldimethylsilyloxy)propyl]-4-phenylthio-2-azetidinon-1-yl)acetate

To a solution of (3R,4S)-3-[1-(S)-(tert-butyldimethylsilyloxy)propyl]-4-phenylthio-2-azetidinone (1.53 g, 4.35 mmol) in dry N,N-dimethylformamide (6.7 ml), p-nitrobenzyl iodoacetate (1.66 g, 4.78 mmol) and potassium carbonate (1.82 g, 13.2 mmol) were added under an argon gas stream. The reaction mixture was then heated to 50-55°C. Four hours and thirty minutes later, the reaction mixture was diluted with water (50 ml) and then extracted with methylene chloride (100 ml and 50 ml). The organic layers were combined, followed by washing with a saturated aqueous solution of sodium chloride (100 ml).

The organic layer was dried over anhydrous sodium sulfate, the solvent was distilled off under reduced pressure, and the residue was subjected to column chromatography by using silica gel (38 g). From ethyl acetate-hexane (1:8, V/V), p-nitrobenzyl 2-[(3R,4S)-3-(1-(S)-(tert-butyldimethylsilyloxy)propyl)-4-phenylthio-2-azetidinon-1-yl)acetate was obtained as a slightly yellowish oil (2.20 g, 93% yield).

NMR δ ($CDCl_3$):

0.01 (3H,s), 0.06 (3H,s), 0.86 (9H,s), 0.91 (3H,t,J=7.5Hz), 1.55-1.7 (2H,m), 3.17 (1H,dd,J=2.1Hz,2.8Hz), 3.93 (1H,d,J=17.8Hz), 4.05-4.1 (1H,m), 4.25 (1H,d,J=17.8Hz), 5.16 (1H,d,J=13.2Hz), 5.22 (1H,d,J=13.2Hz), 5.30 (1H,d, J=2.1Hz), 7.25-7.35 (3H,m), 7.4-7.5 (2H,m), 7.47 (2H,d,J=8.7Hz), 8.22 (2H,d,J=8.7Hz).

Example 39

Synthesis of p-nitrobenzyl 2-[bis(benzoylthio)-methylidene]-2-[(3R,4S)-3-(1-(S)-(tert-butyldimethylsilyloxy)propyl)-4-phenylthio-2-azetidinon-1-yl]acetate

To a solution of hexamethyldisilazane (1.65 g, 10.2 mmol) in distilled tetrahydrofuran (25 mℓ), a 1.71 N hexane solution of n-butyllithium (5.3 mℓ, 9.06 mmol) was added under an argon gas stream at room temperature. Thirty minutes later, the reaction mixture was cooled to -78°C, followed by the addition of a solution of p-nitrobenzyl 2-[(3R, 4S)-3-(1-(S)-(tert-butyldimethylsilyloxy)propyl)-4-phenylthio-2-azetidinon-1-yl]acetate (2.50 g, 4.59 mmol) in distilled tetrahydrofuran (5 mℓ).

Ten minutes later, the reaction mixture was added successively with carbon disulfide (0.55 mℓ, 9.14 mmol) and a solution of benzoyl chloride (1.6 mℓ, 13.8 mmol) in distilled tetrahydrofuran (5 mℓ). Ten minutes later, the reaction mixture was added with acetic acid (0.45 mℓ, 7.84 mmol) and the resultant mixture was poured into ethyl acetate (200 mℓ). The thus-obtained mixture was washed successively with a saturated aqueous solution of sodium chloride (100 mℓ), a saturated aqueous solution of sodium hydrogencarbonate (100 mℓ) and a saturated aqueous solution of sodium chloride (100 mℓ). The organic layer was dried over anhydrous sodium sulfate and the solvent was distilled off under reduced pressure. The residue was subjected to column chromatography by using silica gel (50 g). From ethyl acetate-hexane (1:3, V/V), p-nitrobenzyl 2-[bis(benzoylthio)methylidene]-2-[(3R,4S)-3-(1-(S)-(tert-butyldimethylsilyloxy)propyl)-4-phenylthio-2-azetidinon-1-yl)acetate was obtained as a slightly yellowish solid (3.37 g, 88% yield).

NMR δ ($CDCl_3$):

0.01 (3H,s), 0.02 (3H,s), 0.83 (9H,s), 0.90 (3H,t,J=7.5Hz), 1.5-1.65 (2H,m), 3.12 (1H,dd,J=2.5Hz,2.8Hz), 3.95-4.0 (1H,m), 5.24 (1H,d,J=12.9Hz), 5.30 (1H,d,J=12.9Hz), 5.85 (1H,d,J=2.8Hz), 7.2-7.35 (3H,m), 7.35-7.4 (2H,m), 7.4-7.5 (4H,m), 7.5-7.6 (2H,m), 7.69 (2H,d,J=7.1Hz), 7.80 (2H,d,J=7.1Hz), 7.92 (2H,d,J=8.7Hz), 7.99 (2H,d,J=8.7Hz).

Example 40

Synthesis of p-nitrobenzyl (5R,6R)-6-((S)-1-tert-butyldimethylsilyloxypropyl)-2-methylthiopenem-3-carboxylate

To a solution of p-nitrobenzyl 2-[bis(benzoyl-thio)methylidene]-2-[(3R,4S)-3-(1-(S)-tert-butyl-dimethylsilyloxy) propyl)-4-phenylthio-2-azetidinon-1-yl]acetate (2.43 g, 2.93 mmol) in distilled methylene chloride (40 mℓ), sulfuryl chloride (0.44 mℓ, 4.41 mmol) was added under an argon gas stream at -5°C. Fifteen minutes later, the reaction mixture was added successively with allyl acetate (1.6 mℓ, 14.8 mmol) and diphenyl disulfide (639 mg, 2.93 mmol) and 5 minutes later, the reaction mixture was ice-cooled.

Twenty minutes later, the reaction mixture was added with a solution of morpholine (0.77 mℓ, 8.80 mmol) in distilled methylene chloride (4 mℓ) and also with triethylamine (0.60 mℓ, 4.30 mmol) and 10 minutes later, was also added with methyl iodide (0.70 mℓ, 11.2 mmol) and triethylamine (0.40 mℓ, 2.87 mmol). The temperature of the reaction mixture was allowed to rise to room temperature. One hour later, the reaction mixture was poured into ethyl acetate (200 mℓ), followed by successive washing with a saturated aqueous solution of potassium hydrogensulfate (100 mℓ), a saturated aqueous solution of sodium hydrogencarbonate (100 mℓ) and a saturated aqueous solution of sodium chloride (100 mℓ).

The organic layer was dried over anhydrous sodium sulfate, the solvent was distilled off under reduced pressure, and the residue was subjected to column chromatography by using silica gel (50 g). From ethyl acetate-hexane (1:8, V/V), p-nitrobenzyl (5R,6R)-6-((S)-1-tert-butyldimethylsilyloxypropyl) -2-methylthiopenem-3-carboxylate was obtained as a slightly yellowish solid (1.07 g, 70% yield).

NMR δ ($CDCl_3$):

0.12 (3H,s), 0.12 (3H,s), 0.88 (9H,s), 0.99 (3H,t,J=7.5Hz), 1.75-1.9 (2H,m), 2.56 (3H,s), 4.09 (1H,dd,J=4.0Hz, 9.5Hz), 4.35 (1H,dt,J=4.5Hz,9.5Hz), 5.21 (1H,d,J=13.8Hz), 5.47 (1H,d,J=13.8Hz), 5.73 (1H,d,J=4.0Hz), 7.61 (2H,d, J=8.7Hz), 8.21 (2H,d,J=8.7Hz).

Example 41

Synthesis of n-nitrobenzyl (5R,6R)-6-((S)-1-tert-butyldimethylsilyloxypropyl)-2-methylsulfinylpenem-3-carboxylate

To a solution of p-nitrobenzyl (5R,6R)-6-((S)-1-tert-butyldimethylsilyloxypropyl)-2-methylthiopenem-3-carboxylate (1.068 g, 2.03 mmol) in distilled methylene chloride (20 mℓ), m-chloroperbenzoic acid (393 mg, 2.28 mmol) was added under an argon gas stream at -30°C.

One hour later, the reaction mixture was poured into ethyl acetate (200 mℓ), followed by successive washing with a 0.01 N aqueous solution of sodium thiosulfate (30 mℓ), a saturated aqueous solution of sodium hydrogencarbonate (100 mℓ) and a saturated aqueous solution of sodium chloride (100 mℓ). The organic layer was dried over anhydrous sodium sulfate, the solvent was distilled off under reduced pressure and the residue was subjected to column chromatography by using silica gel (50 g). From ethyl acetate-hexane (1:1, V/V), p-nitrobenzyl (5R,6R)-6-((S)-1-tert-butyldimethylsilyloxypropyl)-2-methylsulfinylpenem-3-carboxylate (a mixture of two types of isomers) was obtained as a pale yellow solid (793 mg, 72% yield).

NMR δ ($CDCl_3$) :

0.13 (6H,s), 0.88 (9H,s), 1.00 (3H,t,J=7.4Hz), 1.75-1.85 (2H,m), 2.95 (3H,s), 4.18 (1H,dt,J=4.2Hz,10.2Hz), 4.35-4.45 (1H,m), 5.23 (0.6H,d,J=13.5Hz), 5.24 (0.4H,d,J=13.5Hz), 5.44 (0.4H,d,J=13.5Hz), 5.44 (0.6H,d,J=13.5Hz), 5.78 (0.4H,d,J=4.2Hz), 5.93 (0.6H,d,J=4.2Hz), 7.58 (1.2H,d,J=8.7Hz), 7.60 (0.8H,d,J=8.7Hz), 8.24 (2H,d,J=8.7Hz).

Example 42

Synthesis of p-nitrobenzyl (5R,6R)-2-((S)-1-benzylpyrrolidin-3-yl)thio-6-((S)-1-tert-butyldimethylsilyloxypropyl)penem-3-carboxylate

To a solution of p-nitrobenzyl (5R,6R)-6-((S)-1-tert-butyldimethylsilyloxypropyl)-2-methylsulfinylpenem-3-carboxylate (179 mg, 0.33 mmol) in dry dimethylformamide (6 mℓ), a solution of diisopropylethylamine (75 μℓ, 0.43 mmol) in dry dimethylformamide (2 mℓ) and a solution of (S)-3-mercapto-1-benzylpyrrolidine (84 mg, 0.43 mmol) in dry dimethylformamide (2 mℓ) were added under an argon gas stream at -30°C.

Twenty minutes later, the reaction mixture was poured into ethyl acetate (100 mℓ), followed by successive washing with a saturated aqueous solution of potassium hydrogensulfate (50 mℓ), a saturated aqueous solution of sodium hydrogencarbonate (50 mℓ) and a saturated aqueous solution of sodium chloride (50 mℓ). The organic layer was dried over anhydrous sodium sulfate, the solvent was distilled off under reduced pressure, and column chromatography was conducted using silica gel (15 g). From ethyl acetate-hexane (1:8, V/V), p-nitrobenzyl (5R,6R)-2-((S)-1-benzylpyrrolidine-3-yl)thio-6-((S)-1-tert-butyldimethylsilyloxypropyl)-penem-3-carboxylate was obtained as a pale yellow oil (110 mg, 50% yield).

NMR δ ($CDCl_3$):

0.10 (3H,s), 0.11 (3H,s), 0.87 (9H,s), 0.99 (3H,t,J=7.4Hz), 1.7-1.95 (3H,m), 2.3-2.45 (1H,m), 2.45-2.55 (1H,m), 2.55-2.65 (1H,m), 2.65-2.75 (1H,m), 3.1-3.2 (1H,m), 3.63 (2H,s), 3.75-3.9 (1H,m), 4.08 (1H,dd,J=4.0Hz,9.8Hz), 4.3-4.4 (1H,m), 5.20 (1H,d,J=13.8Hz), 5.46 (1H,d,J=13.8Hz), 5.69 (1H,d,J=4.0Hz), 7.61 (2H,d,J=8.7Hz), 8.21 (2H,d,J=8.7Hz).

Example 43

Synthesis of p-nitrobenzyl (5R,6R)-2-((S)-1-benzylpyrrolidin-3-yl)thio-6-((S)-1-hydroxypropyl)penem-3-carboxylate

To a solution of p-nitrobenzyl (5R,6R)-2-((S)-1-benzylpyrrolidin-3-yl)thio-6-((S)-1-tert-butyldimethylsilyloxypropyl)penem-3-carboxylate (108 mg, 0.16 mmol) in distilled tetrahydrofuran (120 μℓ), acetic acid (60 μℓ, 1.05 mmol) and a 1 M tetrahydrofuran solution of n-tetrabutylammoniumm fluoride (0.53 mℓ, 0.53 mmol) were added. Seventeen hours later, ethyl acetate (50 mℓ) was poured into the reaction mixture, followed by successive washing with a saturated aqueous solution of potassium hydrogensulfate (10 mℓ), a saturated aqueous solution of sodium chloride (10 mℓ), a saturated aqueous solution of sodium hydrogencarbonate (20 mℓ) and a saturated aqueous solution of sodium chloride (10 mℓ).

The organic layer was dried over anhydrous sodium sulfate, the solvent was distilled off under reduced pressure, and column chromatography was conducted using silica gel (10 g). From ethyl acetate-hexane (2:1, V/V), p-nitrobenzyl (5R,6R)-2-((S)-1-benzylpyrrolidin-3-yl)thio-6-((S)-1-hydroxypropyl)penem-3-carboxylate was obtained as a colorless oil (79 mg, 88% yield).

NMR δ ($CDCl_3$) :

1.06 (3H,t,J=7.4Hz), 1.55-1.65 (1H,m), 1.65 (1H,bs), 1.8-1.9 (1H,m), 1.9-2.05 (1H,m), 2.3-2.45 (1H,m), 2.57 (1H, dd,J=5.8Hz,10.2Hz), 2.65 (2H,t,J=6.9Hz), 3.15 (1H,dd,J=7.4Hz,10.2Hz), 3.61 (1H,d,J=13.0Hz), 3.65 (1H,d,J=13.0Hz), 3.75-3.85 (1H,m), 3.92 (1H,dd,J=4.0Hz,10.5Hz), 4.05-4.15 (1H,m), 5.20 (1H,d,J=13.8Hz), 5.46 (1H,d,J=13.8Hz), 5.73 (1H,d,J=4.0Hz), 7.3-7.35 (5H,m), 7.61 (2H,d,J=8.8Hz), 8.21 (2H,d,J=8.8Hz).

Example 44

Synthesis of (5R,6R)-2-((S)-1-benzylpyrrolidin-3-yl)thio-6-((S)-1-hydroxypropyl)penem-6-carboxylic acid

A 0.1 M phosphate buffer (pH 7.0) (2.6 mℓ) was added to 10% palladium carbon (150 mg). Subsequent to substitution with hydrogen gas at atmospheric pressure and room temperature, a solution of p-nitrobenzyl (5R,6R)-2-((S)-1-benzylpyrrolidin-3-yl)thio-6-((S)-1-hydroxypropyl)penem-3-carboxylate (76 mg, 0.16 mmol) in tetrahydrofuran (3.9 mℓ) was added.

Two hours and thirty minutes later, insoluble matter was eliminated, the tetrahydrofuran was distilled off under reduced pressure, and the residue was lyophilized. The residue was dissolved in a mixed solvent of water and acetonitrile (1 mM ammonium formate) (95:5, V/V). After insoluble matter was eliminated, high-performance liquid chromatography was conducted using a column (20 mm in diameter x 250 mm) packed with octadecylsilylated silica gel [gradient elution: water-acetonitrile (1 mM ammonium formate), 86:14 to 32:68, V/V]. By lyophilization, (5R,6R)-2-((S)-1-benzylpyrrolidin-3-yl)thio-6-((S)-1-hydroxypropyl)penem-6-carboxylic acid was obtained as a white solid (7.0 mg, 12% yield).

NMR δ ($D_2O$):

1.00 (3H,t,J=7.4Hz), 1.45-1.65 (1H,m), 1.75-1.95 (1H,m), 1.95-2.15 (1H,m), 2.5-2.7 (1H,m), 3.3-3.5 (2H,m), 3.5-3.6 (1H,m), 3.7-3.8 (1H,m), 4.0-4.1 (2H,m), 4.1-4.2 (1H,m), 4.3-4.4 (2H,m), 5.74 (1H,bs), 7.51 (5H,bs).

IR $\nu_{max}$(NaCl):

1762, 1560, 1374 $cm^{-1}$.

Example 45

Synthesis of p-nitrobenzyl (5R,6R)-2-((S)-1-allyloxycarbonylpyrrolidin-3-yl)thio-6-((S)-1-tert-butyldimethylsilyloxypropyl)penem-3-carboxylate.

From p-nitrobenzyl (5R,6R)-2-methylsulfinyl-6-((S)-1-tert-butyldimethylsilyloxypropyl)penem-3-carboxylate (540 mg, 1.0 mmol), the title compound was obtained as a yellow amorphous (505 mg, 76% yield) in a similar manner as in Example 42.

NMR δ ($CDCl_3$):

8.21 (2H,d,J=8.8Hz), 7.60 (2H,d,J=8.8Hz), 5.88-6.00 (1H,m), 5.75 (1H,d,J=4.0Hz), 5.46 (1H,d,J=13.7Hz), 5.30 (1H,d,J=16.0Hz), 5.17-5.23 (2H,m), 4.60 (2H,d,J=5.4Hz), 4.30-4.38 (1H,m), 4.12 (1H,dd,J=4.1Hz,9.4Hz), 3.82-3.95 (2H,m), 3.41-3.77 (1H,m), 2.30-2.42 (1H,m), 1.98-2.12 (1H,m), 1.85-1.90 (1H,m), 1.45-1.60 (1H,m), 0.99 (3H,t, J=7.5Hz), 0.88 (9H,s), 0.12 (3H,s), 0.11 (3H,s).

IR $\nu_{max}$(NaCl):

1790, 1704 $cm^{-1}$.

Example 46

Synthesis of p-nitrobenzyl (5R,6R)-2-((S)-1-allyloxycarbonylpyrrolidin-3-yl)thio-6- ((S)-1-hydroxypropyl)penem-3-carboxylate.

From p-nitrobenzyl (5R,6R)-2-((S)-1-allyloxycarbonylpyrrolidin-3-yl)thio-6-((S)-1-tert-butyldimethylsilyloxypropyl)penem-3-carboxylate (495 mg, 0.75 mmol), the title compound was obtained as a yellow amorphous (320 mg, 78% yield) in a similar manner as in Example 43.

NMR $\delta$ ($CDCl_3$):

8.22 (2H,d,J=8.8Hz), 7.60 (2H,d,J=8.8Hz), 5.88-6.00 (1H,m), 5.79 (1H,d,J=4.0Hz), 5.46 (1H,d,J=16.7Hz), 5.30 (1H,d,J=17.3Hz), 5.15-5.26 (2H,m), 4.60 (2H,d,J=5.6Hz), 4.08-4.18 (1H,m), 3.84-3.99 (3H,m), 3.42-3.67 (3H,m), 2.29-2.43 (1H,m), 1.92-2.10 (1H,m), 1.44-1.69 (1H,m), 1.07 (3H,t,J=7.4Hz).

IR $\nu_{max}$(NaCl):

3415, 1785, 1694 $cm^{-1}$.

Example 47

Synthesis of p-nitrobenzyl (5R,6R)-2-((S)-1-allylpyrrolidin-3-yl)thio-6-((S)-1-hydroxypropyl)penem-3-carboxylate and p-nitrobenzyl (5R,6R)-2-((S)-1-(5,5-dimethyl-3-oxocyclohexen-1-yl)pyrrolidin-3-yl)thio-6-((S)-1-hydroxypropyl)-penem-3-carboxylate

p-Nitrobenzyl (5R,6R)-2-((S)-1-allyloxycarbonylpyrrolidin-3-yl)thio-6-((S)-1-hydroxypropyl)penem-3-carboxylate (247 mg, 0.45 mmol) was dissolved in methylene chloride. Tetrakistriphenylphosphine palladium and dimedone were added at room temperature, followed by stirring for 30 minutes. The solvent was eliminated and the residue was purified by column chromatography.

The title compounds, p-nitrobenzyl (5R,6R)-2-((S)-1-allylpyrrolidin-3-yl)thio-6-((S)-1-hydroxypropyl)penem-3-carboxylate and p-nitrobenzyl (5R,6R)-2-((S)-1-(5,5-dimethyl-3-oxocyclohexen-1-yl)pyrrolidin-3-yl)thio-6-((S)-1-hydroxypropyl)penem-3-carboxylate, were obtained as a brown amorphous (52 mg, 23% yield) and as a brown amorphous (188 mg, 71% yield), respectively.

NMR $\delta$ ($CDCl_3$):

8.41 (2H,d,J=8.7Hz), 7.60 (2H,d,J=8.7Hz), 5.80-5.94 (1H,m), 5.75 (1H,d,J=4.0Hz), 5.46 (1H,d,J=13.7Hz), 5.2 (2H,d,J=13.7Hz), 5.13 (1H,d,J=10.1Hz), 4.07-4.16 (1H,m), 3.93 (1H,dd,J=4.0Hz,10.3Hz), 3.74-3.86 (1H,m), 3.14-3.22 (1H,m), 2.48-2.75 (3H,m), 2.30-2.48 (1H,m) 1.91-2.06 (1H,m), 1.80-1.91 (1H,m), 1.50-1.72 (1H,m), 1.06 (3H,t, J=7.4Hz).

IR $\nu_{max}$(NaCl):

1780, 1684 $cm^{-1}$.

NMR $\delta$ ($CDCl_3$):

8.21 (2H,d,J=8.6Hz), 7.60 (2H,d,J=8.6Hz), 5.81 (1H,d,J=3.9Hz), 5.47 (1H,d,J=13.6Hz), 5.20 (1H,d,J=13.6Hz), 5.05 (1H,s), 4.09-4.19 (1H,m), 3.92-4.02 (2H,m), 3.25-3.68 (4H,m), 2.36-2.53 (1H,m), 2.09-2.22 (1H,m), 1.92-2.09 (1H,m), 2.28 (2H,s), 2.17 (2H,s), 1.44-1.65 (1H,m), 1.00-1.14 (9H,m).

IR $\nu_{max}$(NaCl):

1785, 1685 $cm^{-1}$.

Example 48

Synthesis of (5R,6R)-2-((S)-1-propylpyrrolidin-3-yl)thio-6-((S)-1-hydroxypropyl)penem-3-carboxylic acid

A 0.1 M phosphate buffer (pH 7.0) (5.5 mℓ) was added to 10% palladium carbon (110 mg). Subsequent to substitution with hydrogen gas, a solution of p-nitrobenzyl (5R,6R)-2-((S)-l-allylpyrrolidin-3-yl)thio-6-((S)-1-hydroxypropyl)penem-3-carboxylate (56 mg, 0.11 mmol) in tetrahydrofuran (5.5 mℓ) was added. Under atmospheric pressure, the resultant mixture was stirred at room temperature. Subsequent to elimination of the catalyst, the tetrahydrofuran was distilled off under reduced pressure, followed by lyophilization. High performance liquid chromatography was conducted using a column (20 mm in diameter x 250 mm) packed with octadecylated silica gel [gradient elution: water-acetonitrile (1 mM ammonium formate)]. After lyophilization, the title compound was obtained as a white powder (16 mg, 39% yield).

NMR $\delta$ ($D_2O$):

5.81 (1H,d,J=3.5Hz), 4.03-4.30 (3H,m), 3.11-4.00 (6H,m), 2.51-2.80 (1H,m), 1.99-2.27 (1H,m), 1.68-1.99 (3H, m), 1.50-1.68 (1H,m), 0.90-1.14 (6H,m).

IR $\nu_{max}$(KBr):

3356, 1774, 1584 $cm^{-1}$.

Example 49

Synthesis of (5R,6R)-2-((S)-1-propoxycarbonylpyrrolidin-3-yl)thio-6-((S)-1-hydroxypropyl)-penem-3-carboxylic acid and (5R,6R)-2-((S)-pyrrolidin-3-yl)thio-6-((S)-1-hydroxypropyl)-penem-3-carboxylic acid

From p-nitrobenzyl (5R,6R)-2-((S)-l-allyloxycarbonylpyrrolidin-3-yl)thio-6-((S)-1-hydroxypropyl)-penem-3-carboxylate (40 mg, 0.07 mmol), the title compounds, (5R,6R)-2-((S)-1-propoxycarbonylpyrrolidin-3-yl)thio-6-((S)-1-hydroxypropyl)penem-3-carboxylic acid and (5R,6R)-2-((S)-pyrrolidin-3-yl)thio-6-((S)-1-hydroxypropyl)penem-3-carboxylic acid were obtained as a white powder (4 mg, 13% yield) and as a white powder (14 mg, 58% yield), respectively, in a similar manner as in Example 48.
NMR $\delta$ ($D_2O$)
5.74 (1H,s), 3.89-4.08 (5H,m), 3.41-3.61 (3H,m), 2.28-2.45 (1H,m), 1.80-2.09 (2H,m), 1.58-1.71 (2H,m), 1.43-1.58 (1H,m), 1.02 (3H,t,J=7.4Hz), 0.96 (3H,t,J=7.4Hz).
NMR $\delta$ ($D_2O$)
5.81 (1H,d,J=3.6Hz), 4.03-4.22 (3H,m), 3.78 (1H,dd,J=6.5Hz,12.8Hz), 3.39-3.61 (4H,m), 2.48-2.61 (1H,m), 2.06-2.19 (1H,m), 1.80-2.94 (1H,m), 1.49-1.65 (1H,m) 1.00 (3H,t,J=7.4Hz).
IR $\nu_{max}$(KBr):
3420, 1764, 1596 $cm^{-1}$.

Example 50

Synthesis of (5R,6R)-2-((S)-1-(5,5-dimethyl-3-oxocyclohexen-1-yl)pyrrolidin-3-yl)thio-6-((S)-1-hydroxypropyl)penem-3-carboxylic acid

From p-nitrobenzyl (5R,6R)-2-((S)-1-(5,5-dimethyl-3-oxocyclohexen-1-yl)pyrrolidin-3-yl)thio-6-((S)-1-hydroxypropyl)penem-3-carboxylate (60 mg, 0.13 mmol), the title compound was obtained as a white powder (31 mg, 53% yield) in a similar manner as in Example 44.
NMR $\delta$ ($D_2O$)
5.80 (1H,d,J=3.5Hz), 4.01-4.20 (4H,m), 3.66-3.98 (2H,m), 3.41-3.66 (2H,m), 2.39-2.58 (1H,m), 2.46 (2H,d, J=10.2Hz), 2.18 (1H,s), 2.04-2.20 (1H,m), 1.80-1.95 (1H,m), 1.49-1.67 (1H,m), 1.07 (6H,s), 1.00 (3H,t,J=7.4Hz).
IR $\nu_{max}$(KBr):
3385, 1770, 1540 $cm^{-1}$.

Example 51

Synthesis of p-nitrobenzyl (5R,6R)-6-((S)-1-hydroxypropyl)-2-methylthiopenem-3-carboxylate

In a similar manner as in Example 43 except for the use of p-nitrobenzyl (5R,6R)-6-((S)-1-tert-butyldimethylsilyloxypropyl)-2-methylthiopenem-3-carboxylate instead of p-nitrobenzyl (5R,6R)-2-((S)-1-benzylpyrrolidin-3-yl)thio-6-((S)-1-tert-butyldimethylsilyloxypropyl)penem-3-carboxylate, the title compound was obtained as a brown oil (681 mg, 83% yield).

Example 52

Synthesis of p-nitrobenzyl (5R,6R)-6-((S)-1-hydroxypropyl)-2-methylsulfinylpenem-3-carboxylate

In a similar manner as in Example 9 except for the use of p-nitrobenzyl (5R,6R)-6-((S)-1-hydroxypropyl)-2-methylthiopenem-3-carboxylate instead of allyl (5R,6R)-6-((S)-1-tert-butyldimethylsilyloxypropyl)-2-methylthiopenem-3-carboxylate, the title compound was obtained as a white powder (317 mg, 64% yield).

Example 53

Synthesis of p-nitrobenzyl (5R,6R)-6-((S)-1-hydroxypropyl)-2-((S)-1-(p-nitrobenzyloxycarbonyl)pyrrolidin-3-yl) thiopenem-3-carboxylate

In a similar manner as in Example 10 except for the use of p-nitrobenzyl (5R,6R)-6-((S)-1-hydroxypropyl)-2-methylsulfinylpenem-3-carboxylate (96 mg, 0.23 mmol) instead of allyl (5R,6R)-6-((S)-1-tert-butyldimethylsilyloxypropyl)-2-methylsulfinylpenem-3-carboxylate and the use of (S)-3-mercapto-1-(p-nitro-benzyloxycarbonyl)pyrrolidine (194 mg, 0.6 mmol) instead of 1-allyloxycarbonyl-3-mercaptopyrrolidine, the title compound was obtained (130 mg, 90% yield).

Example 54

Synthesis of p-nitrobenzyl (5R,6R)-6-((S)-1-hydroxypropyl)-2-(1-phenylpyrrolidin-3-yl)thiopenem-3-carboxylate

In a similar manner as in Example 10 except for the use of p-nitrobenzyl (5R,6R)-6-((S)-1-hydroxy-propyl)-2-methylsulfinylpenem-3-carboxylate (64 mg, 0.15 mmol) instead of allyl (5R,6R)-6-((S)-1-tert-butyldimethylsilyloxypropyl)-2-methylsulfinylpenem-3-carboxylate and the use of 1-phenyl-3-acetylthiopyrrolidine (171 mg, 0.6 mmol) subsequent to conversion of the acetylthio group into a mercapto group in a similar manner as in Production Example 46 instead of 1-allyloxycarbonyl-3-mercaptopyrrolidine, the title compound was obtained (63 mg, 77% yield).

Example 55

Synthesis of p-nitrobenzyl (5R,6R)-6-((S)-1-hydroxypropyl)-2-((S)-1-phenethylpyrrolidin-3-yl)thiopenem-3-carboxylate

In a similar manner as in Example 10 except for the use of p-nitrobenzyl (5R,6R)-6-((S)-1-hydroxypropyl)-2-methylsulfinylpenem-3-carboxylate (20 mg) instead of allyl (5R,6R)-6-((S)-1-tert-butyldimethylsilyloxypropyl)-2-methylsulfinylpenem-3-carboxylate and the use of (S)-3-benzoylthio-1-phenethylpyrrolidine (50 mg, 0.16 mmol) subsequent to conversion of the benzoylthio group into a mercapto group in a similar manner as in Production Example 46 instead of 1-allyloxycarbonyl-3-mercaptopyrrolidine, the title compound was obtained (16 mg, 61% yield).

Example 56

Synthesis of p-nitrobenzyl (5R,6R)-6-((S)-1-hydroxypropyl)-2-(1-((S)-2-hydroxy-2-phenylethyl)pyrrolidin-3-yl) thiopenem-3-carboxylate

In a similar manner as in Example 10 except for the use of p-nitrobenzyl (5R,6R)-6-((S)-1-hydroxypropyl)-2-methylsulfinylpenem-3-carboxylate (39 mg, 0.1 mmol) instead of allyl (5R,6R)-6-((S)-1-tert-butyldimethylsilyloxypropyl)-2-methylsulfinylpenem-3-carboxylate and the use of (S)-3-benzoylthio-1-((S)-2-hydroxy-2-phenylethyl)pyrrolidine (67 mg, 0.21 mmol) subsequent to conversion of the benzoylthio group into a mercapto group in a similar manner as in Production Example 46 instead of 1-allyloxycarbonyl-3-mercaptopyrrolidine, the title compound was obtained (36 mg, 68% yield).

Example 57

Synthesis of p-nitrobenzyl (5R,6R)-2-(1-((R)-2-hydroxy-2-phenylethyl)pyrrolidin-3-yl)thio-6-((S)-1-hydroxypropyl) penem-3-carboxylate

In a similar manner as in Example 10 except for the use of p-nitrobenzyl (5R,6R)-6-((S)-1-hydroxypropyl)-2-methylsulfinylpenem-3-carboxylate (39 mg, 0.1 mmol) instead of allyl (5R,6R)-6-((S)-1-tert-butyldimethylsilyloxypropyl)-2-methylsulfinylpenem-3-carboxylate and the use of 3-(S)-benzoylthio-1-((R)-2-hydroxy-2-phenylethyl)pyrrolidine (50 mg, 0.15 mmol) subsequent to conversion of the benzoylthio group into a mercapto group in a similar manner as in Production Example 46 instead of 1-allyloxycarbonyl-3-mercaptopyrrolidine, the title compound was obtained (26 mg, 49% yield).

Example 58

Synthesis of p-nitrobenzyl (5R,6R)-2-((S)-1-benzoylpyrrolidin -3-yl)thio-6-((S)-1-hydroxypropyl)penem-3-carboxylate

In a similar manner as in Example 10 except for the use of p-nitrobenzyl (5R,6R)-6-((S)-1-hydroxypropyl)-2-methylsulfinylpenem-3-carboxylate (64 mg, 0.15 mmol) instead of allyl (5R,6R)-6-((S)-1-tert-butyldimethylsilyloxypropyl)-2-methylsulfinylpenem-3-carboxylate and the use of (S)-3-benzoylthio-1-benzoylpyrrolidine (189 mg, 0.6 mmol) subsequent to conversion of the benzoylthio group into a mercapto group in a similar manner as in Production Example 46 instead of 1-allyloxycarbonyl-3-mercaptopyrrolidine, the title compound was obtained (100 mg, 100%

yield).

Example 59

Synthesis of p-nitrobenzyl (5R,6R)-2-((S)-1-acetonylpyrrolidin -3-yl)thio-6-((S)-1-hydroxypropyl)penem-3-carboxylate

In a similar manner as in Example 10 except for the use of p-nitrobenzyl (5R,6R)-6-((S)-1-hydroxypropyl)-2-methylsulfinylpenem-3-carboxylate (64 mg, 0.15 mmol) instead of allyl (5R,6R)-6-((S)-1-tert-butyldimethylsilyloxypropyl)-2-methylsulfinylpenem-3-carboxylate and the use of (S)-1-acetonyl-3-benzoylthiopyrrolidine (162 mg, 0.6 mmol) subsequent to conversion of the benzoylthio group into a mercapto group in a similar manner as in Production Example 46 instead of 1-allyloxycarbonyl-3-mercaptopyrrolidine, the title compound was obtained (19 mg, 24% yield).

Example 60

Synthesis of p-nitrobenzyl (5R,6R)-6-((S)-1-hydroxypropyl)-2-((S)-1-phenacylpyrrolidin-3-yl)thiopenem-3-carboxylate

In a similar manner as in Example 10 except for the use of p-nitrobenzyl (5R,6R)-6-((S)-1-hydroxypropyl)-2-methylsulfinylpenem-3-carboxylate (64 mg, 0.15 mmol) instead of allyl (5R,6R)-6-((S)-1-tert-butyldimethylsilyloxypropyl)-2-methylsulfinylpenem-3-carboxylate and the use of (S)-3-mercapto-N-phenacylpyrrolidine (195 mg, 0.6 mmol) instead of 1-allyloxycarbonyl-3-mercaptopyrrolidine, the title compound was obtained (69 mg, 79% yield).

Example 61

Synthesis of p-nitrobenzyl (5R,6R)-2-((S)-1-(2-p-fluorophenyl-2-oxoethyl)pyrrolidin-3-yl)thio-6-((S)-1-hydroxypropyl) penem-3-carboxylate

In a similar manner as in Example 10 except for the use of p-nitrobenzyl (5R,6R)-6-((S)-1-hydroxypropyl)-2-methylsulfinylpenem-3-carboxylate (48 mg, 0.11 mmol) instead of allyl (5R,6R)-6-((S)-1-tert-butyldimethylsilyloxypropyl)-2-methylsulfinylpenem-3-carboxylate and the use of (S)-3-benzoylthio-1-(2-p-fluorophenyl-2-oxoethyl)pyrrolidine (103 mg, 0.3 mmol) subsequent to conversion of the benzoylthio group into a mercapto group in a similar manner as in Production Example 46 instead of 1-allyloxycarbonyl-3-mercaptopyrrolidine, the title compound was obtained (37 mg, 60% yield).

Example 62

Synthesis of p-nitrobenzyl (5R,6R)-6-((S)-1-hydroxypropyl)-2-((S)-1-(2-oxo-2-p-tolylethyl)-pyrrolidin-3-yl)thiopenem-3-carboxylate

In a similar manner as in Example 10 except for the use of p-nitrobenzyl (5R,6R)-6-((S)-1-hydroxypropyl)-2-methylsulfinylpenem-3-carboxylate (41 mg, 0.1 mmol) instead of allyl (5R,6R)-6-((S)-1-tert-butyldimethylsilyloxypropyl)-2-methylsulfinylpenem-3-carboxylate and the use of (S)-3-benzoylthio-1-(2-oxo-2-p-tolylethyl)pyrrolidine (68 mg, 0.2 mmol) subsequent to conversion of the benzoylthio group into a mercapto group in a similar manner as in Production Example 46 instead of 1-allyloxycarbonyl-3-mercaptopyrrolidine, the title compound was obtained (38 mg, 63% yield).

Example 63

Synthesis of p-nitrobenzyl (5R,6R)-6-((S)-1-hydroxypropyl)-2-((S)-1-(2-p-methoxyphenyl-2-oxoethyl)pyrrolidin-3-yl) thiopenem-3-carboxylate

In a similar manner as in Example 10 except for the use of p-nitrobenzyl (5R,6R)-6-((S)-1-hydroxypropyl)-2-methylsulfinylpenem-3-carboxylate (42 mg, 0.1 mmol) instead of allyl (5R,6R)-6-((S)-1-tert-butyldimethylsilyloxypropyl)-2-methylsulfinylpenem-3-carboxylate and the use of (S)-3-benzoylthio-1-(2-p-methoxyphenyl-2-oxoethyl)pyrrolidine (67 mg, 0.2 mmol) subsequent to conversion of the benzoylthio group into a mercapto group in a similar manner as in Production Example 46 instead of 1-allyloxycarbonyl-3-mercaptopyrrolidine, the title compound was obtained (23 mg, 38% yield).

Example 64

Synthesis of p-nitrobenzyl (5R,6R)-6-((S)-1-hydroxypropyl)-2-((S)-1-(2-p-phenylphenyl-2-oxoethyl)pyrrolidin-3-yl) thiopenem-3-carboxylate

In a similar manner as in Example 10 except for the use of p-nitrobenzyl (5R,6R)-6-((S)-1-hydroxy-propyl)-2-methylsulfinylpenem-3-carboxylate (40 mg, 0.1 mmol) instead of allyl (5R,6R)-6-((S)-1-tert-butyldimethylsilyloxypropyl)-2-methylsulfinylpenem-3-carboxylate and the use of (S)-3-benzoylthio-1-(2-p-phenylphenyl-2-oxoethyl)pyrrolidine (80 mg, 0.2 mmol) subsequent to conversion of the benzoylthio group into a mercapto group in a similar manner as in Production Example 46 instead of 1-allyloxycarbonyl-3-mercaptopyrrolidine, the title compound was obtained (27 mg, 44% yield).

Example 65

Synthesis of p-nitrobenzyl (5R,6R)-2-((S)-1-(2-benzoylethyl)pyrrolidin-3-yl)thio-6-((S)-1-hydroxypropyl)penem-3-carboxylate

In a similar manner as in Example 10 except for the use of p-nitrobenzyl (5R,6R)-6-((S)-1-hydroxypropyl)-2-methylsulfinylpenem-3-carboxylate (42 mg, 0.1 mmol) instead of allyl (5R,6R)-6-((S)-1-tert-butyldimethylsilyloxypropyl)-2-methylsulfinylpenem-3-carboxylate and the use of (S)-1-(2-benzoylethyl)-3-benzoylthiopyrrolidine (102 mg, 0.30 mmol) subsequent to conversion of the benzoylthio group into a mercapto group in a similar manner as in Production Example 46 instead of l-allyloxycarbonyl-3-mercaptopyrrolidine, the title compound was obtained (14 mg, 23% yield).

Example 66

Synthesis of p-nitrobenzyl (5R,6R)-2-((S)-1-(1-benzoylethyl)pyrrolidin-3-yl)thio-6-((S)-1-hydroxypropyl)penem-3-carboxylate

In a similar manner as in Example 10 except for the use of p-nitrobenzyl (5R,6R)-6-((S)-1-hydroxypropyl)-2-methylsulfinylpenem-3-carboxylate (64 mg, 0.15 mmol) instead of allyl (5R,6R)-6-((S)-1-tert-butyldimethylsilyloxy-propyl)-2-methylsulfinylpenem-3-carboxylate and the use of (S)-1-(1-benzoylethyl)-3-benzoylthiopyrrolidine (93 mg, 0.3 mmol) subsequent to conversion of the benzoylthio group into a mercapto group in a similar manner as in Production Example 46 instead of 1-allyloxycarbonyl-3-mercaptopyrrolidine, the title compound was obtained (37 mg, 41% yield).

Example 67

Synthesis of p-nitrobenzyl (5R,6R)-6-((S)-1-hydroxypropyl)-2-((S)-1-phenylaminocarbonylmethylpyrrolidin-3-yl) thiopenem-3-carboxylate

In a similar manner as in Example 10 except for the use of p-nitrobenzyl (5R,6R)-6-((S)-1-hydroxypropyl)-2-methylsulfinylpenem-3-carboxylate (42 mg, 0.10 mmol) instead of allyl (5R,6R)-6-((S)-1-tert-butyldimethylsilyloxy-propyl)-2-methylsulfinylpenem-3-carboxylate and the use of 3-(S)-benzoylthio-N-phenylaminocarbonylmethylpyrrolidine (110 mg, 0.3 mmol) subsequent to conversion of the benzoylthio group into a mercapto group in a similar manner as in Production Example 46 instead of 1-allyloxycarbonyl-3-mercaptopyrrolidine, the title compound was obtained (52 mg, 87% yield).

Example 68

Synthesis of p-nitrobenzyl (5R,6R)-2-((S)-1-benzylaminocarbonylmethylpyrrolidin-3-yl)thio-6-((S)-1-hydroxypropyl) penem-3-carboxylate

In a similar manner as in Example 10 except for the use of p-nitrobenzyl (5R,6R)-6-((S)-1-hydroxypropyl)-2-methylsulfinylpenem-3-carboxylate (43 mg, 0.10 mmol) instead of allyl (5R,6R)-6-((S)-1-tert-butyldimethylsilyloxy-propyl)-2-methylsulfinylpenem-3-carboxylate and the use of 3-(S)-benzoylthio-N-benzylaminocarbonylmethylpyrrolidine (106 mg, 0.3 mmol) subsequent to conversion of the benzoylthio group into a mercapto group in a similar manner as in Production Example 46 instead of 1-allyloxycarbonyl-3-mercaptopyrrolidine, the title compound was obtained (48 mg, 78% yield).

Example 69

Synthesis of p-nitrobenzyl (5R,6R)-6-((S)-1-hydroxypropyl)-2-((S)-1-(1-indanon-2-yl)-pyrrolidin-3-yl)thiopenem-3-carboxylate

In a similar manner as in Example 10 except for the use of p-nitrobenzyl (5R,6R)-6-((S)-1-hydroxypropyl)-2-methylsulfinylpenem-3-carboxylate (42 mg, 0.1 mmol) instead of allyl (5R,6R)-6-((S)-1-tert-butyldimethylsilyloxypropyl)-2-methylsulfinylpenem-3-carboxylate and the use of (S)-3-benzoylthio-1-(1-indanon-2-yl)pyrrolidine (185 mg, 0.55 mmol) subsequent to conversion of the benzoylthio group into a mercapto group in a similar manner as in Production Example 46 instead of 1-allyloxycarbonyl-3-mercaptopyrrolidine, the title compound was obtained (42 mg, 71% yield).

Example 70

Synthesis of p-nitrobenzyl (5R,6R)-6-((S)-1-hydroxypropyl)-2-((S)-1-(1-tetralon-2-yl)-pyrrolidin-3-yl)thiopenem-3-carboxylate

In a similar manner as in Example 10 except for the use of p-nitrobenzyl (5R,6R)-6-((S)-1-hydroxypropyl)-2-methylsulfinylpenem-3-carboxylate (44 mg, 0.1 mmol) instead of allyl (5R,6R)-6-((S)-1-tert-butyldimethylsilyloxypropyl)-2-methylsulfinylpenem-3-carboxylate and the use of (S)-3-benzoylthio-1-(1-tetralon-2-yl)pyrrolidine (74 mg, 0.22 mmol) subsequent to conversion of the benzoylthio group into a mercapto group in a similar manner as in Production Example 46 instead of 1-allyloxycarbonyl-3-mercaptopyrrolidine, the title compound was obtained (10 mg, 17% yield).

Example 71

Synthesis of p-nitrobenzyl (5R,6R)-2-((S)-1-(1-benzosuberon-2-yl)pyrrolidin-3-yl)thio-6-((S)-1-hydroxypropyl)penem-3-carboxylate

In a similar manner as in Example 10 except for the use of p-nitrobenzyl (5R,6R)-6-((S)-1-hydroxypropyl)-2-methylsulfinylpenem-3-carboxylate (39 mg, 0.1 mmol) instead of allyl (5R,6R)-6-((S)-1-tert-butyldimethylsilyloxypropyl)-2-methylsulfinylpenem-3-carboxylate and the use of (S)-3-benzoylthio-1-(1-benzosuberon-2-yl)pyrrolidine (60 mg, 0.16 mmol) subsequent to conversion of the benzoylthio group into a mercapto group in a similar manner as in Production Example 46 instead of 1-allyloxycarbonyl-3-mercaptopyrrolidine, the title compound was obtained (31 mg, 54% yield).

Example 72

Synthesis of p-nitrobenzyl (5R,6R)-6-((S)-1-hydroxypropyl)-2-((S)-1-(2-pyridylmethylpyrrolidin-3-yl)thiopenem-3-carboxylate

In a similar manner as in Example 10 except for the use of p-nitrobenzyl (5R,6R)-6-((S)-1-hydroxypropyl)-2-methylsulfinylpenem-3-carboxylate (123 mg, 0.29 mmol) instead of allyl (5R,6R)-6-((S)-1-tert-butyldimethylsilyloxypropyl)-2-methylsulfinylpenem-3-carboxylate and the use of 3-benzoylthio-1-(2-pyridylmethylpyrrolidine (188 mg, 0.63 mmol) subsequent to conversion of the benzoylthio group into a mercapto group in a similar manner as in Production Example 46 instead of 1-allyloxycarbonyl-3-mercaptopyrrolidine, the title compound was obtained (100 mg, 62% yield).

Example 73

Synthesis of p-nitrobenzyl (5R,6R)-2-(1-benzylpiperidin-3-yl)thio-6-((S)-1-hydroxypropyl)penem-3-carboxylate

In a similar manner as in Example 10 except for the use of p-nitrobenzyl (5R,6R)-6-((S)-1-hydroxypropyl)-2-methylsulfinylpenem-3-carboxylate (85 mg, 0.2 mmol) instead of allyl (5R,6R)-6-((S)-1-tert-butyldimethylsilyloxypropyl)-2-methylsulfinylpenem-3-carboxylate and the use of 3-benzoylthio-1-benzylpiperidine (191 mg, 0.6 mmol) subsequent to conversion of the benzoylthio group into a mercapto group in a similar manner as in Production Example 46 instead of 1-allyloxycarbonyl-3-mercaptopyrrolidine, the title compound was obtained as isomer A (32 mg, 28% yield) and isomer B (29 mg, 26% yield).

Example 74

Synthesis of p-nitrobenzyl (5R,6R)-2-((S)-1-benzylpyrrolidin-2-yl)methylthio-6-((S)-1-hydroxypropyl)penem-3-carboxylate

In a similar manner as in Example 10 except for the use of p-nitrobenzyl (5R,6R)-6-((S)-1-hydroxypropyl)-2-methylsulfinylpenem-3-carboxylate (43 mg, 0.1 mmol) instead of allyl (5R,6R)-6-((S)-1-tert-butyldimethylsilyloxypropyl)-2-methylsulfinylpenem-3-carboxylate and the use of (S)-2-benzoylthiomethyl-1-benzylpyrrolidine (96 mg, 0.3 mmol) subsequent to conversion of the benzoylthio group into a mercapto group in a similar manner as in Production Example 46 instead of l-allyloxycarbonyl-3-mercaptopyrrolidine, the title compound was obtained (30 mg, 52% yield).

Example 75

Synthesis of p-nitrobenzyl (5R,6R)-2-((R)-1-benzylpyrrolidin-2-yl)methylthio-6-((S)-1-hydroxypropyl)penem-3-carboxylate

In a similar manner as in Example 10 except for the use of p-nitrobenzyl (5R,6R)-6-((S)-1-hydroxypropyl)-2-methylsulfinylpenem-3-carboxylate (43 mg, 0.1 mmol) instead of allyl (5R,6R)-6-((S)-1-tert-butyldimethylsilyloxypropyl)-2-methylsulfinylpenem-3-carboxylate and the use of (R)-2-benzoylthiomethyl-1-benzylpyrrolidine (60 mg, 0.19 mmol) subsequent to conversion of the benzoylthio group into a mercapto group in a similar manner as in Production Example 46 instead of 1-allyloxycarbonyl-3-mercaptopyrrolidine, the title compound was obtained (28 mg, 49% yield).

Example 76

Synthesis of p-nitrobenzyl (5R,6R)-2-(1-benzylpiperidin-2-yl)methylthio-6-((S)-1-hydroxypropyl)penem-3-carboxylate

In a similar manner as in Example 10 except for the use of p-nitrobenzyl (5R,6R)-6-((S)-1-hydroxypropyl)-2-methylsulfinylpenem-3-carboxylate (86 mg, 0.2 mmol) instead of allyl (5R,6R)-6-((S)-1-tert-butyldimethylsilyloxypropyl)-2-methylsulfinylpenem-3-carboxylate and the use of 2-benzoylthiomethyl-1-benzylpiperidine (227 mg, 0.7 mmol) subsequent to conversion of the benzoylthio group into a mercapto group in a similar manner as in Production Example 46 instead of 1-allyloxycarbonyl-3-mercaptopyrrolidine, the title compound was obtained (66 mg, 57% yield).

Example 77

Synthesis of p-nitrobenzyl (5R,6R)-6-((S)-1-hydroxypropyl)-2-(2-pyridylmethyl)thiopenem-3-carboxylate

In a similar manner as in Example 10 except for the use of p-nitrobenzyl (5R,6R)-6-((S)-1-hydroxypropyl)-2-methylsulfinylpenem-3-carboxylate (64 mg, 0.15 mmol) instead of allyl (5R,6R)-6-((S)-1-tert-butyldimethylsilyloxypropyl)-2-methylsulfinylpenem-3-carboxylate and the use of 2-mercaptomethylpyridine (184 mg, 1.2 mmol) instead of 1-allyloxycarbonyl-3-mercaptopyrrolidine, the title compound was obtained (59 mg, 81% yield).

Example 78

Synthesis of p-nitrobenzyl (5R,6R)-6-((S)-1-hydroxypropyl)-2-(2-(p-nitrobenzyloxycarbonylamino)ethyl)thiopenem-3-carboxylate

In a similar manner as in Example 10 except for the use of p-nitrobenzyl (5R,6R)-6-((S)-1-hydroxypropyl)-2-methylsulfinylpenem-3-carboxylate (128 mg, 0.3 mmol) instead of allyl (5R,6R)-6-((S)-1-tert-butyldimethylsilyloxypropyl)-2-methylsulfinylpenem-3-carboxylate and the use of 2-p-nitrobenzyloxycarbonylaminoethanethiol (154 mg, 0.6 mmol) instead of 1-allyloxycarbonyl-3-mercaptopyrrolidine, the title compound was obtained (156 mg, 84% yield).

Example 79

Synthesis of p-nitrobenzyl (5R,6R)-6-((S)-1-hydroxypropyl)-2-(2-(methylaminocarbonyl)-ethyl)thiopenem-3-carboxylate

In a similar manner as in Example 10 except for the use of p-nitrobenzyl (5R,6R)-6-((S)-1-hydroxypropyl)-2-methylsulfinylpenem-3-carboxylate (42 mg, 0.1 mmol) instead of allyl (5R,6R)-6-((S)-1-tert-butyldimethylsilyloxypropyl)-2-methylsulfinylpenem-3-carboxylate and the use of 3-acetylthio-N-methylpropionamide (48 mg, 0.3 mmol) subsequent to conversion of the acetylthio group into a mercapto group in a similar manner as in Production Example 46 instead of 1-allyloxycarbonyl-3-mercaptopyrrolidine, the title compound was obtained (18 mg, 37% yield).

Example 80

Synthesis of p-nitrobenzyl (5R,6R)-6-((S)-1-hydroxypropyl)-2-(2-phenylaminocarbonylethyl)-thiopenem-3-carboxylate

In a similar manner as in Example 10 except for the use of p-nitrobenzyl (5R,6R)-6-((S)-1-hydroxypropyl)-2-methylsulfinylpenem-3-carboxylate (51 mg, 0.12 mmol) instead of allyl (5R,6R)-6-((S)-1-tert-butyldimethylsilyloxypropyl)-2-methylsulfinylpenem-3-carboxylate and the use of 3-acetylthio-N-phenylpropionamide (89 mg, 0.4 mmol) subsequent to conversion of the acetylthio group into a mercapto group in a similar manner as in Production Example 46 instead of 1-allyloxycarbonyl-3-mercaptopyrrolidine, the title compound was obtained (25 mg, 39% yield).

Example 81

Synthesis of p-nitrobenzyl (5R,6R)-2-(2-benzylaminocarbonylethyl)thio-6-((S)-1-tert-butyldimethylsilyloxypropyl) penem-3-carboxylate

To a solution of hexamethyldisilazane (0.40 mℓ, 1.89 mmol) in THF (8 mℓ), a 1.56 M hexane solution of n-butyllithium (1.15 mℓ, 1.80 mmol) was added at room temperature, followed by stirring at room temperature for 30 minutes. The reaction mixture was cooled to -78°C, to which a solution of (3R,4S)-3-((S)-1-tert-butyldimethylsilyloxypropyl)-1-(p-nitrobenzyloxycarbonylmethyl)-4-phenylthioazetidin-2-one (490 mg, 0.9 mmol) in THF (1 mℓ) was added dropwise. Ten minutes later, carbon disulfide (0.11 mℓ, 1.8 mmol) was added. Five minutes later, a solution of 3-bromopropionyl chloride (0.15 mℓ, 1.35 mmol) in THF (1 mℓ) was added dropwise. The resulting mixture was stirred for 30 minutes, followed by the addition of acetic acid (90 μℓ). The mixture so obtained was diluted with ethyl acetate, washed with brine, a saturated aqueous solution of sodium hydrogencarbonate and brine, and then dried over sodium sulfate.

The solvent was then distilled off, whereby a yellow oil (652 mg) was obtained. Its roughly-purified product (316 mg) was dissolved in methylene chloride (5 mℓ), to which sulfuryl chloride (73 μℓ, 0.73 mmol) was added under ice cooling. The resultant mixture was stirred for 20 minutes, followed by the addition of allyl acetate (0.15 mℓ, 1.39 mmol). The solvent was eliminated under ice cooling, the residue was dissolved in methylene chloride (5 mℓ), and under ice cooling, diisopropylamine (0.24 mℓ, 1.38 mmol) and benzylamine (0.15 mℓ, 1.37 mmol) were added, followed by stirring for 15 minutes. Subsequent to dilution with ethyl acetate, the resultant mixture was washed with a saturated aqueous solution of potassium hydrogensulfate, a saturated aqueous solution of sodium hydrogencarbonate and brine, and was then dried over sodium sulfate. The solvent was distilled off and the residue was purified by column chromatography, whereby the title compound was obtained as a yellow solid (105 mg, 17% yield).

Example 82

Synthesis of p-nitrobenzyl (5R,6R)-2-(2-benzylaminocarbonylethyl)thio-6-((S)-1-hydroxypropyl)-penem-3-carboxylate

In a similar manner as in Example 43 except for the use of p-nitrobenzyl (5R,6R)-2-(2-benzylaminocarbonylethyl)thio-6-((S)-1-tert-butyldimethylsilyloxypropyl)penem-3-carboxylate (85 mg, 0.13 mmol) instead of p-nitrobenzyl (5R,6R)-2-((S)-1-benzylpyrrolidin-3-yl)thio-6-((S)-1-tert-butyldimethylsilyloxypropyl)-penem-3-carboxylate, the title compound was obtained (15 mg, 21% yield).

Example 83

Synthesis of p-nitrobenzyl (5R,6R)-2-(2-phenethylaminocarbonylethyl)thio-6-((S)-1-hydroxypropyl)penem-3-carboxylate

In a similar manner as in Example 10 except for the use of p-nitrobenzyl (5R,6R)-6-((S)-1-hydroxypropyl)-2-methylsulfinylpenem-3-carboxylate (60 mg, 0.15 mmol) instead of allyl (5R,6R)-6-((S)-1-tert-butyldimethylsilyloxypropyl)-2-methylsulfinylpenem-3-carboxylate and the use of 3-acetylthio-N-phenethylpropionamide (120 mg, 0.5 mmol) subsequent to conversion of the acetylthio group into a mercapto group in a similar manner as in Production Example 46 instead of 1-allyloxycarbonyl-3-mercaptopyrrolidine, the title compound was obtained (50 mg, 58% yield).

Example 84

Synthesis of p-nitrobenzyl (5R,6R)-6-((S)-1-hydroxypropyl)-2-(2-((R)-1-phenylethyl)aminocarbonylethyl)thiopenem-3-carboxylate

In a similar manner as in Example 10 except for the use of p-nitrobenzyl (5R,6R)-6-((S)-1-hydroxypropyl)-2-methylsulfinylpenem-3-carboxylate (43 mg, 0.10 mmol) instead of allyl (5R,6R)-6-((S)-1-tert-butyldimethylsilyloxypropyl)-2-methylsulfinylpenem-3-carboxylate and the use of 3-acetylthio-N-((R)-1-phenylethyl)propionamide (75 mg, 0.3 mmol) subsequent to conversion of the acetylthio group into a mercapto group in a similar manner as in Production Example 46 instead of 1-allyloxycarbonyl-3-mercaptopyrrolidine, the title compound was obtained (49 mg, 86% yield).

Example 85

Synthesis of p-nitrobenzyl (5R,6R)-6-((S)-1-hydroxypropyl)-2-(2-((S)-1-phenylethyl)aminocarbonylethyl)thiopenem-3-carboxylate

In a similar manner as in Example 10 except for the use of p-nitrobenzyl (5R,6R)-6-((S)-1-hydroxypropyl)-2-methylsulfinylpenem-3-carboxylate (43 mg, 0.10 mmol) instead of allyl (5R,6R)-6-((S)-1-tert-butyldimethylsilyloxypropyl)-2-methylsulfinylpenem-3-carboxylate and the use of 3-acetylthio-N-((S)-1-phenylethyl)propionamide (75 mg, 0.3 mmol) subsequent to conversion of the acetylthio group into a mercapto group in a similar manner as in Production Example 46 instead of 1-allyloxycarbonyl-3-mercaptopyrrolidine, the title compound was obtained (50 mg, 89% yield).

Example 86

Synthesis of p-nitrobenzyl (5R,6R)-2-(2-(N-benzyl-N-methyl-aminocarbonyl)ethyl)thio-6-((S)-1-hydroxypropyl)penem-3-carboxylate

In a similar manner as in Example 10 except for the use of p-nitrobenzyl (5R,6R)-6-((S)-1-hydroxypropyl)-2-methylsulfinylpenem-3-carboxylate (64 mg, 0.15 mmol) instead of allyl (5R,6R)-6-((S)-1-tert-butyldimethylsilyloxypropyl)-2-methylsulfinylpenem-3-carboxylate and the use of 3-acetylthio-N-benzyl-N-methylpropionamide (110 mg, 0.5 mmol) subsequent to conversion of the acetylthio group into a mercapto group in a similar manner as in Production Example 46 instead of l-allyloxycarbonyl-3-mercaptopyrrolidine, the title compound was obtained (45 mg, 53% yield).

Example 87

Synthesis of p-nitrobenzyl (5R,6R)-2-(2-benzoylaminoethyl)thio-6-((S)-1-hydroxypropyl)-penem-3-carboxylate

In a similar manner as in Example 10 except for the use of p-nitrobenzyl (5R,6R)-6-((S)-1-hydroxypropyl)-2-methylsulfinylpenem-3-carboxylate (64 mg, 0.15 mmol) instead of allyl (5R,6R)-6-((S)-1-tert-butyldimethylsilyloxypropyl)-2-methylsulfinylpenem-3-carboxylate and the use of 1-benzoylthio-2-benzoylaminoethane (114 mg, 0.6 mmol) subsequent to conversion of the phenylthio group into a mercapto group in a similar manner as in Production Example 46 instead of 1-allyloxycarbonyl-3-mercaptopyrrolidine, the title compound was obtained (63 mg, 77% yield).

Example 88

Synthesis of p-nitrobenzyl (5R,6R)-6-((S)-1-hydroxypropyl)-2-(2-(N-methyl-N-phenacylamino)-ethyl)thiopenem-3-carboxylate

In a similar manner as in Example 10 except for the use of p-nitrobenzyl (5R,6R)-6-((S)-1-hydroxypropyl)-2-methylsulfinylpenem-3-carboxylate (42 mg, 0.1 mmol) instead of allyl (5R,6R)-6-((S)-1-tert-butyldimethylsilyloxypropyl)-2-methylsulfinylpenem-3-carboxylate and the use of N-methyl-N-phenacyl-2-acetylthioethylamine (94 mg, 0.38 mmol) subsequent to conversion of the acetylthio group into a mercapto group in a similar manner as in Production Example 46 instead of 1-allyloxycarbonyl-3-mercaptopyrrolidine, the title compound was obtained (37 mg, 65% yield).

Example 89

Synthesis of p-nitrobenzyl (5R,6R)-6-((S)-1-hydroxypropyl)-2-(2-(pyrrolidin-1-yl)ethyl)-thiopenem-3-carboxylate

In a similar manner as in Example 10 except for the use of p-nitrobenzyl (5R,6R)-6-((S)-1-hydroxypropyl)-2-methylsulfinylpenem-3-carboxylate (55 mg, 0.13 mmol) instead of allyl (5R,6R)-6-((S)-1-tert-butyldimethylsilyloxypropyl)-2-methylsulfinylpenem-3-carboxylate and the use of 1-(2-benzoylthioethyl)-pyrrolidine (69 mg, 0.3 mmol) subsequent to conversion of the benzoylthio group into a mercapto group in a similar manner as in Production Example 46 instead of 1-allyloxycarbonyl-3-mercaptopyrrolidine, the title compound was obtained (37 mg, 58% yield).

Example 90

Synthesis of p-nitrobenzyl (5R,6R)-2-(2-(4-benzylpiperazin-1-yl)ethyl)thio-6-((S)-1-hydroxypropyl)penem-3-carboxylate

In a similar manner as in Example 10 except for the use of p-nitrobenzyl (5R,6R)-6-((S)-1-hydroxypropyl)-2-methylsulfinylpenem-3-carboxylate (61 mg, 0.14 mmol) instead of allyl (5R,6R)-6-((S)-1-tert-butyldimethylsilyloxypropyl)-2-methylsulfinylpenem-3-carboxylate and the use of l-benzyl-4-(2-benzoylthioethyl)piperazine (101 mg, 0.3 mmol) subsequent to conversion of the benzoylthio group into a mercapto group in a similar manner as in Production Example 46 instead of 1-allyloxycarbonyl-3-mercaptopyrrolidine, the title compound was obtained (67 mg, 78% yield).

Example 91

Synthesis of p-nitrobenzyl (5R,6R)-6-((S)-1-hydroxypropyl)-2-(2-(4-(2-pyrimidyl)piperazin-1-yl)ethyl)thiopenem-3-carboxylate

In a similar manner as in Example 10 except for the use of p-nitrobenzyl (5R,6R)-6-((S)-1-hydroxypropyl)-2-methylsulfinylpenem-3-carboxylate (42 mg, 0.1 mmol) instead of allyl (5R,6R)-6-((S)-1-tert-butyldimethylsilyloxypropyl)-2-methylsulfinylpenem-3-carboxylate and the use of 1-(2-benzoylthioethyl)-4-(2-pyrimidyl)piperazine (101 mg, 0.3 mmol) subsequent to conversion of the benzoylthio group into a mercapto group in a similar manner as in Production Example 46 instead of 1-allyloxycarbonyl-3-mercaptopyrrolidine, the title compound was obtained (40 mg, 70% yield).

Example 92

Synthesis of p-nitrobenzyl (5R,6R)-6-((S)-1-hydroxypropyl)-2-(2-(pyrrolidin-2-on-1-yl)ethyl)-thiopenem-3-carboxylate

In a similar manner as in Example 10 except for the use of p-nitrobenzyl (5R,6R)-6-((S)-1-hydroxypropyl)-2-methylsulfinylpenem-3-carboxylate (56 mg, 0.13 mmol) instead of allyl (5R,6R)-6-((S)-1-tert-butyldimethylsilyloxypropyl)-2-methylsulfinylpenem-3-carboxylate and the use of 1-(2-benzoylthioethyl)-pyrrolidin-2-one (104 mg, 0.41 mmol) subsequent to conversion of the benzoylthio group into a mercapto group in a similar manner as in Production Example 46 instead of 1-allyloxycarbonyl-3-mercaptopyrrolidine, the title compound was obtained (54 mg, 81% yield).

Example 93

Synthesis of p-nitrobenzyl (5R,6R)-6-((S)-1-hydroxypropyl)-2-(2-(1-pyrrolyl)ethyl)thiopenem-3-carboxylate

In a similar manner as in Example 10 except for the use of p-nitrobenzyl (5R,6R)-6-((S)-1-hydroxypropyl)-2-methylsulfinylpenem-3-carboxylate (75 mg, 0.18 mmol) instead of allyl (5R,6R)-6-((S)-1-tert-butyldimethylsilyloxypropyl)-2-methylsulfinylpenem-3-carboxylate and the use of 1-(2-benzoylthioethyl)-pyrrole (183 mg, 0.79 mmol) subsequent to conversion of the benzoylthio group into a mercapto group in a similar manner as in Production Example 46 instead of 1-allyloxycarbonyl-3-mercaptopyrrolidine, the title compound was obtained (65 mg, 75% yield).

Example 94

Synthesis of p-nitrobenzyl (5R,6R)-6-((S)-1-hydroxypropyl)-2-(3-phenylaminocarbonylpropyl)-thiopenem-3-carboxylate

In a similar manner as in Example 10 except for the use of p-nitrobenzyl (5R,6R)-6-((S)-1-hydroxypropyl)-2-methylsulfinylpenem-3-carboxylate (64 mg, 0.15 mmol) instead of allyl (5R,6R)-6-((S)-1-tert-butyldimethylsilyloxypropyl)-2-methylsulfinylpenem-3-carboxylate and the use of 4-acetylthio-N-phenybutyrylamide (110 mg, 0.5 mmol) subsequent to conversion of the acetylthio group into a mercapto group in a similar manner as in Production Example 46 instead of 1-allyloxycarbonyl-3-mercaptopyrrolidine, the title compound was obtained (33 mg, 40% yield).

Example 95

Synthesis of p-nitrobenzyl (5R,6R)-2-(3-benzylaminocarbonylpropyl)thio-6-((S)-1-hydroxypropyl)penem-3-carboxylate

In a similar manner as in Example 10 except for the use of p-nitrobenzyl (5R,6R)-6-((S)-1-hydroxypropyl)-2-methylsulfinylpenem-3-carboxylate (60 mg, 0.15 mmol) instead of allyl (5R,6R)-6-((S)-1-tert-butyldimethylsilyloxypropyl)-2-methylsulfinylpenem-3-carboxylate and the use of 4-acetylthio-N-benzylbutyrylamide (78 mg, 0.35 mmol) subsequent to conversion of the acetylthio group into a mercapto group in a similar manner as in Production Example 46 instead of 1-allyloxycarbonyl-3-mercaptopyrrolidine, the title compound was obtained (66 mg, 80% yield).

Example 96

Synthesis of p-nitrobenzyl (5R,6R)-2-(3-benzylaminosulfonylpropyl)thio-6-((S)-1-hydroxypropyl)penem-3-carboxylate

In a similar manner as in Example 10 except for the use of p-nitrobenzyl (5R,6R)-6-((S)-1-hydroxypropyl)-2-methylsulfinylpenem-3-carboxylate (51 mg, 0.12 mmol) instead of allyl (5R,6R)-6-((S)-1-tert-butyldimethylsilyloxypropyl)-2-methylsulfinylpenem-3-carboxylate and the use of 3-acetylthio-N-benzylpropanesulfonamide (86 mg, 0.3 mmol) subsequent to conversion of the acetylthio group into a mercapto group in a similar manner as in Production Example 46 instead of 1-allyloxycarbonyl-3-mercaptopyrrolidine, the title compound was obtained (56 mg, 77% yield).

Example 97

Synthesis of p-nitrobenzyl (5R,6R)-6-((S)-1-tert-butyldimethylsilyloxypropyl)-2-phenylthiopenem-3-carboxylate

In a similar manner as in Example 81 except for the use of (3R,4S)-3-((S)-1-tert-butyldimethylsilyloxypropyl-1-(p-nitrobenzyloxycarbonylmethyl)-4-phenylthioazetidin-2-one (123 mg, 0.22 mmol) and the use of phenyl chlorodithioformate (48 $\mu\ell$, 10.3 mmol) instead of 3-bromopropionyl chloride, the title compound was obtained as a yellow oil (113 mg). It was dissolved at 5°C in dioxane-water (2 m$\ell$, 9:1), followed by the addition of imidazole (37 mg, 0.54 mmol) at room temperature. The resultant mixture was stirred for 20 hours. By similar post-treatment as in Example 81, the title compound was obtained (21 mg, 20% yield).

Example 98

Synthesis of p-nitrobenzyl (5R,6R)-2-phenylthio-6-((S)-1-hydroxypropyl)penem-3-carboxylate

In a similar manner as in Example 43 except for the use of p-nitrobenzyl (5R,6R)-6-((S)-1-tert-butyldimethylsilyloxypropyl-2-phenylthiopenem-3-carboxylate (970 mg, 1.6 mmol) instead of p-nitrobenzyl (5R,6R)-2-((S)-1-benzylpyrrolidin-3-yl)thio-6-((S)-1-tert-butyldimethylsilyloxypropyl)penem-3-carboxylate, the title compound was obtained as a yellow foamy solid (617 mg, 87% yield).

Example 99

Synthesis of p-nitrobenzyl (5R,6R)-6-((S)-1-tert-butyldimethylsilyloxypropyl)-2-(4-phenylthiazol-2-yl)thiopenem-3-carboxylate

To a suspension of sodium hydride (12 mg, 0.48 mmol) in THF (9 mg), 2-mercapto-4-phenylthiazole (88 mg, 0.45 mmol) was added under an argon gas stream at room temperature. Five minutes later, the reaction mixture was added with p-nitrobenzyl (5R,6R)-6-((S)-1-tert-butyldimethylsilyloxypropyl)-2-methylsulfinylpenem-3-carboxylate (134 mg, 0.25 mmol).

Twenty-five minutes later, the reaction mixture was poured into ethyl acetate (100 m$\ell$), followed by successive washing with a saturated aqueous solution of potassium hydrogensulfate (50 m$\ell$), a saturated aqueous solution of sodium hydrogencarbonate (50 m$\ell$) and a saturated aqueous solution of sodium chloride (50 m$\ell$). The organic layer

was dried over anhydrous sodium sulfate, the solvent was distilled off under reduced pressure, and column chromatography was conducted using silica gel (10 mg). From ethyl acetate-hexane (1:10, V/V), the title compound was obtained as a pale yellow oil (109 mg, 66% yield).

Example 100

Synthesis of p-nitrobenzyl (5R,6R)-6-((S)-1-hydroxypropyl)-2-(4-phenyl-thiazol-2-yl)thiopenem-3-carboxylate

In a similar manner as in Example 43 except for the use of p-nitrobenzyl (5R,6R)-6-((S)-1-tert-butyldimethylsilyloxypropyl)-2-(4-phenyl-thiazol-2-yl)thiopenem-3-carboxylate (107 mg, 0.16 mmol) instead of p-nitrobenzyl (5R, 6R)-2-((S)-1-benzylpyrrolidin-3-yl)thio-6-((S)-1-tert-butyldimethylsilyloxypropyl)penem-3-carboxylate, the title compound was obtained as a pale yellow oil (33 mg, 38% yield).

Example 101

Synthesis of p-nitrobenzyl (5R,6R ) -2-((3S,5S)-1-allyloxycarbonyl-5-dimethylaminocarbonylpyrrolidin-3-yl)thio-6-((S)-1-tert-butyldimethylsilyloxypropyl)penem-3-carboxylate

In a similar manner as in Example 42 except for the use of p-nitrobenzyl (5R,6R)-6-((S)-1-tert-butyldimethylsilyloxypropyl)-2-methylsulfinylpenem-3-carboxylate (78 mg, 0.15 mmol) and the use of (3S,5S)-3-acetylthio-1-allyloxycarbonyl-5-dimethylaminocarbonylpyrrolidine (77 mg, 0.26 mmol) subsequent to conversion of the acetylthio group into a mercapto group in a similar manner as in Production Example 46 instead of (S)-3-mercapto-1-benzylpyrrolidine, the title compound was obtained (82 mg, 77% yield).

Example 102

Synthesis of p-nitrobenzyl (5R,6R)-2-((3S,5S)-1-allyloxycarbonyl-5-dimethylaminocarbonylpyrrolidin-3-yl)thio-6-((S)-1-hydroxypropyl) - penem-3-carboxylate

In a similar manner as in Example 43 except for the use of p-nitrobenzyl (5R,6R)-2-((3S,5S)-1-allyloxycarbonyl-5-dimethylaminocarbonylpyrrolidin-3 -yl)thio-6-((S)-1-tert-butyldimethylsilyloxypropyl)penem-3-carboxylate (142 mg, 0.19 mmol) instead of p-nitrobenzyl (5R,6R)-2-((S)-1-benzylpyrrolidin-3-yl)thio-6-((S)-1-tert-butyldimethylsilyloxypropyl)penem-3-carboxylate, the title compound was obtained as a pale yellow oil (101 mg, 84% yield).

Example 103

Synthesis of (5R,6R)-2-((S)-1-(1-iminoethyl)-pyrrolidin-3-yl) thio-6-((S)-1-hydroxypropyl)-penem-3-carboxylic acid

After p-nitrobenzyl (5R,6R)-6-((S)-1-hydroxypropyl)-2-((S)-p-nitrobenzyloxycarbonylpyrrolidin-3-yl)thiopenem-3-carboxylate was subjected to catalytic hydrogenation in the presence of palladium carbon, the catalyst was eliminated and the reaction product was lyophilized. The resultant lyophilizate was dissolved in a 0.1 M phosphate buffer (11 m$\ell$) of pH 8.4, followed by the addition of a solution of methyl acetimidate tetrafluoroboric acid salt (162 mg, 1.1 mmol) in THF (5 m$\ell$) over about 10 minutes at room temperature while maintaining the reaction mixture within a pH range of from 8 to 8.5 with 1 N sodium hydroxide. After the mixture was stirred at room temperature for 30 minutes, it was adjusted to pH 7.5 with 1 N hydrochloric acid, followed by lyophilization. The lyophilizate was purified by HPLC in the manner described in Example 44, whereby the title compound was obtained as a white powder (12 mg, 29% yield).

Example 104

Synthesis of (5R,6R)-2-((S)-1-($\alpha$-iminobenzyl)-pyrrolidin-3-yl)thio-6-((S)-1-hydroxypropyl)-penem-3-carboxylic acid

In a similar manner as in Example 103 except for the use of methyl benzimidate tetrafluoroboric acid salt (45 mg, 0.20 mmol) instead of methyl acetimidate tetrafluoroboric acid salt, the title compound was obtained (5 mg, 29% yield).

Example 105

Synthesis of (5R,6R)-6-((S)-1-hydroxypropyl)-2-(1-phenylpyrrolidin-3-yl)thiopenem-3-carboxylic acid

In a similar manner as in Example 44 except for the use of p-nitrobenzyl (5R,6R)-6-((S)-1-hydroxypropyl)-2-(1-phenylpyrrolidin-3-yl)thiopenem-3-carboxylate (69 mg, 0.13 mmol) instead of p-nitrobenzyl (5R,6R)-2-((S)-1-benzylpyrrolidin-3-yl)thio-6-((S)-1-hydroxypropyl)penem-3-carboxylate, the title compound was obtained as a pale yellow powder (10 mg, 19% yield).

Example 106

Synthesis of (5R,6R)-6-((S)-1-hydroxypropyl)-2-((S)-1-phenethylpyrrolidin-3-yl)thiopenem-3-carboxylic acid

In a similar manner as in Example 44 except for the use of p-nitrobenzyl (5R,6R)-2-((S)-1-phenethylpyrrolidin-3-yl)thio-6-((S)-1-hydroxypropyl)penem-3-carboxylate (16 mg, 0.028 mmol) instead of p-nitrobenzyl (5R,6R)-2-((S)-1-benzylpyrrolidin-3-yl)thio-6-((S)-1-hydroxypropyl)penem-3-carboxylate, the title compound was obtained as a pale yellow powder (3.5 mg, 28% yield).

Example 107

Synthesis of (5R,6R)-6-((S)-1-hydroxypropyl)-2-(1-((S)-2-hydroxy-2-phenylethyl)pyrrolidin-3-yl)thiopenem-3-carboxylic acid

In a similar manner as in Example 44 except for the use of p-nitrobenzyl (5R,6R)-6-((S)-1-hydroxypropyl) -2-(1-((S)-2-hydroxy-2-phenylethyl)pyrrolidin-3-yl)thiopenem-3-carboxylate (36 mg, 0.061 mmol) instead of p-nitrobenzyl (5R,6R)-2-((S)-1-benzylpyrrolidin-3-yl)thio-6-((S)-1-hydroxypropyl)penem-3-carboxylate, the title compound was obtained as a pale yellow powder (9 mg, 33% yield).

Example 108

Synthesis of (5R,6R)-6-((S)-1-hydroxypropyl)-2-(1-((R)-2-hydroxy-2-phenylethyl)pyrrolidin-3-yl)thiopenem-3-carboxylic acid

In a similar manner as in Example 44 except for the use of p-nitrobenzyl (5R,6R)-6-((S)-1-hydroxypropyl)-2-(1-((R)-2-hydroxy-2-phenylethyl)pyrrolidin-3-yl)thiopenem-3-carboxylate (26 mg, 0.044 mmol) instead of p-nitrobenzyl (5R,6R)-2-((S)-1-benzylpyrrolidin-3-yl)thio-6-((S)-1-hydroxypropyl)penem-3-carboxylate, the title compound was obtained as a pale yellow powder (4 mg, 20% yield).

Example 109

Synthesis of (5R,6R)-2-((S)-1-benzoylpyrrolidin-3-yl)thio-6-((S)-1-hydroxypropyl)penem-3-carboxylic acid

In a similar manner as in Example 44 except for the use of p-nitrobenzyl (5R,6R)-2-((S)-1-benzoylpyrrolidin-3-yl)thio-6-((S)-1-hydroxypropyl)penem-3-carboxylate (59 mg, 0.10 mmol) instead of p-nitrobenzyl (5R,6R)-2-((S)-1-benzylpyrrolidin-3-yl)thio-6-((S)-1-hydroxypropyl)penem-3-carboxylate, the title compound was obtained as a pale yellow powder (22 mg, 50% yield).

Example 110

Synthesis of (5R,6R)-2-((S)-1-acetonylpyrrolidin-3-yl)thio-6-((S)-1-hydroxypropyl)penem-3-carboxylic acid

In a similar manner as in Example 44 except for the use of p-nitrobenzyl (5R,6R)-2-((S)-1-acetonylpyrrolidin-3-yl)thio-6-((S)-1-hydroxypropyl)penem-3-carboxylate (19 mg, 0.036 mmol) instead of p-nitrobenzyl (5R,6R)-2-((S)-1-benzylpyrrolidin-3-yl)thio-6-((S)-1-hydroxypropyl)penem-3-carboxylate, the title compound was obtained as a pale yellow powder (6 mg, 43% yield).

Example 111

Synthesis of (5R,6R)-6-((S)-1-hydroxypropyl)-2-((S)-1-phenacylpyrrolidin-3-yl)thiopenem-3-carboxylic acid

In a similar manner as in Example 44 except for the use of p-nitrobenzyl (5R,6R)-6-((S)-1-hydroxypropyl)-2-((S)-1-phenacylpyrrolidin-3-yl)thiopenem-3-carboxylate (59 mg, 0.10 mmol) instead of p-nitrobenzyl (5R,6R)-2-((S)-1-benzylpyrrolidin-3-yl)thio-6-((S)-1-hydroxypropyl)penem-3-carboxylate, the title compound was obtained as a pale yellow powder (9 mg, 20% yield).

Example 112

Synthesis of (5R,6R)-2-((S)-1-(2-p-fluorophenyl-2-oxoethyl)pyrrolidin-3-yl)thio-6-((S)-1-hydroxypropyl)penem-3-carboxylic acid

In a similar manner as in Example 44 except for the use of p-nitrobenzyl (5R,6R)-2-((S)-1-(2-p-fluoro-phenyl-2-oxoethyl)pyrrolidin-3-yl)thio-6-((S)-1-hydroxypropylpenem-3-carboxylate (45 mg, 0.075 mmol) instead of p-nitrobenzyl (5R,6R)-2-((S)-1-benzylpyrrolidin-3-yl)thio-6-((S)-1-hydroxypropyl)penem-3-carboxylate, the title compound was obtained as a pale yellow powder (7 mg, 20% yield).

Example 113

Synthesis of (5R,6R)-6-((S)-1-hydroxypropyl)-2-((S)-1-(2-oxo-2-p-tolylethyl)pyrrolidin-3-yl)-thiopenem-3-carboxylic acid

In a similar manner as in Example 44 except for the use of p-nitrobenzyl (5R,6R)-6-((S)-1-hydroxypropyl)-2-((S)-1-(2-oxo-2-p-tolylethyl)pyrrolidin-3-yl)thiopenem-3-carboxylate (38 mg, 0.064 mmol) instead of p-nitrobenzyl (5R,6R)-2-((S)-1-benzylpyrrolidin-3-yl)thio-6-((S)-1-hydroxypropyl)penem-3-carboxylate, the title compound was obtained as a pale yellow powder (3 mg, 10% yield).

Example 114

Synthesis of (5R,6R)-6-((S)-1-hydroxypropyl)-2-((S)-1-(2-p-methoxyphenyl-2-oxoethyl)pyrrolidin-3-yl)thiopenem-3-carboxylic acid

In a similar manner as in Example 44 except for the use of p-nitrobenzyl (5R,6R)-6-((S)-1-hydroxypropyl)-2-((S)-1-(2-p-methoxyphenyl-2-oxoethyl)-pyrrolidin-3-yl)thiopenem-3-carboxylate (23 mg, 0.037 mmol) instead of p-nitrobenzyl (5R,6R)-2-((S)-1-benzylpyrrolidin-3-yl)thio-6-((S)-1-hydroxypropyl)-penem-3-carboxylate, the title compound was obtained as a pale yellow powder (3.5 mg, 20% yield).

Example 115

Synthesis of (5R,6R)-6-((S)-1-hydroxypropyl)-2-((S)-1-(2-p-phenylphenyl-2-oxoethyl) pyrrolidin-3-yl)thiopenem-3-carboxylic acid

In a similar manner as in Example 44 except for the use of p-nitrobenzyl (5R,6R)-6-((S)-1-hydroxypropyl)-2-((S)-1-(2-p-phenylphenyl-2-oxoethyl)-pyrrolidin-3-yl)thiopenem-3-carboxylate (27 mg, 0.041 mmol) instead of p-nitrobenzyl (5R,6R)-2-((S)-1-benzylpyrrolidin-3-yl)thio-6-((S)-1-hydroxypropyl)-penem-3-carboxylate, the title compound was obtained as a pale yellow powder (2 mg, 9% yield).

Example 116

Synthesis of (5R,6R)-2-((S)-1-(2-benzoylethyl)-pyrrolidin-3-yl)thio-6-((S)-1-hydroxypropyl)-penem-3-carboxylic acid

In a similar manner as in Example 44 except for the use of p-nitrobenzyl (5R,6R)-2-((S)-1-(2-benzoylethyl) pyrrolidin-3-yl)thio-6-((S)-1-hydroxypropyl)-penem-3-carboxylate (14 mg, 0.023 mmol) instead of p-nitrobenzyl (5R, 6R)-2-((S)-1-benzylpyrrolidin-3-yl)-thio-6-((S)-1-hydroxypropyl)penem-3-carboxylate, the title compound was obtained as a pale yellow powder (2 mg, 19% yield).

Example 117

Synthesis of (5R,6R)-2-((S)-1-(1-benzoylethyl)-pyrrolidin-3-yl)thio-6-((S)-hydroxypropyl)penem-3-carboxylic acid

In a similar manner as in Example 44 except for the use of p-nitrobenzyl (5R,6R)-2-((S)-1-(1-benzoylethyl) pyrrolidin-3-yl)thio-6-((S)-1-hydroxypropyl)-penem-3-carboxylate (37 mg, 0.06 mmol) instead of p-nitrobenzyl (5R,6R)-2-((S)-1-benzylpyrrolidin-3-yl)-thio-6-((S)-1-hydroxypropyl)penem-3-carboxylate, the title compound was obtained as a pale yellow powder (8 mg, 28% yield).

Example 118

Synthesis of (5R,6R)-6-((S)-1-hydroxypropyl)-2-((S)-1-phenylaminocarbonylmethylpyrrolidin-3-yl)-thiopenem-3-carboxylic acid

In a similar manner as in Example 44 except for the use of p-nitrobenzyl (5R,6R)-6-((S)-1-hydroxypropyl)-2-((S)-1-phenylaminocarbonylmethylpyrrolidin-3-yl)thiopenem-3-carboxylate (40 mg, 0.067 mmol) instead of p-nitrobenzyl (5R,6R)-2-((S)-1-benzylpyrrolidin-3-yl)thio-6-((S)-1-hydroxypropyl)penem-3-carboxylate, the title compound was obtained as a pale yellow powder (10 mg, 32% yield).

Example 119

Synthesis of (5R,6R)-2-((S)-benzylaminocarbonylmethylpyrrolidin-3-yl)thio-6-((S)-hydroxypropyl)-penem-3-carboxylic acid

In a similar manner as in Example 44 except for the use of p-nitrobenzyl (5R,6R)-2-((S)-1-benzylaminocarbonylmethylpyrrolidin-3-yl)thio-6-((S)-1-hydroxypropyl)penem-3-carboxylate (47 mg, 0.076 mmol) instead of p-nitrobenzyl (5R,6R)-2-((S)-1-benzylpyrrolidin-3-yl)thio-6-((S)-1-hydroxypropyl)penem-3-carboxylate, the title compound was obtained as a pale yellow powder (15 mg, 41% yield).

Example 120

Synthesis of (5R,6R)-6-((S)-1-hydroxypropyl)-2-((S)-1-(1-indanon-2-yl)pyrrolidin-3-yl)thiopenem-3-carboxylic acid

In a similar manner as in Example 44 except that p-nitrobenzyl (5R,6R)-6-((S)-1-hydroxypropyl)-2-((S)-1-(1-indanon-2-yl)pyrrolidin-3-yl)thiopenem-3-carboxylate (28 mg, 0.047 mmol) was used instead of p-nitrobenzyl (5R,6R)-2-((S)-1-benzylpyrrolidin-3-yl)thio-6-((S)-1-hydroxypropyl)penem-3-carboxylate and the reaction product so obtained was fractionated by high-performance liquid chromatography, Isomer A of the title compound was obtained as latter eluate fractions in the form of a pale yellow powder (2.3 mg, 10% yield) and Isomer B of the title compound was obtained as former eluate fractions in the form of a pale yellow powder (3.6 mg, 16% yield).

Example 121

Synthesis of (5R,6R)-6-((S)-1-hydroxypropyl)-2-((S)-1-(1-tetralon-2-yl)pyrrolidin-3-yl)thiopenem-3-carboxylic acid

In a similar manner as in Example 44 except for the use of p-nitrobenzyl (5R,6R)-6-((S)-1-hydroxypropyl)-2-((S)-1-(1-tetralon-2-yl)pyrrolidin-3-yl)thiopenem-3-carboxylate (10 mg, 0.016 mmol) instead of p-nitrobenzyl (5R,6R)-2-((S)-1-benzylpyrrolidin-3-yl)-thio-6-((S)-1-hydroxypropyl)penem-3-carboxylate, the title compound was obtained as a pale yellow powder (2 mg, 26% yield).

Example 122

Synthesis of (5R,6R)-2-((S)-1-(1-benzosuberon-2-yl)pyrrolidin-3-yl)thio-6-((S)-1-hydroxypropyl)-penem-3-carboxylic acid

In a similar manner as in Example 44 except that p-nitrobenzyl (5R,6R)-2-((S)-1-(1-benzosuberon-2-yl)-pyrrolidin-3-yl)thio-6-((S)-1-hydroxypropyl)penem-3-carboxylate (31 mg, 0.050 mmol) was used instead of p-nitrobenzyl (5R,6R)-2-((S)-1-benzylpyrrolidin-3-yl)-thio-6-((S)-1-hydroxypropyl)penem-3-carboxylate and the reaction product so obtained was fractionated by high-performance liquid chromatography, Isomer A of the title compound was obtained

as latter eluate fractions in the form of a pale yellow powder (2.6 mg, 10% yield) and Isomer B of the title compound was obtained as former eluate fractions in the form of a pale yellow powder (4.3 mg, 18% yield).

Example 123

Synthesis of (5R,6R)-6-((S)-1-hydroxypropyl)-2-((S)-1-(2-pyridylmethyl)pyrrolidin-3-yl)thiopenem-3-carboxylic acid

In a similar manner as in Example 44 except for the use of p-nitrobenzyl (5R,6R)-6-((S)-1-hydroxypropyl)-2-((S)-1-(2-pyridylmethyl)pyrrolidin-3-yl)thiopenem-3-carboxylate (30 mg, 0.053 mmol) instead of p-nitrobenzyl (5R, 6R)-2-((S)-l-benzylpyrrolidin-3-yl)-thio-6-((S)-1-hydroxypropyl)penem-3-carboxylate, the title compound was obtained as a pale yellow powder (10 mg, 44% yield).

Example 124

Synthesis of (5R,6R)-6-((S)-1-hydroxypropyl)-2-((S)-N-((1-methyl-2-pyridinio)methyl)-pyrrolidin-3-yl)thiopenem-3-carboxylic acid (Compound A) and (5R,6R)-6-((S)-1-hydroxypropyl)-2-((S)-N-(2-pyridylmethyl)-N-methyl-pyrrolidin-3-yl)thiopenem-3-carboxylic acid (Compound B)

In a similar manner as in Example 130, which will be described subsequently herein, except for the use of p-nitrobenzyl (5R,6R)-6-((S)-1-hydroxypropyl)-2-((S)-N-(2-pyridylmethyl)-pyrrolidin-3-yl)thiopenem-3-carboxylate (66 mg, 0.12 mmol) was used instead of p-nitrobenzyl (5R,6R)-6-((S)-1-hydroxypropyl)-2-(2-pyridylmethylthio)penem-3-carboxylate, Isomer A of the title compound was obtained as latter eluate fractions in the form of a pale yellow powder (15 mg, 28% yield) and Isomer B of the title compound was obtained as former eluate fractions in the form of a pale yellow powder (8 mg, 15% yield).

Example 125

Synthesis of (5R,6R)-2-(1-benzylpiperidin-3-yl)thio-6-((S)-1-hydroxypropyl)penem-3-carboxylic acid

In a similar manner as in Example 44 except that p-nitrobenzyl (5R,6R)-2-(1-benzylpiperidin-3-yl)thio-6-((S)-1-hydroxypropyl)penem-3-carboxylate (32 mg, 0.056 mmol) was used instead of p-nitrobenzyl (5R,6R)-2-((S)-1-benzylpyrrolidin-3-yl)thio-6-((S)-1-hydroxypropyl)penem-3-carboxylate and the reaction product so obtained was fractionated by high-performance liquid chromatography, Isomer A of the title compound was obtained as latter eluate fractions in the form of a pale yellow powder (5 mg, 20% yield) and Isomer B of the title compound was obtained as former eluate fractions in the form of a pale yellow powder (9 mg, 36% yield).

Example 126

Synthesis of (5R,6R)-2-((S)-1-benzylpyrrolidin-2-yl)methylthio-6-((S)-1-hydroxypropyl)penem-3-carboxylic acid

In a similar manner as in Example 44 except for the use of p-nitrobenzyl (5R,6R)-2-((S)-1-benzylpyrrolidin-2-yl)methylthio-6-((S)-1-hydroxypropyl)-penem-3-carboxylate (34 mg, 0.06 mmol) instead of p-nitrobenzyl (5R,6R)-2-((S)-1-benzylpyrrolidin-3-yl)-thio-6-((S)-1-hydroxypropyl)penem-3-carboxylate, the title compound was obtained as a pale yellow powder (10 mg, 38% yield).

Example 127

Synthesis of (5R,6R)-2-((R)-1-benzylpyrrolidin-2-yl)methylthio-6-((S)-1-hydroxypropyl)penem-3-carboxylic acid

In a similar manner as in Example 44 except for the use of p-nitrobenzyl (5R,6R)-2-((R)-1-benzylpyrrolidin-2-yl)methylthio-6-((S)-1-hydroxypropyl)-penem-3-carboxylate (28 mg, 0.05 mmol) instead of p-nitrobenzyl (5R,6R)-2-((S)-1-benzylpyrrolidin-3-yl)-thio-6-((S)-1-hydroxypropyl)penem-3-carboxylate, the title compound was obtained as a pale yellow powder (9 mg, 41% yield).

Example 128

Synthesis of (5R,6R)-2-(1-benzylpiperidin-2-yl)methylthio-6-((S)-1-hydroxypropyl)penem-3-carboxylic acid

In a similar manner as in Example 44 except for the use of p-nitrobenzyl (5R,6R)-2-(1-benzylpiperidin-2-yl) methylthio-6-((S)-1-hydroxypropyl)penem-3-carboxylate (66 mg, 0.11 mmol) instead of p-nitrobenzyl (5R,6R)-2-((S)-1-benzylpyrrolidin-3-yl)thio-6-((S)-1-hydroxypropyl)penem-3-carboxylate, the title compound was obtained as a pale yellow powder (10 mg, 20% yield).

Example 129

Synthesis of (5R,6R)-6-((S)-1-hydroxypropyl)-2-(2-pyridylmethyl)thiopenem-3-carboxylic acid

In a similar manner as in Example 44 except for the use of p-nitrobenzyl (5R,6R)-6-((S)-1-hydroxypropyl)-2-(2-pyridylmethyl)thiopenem-3-carboxylate (40 mg, 0.082 mmol) instead of p-nitrobenzyl (5R,6R)-2-((S)-1-benzylpyrrolidin-3-yl)thio-6-((S)-1-hydroxypropyl)penem-3-carboxylate, the title compound was obtained as a pale yellow powder (17 mg, 59% yield).

Example 130

Synthesis of (5R,6R)-6-((S)-1-hydroxypropyl)-2-(1-methyl-2-pyridinio)methylthiopenem-3-carboxylic acid

p-Nitrobenzyl (5R,6R)-6-((S)-1-hydroxypropyl)-2-(2-pyridylmethylthio)penem-3-carboxylate (59 mg, 0.12 mmol) was dissolved in distilled methylene chloride, followed by the addition of methyl trifluoromethanesulfonate (31 $\mu\ell$) under ice cooling. The resultant mixture was stirred for 1.5 hours. This methylene chloride solution was added to a mixture of THF (8.4 m$\ell$) and 10% palladium carbon (150 mg) in a 0.1 M phosphate buffer of pH 7 (6.1 m$\ell$), followed by stirring at room temperature under a hydrogen gas atmosphere. One hour and fifteen minutes later, the catalyst was filtered off and the residue was lyophilized. The lyophilizate was purified by high-performance liquid chromatography on a column (20 mm in diameter x 250 mm) packed with octadecylated silica gel [gradient elution: water-acetonitrile (1 mM ammonium formate)]. Subsequent to lyophilization, the title compound was obtained as a pale yellow powder (8 mg, 18% yield).

Example 131

Synthesis of (5R,6R)-2-(2-(1-iminoethylamino)-ethyl)thio-6-((S)-1-hydroxypropyl)penem-3-carboxylic acid

In a similar manner as in Example 103 except for the use of p-nitrobenzyl (5R,6R)-6-((S)-1-hydroxypropyl)-2-(2-p-nitrobenzyloxycarbonylaminoethyl)thiopenem-3-carboxylate (52 mg, 0.09 mmol) instead of p-nitrobenzyl (5R, 6R)-6-((S)-1-hydroxypropyl)-2-((S)-p-nitrobenzyloxycarbonylpyrrolidin-3-yl)thiopenem-3-carboxylate and the use of methyl acetimidate tetrafluoroboric acid salt (131 mg, 0.9 mmol), the title compound was obtained as a pale yellow powder (3.8 mg, 12% yield).

Example 132

Synthesis of (5R,6R)-2-(2-(a-iminobenzyl)-aminoethyl)thio-6-((S)-1-hydroxypropyl)penem-3-carboxylic acid

In a similar manner as in Example 103 except for the use of p-nitrobenzyl (5R,6R)-6-((S)-1-hydroxypropyl)-2-(2-p-nitrobenzyloxycarbonylaminoethyl)thiopenem-3-carboxylate (52 mg, 0.09 mmcl) instead of p-nitrobenzyl (5R, 6R)-6-((S)-1-hydroxypropyl)-2-((S)-p-nitrobenzyloxycarbonylpyrrolidin-3-yl)thiopenem-3-carboxylate and the use of methyl benzimidate tetrafluoroboric acid salt (96 mg, 0.43 mmol) instead of methyl acetimidate tetrafluoroboric acid salt,the title compound was obtained as a pale yellow powder (6 mg, 16% yield).

Example 133

Synthesis of (5R,6R)-6-((S)-1-hydroxypropyl)-2-(2-(methylaminocarbonyl)ethyl)thiopenem-3-carboxylic acid

In a similar manner as in Example 44 except for the use of p-nitrobenzyl (5R,6R)-6-((S)-1-hydroxypropyl) -2-(2- (methylaminocarbonyl) ethyl)thiopenem-3-carboxylate (18 mg, 0.056 mmol) instead of p-nitrobenzyl (5R,6R)-2-((S)-1-benzylpyrrolidin-3-yl)thio-6-((S)-1-hydroxypropyl)penem-3-carboxylate, the title compound was obtained as a pale yellow powder (6 mg, 30% yield).

Example 134

Synthesis of (5R,6R)-6-((S)-1-hydroxypropyl)-2-(2-phenylaminocarbonylethyl)thiopenem-3-carboxylic acid

In a similar manner as in Example 44 except for the use of p-nitrobenzyl (5R,6R)-6-((S)-1-hydroxypropyl)-2-(2-phenylaminocarbonylethyl)thiopenem-3-carboxylate (25 mg, 0.046 mmol) instead of p-nitrobenzyl (5R,6R)-2-((S)-1-benzylpyrrolidin-3-yl)thio-6-((S)-1-hydroxypropyl)penem-3-carboxylate, the title compound was obtained as a pale yellow powder (9 mg, 47% yield).

Example 135

Synthesis of (5R,6R)-2-(2-benzylaminocarbonylethyl)thio-6-((S)-1-hydroxypropyl)penem-3-carboxylic acid

In a similar manner as in Example 44 except for the use of p-nitrobenzyl (5R,6R)-2-(2-benzylaminocarbonylethyl)thio-6-((S)-1-hydroxypropyl)penem-3-carboxylate (50 mg, 0.09 mmol) instead of p-nitrobenzyl (5R,6R)-2-((S)-1-benzylpyrrolidin-3-yl)thio-6-((S)-1-hydroxypropyl)penem-3-carboxylate, the title compound was obtained as a pale yellow powder (22 mg, 56% yield).

Example 136

Synthesis of (5R,6R)-6-((S)-1-hydroxypropyl)-2-(2-phenethylaminocarbonylethyl)thiopenem-3-carboxylic acid

In a similar manner as in Example 44 except for the use of p-nitrobenzyl (5R,6R)-6-((S)-1-hydroxypropyl)-2-(2-phenethylaminocarbonylethyl)thiopenem-3-carboxylate (50 mg, 0.09 mmol) instead of p-nitrobenzyl (5R,6R)-2-((S)-1-benzylpyrrolidin-3-yl)thio-6-((S)-1-hydroxypropyl)penem-3-carboxylate, the title compound was obtained as a pale yellow powder (22 mg, 56% yield).

Example 137

Synthesis of (5R,6R)-6-((S)-1-hydroxypropyl)-2-(2-((R)-1-phenylethyl)aminocarbonylethyl)thiopenem-3-carboxylic acid

In a similar manner as in Example 44 except for the use of p-nitrobenzyl (5R,6R)-6-((S)-1-hydroxypropyl)-2-(2-((R)-1-phenylethyl)aminocarbonylethyl)-thiopenem-3-carboxylate (49 mg, 0.086 mmol) instead of p-nitrobenzyl (5R,6R)-2-((S)-1-benzylpyrrolidin-3-yl)-thio-6-((S)-1-hydroxypropyl)penem-3-carboxylate, the title compound was obtained as a pale yellow powder (14 mg, 37% yield).

Example 138

Synthesis of (5R,6R)-6-((S)-1-hydroxypropyl)-2-(2-((S)-1-phenylethyl)aminocarbonylethyl)thiopenem-3-carboxylic acid

In a similar manner as in Example 44 except for the use of p-nitrobenzyl (5R,6R)-6-((S)-1-hydroxypropyl)-2-(2-((S)-1-phenylethyl)aminocarbonylethyl)-thiopenem-3-carboxylate (50 mg, 0.086 mmol) instead of p-nitrobenzyl (5R,6R)-2-((S)-1-benzylpyrrolidin-3-yl)-thio-6-((S)-1-hydroxypropyl)penem-3-carboxylate, the title compound was obtained as a pale yellow powder (14 mg, 37% yield).

Example 139

Synthesis of (5R,6R)-2-(2-(N-benzyl-N-methylaminocarbonyl)ethyl)thio-6-((S)-1-hydroxypropyl)-penem-3-carboxylic acid

In a similar manner as in Example 44 except for the use of p-nitrobenzyl (5R,6R)-2-(2-(N-benzyl-N-methylaminocarbonyl)ethyl)thio-6-((S)-1-hydroxypropyl)penem-3-carboxylate (61 mg, 0.11 mmol) instead of p-nitrobenzyl (5R,6R)-2-((S)-1-benzylpyrrolidin-3-yl)thio-6-((S)-1-hydroxypropyl)penem-3-carboxylate, the title compound was obtained as a pale yellow powder (16 mg, 33% yield).

Example 140

Synthesis of (5R,6R)-2-(2-aminoethyl)thio-6-((S)-1-hydroxypropyl)penem-3-carboxylic acid

In a similar manner as in Example 44 except for the use of p-nitrobenzyl (5R,6R)-6-((S)-1-hydroxypropyl)-2-(2-(p-nitrobenzyloxycarbonylamino)ethyl)thiopenem-3-carboxylate (33 mg, 0.075 mmol) instead of p-nitrobenzyl (5R,6R)-2-((S)-1-benzylpyrrolidin-3-yl)-thio-6-((S)-1-hydroxypropyl)penem-3-carboxylate, the title compound was obtained as a pale yellow powder (4 mg, 18% yield).

Example 141

Synthesis of (5R,6R)-2-(2-benzoylaminoethyl)thio-6-((S)-1-hydroxypropyl)penem-3-carboxylic acid

In a similar manner as in Example 44 except for the use of p-nitrobenzyl (5R,6R)-2-(2-benzoylaminoethyl) thio-6-((S)-1-hydroxypropyl) penem-3-carboxylate (58 mg, 0.11 mmol) instead of p-nitrobenzyl (5R,6R)-2-((S)-1-benzylpyrrolidin-3-yl)thio-6-((S)-1-hydroxypropyl)penem-3-carboxylate, the title compound was obtained as a pale yellow powder (27 mg, 62% yield).

Example 142

Synthesis of (5R,6R)-6-((S)-1-hydroxypropyl)-2-. (2-(N-methyl-N-phenacylamino)ethyl)thiopenem-3-carboxylic acid

In a similar manner as in Example 44 except for the use of p-nitrobenzyl (5R,6R)-6-((S)-1-hydroxypropyl)-2-(2-(N-methyl-N-phenacylamino)ethyl)thiopenem-3-carboxylate (37 mg, 0.063 mmol) instead of p-nitrobenzyl (5R, 6R)-2-((S)-1-benzylpyrrolidin-3-yl)thio-6-((S)-1-hydroxypropyl)penem-3-carboxylate, the title compound was obtained as a pale yellow powder (5 mg, 18% yield).

Example 143

Synthesis of (5R,6R)-6-((S)-1-hydroxypropyl)-2-(2-(pyrrolidin-1-yl)ethyl)thiopenem-3-carboxylic acid

In a similar manner as in Example 44 except for the use of p-nitrobenzyl (5R,6R)-6-((S)-1-hydroxypropyl)-2-(2-(pyrrolidin-1-yl)ethyl)thiopenem-3-carboxylate (37 mg, 0.075 mmol) instead of p-nitrobenzyl (5R,6R)-2-((S)-1-benzylpyrrolidin-3-yl)thio-6-((S)-1-hydroxypropyl)penem-3-carboxylate, the title compound was obtained as a pale yellow powder (10 mg, 37% yield).

Example 144

Synthesis of (5R,6R)-2-(2-(4-benzylpiperazin-1-yl)ethyl)thio-6-((S)-1-hydroxypropyl)penem-3-carboxylic acid

In a similar manner as in Example 44 except for the use of p-nitrobenzyl (5R,6R)-2-(2-(4-benzylpiperazin-1-yl)ethyl)thio-6-(S)-1-hydroxypropyl)penem-3-carboxylate (74 mg, 0.12 mmol) instead of p-nitrobenzyl (5R,6R)-2-((S)-1-benzylpyrrolidin-3-yl)thio-6-((S)-1-hydroxypropyl)penem-3-carboxylate, the title compound was obtained as a pale yellow powder (7 mg, 12% yield).

Example 145

Synthesis of (5R,6R)-6-((S)-1-hydroxypropyl)-2-(2-(4-(2-pyrimidyl)piperazin-1-yl)ethyl)thio-penem-3-carboxylic acid

In a similar manner as in Example 44 except for the use of p-nitrobenzyl (5R,6R)-6-((S)-1-hydroxypropyl)-2-(2-(4-(2-pyrimidyl)piperazin-1-yl)ethyl)thiopenem-3-carboxylate (40 mg, 0.068 mmol) instead of p-nitrobenzyl (5R, 6R)-2-((S)-1-benzylpyrrolidin-3-yl)thio-6-((S)-1-hydroxypropyl)penem-3-carboxylate, the title compound was obtained as a pale yellow powder (11 mg, 36% yield).

Example 146

Synthesis of (5R,6R)-6-((S)-1-hydroxypropyl)-2-(2-(pyrrolidin-2-on-1-yl)ethylthio)-((S)-1-hydroxypropyl)penem-3-carboxylic acid

In a similar manner as in Example 44 except for the use of p-nitrobenzyl (5R,6R)-6-((S)-1-hydroxypropyl)-2-(2-(pyrrolidin-2-on-1-yl)ethylthio)-((S)-1-hydroxypropyl)-penem-3-carboxylate (65 mg, 0.12 mmol) instead of p-nitrobenzyl (5R,6R)-2-((S)-1-benzylpyrrolidin-3-yl)thio-6-((S)-1-hydroxypropyl)penem-3-carboxylate, the title compound was obtained as a pale yellow powder (21 mg, 47% yield).

Example 147

Synthesis of (5R,6R)-6-((S)-1-hydroxypropyl)-2-(2-(1-pyrrolyl)ethyl)thiopenem-3-carboxylic acid

In a similar manner as in Example 44 except for the use of p-nitrobenzyl (5R,6R)-6-((S)-1-hydroxypropyl)-2-(2-(1-pyrrolyl)ethyl)thiopenem-3-carboxylate (65 mg, 0.13 mmol) instead of p-nitrobenzyl (5R,6R)-2-((S)-1-benzylpyrrolidin-3-yl)thio-6-((S)-1-hydroxypropyl)penem-3-carboxylate, the title compound was obtained as a pale yellow powder (15 mg, 30% yield).

Example 148

Synthesis of (5R,6R)-6-((S)-1-hydroxypropyl)-2-(3-phenylaminocarbonylpropyl)thiopenem-3-carboxylic acid

In a similar manner as in Example 44 except for the use of p-nitrobenzyl (5R,6R)-6-((S)-1-hydroxypropyl)-2-(3-phenylaminocarbonylpropyl)thiopenem-3-carboxylate (33 mg, 0.059 mmol) instead of p-nitrobenzyl (5R,6R)-2-((S)-1-benzylpyrrolidin-3-yl)thio-6-((S)-1-hydroxypropyl)penem-3-carboxylate, the title compound was obtained as a pale yellow powder (10 mg, 40% yield).

Example 149

Synthesis of (5R,6R)-2-(3-benzylaminocarbonylpropyl)thio-6-((S)-1-hydroxypropyl)penem-3-carboxylic acid

In a similar manner as in Example 44 except for the use of p-nitrobenzyl (5R,6R)-2-(3-benzylaminocarbonylpropyl)thio-6-((S)-1-hydroxypropyl)penem-3-carboxylate (66 mg, 0.11 mmol) instead of p-nitrobenzyl (5R,6R)-2-((S)-1-benzylpyrrolidin-3-yl)thio-6-((S)-1-hydroxypropyl)penem-3-carboxylate, the title compound was obtained as a pale yellow powder (13 mg, 27% yield).

Example 150

Synthesis of (5R,6R)-2-(3-benzylaminosulfonylpropyl)thio-6-((S)-1-hydroxypropyl)penem-3-carboxylic acid

In a similar manner as in Example 44 except for the use of p-nitrobenzyl (5R,6R)-2-(3-benzylaminosulfonylpropyl)thio-6-((S)-1-hydroxypropyl)penem-3-carboxylate (56 mg, 0.094 mmol) instead of p-nitrobenzyl (5R,6R)-2-((S)-1-benzylpyrrolidin-3-yl)thio-6-((S)-1-hydroxypropyl)penem-3-carboxylate, the title compound was obtained as a pale yellow powder (7.3 mg, 17% yield).

Example 151

Synthesis of (5R,6R)-6-((S)-1-hydroxypropyl)-2-phenylthiopenem-3-carboxylic acid

In a similar manner as in Example 44 except for the use of p-nitrobenzyl (5R,6R)-6-((S)-1-hydroxypropyl)-2-phenylthiopenem-3-carboxylate (47 mg, 0.1 mmol) instead of p-nitrobenzyl (5R,6R)-2-((S)-1-benzylpyrrolidin-3-yl)thio-6-((S)-1-hydroxypropyl)-penem-3-carboxylate, the title compound was obtained as a pale yellow powder (16 mg, 47% yield).

Example 152

Synthesis of (5R,6R)-6-((S)-1-hydroxypropyl)-2-(4-phenylthiazol-2-yl)thiopenem-3-carboxylic acid

In a similar manner as in Example 44 except for the use of p-nitrobenzyl (5R,6R)-6-((S)-1-hydroxypropyl) -2-(4-phenylthiazol-2-yl) thiopenem-3-carboxylate (33 mg, 0.06 mmol) instead of p-nitrobenzyl (5R,6R)-2-((S)-1-benzylpyrrolidin-3-yl)thio-6-((S)-1-hydroxypropyl)penem-3-carboxylate, the title compound was obtained as a pale yellow powder (18 mg, 72% yield).

Example 153

Synthesis of (5R,6R)-2-((3S,5S)-5-dimethylaminocarbonylpyrrolidin-3-yl)thio-6-((S)-1-hydroxypropyl)penem-3-carboxylic acid

To a solution of p-nitrobenzyl (5R,6R)-2-((3S,5S)-1-allyloxycarbonyl-5-dimethylaminocarbonylpyrrolidin-3-yl) thio-6-((S)-1-hydroxypropyl)penem-3-carboxylate (97 mg, 0.16 mmol) in THF (2 mℓ), acetic acid (30 μℓ, 0.52 mmol), tetrakistriphenylphosphine palladium (17 mg, 0.015 mmol) and tributyltin hydride (8.0 mg, 0.26 mmol) were added under an argon gas stream at -28°C.

Three hours later, the reaction mixture was diluted with THF (1.9 mℓ) and subsequent to substitution by hydrogen gas under atmospheric pressure, 10% palladium carbon in a 0.1 M phosphate buffer of pH 7.0 (2.6 mℓ) was added. Three hours and thirty minutes later, the catalyst was eliminated, THF was distilled off under reduced pressure, and the residue was lyophilized. The lyophilizate was dissolved in water and then subjected to high-performance liquid chromatography on a column (20 mm in diameter x 250 mm) packed with octadecylsilylated silica gel [gradient elution: water-acetonitrile (1 mM ammonium formate) (86:14 to 32:68, V/V). By lyophilization, the title compound was obtained as a white solid (16 mg, 25% yield).

Physical data of the compounds obtained in Examples 51 to 153 are shown in Tables 27 to 62.

Table 27

| Example No. | Chemical Structure | IR($cm^{-1}$) | NMR($\delta$ ppm) |
|---|---|---|---|
| 51 | 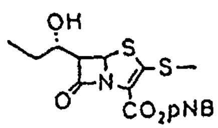 | 3520, 1780, 1684, 1522 | ($CDCl_3$) 8.21(2H,d,J=8.7Hz),7.61(2H,d,J=8.9Hz),5.76(1H,d,J=4.0Hz),5.47(2H,d,J=13.7Hz),5.21(2H,d,J=13.7Hz),4.06-4.17(1H,m),3.93(1H,dd,J=4.0Hz,10.4Hz),2.57(3H,s),1.92-2.08(1H,m),1.46-1.70(1H,m),1.06(3H,t,J=7.5Hz) |
| 52 | 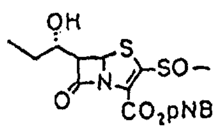 | | ($CDCl_3$) 1.06,1.08(total 3H,t,J=7.5Hz),1.6-1.7(1H,m),1.9-2.0(1H,m),1.96,2.04(total 1H,d,J=5.2Hz),2.96(3H,s),4.02,4.05(total 1H,dd,J=4.2Hz,10.3Hz),5.24,5.25(total 1H,d,J=13.5Hz),5.44,5.45(total 1H,d,J=13.5Hz),5.80,5.94(total 1H,d,J=4.2Hz),7.58,7.61(total 2H,d,J=8.8Hz),8.24(2H,d,J=8.8Hz) |
| 53 | 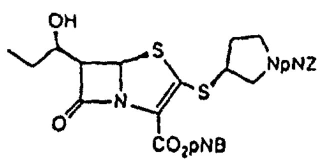 | 3447, 1785, 1684, 1552 | ($CDCl_3$) 8.22(2H,d,J=8.8Hz),8.21(2H,d,J=8.7Hz),7.61(2H,d,J=8.5Hz),7.51(2H,d,J=8.5Hz),5.78(1H,d,J=4.0Hz),5.47(1H,d,J=13.7Hz),5.60(2H,s),5.20(1H,d,J=13.8Hz),4.07-4.18(1H,m),3.87-4.00(2H,m),3.45-3.71(4H,m),2.32-2.48(1H,m),1.94-2.15(2H,m),1.50-1.70(1H,m),1.07(3H,t,J=7.5Hz) |

Table 28

| Example No. | Chemical Structure | IR($cm^{-1}$) | NMR($\delta$ ppm) |
|---|---|---|---|
| 54 | 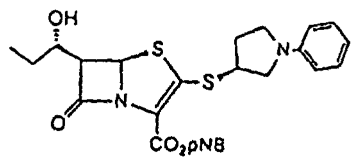 | 3500, 1785, 1684 | ($CDCl_3$) 8.20(2H,d,J=8.6Hz),7.60(1H,d,J=8.7Hz),7.60(1H,d, J=8.7Hz),7.23(1H,d,J=7.7Hz),6.73(1H,t,J=7.2Hz), 6.56(1H,d,J=8.2Hz),5.80(0.5H,d,J=4.0Hz),5.78(0.5H, d,J=4.0Hz),5.46(0.5H,d,J=13.7Hz),5.45(0.5H,d,J=13.7Hz), 5.21(0.5H,d,J=13.7Hz),5.20(0.5H,d,J=13.7Hz),4.07-4.20 (1H,m),3.99-4.07(1H,m),3.96(0.5H,dd,J=4.0Hz,10.5Hz), 3.95(0.5H,dd,J=4.0Hz,10.5Hz),3.31-3.56(4H,m),2.46-2.61 (1H,m),2.11-2.29(1H,m),1.95-2.11(1H,m),1.55-1.70(1H,m), 1.02-1.13(3H,m) |
| 55 | 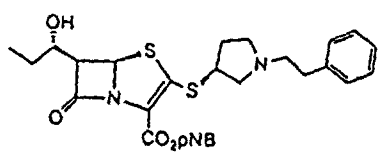 | 1696, 1790, 2812 | ($CDCl_3$) 1.06(3H,t,J=7Hz),1.61(1H,m),1.88(1H,m),1.99(1H,m),2.39(1H, m),2.61(1H,dd,J=6Hz,10Hz),2.66-2.82(6H,m),3.23(1H,dd,J=7Hz, 10Hz),3.82(1H,m),3.94(1H,dd,J=4Hz,10Hz),4.12(1H,m),5.21(1H, d,J=14Hz),5.46(1H,d,J=14Hz),5.76(1H,d,J=4Hz),7.19(3H,m), 7.28(2H,m),7.61(2H,d,J=9Hz),8.21(2H,d,J=9Hz) |
| 56 | 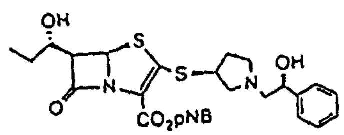 | 1607, 1691, 1789, 2890, 3460 | ($CDCl_3$) 1.07(3H,t,J=7Hz),1.58(1H,m),1.91(1H,m),1.99(1H,m),2.42(1H, m),2.56(1H,dd,J=3Hz,12Hz),2.64(1H,dd,J=2Hz,8Hz),2.71-2.81 (2H,m),2.97(1H,m),3.32(1H,dd,J=7Hz,10Hz),3.83(1H,m),3.95 (1H,dd,J=4Hz,10Hz),4.12(1H,m),4.71(1H,dd,J=3Hz,10Hz),5.22 (1H,d,J=14Hz),5.48(1H,d,J=14Hz),5.77(1H,d,J=4Hz),7.26-7.37(5H,m),7.62(2H,d,J=9Hz),8.22(2H,d,J=9Hz) |

Table 29

| Example No. | Chemical Structure | IR($cm^{-1}$) | NMR($\delta$ ppm) |
|---|---|---|---|
| 57 | 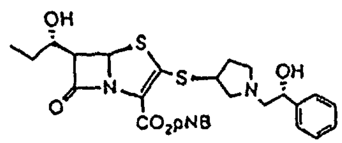 | | ($CDCl_3$) 1.07(3H,m),1.59(1H,m),1.88-2.10(2H,m),2.43(1H,m),2.55(1H,dd, J=3Hz,12Hz),2.72-2.93(4H,m),3.18(1H,dd,J=7Hz,10Hz),3.85(1H, m),3.95(1H,dd,J=4Hz,10Hz),4.10(1H,m),4.71(1H,dd,J=3Hz,10Hz), 5.21(1H,d,J=14Hz),5.46(1H,d,J=14Hz),5.78(1H,d,J=4Hz),7.28-7.37(5H,m),7.61(2H,d,J=9Hz),8.21(2H,d,J=9Hz) |
| 58 | 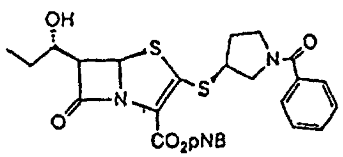 | 3422,1782, 1617 | ($CDCl_3$) 8.22(2H,d,J=8.7Hz),7.55-7.66(4H,m),7.38-7.56(3H,m), 5.75-5.87(1H,bs),5.41-5.52(2H,m),5.21(1H,d,J=14.1Hz), 4.17-4.26(1H,m),4.05-4.17(1H,m),3.35-4.05(5H,m), 2.30-2.55(1H,m),1.90-2.12(1H,m),1.32-1.55(1H,m),1.06 (3H,t,J=7.5Hz) |
| 59 | 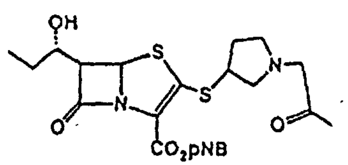 | 1695,1710, 1790,2800, 2958 | ($CDCl_3$) 1.06(3H,t,J=7Hz),1.61(1H,m),1.84-1.92(1H,m),1.94-2.04(1H,m), 2.14(3H,s),2.37-2.46(1H,m),2.61(1H,dd,J=6Hz,10Hz),2.68(1H,dd, J=9Hz,14Hz),2.81(1H,m),3.33(1H,dd,J=7Hz,10Hz),3.38(2H,s), 3.85(1H,m),3.94(1H,dd,J=3Hz,10Hz),4.12(1H,m),5.20(1H,d, J=14Hz),5.46(1H,d,J=14Hz),5.75(1H,d,J=4Hz),7.61(2H,d,J=9Hz), 8.21(2H,d,J=9Hz) |

Table 30

| Example No. | Chemical Structure | IR($cm^{-1}$) | NMR(δ ppm) |
|---|---|---|---|
| 60 | 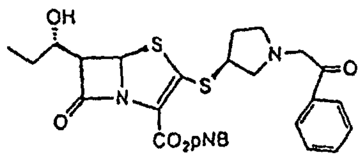 | 3400, 1782, 1690 | ($CDCl_3$) 8.21(2H,d,J=8.8Hz),7.96(2H,d,J=7.2Hz),7.54-7.70(3H,m), 7.37-7.54(2H,m),5.75(1H,d,J=4.0Hz),5.46(1H,d,J=13.7Hz), 5.21(1H,d,J=13.7Hz),4.07-4.20(1H,m),4.00(2H,s),3.85-3.96 (1H,m),3.94(1H,dd,J=4.1Hz,10.5Hz),3.49(1H,dd,J=7.3Hz,10.0Hz), 2.90-3.01(1H,m),2.62-2.85(2H,m),1.40-1.53(1H,m),1.87-2.09(2H,m),1.51-1.70(1H,m),1.06(3H,t,J=7.5Hz) |
| 61 | 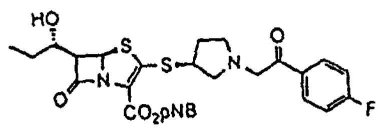 | 1599, 1694, 1788, 2793, 3018 | ($CDCl_3$) 1.06(3H,t,J=7Hz),1.58(1H,m),1.86-1.99(2H,m),2.31(1H,m), 2.69(1H,m),2.76(1H,m),2.86-2.96(2H,m),3.25(1H,dd,J=7Hz, 10Hz),3.56(1H,m),3.93(1H,m),3.96(2H,s),4.12(1H,m),5.21 (1H,d,J=14Hz),5.47(1H,d,J=14Hz),5.75(1H,d,J=4Hz),7.12(2H, m),7.61(2H,d,J=9Hz),8.03(2H,m),8.21(2H,d,J=9Hz) |
| 62 | 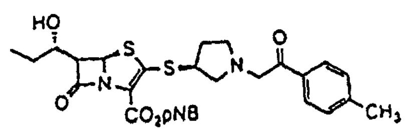 | 1607, 1694, 1790, 2969 | ($CDCl_3$) 1.07(3H,t,J=7Hz),1.63(1H,m),1.86-2.00(2H,m),2.30(1H,m), 2.40(3H,s),2.67(1H,dd,J=6Hz,10Hz),2.74(1H,dd,J=8Hz,16Hz), 2.92(1H,m),3.28(1H,dd,J=7Hz,10Hz),3.53(1H,m),3.97(2H,s), 4.02(1H,dd,J=4Hz,10Hz),4.16(1H,m),5.21(1H,d,J=14Hz),5.46 (1H,d,J=14Hz),5.75(1H,d,J=4Hz),7.25(2H,m),7.61(2H,d,J=9Hz), 7.87(2H,d,J=9Hz),8.21(2H,d,J=9Hz) |

Table 31

| Example No. | Chemical Structure | IR($cm^{-1}$) | NMR(δ ppm) |
|---|---|---|---|
| 63 | 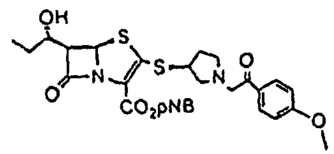 | 1602,1690,<br>1792,2962 | ($CDCl_3$)<br>1.06(3H,t,J=7Hz),1.59(1H,m),1.89-2.01(2H,m),2.46(1H,m),<br>2.68(1H,m),2.73(1H,m),2.96(1H,m),3.49(1H,dd,J=7Hz,10Hz),3.87<br>(3H,s),3.93(1H,m),3.96(2H,s),4.13(1H,m),5.21(1H,d,J=14Hz),<br>5.86(1H,d,J=14Hz),5.75(1H,d,J=4Hz),6.93(2H,d,J=9Hz),7.61(2H,<br>d,J=9Hz),7.96(2H,d,J=9Hz),8.21(2H,d,J=9Hz) |
| 64 | 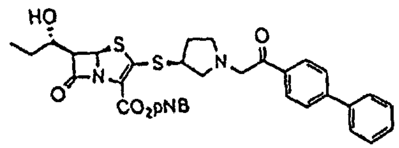 | 1604,1690,<br>1786,2940 | ($CDCl_3$)<br>1.06(3H,t,J=7Hz),1.61(1H,m),1.90-2.04(2H,m),2.34(1H,m),<br>2.72(1H,m),2.79(1H,m),2.95(1H,m),3.31(1H,dd,J=7Hz,10Hz),<br>3.59(1H,m),3.94(1H,dd,J=4Hz,10Hz),4.04(2H,s),4.10(1H,m),<br>5.21(1H,d,J=14Hz),5.46(1H,d,J=14Hz),5.76(1H,d,J=4Hz),<br>7.38-7.49(3H,m),7.60-7.72(6H,m),8.06(2H,d,J=8Hz),<br>8.21(2H,d,J=9Hz) |
| 65 | 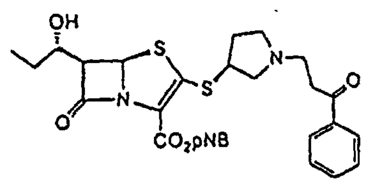 | 1608,1685,<br>1791,2362,<br>2941 | ($CDCl_3$)<br>1.06(3H,t,J=7Hz),1.60(1H,m),1.86(1H,m),1.99(1H,m),2.37(1H,m),<br>2.66(1H,dd,J=5Hz,10Hz),2.70(2H,t,J=7Hz),2.95(2H,m),3.18(2H,t,<br>J=7Hz),3.81(1H,m),3.93(1H,dd,J=4Hz,10Hz),4.12(1H,m),5.20(1H,<br>d,J=14Hz),5.46(1H,d,J=14Hz),5.75(1H,d,J=4Hz),7.47(2H,d,J=<br>7Hz),7.54-7.62(3H,m),7.96(2H,d,J=9Hz),8.21(2H,d,J=9Hz) |

Table 32

| Example No. | Chemical Structure | IR(cm⁻¹) | NMR(δ ppm) |
|---|---|---|---|
| 66 | 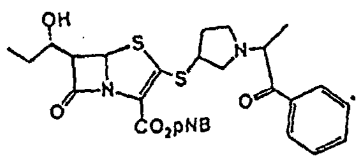 | 1654, 1684, 1780, 2950 | ($CDCl_3$) 1.06(3H,t,J=7Hz),1.38(3H,d,J=7Hz),1.71-2.04(3H,m),2.40-2.58 (1H,m),2.59-2.87(3H,m),3.41(1H,m),3.85(1H,m),3.29(1H,m), 4.06-4.15(2H,m),5.20(1H,m),5.46(1H,m),5.73(1H,d,J=6Hz), 7.40-7.49(2H,m),7.53-7.63(3H,m),8.06(2H,m),8.20(2H,m) |
| 67 | 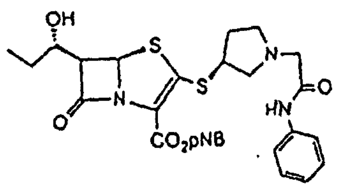 | 1602, 1685, 1791, 2822, 3336 | ($CDCl_3$) 1.06(3H,t,J=7Hz),1.65(1H,m),1.98(2H,m),2.40-2.51(1H,m), 2.62-2.71(2H,m),3.05(2H,m),3.33(2H,dd,J=17Hz,36Hz), 3.86-3.95(2H,m),4.08-4.15(1H,m),5.20(1H,d,J=14Hz),5.49(1H, d,J=14Hz),5.75(1H,d,J=4Hz),7.10(1H,m),7.30(2H,d,J= 8Hz),7.60(4H,d,J=7Hz),8.22(2H,d,J=9Hz) |
| 68 | 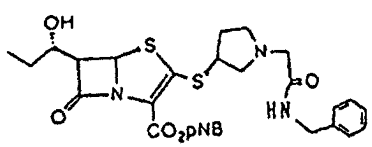 | 1608, 1674, 1790, 2360, 2965, 3388 | ($CDCl_3$) 1.06(3H,t,J=7Hz),1.60(1H,m),1.86(1H,m),1.96(1H,m),2.37(1H, m),2.61(1H,m),2.83-2.92(2H,m),3.06(1H,dd,J=7Hz,10Hz), 3.24(2H,d,J=4Hz),3.79(1H,m),3.95(1H,dd,J=4Hz,10Hz),4.08(1H, m),4.47(2H,m),5.17(1H,d,J=14Hz),5.45(1H,d,J=14Hz),5.74(1H, d,J=4Hz),7.27-7.39(5H,m),7.60(2H,d,J=9Hz),8.21(2H,d,J=9Hz) |

Table 33

| Example No. | Chemical Structure | IR($cm^{-1}$) | NMR($\delta$ ppm) |
|---|---|---|---|
| 69 | 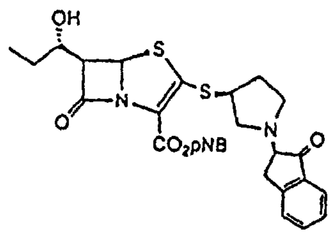 | 1604, 1710, 1788, 2358, 2936, 3023 | ($CDCl_3$) 1.06(3H, t, J=7Hz), 1.59(1H, m), 1.87(1H, m), 1.98(1H, m), 2.37(1H, m), 2.58(1H, m), 2.66-2.84(4H, m), 3.05-3.22(1H, m), 3.79(1H, m), 3.93(1H, dd, J=4Hz, 10Hz), 4.11(1H, m), 4.68(1H, m), 5.20(1H, d, J=14Hz), 5.46(1H, d, J=14Hz), 5.73(1H, m), 7.45(2H, d, J=7Hz), 7.59-7.65(3H, m), 7.76(1H, d, J=8Hz), 8.21(1H, d, J=9Hz) |
| 70 | 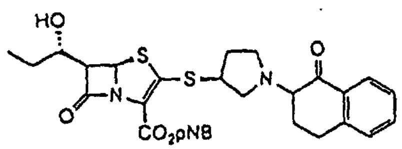 | 1604, 1684, 1787, 2800, 2962 | ($CDCl_3$) 1.06(3H, m), 1.60(1H, m), 1.87-2.02(2H, m), 2.14-2.20(1H, m), 2.25-2.32(2H, m), 2.44-2.58(3H, m), 2.72-2.84(1H, m), 2.93-3.03(1H, m), 3.20-3.40(1H, m), 3.59(1H, m), 3.76(1H, m), 3.94(1H, m), 4.11(1H, m), 5.20(1H, m), 5.46(1H, m), 5.72(1H, m), 7.40(2H, m), 7.50(1H, m), 7.61(2H, m), 8.01(1H, d, J=8Hz), 8.21(2H, m) |
| 71 | 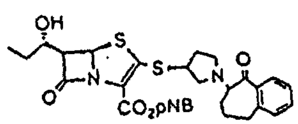 | 1602, 1682, 1788, 2950, 3035 | ($CDCl_3$) 1.06(3H, t, J=7Hz), 1.68(1H, m), 1.75-1.90(3H, m), 1.96(1H, m), 2.10-2.47(4H, m), 2.50-2.60(1H, m), 2.68-2.95(2H, m), 3.34(1H, dd, J=7Hz, 10Hz), 3.45(1H, dd, J=4Hz, 4Hz), 3.60-3.80(1H, m), 3.93(1H, m), 4.09(1H, m), 5.20(1H, m), 5.46(1H, m), 5.72(1H, m), 7.22(1H, dd, J=8Hz, 8Hz), 7.33-7.47(3H, m), 7.62(2H, m), 8.21(2H, m) |

Table 34

| Example No. | Chemical Structure | IR($cm^{-1}$) | NMR($\delta$ ppm) |
|---|---|---|---|
| 72 | 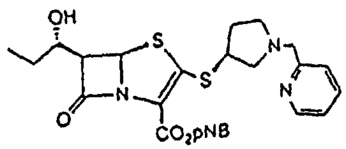 | 3290, 1792, 1676 | ($CDCl_3$)<br>8.55(1H,d,J=5.1Hz),8.21(2H,d,J=8.7Hz),7.66(1H,t,J=7.6Hz), 7.61(2H,d,J=8.7Hz),7.38(1H,d,J=7.9Hz),7.14-7.21(1H,m),5.73 (1H,d,J=3.9Hz),5.46(1H,d,J=13.7Hz),5.20(1H,d,J=13.7Hz), 4.08-4.17(1H,m),3.92(1H,dd,J=4.0Hz,10.5Hz),3.78-3.90(1H,m), 3.80(1H,d,J=2.9Hz),3.25(1H,dd,J=7.3Hz,10.2Hz),2.56(1H,t,J=7.0Hz),2.67(1H,dd,J=5.7Hz,10.2Hz),2.35-2.49(1H,m),1.95-2.05 (1H,m),1.85-1.95(1H,m),1.49-1.67(1H,m),1.06(3H,t,J=7.4Hz) |
| 73 | 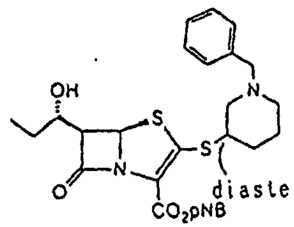<br>diastereoisomer | | (Isomer A)<br>($CDCl_3$)<br>1.05(3H,t,J=7.4Hz),1.55-1.7(2H,m),1.7-1.85(3H,m),1.95-2.1(2H,m),2.2-2.3(1H,m),2.85-3.0(2H,m),3.1-3.3(2H,m),3.36 (1H,d,J=13.0Hz),3.92(1H,dd,J=4.0Hz,10.3Hz),4.02(1H,d,J=13.0Hz),4.05-4.15(1H,m),5.22(1H,d,J=13.8Hz),5.48(1H,d, J=13.8Hz),5.71(1H,d,J=4.0Hz),7.2-7.35(5H,m),7.62(2H,d,J=8.5Hz),8.19(2H,d,J=8.5Hz)<br><br>(Isomer B)<br>($CDCl_3$)<br>1.06(3H,t,J=7.4Hz),1.55-1.65(2H,m),1.65-1.8(3H,m),1.9-2.1 (2H,m),2.2-2.3(1H,m),2.85-3.0(2H,m),3.1-3.3(2H,m),3.39(1H,d, J=13.1Hz),3.91(1H,dd,J=4.0Hz,10.3Hz),4.02(1H,d,J=13.1Hz), 4.05-4.15(1H,m),5.22(1H,d,J=13.8Hz),5.48(1H,d,J=13.8Hz), 5.69(1H,d,J=4.0Hz),7.2-7.35(5H,m),7.62(2H,d,J=8.5Hz), 8.19(2H,d,J=8.5Hz) |

Table 35

| Example No. | Chemical Structure | IR($cm^{-1}$) | NMR($\delta$ ppm) |
|---|---|---|---|
| 74 | 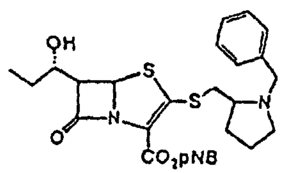 | | ($CDCl_3$) 1.06(3H,t,J=7.4Hz),1.55-1.85(5H,m),1.95-2.1(2H,m),2.2-2.3(1H,m),2.85-3.0(2H,m),3.11(1H,dd,J=7.3Hz,12.1Hz),3.29(1H,dd,J=2.9Hz,12.1Hz),3.34(1H,d,J=13.1Hz),3.93(1H,dd,J=4.0Hz,10.4Hz),4.03(1H,d,J=13.1Hz),4.1-4.15(1H,m),5.22(1H,d,J=13.8Hz),5.49(1H,d,J=13.8Hz),5.72(1H,d,J=4.0Hz),7.2-7.4(5H,m),7.63(2H,d,J=8.5Hz),8.20(2H,d,J=8.5Hz) |
| 75 | 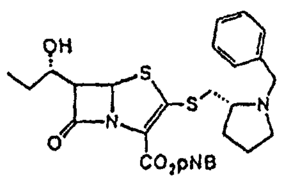 | | ($CDCl_3$) 1.07(3H,t,J=7.4Hz),1.55-1.8(5H,m),1.95-2.1(2H,m),2.2-2.3(1H,m),2.85-3.0(2H,m),3.19(1H,d,J=2.8Hz),3.20(1H,s),3.38(1H,d,J=13.1Hz),3.92(1H,dd,J=3.9Hz,10.5Hz),4.02(1H,d,J=13.1Hz),4.05-4.15(1H,m),5.22(1H,d,J=13.8Hz),5.48(1H,d,J=13.8Hz),5.70(1H,d,J=3.9Hz),7.2-7.4(5H,m),7.62(2H,d,J=8.7Hz),8.20(2H,d,J=8.7Hz) |
| 76 | 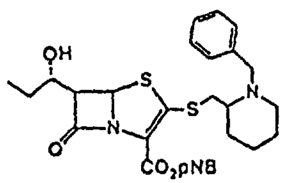 | | ($CDCl_3$) 1.05,1.06(total 3H,t,J=7.3Hz),1.35-1.8(6H,m),1.95-2.15(2H,m),2.7-2.8(3H,m),3.2-3.45(3H,m),3.85-3.95(1H,m),3.95-4.05(1H,m),4.05-4.15(1H,m),5.22(1H,d,J=13.7Hz),5.48,5.49(total 1H,d,J=13.7Hz),5.69,5.70(total 1H,d,J=4.0Hz),7.2-7.4(5H,m),7.63(2H,d,J=8.6Hz),8.20(2H,d,J=8.6Hz) |

Table 36

| Example No. | Chemical Structure | IR(cm$^{-1}$) | NMR(δ ppm) |
|---|---|---|---|
| 77 | 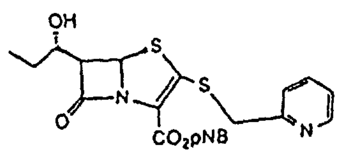 | 3310, 1790, 1730, 1679 | ($CDCl_3$) 8.58(1H,d,J=4.0Hz),8.21(2H,d,J=8.7Hz),7.57-7.75(1H,m),7.60(2H,d,J=8.7Hz),7.40(1H,d,J=7.7Hz),7.17-7.30(1H,m),5.73(1H,d,J=4.0Hz),5.47(1H,d,J=13.7Hz),5.20(1H,d,J=13.7Hz),4.44(1H,d,J=13.5Hz),4.35(1H,d,J=13.5Hz),3.96-4.07(1H,m),3.93(1H,dd,J=4.0Hz,10.3Hz),1.90-2.05(1H,m),1.50-1.67(1H,m),1.05(3H,t,J=7.4Hz) |
| 78 | 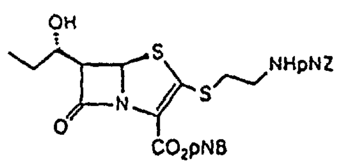 | 3385, 1780, 1700, 1521 | ($CDCl_3$) 8.22(2H,d,J=8.7Hz),8.21(2H,d,J=8.7Hz),7.61(2H,d,J=8.6Hz),7.50(2H,d,J=8.3Hz),5.72(1H,d,J=3.9Hz),5.47(1H,d,J=13.7Hz),5.12-5.29(3H,m),4.05-4.19(1H,m),3.94(1H,dd,J=4.0Hz,10.5Hz),3.49-3.65(2H,m),3.18-3.30(1H,m),3.07-3.18(1H,m),1.92-2.05(1H,m),1.45-1.68(1H,m),1.06(3H,t,J=7.5Hz) |
| 79 | 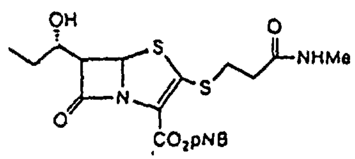 | 1608, 1680, 1789, 2361, 2944, 3016, 3640 | ($CDCl_3$) 1.06(3H,t,J=7Hz),1.58(1H,m),1.91-2.01(1H,m),2.58(2H,t,J=7Hz),2.82(3H,d,J=5Hz),3.24-3.39(2H,m),3.94(1H,dd,J=4Hz,10Hz),4.12(1H,m),5.20(1H,d,J=14Hz),5.46(1H,d,J=14Hz),5.75(1H,d,J=4Hz),7.60(1H,d,J=9Hz),8.21(1H,d,J=9Hz) |

Table 37

| Example No. | Chemical Structure | IR($cm^{-1}$) | NMR(δ ppm) |
|---|---|---|---|
| 8 0 | 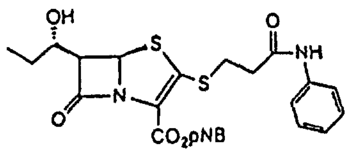 | 1603, 1696, 1790, 3029 | ($CDCl_3$) 1.06(3H,t,J=7Hz),1.59(1H,m),1.92-2.00(1H,m),2.78(2H,t,J=7Hz), 3.31-3.47(2H,m),3.94(1H,dd,J=4Hz,10Hz),4.12(1H,dd,J=7Hz,14Hz), 5.20(1H,d,J=14Hz),5.45(1H,d,J=14Hz),5.74(1H,d,J=4Hz),7.12(1H, d,J=7Hz),7.33(2H,d,J=8Hz),7.49(2H,d,J=8Hz),7.59(2H,d,J=9Hz), 8.19(2H,d,J=9Hz) |
| 8 1. | 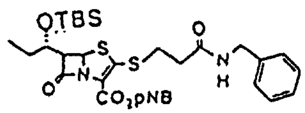 | 1785, 1650, 1522 | ($CDCl_3$) 8.21(2H,d,J=8.6Hz),7.60(2H,d,J=8.6Hz),5.72(1H,d,J=4.0Hz), 5.45(2H,d,J=13.7Hz),5.23(2H,d,J=13.7Hz),5.43(2H,d,J=5.9Hz), 4.30-4.40(1H,m),4.08-4.17(1H,m),3.22-3.47(2H,m),2.56-2.69 (2H,m),1.72-1.89(1H,m),1.49-1.62(1H,m),0.98(3H,t,J=7.3Hz), 0.87(9H,s),0.11(3H,s),0.10(3H,s) |
| 8 2 | 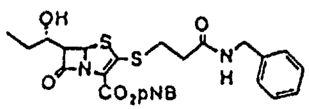 | | ($CDCl_3$) 8.21(2H,d,J=8.7Hz),7.60(2H,d,J=8.6Hz),7.21-7.41(5H,m),5.74 (1H,d,J=4.0Hz),5.46(1H,d,J=13.4Hz),5.21(1H,d,J=13.8Hz), 4.45(1H,d,J=5.5Hz),4.08-4.18(1H,m),3.94(1H,dd,J=4.0Hz,10.3Hz), 3.35-3.45(1H,m),3.24-3.35(1H,m),2.63(2H,t,J=7.1Hz),1.90-2.08(1H,m),1.58-1.79(1H,m),1.26(2H,t,J=7.1Hz),1.06(3H,t, J=7.4Hz) |

Table 38

| Example No. | Chemical Structure | IR(cm$^{-1}$) | NMR(δ ppm) |
|---|---|---|---|
| 83 | 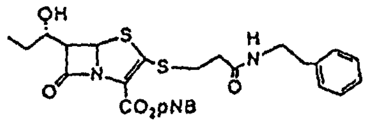 | 3400, 1784, 1654, 1522, 1330, 1118 | ($CDCl_3$) 1.06(t, J=7Hz, 3H), 1.55-1.65(m, 1H), 1.95-2.05(m, 1H), 2.53(t, J=7Hz, 2H), 2.82(t, J=7Hz, 2H), 3.20-3.40(m, 2H), 3.53(q, J=6Hz, 2H), 3.94(dd, J=10Hz, 4Hz, 1H), 4.10-4.18(m, 1H), 5.20(d, J=14Hz, 1H), 5.73(d, J=14Hz, 1H), 5.74(d, J=4Hz, 1H), 7.18(d, J=8Hz, 2H), 7.23(t, J=8Hz, 1H), 7.30(t, J=7Hz, 2H), 7.60(d, J=9Hz, 2H), 8.20(d, J=9Hz, 2H) |
| 84 | 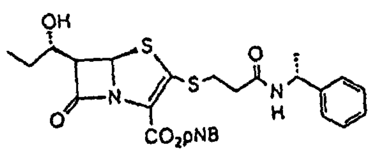 | 1607, 1678, 1787, 2360, 2986, 3432 | ($CDCl_3$) 1.04(3H, t, J=7Hz), 1.49(3H, d, J=7Hz), 1.56(1H, m), 1.70-2.05 (1H, m), 2.40(1H, d, J=6Hz), 2.57(2H, m), 3.26(2H, m), 3.93(1H, dd, J=4Hz, 10Hz), 4.03(1H, m), 5.10(1H, quint., J=7Hz), 5.20(1H, d, J=14Hz), 5.47(1H, d, J=14Hz), 5.73(1H, d, J=4Hz), 5.95(1H, d, J=8Hz), 7.23-7.36(5H, m), 7.61(2H, d, J=9Hz), 8.21(2H, d, J=9Hz) |
| 85 | 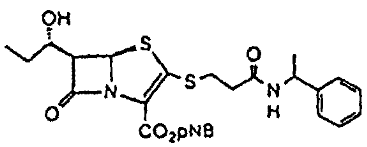 | 1608, 1674, 1786, 2792, 3433 | ($CDCl_3$) 1.06(3H, t, J=7Hz), 1.48(3H, d, J=7Hz), 1.56(1H, m), 1.96(1H, m), 2.57(2H, t, J=7Hz), 3.28(2H, dt, J=5Hz, 7Hz), 3.92(1H, dd, J=4Hz, 10Hz), 4.09(1H, m), 5.10(1H, quint., J=7Hz), 5.20(1H, d, J=14Hz), 5.44(1H, d, J=14Hz), 5.71(1H, d, J=14Hz), 5.93(1H, d, J=7Hz), 7.27-7.32(5H, m), 7.60(2H, d, J=9Hz), 8.20(2H, d, J=7Hz) |

Table 39

| Example No. | Chemical Structure | IR($cm^{-1}$) | NMR($\delta$ ppm) |
|---|---|---|---|
| 86 | 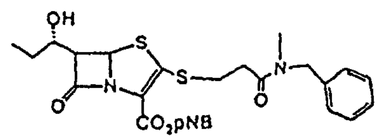 | 3420, 1790, 1694, 1635, 1340 | ($CDCl_3$) 1.00-1.03(m,3H), 1.55-1.70(m,1H), 1.90-2.05(m,1H), 2.75-2.85 (m,2H), 2.91, 2.98(s, total 3H), 3.23-3.45(m,2H), 3.87-3.97(m,1H), 4.10-4.22(m,1H), 4.52, 4.58(s, total 2H), 5.17-5.25(m,1H), 5.40-5.50(m,1H), 5.65, 5.76(each d, J=4Hz, total 1H), 7.10-7.40(m,5H), 7.55-7.62(m,2H), 8.20-8.25(m,2H) |
| 87 | 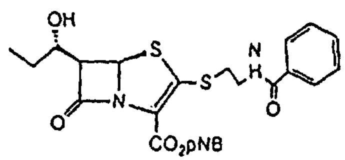 | 3370, 1786, 1688 | ($CDCl_3$) 8.21(2H,d,J=8.8Hz), 7.76(2H,d,J=7.1Hz), 7.61(2H,d,J=8.7Hz), 7.53(1H,t,J=7.3Hz), 7.44(2H,t,J=7.7Hz), 6.50-6.62(1H,m), 5.69 (1H,d,J=3.6Hz), 5.46(1H,d,J=13.7Hz), 5.21(1H,d,J=13.7Hz), 3.95-4.05(1H,m), 3.92(1H,dd,J=3.8Hz,10.5Hz), 3.68-3.88(2H,m), 3.19-3.39(2H,m), 1.84-1.99(1H,m), 1.45-1.70(1H,m), 1.01(3H,t, J=7.4Hz) |
| 88 | 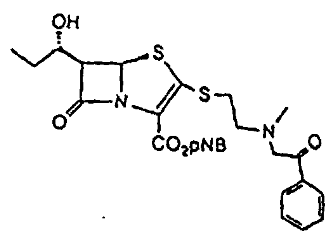 | 1608, 1694, 1787, 2969 | ($CDCl_3$) 1.05(3H,t,J=7Hz), 1.59(1H,m), 1.97(1H,m), 2.47(3H,s), 2.88 (2H,m), 3.16(2H,m), 3.92(1H,m), 3.94(2H,s), 4.09(1H,m), 5.20(1H, d,J=14Hz), 5.46(1H,d,J=14Hz), 5.72(1H,d,J=4Hz), 7.46(2H,d, J=8Hz), 7.56(1H,d,J=7Hz), 7.61(2H,d,J=9Hz), 7.97 (2H,d,J=7Hz), 8.20(2H,d,J=9Hz) |

Table 40

| Example No. | Chemical Structure | IR($cm^{-1}$) | NMR(δ ppm) |
|---|---|---|---|
| 89 | 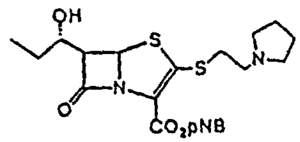 | | ($CDCl_3$) 1.06(3H,t,J=7.4Hz),1.55-1.65(1H,m),1.75-1.85(4H,m),1.95-2.05(1H,m),2.5-2.65(4H,m),2.75-2.9(3H,m),3.09-3.16(1H,m),3.18-3.24(1H,m),3.93(1H,dd,J=4.0Hz,10.4Hz),4.1-4.2(1H,m),5.21(1H,d,J=13.8Hz),5.47(1H,d,J=13.8Hz),5.74(1H,d,J=4.0Hz),7.61(2H,d,J=8.6Hz),8.21(2H,d,J=8.6Hz) |
| 90 | 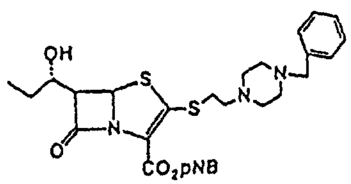 | | ($CDCl_3$) 1.06(3H,t,J=7.4Hz),1.55-1.7(2H,m),1.95-2.05(1H,m),2.47(4H,bs),2.51(4H,bs),2.65-2.75(2H,m),3.07-3.14(1H,m),3.15-3.22(1H,m),3.50(2H,s),3.92(1H,dd,J=4.0Hz,10.4Hz),4.1-4.15(1H,m),5.21(1H,d,J=13.8Hz),5.47(1H,d,J=13.8Hz),5.73(1H,d,J=4.0Hz),7.27-7.31(5H,m),7.61(2H,d,J=8.6Hz),8.21(2H,d,J=8.6Hz) |
| 91 | 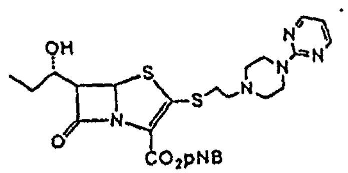 | | ($CDCl_3$) 1.06(3H,t,J=7.4Hz),1.55-1.65(1H,m),1.71(1H,d,J=4.9Hz),1.95-2.05(1H,m),2.55(4H,t,J=5.0Hz),2.7-2.8(2H,m),3.13-3.19(1H,m),3.21-3.28(1H,m),3.83(4H,t,J=5.0Hz),3.94(1H,dd,J=4.0Hz,10.5Hz),4.1-4.2(1H,m),5.21(1H,d,J=13.8Hz),5.48(1H,d,J=13.8Hz),5.76(1H,d,J=4.0Hz),6.49(1H,t,J=4.8Hz),7.62(2H,d,J=8.7Hz),8.22(2H,d,J=8.7Hz),8.30(2H,d,J=4.8Hz) |

Table 41

| Example No. | Chemical Structure | IR($cm^{-1}$) | NMR($\delta$ ppm) |
|---|---|---|---|
| 92 | 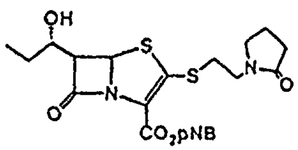 | | ($CDCl_3$) 1.06(3H,t,J=7.4Hz),1.56-1.68(1H,m),1.95-2.01(1H,m),2.03-2.08(3H,m),2.37(2H,t,J=8.1Hz),3.1-3.17(1H,m),3.19-3.26(1H,m),3.46(2H,t,J=7.1Hz),3.52-3.58(1H,m),3.63-3.7(1H,m),3.95(1H,dd,J=4.0Hz,10.3Hz),4.1-4.2(1H,m),5.21(1H,d,J=13.7Hz),5.46(1H,d,J=13.7Hz),5.76(1H,d,J=4.0Hz),7.61(2H,d,J=8.7Hz),8.22(2H,d,J=8.7Hz) |
| 93 | 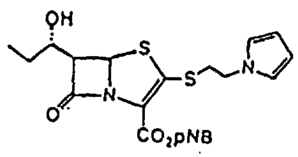 | | ($CDCl_3$) 1.07(3H,t,J=7.4Hz),1.55-1.63(1H,m),1.68(1H,d,J=5.6Hz),1.95-2.01(1H,m),3.23-3.3(1H,m),3.35-3.42(1H,m),3.93(1H,dd,J=4.0Hz,10.3Hz),4.07-4.12(1H,m),4.21(2H,t,J=6.8Hz),5.21(1H,d,J=13.7Hz),5.47(1H,d,J=13.7Hz),5.71(1H,d,J=4.0Hz),6.16(2H,t,J=2.1Hz),6.67(2H,t,J=2.1Hz),7.61(2H,d,J=8.7Hz),8.22(2H,d,J=8.7Hz) |
| 94 | 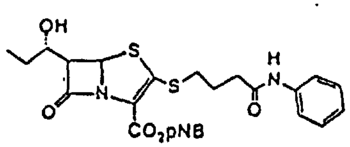 | 3360,1780,1684,1522,1330 | ($CDCl_3$) 1.04(t,J=7Hz,3H),1.60-1.70(m,1H),1.90-2.03(m,1H),2.15-2.25(m,2H),2.50-2.60(m,2H),3.05-3.25(m,2H),3.93(dd,J=10Hz,4Hz,1H),4.05-4.15(m,2H),5.20(d,J=14Hz,1H),5.43(d,J=14Hz,1H),5.72(d,J=4Hz,1H),7.12(t,J=7Hz,1H),7.32(t,J=7Hz,2H),7.49(d,J=7Hz,2H),7.60(d,J=8Hz,2H),8.21(d,J=8Hz,2H) |

Table 42

| Example No. | Chemical Structure | IR($cm^{-1}$) | NMR($\delta$ ppm) |
|---|---|---|---|
| 95 | 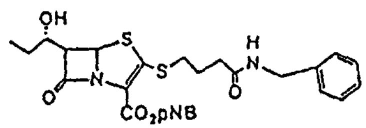 | 3370, 1781, 1654, 1329 | ($CDCl_3$) 1.06(t, J=7Hz, 3H), 1.50-1.60(m, 1H), 1.90-2.00(m, 1H), 2.05-2.15(m, 2H), 2.30-2.40(m, 2H), 3.00-3.15(m, 2H), 3.93(dd, J=10Hz, 4Hz, 1H), 4.10-4.15(m, 1H), 4.43(d, J=6Hz, 2H), 5.20(d, J=14Hz, 1H), 5.45(d, J=14Hz, 1H), 5.72(d, J=4Hz, 1H), 7.20-7.35(m, 5H), 7.60(d, J=8Hz, 2H), 8.20(d, J=8Hz, 2H) |
| 96 | 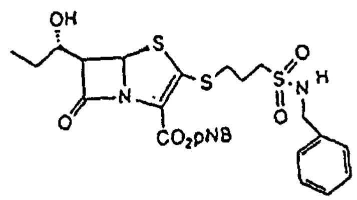 | 1608, 1691, 1790, 2361, 2929, 3030 | ($CDCl_3$) 1.06(3H, t, J=8Hz), 1.55-1.63(1H, m), 1.94-2.00(1H, m), 2.21(2H, t, J=7Hz), 3.05(2H, t, J=7Hz), 3.12(2H, m), 3.94(1H, dd, J=4Hz, 10Hz), 4.12(1H, dd, J=7Hz, 14Hz), 4.31(2H, d, J=6Hz), 5.21(1H, d, J=14Hz), 5.46(1H, d, J=14Hz), 5.74(1H, d, J=4Hz), 7.34(5H, m), 7.61(2H, d, J=9Hz), 8.21(2H, d, J=9Hz) |
| 97 | 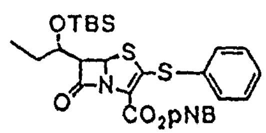 | | ($CDCl_3$) 0.02(6H, s), 0.74(s, 9H), 0.94(3H, t, J=7Hz), 1.7-1.9(m, 2H), 4.01(dd, J=10Hz, 4Hz, 1H), 4.2-4.3(m, 1H), 5.24(d, J=14Hz, 1H), 5.52(d, J=14Hz, 1H), 5.52(d, J=4Hz, 1H), 7.3-7.5(m, 5H), 7.65(d, J=11Hz, 2H), 8.25(d, J=11Hz; 2H) |

Table 43

| Example No. | Chemical Structure | IR($cm^{-1}$) | NMR($\delta$ ppm) |
|---|---|---|---|
| 98 | 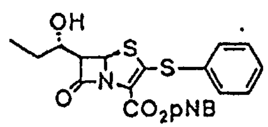 | 3415, 1784, 1684 | ($CDCl_3$) 8.23(2H,d,J=8.8Hz),7.65(2H,d,J=8.8Hz),7.64(2H,d,J=8.0Hz),7.40(3H,t,J=8.0Hz),5.60(1H,d,J=4Hz),5.52(1H,d,J=13.7Hz),5.26(1H,d,J=13.7Hz),3.99-4.08(1H,m),3.86(1H,dd,J=4.1Hz,10.5Hz),1.89-2.01(1H,m),1.45-1.64(1H,m),1.02(3H,t,J=7.5Hz) |
| 99 | 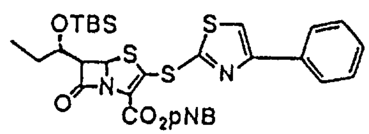 | | ($CDCl_3$) -0.14(3H,s),0.01(3H,s),0.70(9H,s),0.95(3H,t,J=7.5Hz),1.7-1.9(2H,m),4.05(1H,dd,J=4.1Hz,9.8Hz),4.3-4.35(1H,m),5.28(1H,d,J=14.0Hz),5.51(1H,d,J=14.0Hz),5.61(1H,d,J=4.1Hz),7.35-7.5(3H,m),7.63(2H,d,J=8.7Hz),7.66(1H,s),7.89(2H,d,J=8.5Hz),8.24(2H,d,J=8.7Hz) |
| 100 | 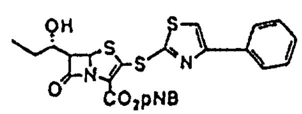 | | ($CDCl_3$) 1.03(3H,t,J=7.4Hz),1.6-1.7(1H,m),1.77(1H,d,J=10.8Hz),1.9-2.0(1H,m),3.91(1H,dd,J=4.1Hz,10.3Hz),4.1-4.2(1H,m),5.28(1H,d,J=13.6Hz),5.51(1H,d,J=13.6Hz),5.68(1H,d,J=4.1Hz),7.35-7.5(3H,m),7.64(2H,d,J=8.5Hz),7.69(1H,s),7.91(2H,d,J=8.4Hz),8.24(2H,d,J=8.5Hz) |

Table 44

| Example No. | Chemical Structure | IR(cm$^{-1}$) | NMR(δ ppm) |
|---|---|---|---|
| 101 | 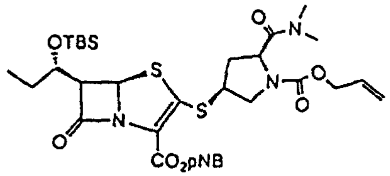 | | ($CDCl_3$) 0.12(3H,s),0.12(3H,s),0.88(9H,s),0.99(3H,t,J=7.4Hz),1.75-1.9(1H,m),1.95-2.05(1H,m),2.65-2.8(1H,m),2.98(3H,s),3.10(3H,s),3.5-3.6(1H,m),3.7-3.85(1H,m),4.1-4.15(1H,m),4.25-4.4(2H,m),4.5-4.7(2H,m),4.7-4.8(1H,m),5.15-5.35(3H,m),5.45(1H,d,J=13.7Hz),5.75(1H,d,J=4.0Hz),5.8-6.0(1H,m),7.60(2H,d,J=8.7Hz),8.22(2H,d,J=8.7Hz) |
| 102 | 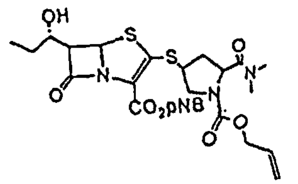 | | ($CDCl_3$) 1.06(3H,t,J=7.4Hz),1.55-1.75(1H,m),1.95-2.1(1H,m),2.7-2.8(1H,m),2.98(3H,s),3.11(3H,s),3.54(1H,t,J=10.2Hz),3.7-3.85(1H,m),3.95(1H,dd,J=4.0Hz,10.2Hz),4.1-4.2(1H,m),4.25-4.4(1H,m),4.5-4.7(2H,m),4.7-4.8(1H,m),5.15-5.35(3H,m),5.45(1H,d,J=13.7Hz),5.78(1H,d,J=4.0Hz),5.85-6.0(1H,m),7.60(2H,d,J=8.6Hz),8.22(2H,d,J=8.6Hz) |
| 103 | 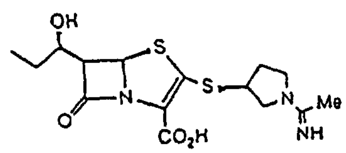 | | ($D_2O$) 5.81,5.82(1H,each d,each J=3.6Hz),4.05-4.25(3H,m),3.99(1H,dd,J=6.4Hz,12.3Hz),3.75-3.95(3H,m),3.54-3.75(2H,m),2.47-2.62(1H,m),2.32-2.29(3H,d,J=4.2Hz),2.10-2.26(1H,m),1.80-1.95(1H,m),1.50-1.67(1H,m),1.00(3H,t,J=7.4Hz) |

Table 45

| Example No. | Chemical Structure | IR($cm^{-1}$) | NMR($\delta$ ppm) |
|---|---|---|---|
| 104 | 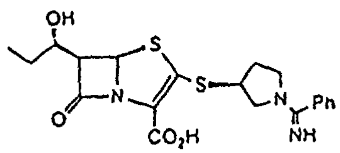 | 3387, 1766, 1606, 1378 | ($D_2O$) 0.9-1.05(m,3H), 1.42-1.62(m,1H), 1.75-1.95(m,1H), 2.00-2.17(m, 0.3H), 2.20-2.35(m,0.7H), 2.35-2.50(m,0.3H), 2.55-2.75(m,0.7H), 3.60-4.35(m,8H), 5.48(d,J=4Hz,0.7H), 5.85(d,J=4Hz,0.3H), 7.50-7.80 (m,5H) |
| 105 | 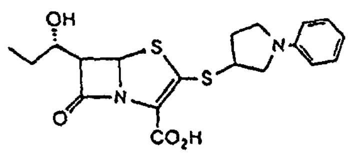 | 3450, 1768, 1599, 1507, 1370 | ($CD_3OD$) 1.02(t,J=7Hz,3H), 1.45-1.60(m,1H), 1.80-1.95(m,1H), 2.05-2.25(m, 1H), 2.50-2.65(m,1H), 3.30-3.50(m,4H), 3.70-3.85(m,1H), 3.90-4.15(m, 2H), 5.78-5.82(m,1H), 6.64(d,J=8Hz,2H), 6.72(t,J=7Hz,1H), 7.24(t,J=7Hz, 2H) |
| 106 | 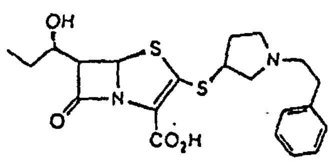 | 1765, 1592, 1376 | ($CD_3OD$) 1.03(t,J=7Hz,3H), 1.45-1.60(m,1H), 1.85-1.95(m,1H), 1.95-2.10 (m,1H), 2.50-2.60(m,1H), 3.00-3.10(m,2H), 3.20-3.45(m,4H), 3.45-3.60(m,2H), 3.90-4.10(m,3H), 5.68(d,J=4Hz,1H), 7.25-7.40 (m,5H) |

Table 46

| Example No. | Chemical Structure | IR($cm^{-1}$) | NMR(δ ppm) |
|---|---|---|---|
| 107 | 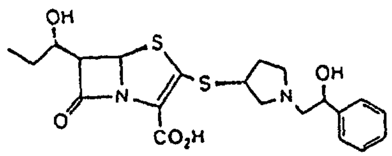 | 3360, 1764, 1577, 1376 | ($CD_3OD$) 1.03(t, J=7Hz, 3H), 1.45-1.55(m, 1H), 1.85-1.95(m, 1H), 2.05-2.12(m, 1H), 2.52-2.62(m, 1H), 3.35-3.45(m, 3H), 3.53-3.65(m, 2H), 3.75-3.85(m, 1H), 3.90(dd, J=10Hz, 4Hz, 1H), 3.93-4.02(m, 1H), 4.05-4.52(m, 1H), 5.00-5.05(m, 1H), 5.71(d, J=4Hz, 1H), 7.31(d, J=7Hz, 1H), 7.38(t, J=7Hz, 1H), 7.44(d, J=7Hz, 1H) |
| 108 | 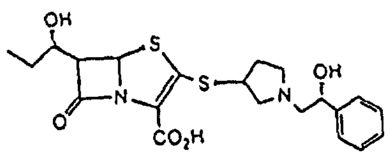 | 3350, 1762, 1582, 1381 | ($CD_3OD$) 1.03(t, J=7Hz, 3H), 1.45-1.58(m, 1H), 1.85-1.95(m, 1H), 2.00-2.10 (m, 1H); 2.50-2.60(m, 1H), 3.20-3.45(m, 4H), 3.45-3.55(m, 1H), 3.65-3.75(m, 1H), 3.89(dd, J=10Hz, 4Hz, 1H), 3.95-4.00(m, 1H), 4.00-4.08(m, 1H), 4.97(dd, J=10Hz, 3Hz, 1H), 5.69(d, J=4Hz, 1H), 7.25-7.32 (m, 1H); 7.37(t, J=7Hz, 2H), 7.43(d, J=7Hz, 2H) |
| 109 | 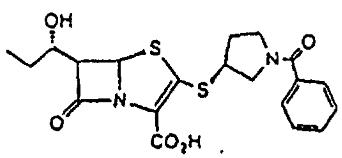 | 3430, 1782, 1612, 1436 | ($CD_3OD$) 0.98-1.03(m, 3H), 1.45-1.60(m, 1H), 1.80-1.98(m, 1H), 2.00-2.15(m, 1H), 2.40-2.55(m, 1H), 3.55-4.20(m, 7H), 5.60, 5.80(bs, total 1H), 7.40-7.55(m, 5H) |

Table 47

| Example No. | Chemical Structure | IR (cm$^{-1}$) | NMR (δ ppm) |
|---|---|---|---|
| 110 | 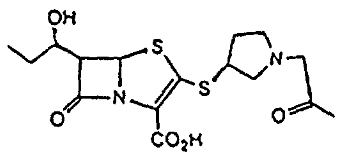 | 3400, 1762, 1580, 1375 | ($CD_3OD$) 1.03(t, J=7Hz, 3H), 1.45-1.55(m, 1H), 1.85-2.03(m, 2H), 2.16(s, 3H), 2.40-2.50(m, 1H), 3.04-3.18(m, 3H), 3.45-3.55(m, 1H), 3.89(s, 2H), 3.90-4.00(m, 3H), 5.72(d, J=4Hz, 1H) |
| 111 | 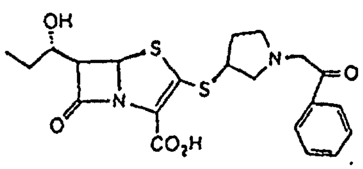 | 3430, 1772, 1694, 1595, 1376 | ($CD_3OD$) 1.03(t, J=7Hz, 3H), 1.50-1.60(m, 1H), 1.85-1.95(m, 1H), 1.95-2.05(m, 1H), 2.50-2.60(m, 1H), 3.20-3.30(3H, m), 3.62-3.70(m, 1H), 3.90-4.08(m, 3H), 4.58(s, 2H), 5.72(d, J=3Hz, 1H), 7.53(t, J=7Hz, 2H), 7.66(t, J=7Hz, 1H), 8.00(d, J=7Hz, 2H) |
| 112 | 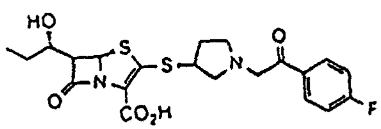 | 3400, 1768, 1600, 1377, 1237 | ($CD_3OD$) 1.03(t, J=7Hz, 3H), 1.45-1.58(m, 1H), 1.85-1.95(m, 1H), 1.95-2.05(m, 1H), 2.45-2.60(m, 1H), 3.15-3.40(m, 3H), 3.60-3.70(m, 1H), 3.90-4.00(m, 2H), 4.00-4.08(m, 1H), 4.53(s, 2H), 5.72(d, J=5Hz, 2H), 7.26(t, J=9Hz, 2H), 8.09(dd, J=12Hz, 5Hz, 2H) |

Table 48

| Example No. | Chemical Structure | IR($cm^{-1}$) | NMR($\delta$ ppm) |
|---|---|---|---|
| 113 | 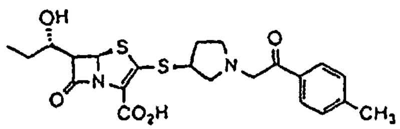 | 3410, 1770, 1605, 1376 | ($CD_3OD$) 1.03(t, J=7Hz, 3H), 1.45-1.55(m, 1H), 1.85-1.95(m, 1H), 1.97-2.05(m, 1H), 2.42(s, 3H), 2.47-2.57(m, 1H), 3.20-3.35(m, 2H), 3.65-3.72(m, 1H), 3.90-4.08(m, 4H), 4.57(s, 2H), 5.71(d, J=4Hz, 1H), 7.35(d, J=8Hz, 2H), 7.90(d, J=8Hz, 2H) |
| 114 | 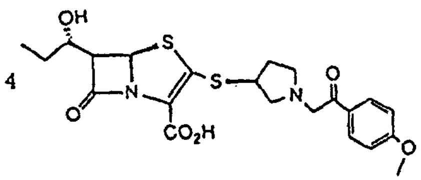 | 3450, 1772, 1600, 1378 | ($CD_3OD$) 1.02(t, J=7Hz, 3H), 1.42-1.55(m, 1H), 1.82-1.95(m, 1H), 1.95-2.10(m, 1H), 2.45-2.65(m, 1H), 3.20-3.45(m, 4H), 3.74(dd, J=12Hz, 7Hz, 1H), 3.89(s, 3H), 3.90-3.95(m, 2H), 4.00-4.10(m, 1H), 4.66(s, 2H), 5.71(d, J=3Hz, 1H), 7.05(d, J=9Hz, 2H), 8.00(d, J=9Hz, 2H) |
| 115 | 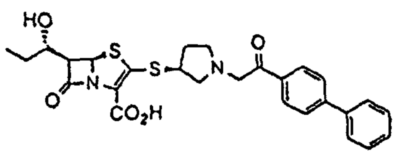 | 3394, 1770, 1688, 1604, 1373 | ($CD_3OD$) 1.03(t, J=7Hz, 3H), 1.45-1.57(m, 1H), 1.85-1.95(m, 1H), 1.95-2.05(m, 1H), 2.50-2.58(m, 1H), 3.12-3.18(m, 1H), 3.2-3.3(bs, 1H), 3.62-3.70(m, 1H), 3.90-4.08(m, 3H), 4.54(s, 2H), 5.71(d, J=4Hz, 1H), 7.43(q, J=4Hz, 1H), 7.48(t, J=8Hz, 2H), 7.69(d, J=7Hz, 2H), 7.79(d, J=8Hz, 2H), 8.09(d, J=8Hz, 2H) |

Table 49

| Example No. | Chemical Structure | IR($cm^{-1}$) | NMR($\delta$ ppm) |
|---|---|---|---|
| 116 | 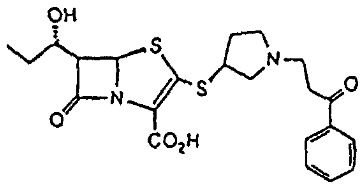 | 3390,1765, 1682,1581, 1378 | ($CD_3OD$) 1.01(t,J=7Hz,3H),1.45-1.55(m,1H),1.80-2.05(m,2H),2.45-2.55(m, 1H),3.1-3.7(m,8H),3.87(dd,J=10Hz,4Hz,1H),3.90-4.05(m,2H),5.67(d, J=4Hz,1H),7.51(t,J=7Hz,2H),7.61(t,J=7Hz,1H),8.01(d,J=7Hz,2H) |
| 117 | 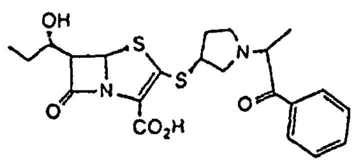 | 3400,1772, 1590,1375 | ($CD_3OD$) 1.02(t,J=7Hz,3H),1.49(d,3H,J=6Hz),1.45-1.55(m,1H),1.85-2.10(m, 2H),2.45-2.55(m,1H),3.05-3.25(m,2H),3.50-3.60,3.65-3.75(total 1H,m),3.87-4.05(m,3H),5.69,5.71(d,each J=3Hz,total 1H), 7.55-7.60(m,2H),7.67(t,J=7Hz,1H),8.05-8.10(m,2H) |
| 118 | 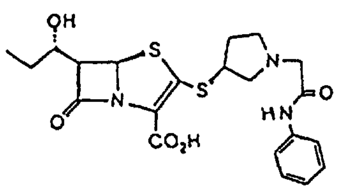 | 3490,1769, 1600,1558, 1380 | ($CD_3OD$-$CD_3CO_2D$-$D_2O$) 1.03(t,J=7Hz,3H),1.40-1.60(m,1H),1.80-2.10(m,2H),2.5-2.6(m,1H), 3.15-3.4(m,3H),3.60(dd,J=9Hz,6Hz,1H),3.90(ABq,J=30Hz,18Hz,2H), 3.92-4.00(m,2H),4.03-4.14(m,1H),5.76(d,J=3Hz,1H),7.12(t,J=9Hz, 1H),7.33(t,J=9Hz,2H),7.57(dd,J=9Hz,1Hz,2H) |

Table 50

| Example No. | Chemical Structure | IR($cm^{-1}$) | NMR($\delta$ ppm) |
|---|---|---|---|
| 119 | 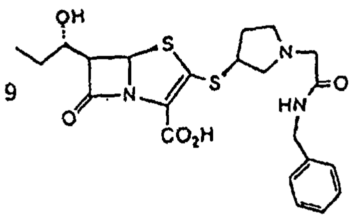 | 3320, 1768, 1682, 1581, 1377 | ($CD_3OD$) 1.03(t, J=7Hz, 3H), 1.45-1.57(m, 1H), 1.82-1.95(m, 2H), 2.37-2.50(m, 1H), 2.80-2.87(m, 1H), 2.94(dd, J=10Hz, 4Hz, 1H), 2.97-3.05(m, 1H), 3.23(dd, J=10Hz, 7Hz, 1H), 3.40(ABq, J=25Hz, 15Hz, 2H), 3.85-3.97(m, 3H), 4.40(ABq, J=23Hz, 15Hz, 2H), 5.70(d, J=3Hz, 1H), 7.2-7.35(m, 5H) |
| 120 | 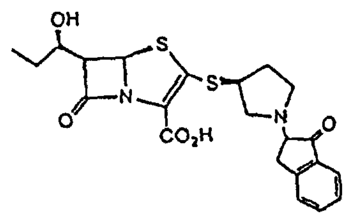 | 1764, 1720, 1381, 1284 | (Isomer A) ($CD_3OD-CD_3CO_2D-D_2O$) 1.01(t, J=7Hz, 3H), 1.50-1.60(m, 1H), 1.80-1.90(m, 1H), 2.10-2.20(m, 1H), 2.60-2.73(m, 1H), 3.03(dd, J=9Hz, 2Hz, 1H), 3.50-3.70(m, 2H), 3.60-3.70(m, 1H), 3.92-4.02(m, 2H), 4.07(dd, J=10Hz, 4Hz, 1H), 4.20-4.30(m, 1H), 5.40(br.d, J=5Hz, 1H), 5.80(d, J=4Hz, 1H), 7.76(t, J=7Hz, 1H), 7.88-8.00(m, 3H) |
| | 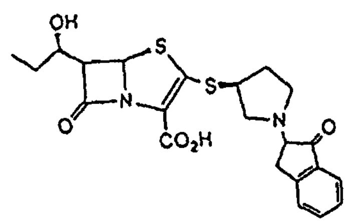 | 1763, 1724, 1373, 1284 | (Isomer B) ($CD_3OD-CD_3CO_2D-D_2O$) 1.01(t, J=7Hz, 3H), 1.50-1.60(m, 1H), 1.80-1.92(m, 1H), 2.10-2.20(m, 1H), 2.65-2.75(m, 1H), 3.00-3.10(m, 1H), 3.42-3.52(m, 2H), 3.60-3.70(m, 1H), 3.90-4.00(m, 2H), 4.05(dd, J=10Hz, 4Hz, 1H), 5.38(br.d, 1H), 5.80(d, J=4Hz, 1H), 7.75(t, J=7Hz, 1H), 7.88-7.98(m, 3H) |

Table 51

| Example No. | Chemical Structure | IR(cm⁻¹) | NMR(δ ppm) |
|---|---|---|---|
| 121 | 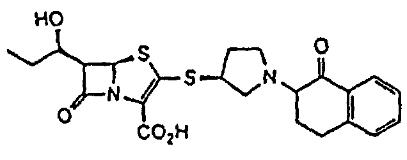 | 1775, 1370 | ($CD_3OD$)<br>1.02(t,J=7Hz,3H),1.45-1.57(m,1H),1.75-1.95(m,2H),2.15-2.25(m,1H),2.30-2.45(m,2H),2.50-2.60(m,2H),2.85-3.05(m,3H),3.40-3.50(m,1H),3.65-3.70(m,1H),3.8-4.0(m,2H),5.70-5.75(m,1H),7.45(t,J=8Hz,1H),7.50(t,J=7Hz,1H),7.58(t,J=8Hz,1H),7.95(d,J=8Hz,1H) |
| 122 | 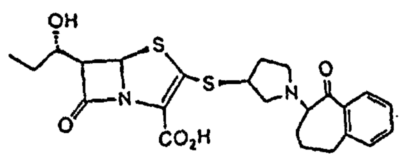 | 3440, 1772, 1560, 1380 | (Isomer A)<br>($CD_3OD$)<br>1.02(t,J=7Hz,3H),1.34-1.50(m,2H),1.72-2.0(m,4H),3.20-3.45(m,3H),2.70-2.90(m,4H),3.16(dd,J=11Hz,7Hz,1H),3.8-3.9(m,1H),3.9-4.0(bs,3H),5.7(bs,1H),7.3-7.55(m,4H) |
| | 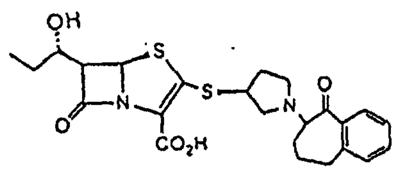 | 1772, 1558, 1374 | (Isomer B)<br>($CD_3OD$)<br>1.02(t,J=7Hz,3H),1.34-1.60(m,2H),1.75-1.95(m,4H),2.2-2.4(m,3H),2.65-2.90(m,4H),3.40-3.50(m,1H),3.85-3.95(bs,3H),3.95-4.05(m,1H),5.70(bs,1H),7.3-7.55(m,4H) |

Table 52

| Example No. | Chemical Structure | IR(cm⁻¹) | NMR(δ ppm) |
|---|---|---|---|
| 123 | 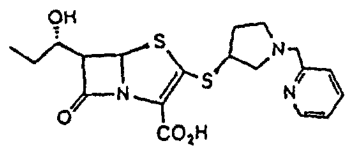 | 3400, 1769, 1672, 1376 | ($CD_3OD$) 1.02(t, J=7Hz, 3H), 1.43-1.57(m, 1H), 1.82-1.95(m, 1H), 2.00-2.12 (m, 1H), 2.52-2.62(m, 1H), 3.30-3.42(m, 3H), 3.62-3.72(m, 1H), 3.90-3.98(m, 2H), 4.05-4.12(m, 1H), 4.34(s, 2H), 5.71(d, J=4Hz, 1H), 7.39(t, J=5Hz, 1H), 7.49(d, J=7Hz, 1H), 7.86(dt, J=8Hz, 2Hz, 1H), 8.60(d, J=5Hz, 1H) |
| 124 | 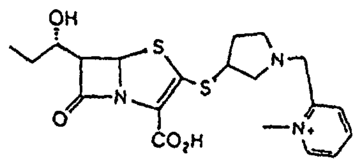 | 3380, 1762, 1580, 1360 | ($CD_3OD$) 1.01(t, J=7Hz, 3H), 1.42-1.58(m, 1H), 1.77-1.95(m, 2H), 2.35-2.45(m, 1H), 2.66(q, J=8Hz, 1H), 2.85-2.92(m, 1H), 2.92-3.05(m, 2H), 3.83(dd, J=10Hz, 4Hz, 2H), 3.90-4.00(m, 1H), 4.41(s, 3H), 5.58(d, J=4Hz, 1H), 7.93(t, J=7Hz, 1H), 8.20(d, J=8Hz, 1H), 8.54(t, J=8Hz, 1H), 8.86(d, J=6Hz, 1H) |
| | 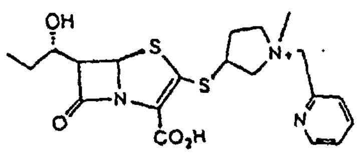 | 3400, 1762, 1586, 1375 | ($CD_3OD$) 1.03(t, J=7Hz, 3H), 1.45-1.57(m, 1H), 1.85-1.97(m, 1H), 2.20-2.30(m, 1H), 2.70-2.85(m, 1H), 3.25(s, 3H), 3.67-3.85(m, 2H), 3.87-4.05(m, 3H), 4.20-4.30(m, 1H), 4.42-4.50(m, 1H), 4.67(ABq, J=17Hz, 3Hz, 2H), 5.70(d, J=4Hz, 1H), 7.52(dd, J=8Hz, 5Hz, 1H), 7.63(d, J=7Hz, 1H), 7.95 (dt, J=8Hz, 2Hz, 1H), 8.72(d, J=4Hz, 1H) |

Table 53

| Example No. | Chemical Structure | IR($cm^{-1}$) | NMR($\delta$ ppm) |
|---|---|---|---|
| 125 | 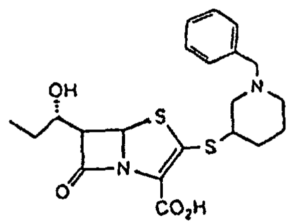 | 3400, 1772, 1593, 1376 | (Isomer A) ($CD_3OD-D_2O$) 1.05(t, J=7Hz, 3H), 1.55-1.65(m, 1H), 1.85-2.30(m, 6H), 2.42-2.55(m, 1H), 3.10-3.50(m, 5H), 3.55-3.72(m, 1H), 3.80-4.00(m, 2H), 4.08(dd, J=10Hz, 4Hz, 1H), 4.40(ABq, J=33Hz, 14Hz, 2H), 5.72(d, J=4Hz, 1H), 7.40-7.55(m, 5H) |
| | 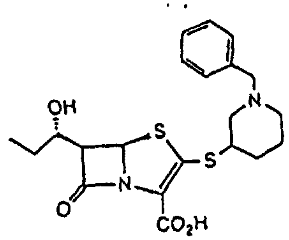 | 3422, 1768, 1594, 1376 | (Isomer B) ($CD_3OD-D_2O$) 7.40-7.60(5H, m), 5.64(1H, d, J=3.5Hz), 4.45, 4.34(each 1H, d, J=12.9Hz), 4.05-4.20(1H, m), 4.04(1H, dd, J=4.0Hz, 10.4Hz), 3.87-4.06(1H, m), 3.58-3.72(1H, m), 3.37-3.50(1H, m), 3.21-3.36(1H, m), 2.99-3.12(1H, m), 2.42-2.62(1H, m), 2.03-2.32(2H, m), 1.89-2.01(1H, m), 1.71-1.89(1H, m), 1.42-1.60(1H, m), 0.97(3H, t, J=7.4Hz) |
| 126 | 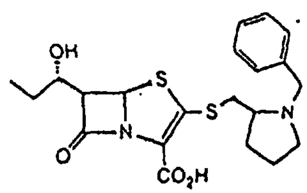 | 3426, 1769, 1578, 1377 | ($D_2O-CD_3OD$) 7.53(2H, d, J=3.5Hz), 7.45(3H, s), 5.74(1H, d, J=3.8Hz), 4.49(1H, d, J=13.1Hz), 4.34(1H, d, J=13.1Hz), 4.09(1H, dd, J=3.9Hz, 10.5Hz), 3.94-4.05(1H, m), 3.84-3.94(1H, m), 3.62-3.75(1H, m), 3.40-3.51(1H, m), 3.13-3.34(2H, m), 2.45-2.59(1H, m), 2.20-2.31(1H, m), 2.10-2.20(1H, m), 1.97-2.10(1H, m), 1.84-1.97(1H, m), 1.55-1.70(1H, m), 1.05(3H, t, J=7.4Hz) |

Table 54

| Example No. | Chemical Structure | IR($cm^{-1}$) | NMR(δ ppm) |
|---|---|---|---|
| 127 | 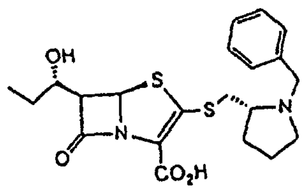 | 3404, 1770, 1586, 1376 | ($D_2O$-MeOD) 7.41-7.60(5H,m), 5.72(1H,d,J=3.4Hz), 4.50(1H,d,J=12.9Hz), 4.31(1H,d,J=13.4Hz), 4.13-4.28(1H,m), 4.09(1H,dd,J=3.5Hz, 9.9Hz), 3.91-4.03(1H,m), 3.61-3.77(1H,m), 3.40-3.54(1H,m), 3.24-3.40(1H,m), 3.05(1H,dd,J=10.9Hz,15.8Hz), 3.48-3.65(1H,m), 2.07-2.34(2H,m), 1.89-2.07(1H,m), 1.71-1.89(1H,m), 1.45-1.61(1H,m), 0.97(3H,t,J=7.4Hz) |
| 128 | 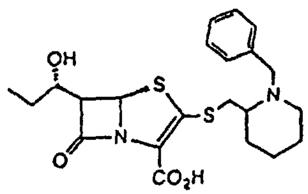 | 3430, 1774, 1596, 1371 | ($CD_3OD$-$D_2O$) 0.99, 0.96(3H, each t,J=7.4Hz,7.9Hz), 1.48-1.61(1H,m), 1.61-2.04(6H,m), 2.04-2.41(1H,m), 3.13-3.36(1H,m), 3.36-3.67(4H,m), 3.74-3.86(1H,m), 3.90-4.16(2H,m), 4.22-4.57(1H,m), 5.64, 5.47(1H, each d,J=4.4Hz), 7.51(5H,s) |
| 129 | 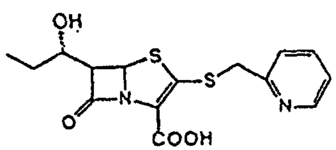 | 3410, 1758, 1597 | ($D_2O$) 8.44-8.55(1H,m), 7.85(1H,t,J=7.7Hz), 7.53(1H,d,J=8.0Hz), 7.33-7.43(1H,m), 5.62(1H,d,J=3.9Hz), 4.42(1H,d,J=14.2Hz), 4.20(1H,d,J=14.2Hz), 3.98(1H,dd,J=3.7Hz,10.3Hz), 3.83-3.94(1H,m), 1.76-1.93(1H,m), 1.42-1.62(1H,m), 0.98(3H,t,J=7.4Hz) |

Table 55

| Example No. | Chemical Structure | IR($cm^{-1}$) | NMR(δ ppm) |
|---|---|---|---|
| 130 | 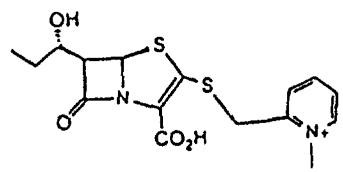 | 3380,1762, 1577,1375 | ($CD_3OD-D_2O$) 1.02(t,J=7Hz,3H),1.40-1.55(m,1H),1.82-1.94(m,1H),3.75-3.82(m,1H),3.88(dd,J=11Hz,4Hz,1H),4.45(s,3H),7.94(t,J=6Hz,1H),8.03(d,J=7Hz,1H),8.46(t,J=7Hz,1H),8.86(d,J=6Hz,1H) |
| 131 | 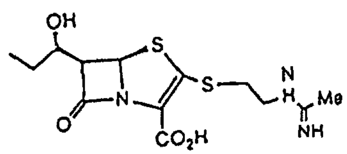 | 3256,1766, 1584,1378 | ($CD_3OD$) 1.02(t,J=7Hz,3H),1.45-1.55(m,1H),1.85-1.95(m,1H),2.23(s,3H),2.98-3.05(m,1H),3.18-3.26(m,1H),3.57(t,J=6Hz,2H),3.88(dd,J=10Hz,4Hz),3.90-4.00(m,1H),5.67(d,J=4Hz,1H) |
| 132 | 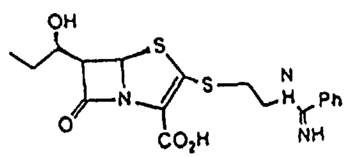 | 3400,1762, 1379 | ($CD_3OD$) 0.99(t,3H,J=7Hz),1.45-1.55(m,1H),1.80-1.95(m,1H),3.10-3.20(m,1H),3.78(t,J=6Hz,2H),3.85-3.95(m,2H),5.56(d,J=3Hz,1H),7.60(t,J=7Hz,2H),7.72(t,J=7Hz,1H),7.76(d,J=7Hz,2H) |

Table 56

| Example No. | Chemical Structure | IR($cm^{-1}$) | NMR(δ ppm) |
| --- | --- | --- | --- |
| 133 | 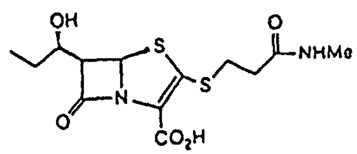 | 3330, 1762, 1578, 1380 | ($CD_3OD$) 1.02(t, J=7Hz, 3H), 1.42-1.55(m, 1H), 1.82-1.98(m, 1H), 2.56(dt, J=8Hz, 2Hz, 2H), 2.71(s, 3H), 3.00-3.30(m, 2H), 3.84(dd, J=10Hz, 4Hz, 1H), 3.90-4.00(m, 1H), 5.66(d, J=4Hz, 1H) |
| 134 | 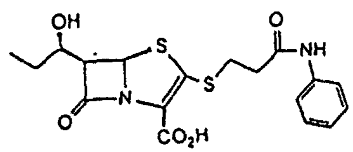 | 3420, 1768, 1668, 1599, 1551, 1500, 1378 | ($CD_3OD$) 1.01(t, J=7Hz, 3H), 1.45-1.55(m, 1H), 1.82-1.95(m, 1H), 2.77-2.85(m, 2H), 3.15-3.25(m, 1H), 3.86(dd, J=10Hz, 4Hz, 1H), 3.92-4.00(m, 1H), 5.68(d, J=4Hz, 1H), 7.07(t, 1H, J=7Hz, 1H), 7.28(t, J=7Hz, 2H), 7.52(d, J=7Hz, 2H) |
| 135 | 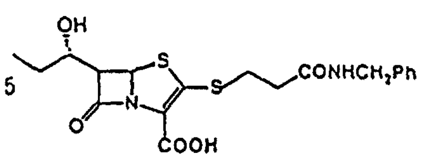 | 3384, 1758, 1595 | ($D_2O$) 7.30-7.50(5H, m), 5.72(1H, s), 4.37-4.52(2H, m), 3.99-4.11 (2H, m), 3.29-3.40(1H, m), 3.14-3.29(1H, m), 2.75(2H, t, J=6.7Hz), 1.77-1.92(1H, m), 1.57-1.63(1H, m), 0.97(3H, t, J=7.5Hz) |

Table 57

| Example No. | Chemical Structure | IR(cm⁻¹) | NMR(δ ppm) |
|---|---|---|---|
| 136 | 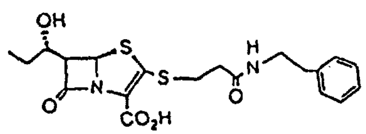 | 3330, 1766, 1651, 1562, 1380 | ($CD_3OD-D_2O$) 0.99(t,3H,J=7Hz),1.45-1.60(m,1H),1.75-1.95(m,1H),2.58(t,J=7Hz,2H),2.80(t,J=7Hz,2H),3.02-3.25(m,2H),3.40-3.50(m,2H),3.92-4.05(m,2H),5.74(d,J=3Hz,1H),7.20-7.35(m,5H) |
| 137 | 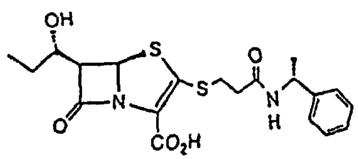 | 3330, 1772, 1651, 1376 | ($CD_3OD$) 1.02(t,J=7Hz,3H),1.44(d,J=7Hz,3H),1.47-1.55(m,1H),1.85-1.95(m,1H),2.65(t,J=7Hz,2H),3.10-3.20(m,1H),3.20-3.30(m,1H),3.89(dd,J=10Hz,4Hz,1H),3.95-4.00(m,1H),5.00(quint,J=6Hz,1H),5.70(d,J=4Hz,1H),7.2(bs,1H),7.27-7.35(m,5H) |
| 138 | 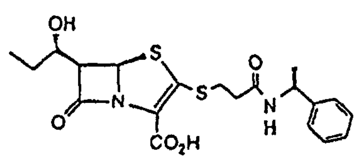 | 3280, 1770, 1654, 1540, 1380 | ($CD_3OD$) 1.02(t,J=7Hz,3H),1.44(d,J=7Hz,3H),1.45-1.58(m,1H),1.85-1.95(m,1H),2.65(dt,J=7Hz,3Hz,2H),3.10-3.20(m,1H),3.25-3.30(m,1H),3.88-3.98(m,2H),4.95-5.05(m,1H),5.71(d,J=4Hz,1H),7.2(bs,1H),7.25-7.35(m,5H) |

Table 58

| Example No. | Chemical Structure | IR($cm^{-1}$) | NMR($\delta$ ppm) |
|---|---|---|---|
| 139 | 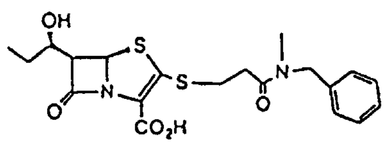 | 3400, 1773, 1625, 1494, 1406, 1124 | ($CD_3OD-D_2O$) 0.90-1.05(m,6H), 1.40-1.60(m,1H), 1.70-1.90(m,1H), 2.90-2.98(m,2H), 3.00, 3.01(s, total 3H), 3.20-3.45, 3.60-3.70(m, total 2H), 3.90-4.10(m,2H), 4.60, 4.65(s, total 2H), 5.50, 5.77(d,J=4Hz, total 1H), 7.20-7.40(m,5H) |
| 140 | 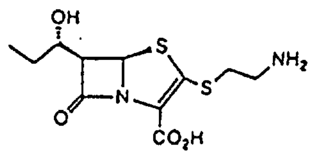 | 3400, 1771, 1560, 1374 | ($D_2O$) 1.02(t,J=7Hz,3H), 1.42-1.65(m,1H), 1.80-1.95(m,1H), 2.95-3.20(m,4H), 3.95-4.08(m,1H), 5.78(d,J=3Hz,1H) |
| 141 | 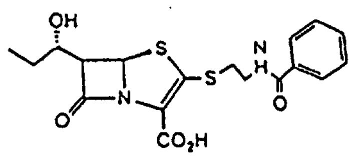 | 3400, 1763, 1642, 1578, 1380, 1297 | ($D_2O$) 0.79(t,J=7Hz,3H), 1.25-1.45(m,1H), 1.45-1.65(m,1H), 3.12-3.25(m,1H), 3.30-3.52(m,2H), 3.52-3.65(m,1H), 3.72-3.82(m,1H), 3.85(dd,J=10Hz,4Hz,1H), 5.38(d,J=4Hz,1H), 7.53(t,J=8Hz,2H), 7.63(t,J=7Hz,1H), 7.74(d,J=7Hz,2H) |

Table 59

| Example No. | Chemical Structure | IR ($cm^{-1}$) | NMR (δ ppm) |
|---|---|---|---|
| 142 | 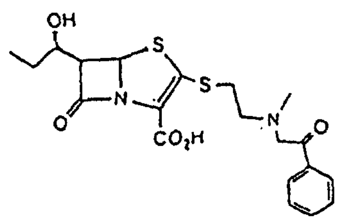 | 3390, 1764, 1693, 1596, 1377 | ($CD_3OD$) 1.01(t, J=7Hz, 3H), 1.45-1.58(m, 1H), 1.80-1.95(m, 1H), 2.72(s, 3H), 3.15-3.30(m, 4H), 3.87-4.02(m, 2H), 4.50(s, 2H), 5.73(d, J=4Hz, 1H), 7.53(t, J=8Hz, 2H), 7.65(t, J=8Hz, 1H), 8.02(dd, J=8Hz, 11Hz, 2H) |
| 143 | 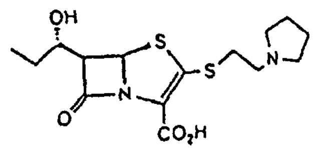 | 3422, 1774, 1686, 1377 | ($CD_3OD$-$D_2O$) 5.85(1H, d, J=4.0Hz), 4.01-4.20(2H, m), 3.62-3.79(2H, m), 3.56(2H, t, J=6.9Hz), 3.20-3.50(3H, m), 3.01-3.20(1H, m), 1.93-2.26(4H, m), 1.85-1.93(1H, m), 1.47-1.63(1H, m), 0.99(3H, t, J=7.4Hz) |
| 144 | 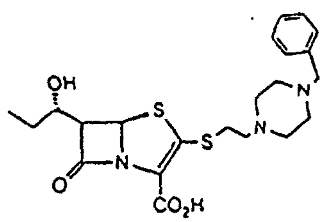 | 3420, 1764, 1578, 1376 | ($CD_3OD$-$D_2O$) 7.49(5H, s), 5.76(1H, s), 4.00-4.20(2H, m), 4.04(2H, s), 2.75-3.28(10H, m), 1.79-2.92(1H, m), 1.48-1.60(1H, m), 1.00(3H, t, J=5.9Hz) |

Table 60

| Example No. | Chemical Structure | IR($cm^{-1}$) | NMR($\delta$ ppm) |
|---|---|---|---|
| 145 | 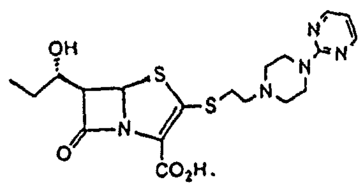 | 3424, 1770, 1586, 1366 | ($D_2O$) 8.45(2H, d, J=5.0Hz), 6.87(1H, t, J=4.9Hz), 5.88(1H, d, J=3.5Hz), 3.99-4.20(2H, m), 3.32-3.69(10H, m), 3.25-3.32(2H, m), 1.75-1.91 (1H, m), 1.45-1.62(1H, m), 0.99(3H, t, J=7.4Hz) |
| 146 | 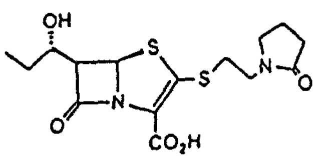 | 3397, 1768, 1666, 1377 | ($CD_3OD-D_2O$) 5.75(1H, s), 3.96-4.10(2H, m), 3.49-3.69(4H, m), 3.20-3.30 (1H, m), 3.01-3.12(1H, m), 2.34-2.49(2H, m), 1.98-2.11(2H, m), 1.78-1.92(1H, m), 1.46-1.61(1H, m), 0.99, 1.00(3H, each t, J=7.3Hz) |
| 147 | 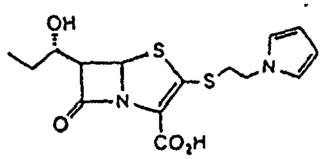 | 3431, 1765, 1676, 1499, 1383 | ($CD_3OD-D_2O$) 6.81(2H, t, J=2.0Hz), 6.14(2H, t, J=2.0Hz), 5.68(1H, dd, J=1.5Hz, 2.5Hz), 4.20-4.30(2H, m), 3.95-4.04(2H, m), 3.35-3.51(1H, m), 3.18-3.35(1H, m), 1.75-1.90(1H, m), 1.40-1.61(1H, m), 0.99(3H, t, J=7.4Hz) |

Table 61

| Example No. | Chemical Structure | IR($cm^{-1}$) | NMR(δ ppm) |
| --- | --- | --- | --- |
| 148 | 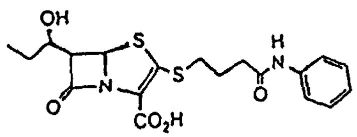 | 3400,1770, 1664,1600, 1546,1445 | ($CD_3OD$) 0.98(t,J=7Hz,3H),1.45-1.60(m,1H),1.75-1.90(m,1H),2.12(quint, J=7Hz,2H),2.56(t,J=7Hz,2H),3.00-3.25(m,2H),3.90-4.05(m,2H), 5.72(d,J=4Hz,1H),7.20(t,J=7Hz,1H),7.30-7.50(m,4H) |
| 149 | 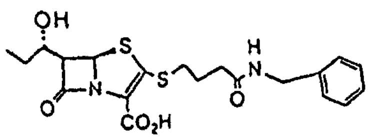 | 3400,1768, 1649 | ($CD_3OD$) 0.99(t,J=7Hz,3H),1.45-1.60(m,1H),1.75-1.90(m,1H),2.03(quint, J=7Hz,2H),2.42(t,J=7Hz,2H),2.90-3.00(m,1H),3.00-3.10(m,1H), 3.92-4.05(m,2H),4.36(s,2H),5.73(d,J=4Hz,1H),7.28-7.40(m,5H) |
| 150 | 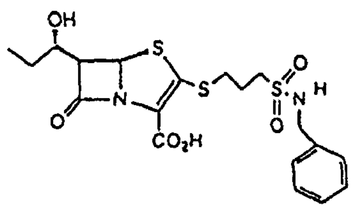 | 3400,1772, 1304,1138 | ($CD_3OD$) 1.02(t,J=7Hz,3H),1.45-1.58(m,1H),1.85-1.95(m,1H),2.08-2.20(m,1H), 2.92-3.02(m,1H),3.93-3.98(m,2H),4.23(s,2H),5.72(d,J=4Hz,1H),7.25-7.40(m,5H) |

Table 62

| Example No. | Chemical Structure | IR($cm^{-1}$) | NMR(δ ppm) |
|---|---|---|---|
| 1 5 1 | 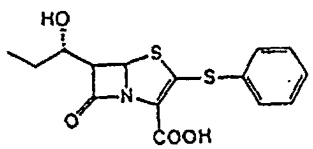 | 3374, 1773, 1684 | ($CD_3OD$) 7.64(2H,d,J=6.8Hz),7.33-7.52(3H,m),5.57(1H,d,J=3.5Hz),3.80-3.92(2H,m),1.80-1.92(1H,m),1.38-1.53(1H,m),0.98(3H,t,J=7.4Hz) |
| 1 5 2 | 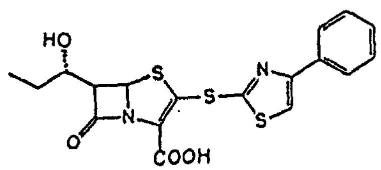 | 1766, 1596, 1386 | ($D_2O$) 0.93(3H,t,J=7.3Hz),1.4-1.55(1H,m),1.7-1.85(1H,m),3.9-4.1(2H,m),5.69(1H,s),7.4-7.6(3H,m),7.87(2H,d,J=7.6Hz),8.00(1H,s) |
| 1 5 3 | 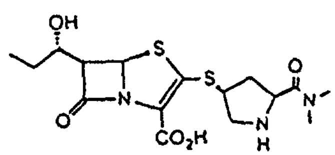 | 3419, 1767, 1631, 1376 | ($D_2O$) 1.01(t,J=7Hz,3H),1.50-1.65(m,1H),1.80-1.95(m,2H),2.90-3.00(m,1H),2.99(s,3H),3.08(s,3H),3.40-3.47(m,1H),3.57-3.65(m,1H),4.02-4.18(m,3H),4.48(t,J=8Hz,1H),5.81(d,J=3Hz,1H) |

Examples 154 to 183

Following the procedures of Example 10 except that p-nitrobenzyl (5R,6R)-6-((S)-1-hydroxypropyl)-2-methylsulfinylpenem-3-carboxylate was used instead of allyl (5R,6R)-6-((S)-1-tert-butyldimethylsilyloxypropyl)-2-methylsulfinylpenem-3-carboxylate and compounds obtained from the compounds described in Production Examples 71 to 95, 97 and 99 to 113 by converting their benzoylthio group or acetylthio group into a mercapto group in a similar manner as in Production Example 46 were employed instead of 1-allyloxycarbonyl-3-mercaptopyrrolidine, the corresponding penem-3-carboxylic acid derivatives were obtained.

Physical data of the thus-obtained compounds are shown in Tables 63 to 70.

Table 63

| Chemical Structure | N M R | I R |
|---|---|---|
| 1 5 4 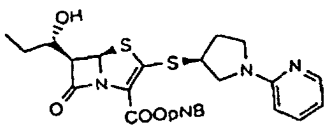 | (CDCl₃) 1.07(3H,d,J=7Hz),1.58-1.64 (1H,m),1.96-2.02(1H,m), 2.15-2.20(1H,m),2.50-2.55 (1H,m),3.51-3.56(1H,m), 3.61-3.70(2H,m),3.95(1H, dd,J=4Hz,10Hz),4.01-4.07 (2H,m),5.19(1H,d,J=14Hz), 5.44(1H,d,J=14Hz),5.79(1H, d,J=4Hz),6.36(1H,d,J=8Hz), 6.58(1H,dd,J=5Hz,7Hz), 7.46(1H,m),7.59(2H,d,J=9Hz), 8.16(1H,dd,J=1Hz,5Hz), 8.19(2H,d,J=9Hz), | |
| 1 5 5 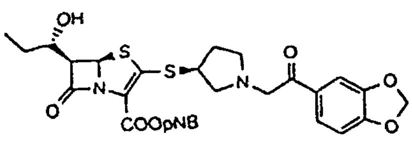 | (CDCl₃) 1.06(3H,t,J=7Hz),1.57-1.61 (1H,m),1.87-1.91(2H,m),2.23-2.33(1H,m),2.40-2.50(1H,m), 2.64-2.71(1H,m),2.72-2.80 (1H,m),2.85-2.94(1H,m),3.24 (1H,dd,J=7Hz,10Hz),3.40-3.48 (1H,m),3.50-3.57(1H,m),3.89 (2H,d,J=5Hz),5.20(1H,d,J= 14Hz),5.46(1H,d,J=14Hz), 5.74(1H,d,J=4Hz),6.03(2H,s), 6.84(1H,d,J=8Hz),7.46(1H,d, J=8Hz),7.58-7.62(3H,m), 8.20(2H,d,J=9Hz) | |
| 1 5 6 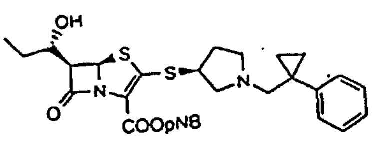 | (CDCl₃) 0.72(2H,m),0.86(2H,m),1.05 (3H,t,J=7Hz),1.55-1.64(1H,m), 1.73-1.79(1H,m),1.95-2.03 (1H,m),2.25-2.30(1H,m),2.46 (1H,dd,J=6Hz,10Hz),2.53 (1H,m),2.65(2H,d,J=5Hz), 2.67-2.72(1H,m),3.18(1H,dd, J=7Hz,10Hz),3.69-3.72(1H,m), 3.91(1H,dd,J=4Hz,10Hz), 4.09-4.12(1H,m),5.19(1H,d, J=14Hz),5.45(1H,d,J=14Hz),5.72 (1H,d,J=4Hz),7.16(1H,m), 7.25(2H,m),7.31(2H,m),7.60 (2H,d,J=9Hz),8.20(2H,d,J=9Hz) | |

Table 64

| Chemical Structure | N M R | I R |
|---|---|---|
| 1 5 7 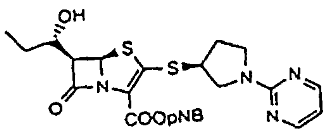 | $(CDCl_3)$ 1.07(3H,t,J=7Hz),1.57-1.64 (1H,m),1.97-2.02(1H,m), 2.14-2.20(1H,m),2.48-2.53 (1H,m),3.70-3.82(3H,m), 3.95(1H,dd,J=4Hz,10Hz), 3.99-4.05(1H,m),4.09-4.18 (2H,m),5.19(1H,d,J=14Hz), 5.45(1H,d,J=14Hz),5.79 (1H,d,J=4Hz),6.53(1H,dd, J=5Hz,5Hz),7.59(2H,d,J=9Hz), 8.19(2H,d,J=9Hz),8.33 (2H,d,J=5Hz) | 2985 1790 1695 1585 $(CHCl_3)$ |
| 1 5 8 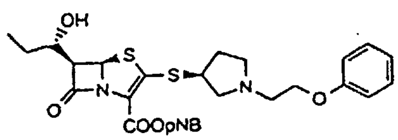 | $(CDCl_3)$ 1.06(3H,t,J=7Hz),1.59-1.63 (1H,m),1.87-1.90(1H,m), 1.98-2.00(1H,m),2.39-2.42 (1H,m),2.65-2.83(3H,m), 2.89-2.95(2H,m),3.32(1H,dd, J=7Hz,10Hz),3.83(1H,brs), 3.91-3.94(1H,m),4.08-4.14 (3H,m),5.20(1H,d,J=14Hz), 5.46(1H,d,J=14Hz),5.75(1H, d,J=4Hz),5.89-6.96(3H,m), 7.26-7.30(2H,m),7.61(2H,d, J=9Hz),8.21(2H,d,J=9Hz) | 1785 1690 1600 $(CHCl_3)$ |
| 1 5 9 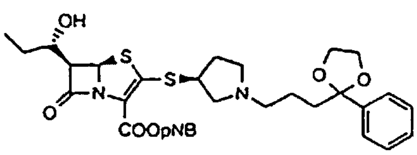 | $(CDCl_3)$ 1.07(3H,m),1.54-1.70(3H,m), 1.91(3H,m),2.38-2.78(6H,m), 2.97-3.04(1H,m),3.76(3H,m), 3.89-4.02(3H,m),4.62(1H,m), 5.19(1H,d,J=14Hz),5.46(1H,d, J=14Hz),5.74(1H,d,J=4Hz), 7.32(3H,m),7.42(2H,m),7.60 (2H,d,J=9Hz),8.20(2H,d,J=9Hz) | 2960 1790 1690 $(CHCl_3)$ |
| 1 6 0 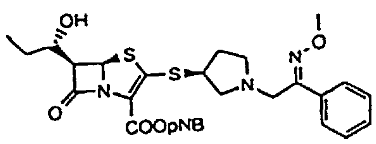 | $(CDCl_3)$ 1.05(3H,t,J=7Hz),1.56-1.62 (1H,m),1.78-1.85(1H,m), 1.95-2.01(1H,m),2.29-2.35 (1H,m),2.62(1H,dd,J=6Hz, 10Hz),2.69(2H,t,J=7Hz), 3.18(1H,dd,J=7Hz,10Hz), 3.71-3.78(3H,m),3.91(1H, dd,J=4Hz,10Hz),3.97(3H,s), 4.09-4.14(1H,m),5.19(1H, d,J=14Hz),5.46(1H,d,J=14Hz), 5.72(1H,d,J=4Hz),7.34(3H,m), 7.60(2H,d,J=9Hz),7.75(2H,m), 8.20(2H,d,J=9Hz) | 2940 1790 1695 $(CHCl_3)$ |

Table 65

| Chemical Structure | N M R | I R |
|---|---|---|
| 1 6 1 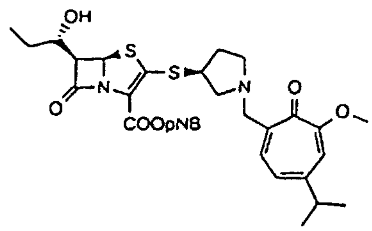 | ($CDCl_3$) 1.06(3H,t,J=7Hz),1.28(6H, d,J=7Hz),1.58-1.63(1H,m), 1.90-2.02(2H,m),2.37-2.43 (1H,m),2.64-2.67(1H,m),2.78-2.90(3H,m),3.13-3.18(1H,m), 3.78(2H,s),3.44(3H,s),5.21 (1H,d,J=14Hz),5.47(1H,t,J= 14Hz),5.74(1H,d,J=4Hz),6.71 (1H,s),6.83(1H,d,J=10Hz), 7.60-7.64(3H,m), 8.21(2H,d,J=9Hz) | 1785 1690 1600 1570 ($CHCl_3$) |
| 1 6 2 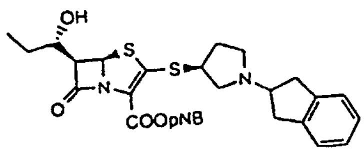 | ($CDCl_3$) 1.07(3H,t,J=7Hz),1.59 (1H,m),1.85-2.03(2H,m), 2.38-2.46(1H,m),2.57-2.94(5H,m),3.01-3.17 (3H,m),3.30(1H,dd,J= 7Hz,10Hz),3.80-3.97 (2H,m),4.08-4.16(1H,m), 5.21(1H,d,J=14Hz),5.47 (1H,d,J=14Hz),5.77(1H,d, J=4Hz),7.12-7.20(4H,m), 7.61(2H,d,J=9Hz),8.22 (2H,d,J=9Hz) | 1785 1690 ($CHCl_3$) |
| 1 6 3 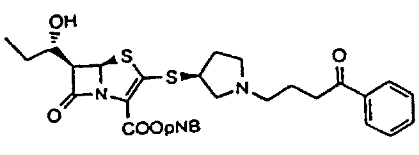 | ($CDCl_3$) 1.06(3H,t,J=8Hz),1.56-1.63 (1H,m),1.80-1.86(1H,m), 1.94(3H,m),2.30-2.35(1H,m), 2.50-2.64(5H,m),3.03(2H,t, J=7Hz),3.11(1H,dd,J=7Hz, 10Hz),3.74-3.78(1H,m),3.93 (1H,dd,J=4Hz,10Hz),4.09-4.14(1H,m),5.20(1H,d, J=14Hz),5.45(1H,d,J=14Hz), 5.75(1H,d,J=9Hz),7.45(2H, dd,J=8Hz,8Hz),7.54(1H,dd, J=7Hz,7Hz),7.60(2H,d,J=9Hz), 7.96(2H,d,J=7Hz),8.20(2H,d,J=9Hz) | 1790 1685 ($CHCl_3$) |
| 1 6 4 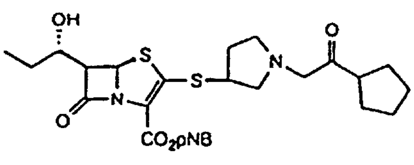 | ($CDCl_3$) 1.06(3H,t,J=7.5Hz),1.48-1.92(8H,m),1.96-2.05(1H,m), 2.35-2.48(1H,m),2.59(1H,dd, J=6.1 and 10.0Hz),2.60-2.70 (1H,m),2.82-2.94(2H,m),3.35-3.41(1H,m),3.44(2H,s),3.80-3.88(1H,m),3.94(1H,dd,J=4.1 and 10.4Hz),4.08-4.17(1H,m), 5.20(1H,d,J=13.6Hz),5.45(1H,d, J=13.6Hz),5.75(1H,d,J=4.0Hz), 7.59-7.64(2H,m),8.20-8.25(2H,m) | 2960 1786 1685 1522 (NaCl) |

Table 66

| Chemical Structure | N M R | I R |
|---|---|---|
| 1 6 5 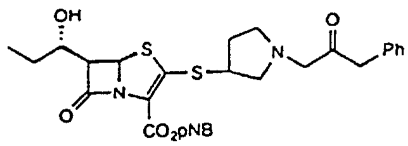 | ($CDCl_3$) 1.06(3H,t,J=7.3Hz),1.54-1.66 (1H,m),1.76-1.90(1H,m),1.95-2.05(1H,m),2.34-2.46(1H,m), 2.56-2.81(3H,m),3.02-3.14 (1H,m),3.26(1H,dd,J=7.2 and 9.9Hz),3.38-3.41(2H,m),3.70-3.73(2H,m),3.75-3.90(1H,m), 3.93(1H,dd,J=4.0 and 10.3Hz), 5.19(1H,d,J=13.8Hz),5.44 (1H,d,J=13.8Hz),5.73(1H,d, J=4.0Hz),7.19-7.36(5H,m), 7.59-7.64(2H,m),8.18-8.23(2H,m) | 3421 2964 1783 1722 1520 (NaCl) |
| 1 6 6 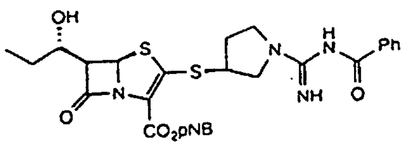 | ($CDCl_3$) 1.06(3H,t,J=7.4Hz),1.55-1.66 (1H,m),1.92-2.22(3H,m),2.45-2.57(1H,m),3.45-3.82(5H,m), 3.45-4.09(2H,m),5.20(1H,d,J= 13.6Hz),5.45(1H,d,J=13.6Hz), 5.81(1H,d,J=4.0Hz),7.36-7.71 (5H,m),8.18-8.26(4H,m) | 3356 1785 1559 1346 (KBr) |
| 1 6 7 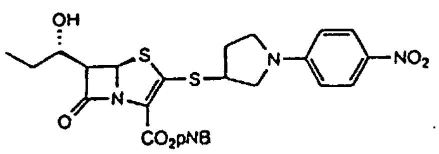 | ($CDCl_3$) 1.08(3H,t,J=7.4Hz),1.56-1.68(1H,m),1.95-2.06(1H,m), 2.13-2.27(1H,m),2.52-2.62 (1H,m),3.39(1H,d,J=11.0Hz), 3.47-3.66(3H,m),3.94-4.00 (1H,m),4.03-4.16(1H,m), 4.65-4.70(1H,brs),5.19 (1H,d,J=13.7Hz),5.45(1H,d, J=13.7Hz),5.83(1H,d, J=4.0Hz),6.45-6.51(2H,m), 7.58-7.63(2H,m),8.10-8.16(2H,m),8.18-8.23(2H,m) | 3649 2862 1784 1600 1520 (NaCl) |
| 1 6 8 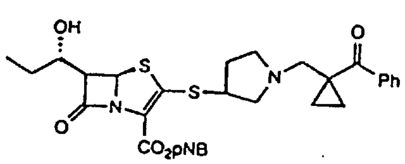 | ($CDCl_3$) 0.76-0.82(2H,m),1.06(3H,t, J=7.4Hz),1.23-1.31(2H,m), 1.52-1.64(1H,m),1.68-1.80 (1H,m),1.93-2.05(1H,m), 2.15-2.28(1H,m),2.38-2.59 (3H,m),2.77(1H,d,J=12.8Hz), 2.82(1H,d,J=12.8Hz),3.05 (1H,dd,J=7.4 and 10.0Hz), 3.62-3.70(1H,m),3.92(1H,dd, J=4.0 and 10.5Hz),4.08-4.16 (1H,m),5.19(1H,d,J=13.8Hz), 5.45(1H,d,J=13.8Hz),5.72 (1H,d,J=4.0Hz),7.38-7.46 (2H,m),7.47-7.53(1H,m),7.59-7.64(2H,m),7.79-7.84(2H,m), 8.18-8.25(2H,m) | 2965 1782 1672 1552 1346 (NaCl) |

Table 67

| Chemical Structure | N M R | I R |
|---|---|---|
| 169 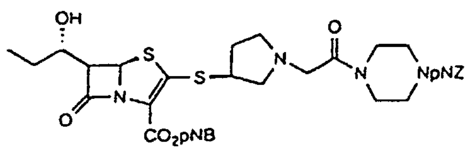 | ($CDCl_3$) 1.06(3H,t,J=7.4Hz),1.56-1.65 (1H,m),1.78-2.04(2H,m),2.33-2.45(1H,m),2.58-2.86(3H,m), 3.10-3.21(1H,m),3.30-3.39 (2H,m),3.45-3.78(8H,m), 3.79-3.88(1H,m), 3.93(1H,dd,J=4.0 and 10.6Hz),4.07-4.16(1H,m), 5.15-5.48(4H,m),5.74 (1H,d,J=4.0Hz),7.51(2H,d, J=8.5Hz),7.59(2H,d,J=8.5Hz), 8.18-8.27(4H,m) | 3400 2934 1783 1702 1522 1347 (KBr) |
| 170 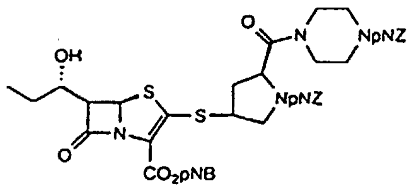 | ($CDCl_3$) 1.06(3H,t,J=7.4Hz),1.50-1.70(2H,m),1.94-2.08(1H,m), 2.69-2.85(1H,m),3.26-3.87 (11H,m),3.95(1H,dd,J=3.9 and 10.4Hz),4.25-4.35(1H,m), 4.70-4.79(1H,m),5.18-5.48 (6H,m),5.78(1H,d,J=3.9Hz), 7.48-7.65(6H,m),8.15-8.26(6H,m) | 3402 2930 1786 1706 1347 (KBr) |
| 171 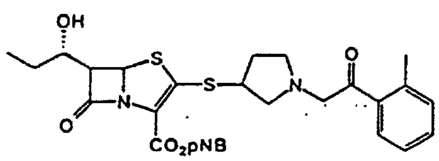 | ($CDCl_3$) 1.06(3H,t,J=7.3Hz),1.45-1.65(1H,m),1.85-1.95(1H,m), 1.95-2.05(1H,m),2.25-2.4 (1H,m),2.49(3H,s),2.6-2.8 (2H,m),2.9-3.0(1H,m),3.25-3.35(1H,m),3.8-3.9(3H,m), 3.9-3.95(1H,m),4.1-4.2 (1H,m),5.21(1H,d,J=13.4Hz), 5.46(1H,d,J=13.4Hz),5.75 (1H,d,J=4.0Hz),7.2-7.3(2H,m), 7.3-7.4(1H,m),7.55-7.7(3H,m), 8.21(2H,d,J=8.7Hz) | 1346 1522 1691 1786 2962 (KBr) |
| 172 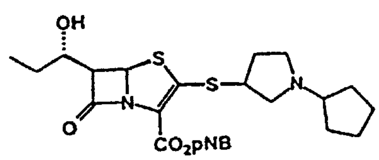 | ($CDCl_3$) 1.06(3H,t,J=7.4Hz),1.35-1.9(11H,m),2.2-2.3(1H,m), 3.0-3.2(2H,m),3.2-3.3(1H,m), 3.4-3.55(2H,m),3.93(1H,dd, J=4.0Hz,10.4Hz),4.1-4.2 (1H,m),5.20(1H,d,J=14.0Hz), 5.46(1H,d,J=14.0Hz),5.75 (1H,d,J=4.0Hz),7.61(2H,d, J=8.7Hz),8.21(2H,d,J=8.7Hz) | 1346 1522 1685 1786 2957 (KBr) |

Table 68

| Chemical Structure | N M R | I R |
|---|---|---|
| 1 7 3 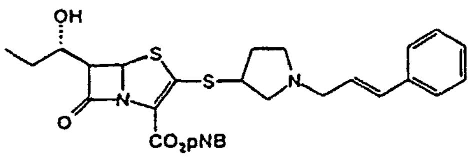 | (CDCl₃) 1.06(3H,t,J=7.4Hz),1.55-1.7(1H,m),1.8-1.95(1H,m),2.25-2.35(1H,m),2.6-2.65(2H,m),2.65-2.75(2H,m),3.05-3.1(1H,m),3.2-3.35(2H,m),3.5-3.6(1H,m),3.92(1H,dd,J=4.0Hz,10.4Hz),4.1-4.15(1H,m),5.20(1H,d,J=13.6Hz),5.46(1H,d,J=13.6Hz),5.74(1H,d,J=4.0Hz),6.25-6.35(1H,m),6.54(1H,d,J=15.7Hz),7.2-7.45(5H,m),7.61(2H,d,J=8.7Hz),8.21(2H,d,J=8.7Hz) | 1346 1497 1522 1785 2960 (KBr) |
| 1 7 4 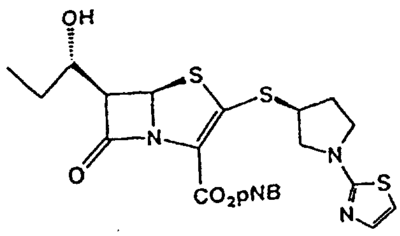 | (CDCl₃) 1.08(t,J=7Hz,3H),1.50-1.65(m,1H),1.95-2.05(m,1H),2.15-2.25(m,1H),2.50-2.60(m,1H),3.50-3.70(m,4H),3.95(dd,J=10Hz,4Hz,1H),4.00-4.20(m,2H),5.20(d,J=14Hz,1H),5.31(d,J=14Hz,1H),5.78(d,J=4Hz,1H),6.53(d,J=3Hz,1H),7.20(d,J=3Hz,1H),7.59(d,J=8Hz,2H),8.19(d,J=8Hz,2H) | 1782 1540 1332 (KBr) |
| 1 7 5 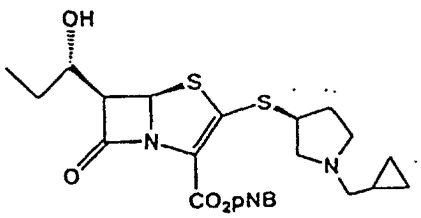 | (CDCl₃) 0.12(2H,m),0.52(2H,m),0.89(1H,m),1.06(3H,t,J=7Hz),1.61(1H,m),1.85 (1H,m),1.90(1H,m),2.33(3H,m),2.50-2.63(2H,m),2.77(1H,m),3.29(1H,dd,J=7Hz,10Hz),3.84(1H,m),3.93(1H,m),4.12(1H,m),5.20(1H,d,J=14Hz),5.46(1H,d,J=14Hz),5.76(1H,d,J=4Hz),7.61(2H,d,J=9Hz),8.21(2H,d,J=9Hz) | 2965 1785 1685 (NaCl) |
| 1 7 6 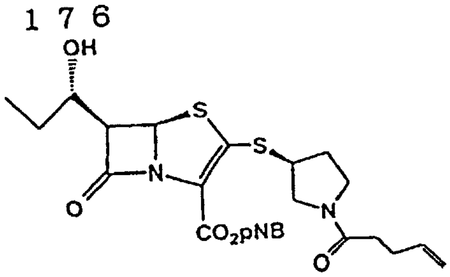 | (CDCl₃) 1.07(t,7Hz,3H),1.95-2.05(m,2H),2.30-2.50(m,4H),3.50-3.75(m,4H),3.84-4.2(m,3H),4.95-5.10(m,2H),5.15-5.25(m,1H),5.45(d,J=13Hz,1H),5.78-5.92(m,2H),7.60(d,J=9Hz,2H),8.21(d,J=9Hz,2H) | |

Table 69

| Chemical Structure | N M R | I R |
|---|---|---|
| 177 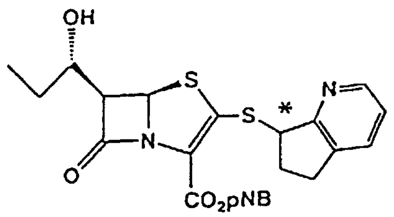 (*:Isomer A) | ($CDCl_3$) 1.06(t,7Hz,3H),1.55-1.65 (m,1H),1.95-2.05(m,1H),2.35-2.45(m,1H),2.70-2.82(m,1H), 2.90-3.00(m,1H),3.10-3.20 (m,1H),3.93(dd,J=7Hz,4Hz,1H), 4.20-4.27(m,1H),4.90-4.98 (m,1H),5.18(d,J=13Hz,1H), 5.43(d,J=13Hz,1H),5.78(d,J=4Hz,1H) 7.14(dd,J=7Hz,5Hz,1H),7.55 (d,J=7Hz,1H),7.58(d,J=9Hz,2H), 8.18(d,J=9Hz,2H),8.43(d,J=5Hz,1H) | 3400 1784 1520 (KBr) |
| 178 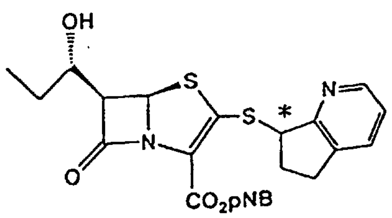 (*:Isomer B) | ($CDCl_3$) 1.08(t,J=7Hz,3H),1.57-1.65 (m,1H),1.95-2.05(m,1H), 2.30-2.40(m,1H),2.65-2.80 (m,1H),2.90-3.00(m,1H), 3.10-3.20(m,1H),3.95(dd,J=10Hz,4Hz,1H),4.10-4.20(m,1H), 4.95(dd,J=8Hz,4Hz,1H),5.18 (d,J=14Hz,1H),5.43(d,J=14Hz,1H), 5.79(d,J=4Hz,1H),7.15(dd,J=8Hz, 5Hz,1H),7.55(d,J=8Hz,1H),7.59 (d,J=9Hz,2H),8.18(d,J=9Hz,2Hz), 8.46(d,J=5Hz,1H) | 3400 1789 1520 (KBr) |
| 179 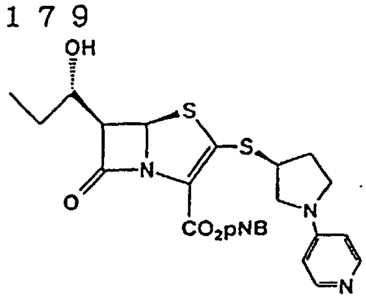 | ($CDCl_3$) 1.07(t,J=7Hz,3H),1.57-1.67(m,1H), 1.95-2.05(m,1H),2.20-2.30(m,1H), 2.53-2.65(m,1H),3.50-3.65(m,3H), 3.95-4.03(m,2H),4.05-4.17(m,2H), 5.18(d,J=14Hz,1H),5.45(d,J=14Hz,1H), 5.81(d,J=4Hz,1H),6.49(d,J=7Hz,2H), 7.59(d,J=9Hz,2H),8.18-8.23(m,4H) | |
| 180 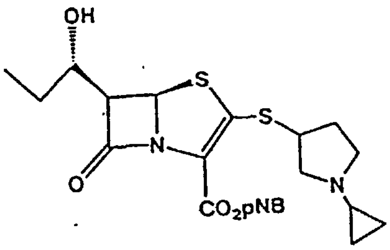 | ($CDCl_3$) 0.40-0.48(m,4H),1.05-1.12(m,3H), 1.58-1.70(m,1H),1.80-1.20(m,2H), 1.65-1.80(m,3H),3.18-3.32(m,1H), 3.40-3.60(m,1H),3.70-3.82(m,1H), 3.90-3.98(m,1H),4.10-4.25(m,2H), 5.25(d,J=13Hz,1H), 5.42(d,J=13Hz,1H), 5.74(d,J=4Hz,1/2H), 5.75(d,J=4Hz,1/2H), 7.60(d,J=9Hz,2H),8.23(d,J=9Hz,2H) | 1786 1522 1347 (KBr) |

Table 70

| Chemical Structure | N M R | I R |
|---|---|---|
| 181 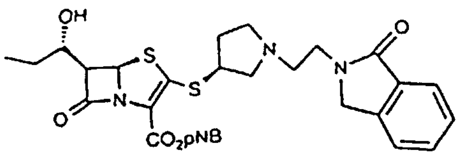 | (CDCl₃) 1.06(3H,t,J=7Hz),1.56-1.68 (1H,m),1.80-1.89(1H,m), 1.94-2.02(1H,m),2.28-2.35 (1H,m),2.69-2.81(5H,m), 3.11-3.18(1H,m),3.72-3.75 (3H,m),3.94(1H,dd,J=4Hz,10Hz), 4.11(1H,m),4.49(2H,s),5.18 (1H,d,J=14Hz),5.44(1H,d, J=14Hz),5.74(1H,d,J=4Hz), 7.44(2H,m),7.52(1H,m),7.60 (2H,d,J=9Hz),7.84(1H,m), 8.20(2H,d,J=9Hz) | |
| 182 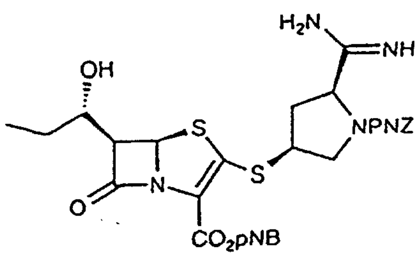 | (CDCl₃) 1.02-1.11(3H,m),1.55-1.68 (1H,m),1.92-2.06(1H,m), 2.40-2.87(2H,m),3.51-3.61 (1H,m),3.80-4.28(4H,m), 4.34-4.54(1H,m),5.16-5.50 (4H,m),5.75-5.82(1H,m), 7.50-7.67(4H,m), 8.18-8.29(4H,m) | 3337 1787 1686 1345 (NaCl) |
| 183 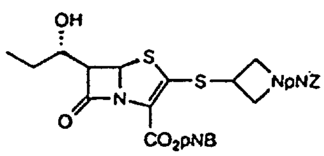 | (CDCl₃) 1.08(3H,t,J=7.3Hz),1.54-1.65(1H,m),1.92-2.04(1H,m), 3.88-4.21(5H,m),4.37-4.52 (2H,m),5.19 (2H,s),5.22 (1H,d,J=13.6Hz),5.46 (1H,d,J=13.6Hz),5.78 (1H,d,J=4.1Hz),7.46-7.51 (2H,m),7.56-7.62(2H,m), 8.18-8.23(4H,m) | |

Example 184

Synthesis of p-nitrobenzyl 2-[(1,5-dihydro-1,5-dioxo-2,4-benzothiepin)-3-ylidene]-2-[(3R,4S)-3-((S)-1-tert-butyldimethylsilyloxypropyl)-4-phenylthio-2-azetidinon-1-yl]acetate

To a solution of p-nitrobenzyl 2-[(3R,4S)-3-((S)-1-tert-butyldimethylsilyloxypropyl)-4-phenylthio-2-azetidinon-1-yl]acetate (2.55 g, 4.68 mmol) in dry tetrahydrofuran (20 mℓ), a 1 N tetrahydrofuran solution of lithium hexamethyldisilazide (7.0 mℓ, 7.00 mmol) was added under an argon gas stream at -78°C. Five minutes later, carbon disulfide (0.56 mℓ, 9.31 mmol) was added to the reaction mixture. Ten minutes later, a solution of phthaloyl chloride (0.81 mℓ, 5.62 mmol) in dry tetrahydrofuran (0.5 mℓ) was also added. Thirty minutes later, acetic acid (0.40 mℓ, 6.99 mmol) was added to the reaction mixture, into which ethyl acetate (100 mℓ) was then poured. The resultant mixture was washed

successively with a saturated aqueous solution of sodium chloride (50 mℓ), a saturated aqueous solution of sodium hydrogencarbonate (50 mℓ) and a saturated aqueous solution of sodium chloride (50 mℓ). The organic phase was dried over anhydrous sodium sulfate and the solvent was distilled off under reduced pressure, whereby the title compound was obtained in a crude form (3.70 g).

NMR ($CDCl_3$):

0.01 (3H,s), 0.04,0.05 (total 3H,s), 0.81,0.82 (total 9H,s), 0.93,0.95 (total 3H,t,7.5Hz), 1.45-1.8 (2H,m), 3.15-3.2 (1H,m), 4.0-4.1 (1H,m), 5.09,5.16 (total 1H,d,J=13.3Hz), 5.21,5.31 (total 1H,d,J=13.3Hz), 5.45,5.51 (total 1H,d, J=2.7Hz), 7.3-7.4 (3H,m), 7.4-7.6 (4H,m), 7.6-7.7 (1H,m), 7.8-7.95 (2H,m), 8.0-8.15 (1H,m), 8.22,8.22 (total 2H,d, J=8.7Hz).

Example 185

Synthesis of p-nitrobenzyl 2-[(1,5-dihydro-1,5-dioxo-2,4-benzothiepin)-3-ylidene]-2-[(3R,4S)-3-((S)-1-tert-butyldimethylsilyloxypropyl)-4-chloro-2-azetidinon-1-yl]acetate

To a solution of crude p-nitrobenzyl 2-[(1,5-dihydro-1,5-dioxo-2,4-benzothiepin)-3-ylidene]-2-[(3R,4S)-3-((S)-1-tert-butyldimethylsilyloxypropyl)-4-phenylthio-2-azetidinon-1-yl]acetate (3.70 g) in dry methylene chloride (20 mℓ), sulfuryl chloride (0.70 mℓ, 8.57 mmol) was added under ice cooling and an argon gas stream. Fifteen minutes later, allyl acetate (1.5 mℓ, 13.9 mmol) was added to the reaction mixture, into which ethyl acetate (100 mℓ) was then poured. The resultant mixture was washed successively with a saturated aqueous solution of sodium hydrogencarbonate (50 mℓ) and a saturated aqueous solution of sodium chloride (50 mℓ). The organic phase was dried over anhydrous sodium sulfate and the solvent was distilled off under reduced pressure. The residue was subjected to column chromatography by using silica gel (25 g) (ethyl acetate-hexane 1:3, V/V), whereby the title compound was obtained as a slightly yellowish solid (2.32 g, 73% yield).

NMR ($CDCl_3$):

0.02 (3H,s), 0.05 (3H,s), 0.82,0.84 (total 9H,s), 0.94 (3H,t,J=7.5Hz), 1.55-1.7 (2H,m), 3.57 (1H,brs), 4.0-4.1 (1H, m), 5.22,5.24 (total 1H,d,J=13.2Hz), 5.43,5.45 (total 1H,d,J=13.2Hz), 5.93,5.95 (total 1H,brs), 7.54,7.55 (total 2H,d, J=8.6Hz), 7.6-7.7 (1H,m), 7.8-7.95 (2H,m), 8.1-8.2 (1H,m), 8.24 (2H,d,J=8.6Hz).

Example 186

Synthesis of p-nitrobenzyl (5R,6R)-6-((S)-1-tert-butyldimethylsilyloxypropyl)-2-mercaptopenem-3-carboxylate (triethylamine complex)

To a solution of p-nitrobenzyl 2-[(1,5-dihydro-1,5-dioxo-2,4-benzothiepin)-3-ylidene]-2-[(3R,4S)-3-((S)-1-tert-butyldimethylsilyloxypropyl)-4-chloro-2-azetidinon-1-yl]acetate (2.31 g, 3.42 mmol) in dry methylene chloride (15 mℓ), a solution of a 30% methanol solution (1.06 mℓ) of methylamine (10.2 mmol) in dry methylene chloride (1 mℓ), and triethylamine (1.43 mℓ, 10.3 mmol) were added under ice cooling and an argon gas stream. The temperature of the reaction mixture was allowed to rise to room temperature and one hour later, was poured into methylene chloride (150 mℓ). The resultant mixture was washed with water (150 mℓ). The organic phase was dried over anhydrous sodium sulfate and the solvent was distilled off under reduced pressure, whereby the title compound was obtained as a brown solid (2.09 g, 100% yield).

NMR ($CDCl_3$):

0.10 (3H,s), 0.11 (3H,s), 0.87 (9H,s), 0.99 (3H,t,J=7.4Hz), 1.17 (7H,t,J=7.3Hz), 1.8-1.9 (2H,m), 2.8-3.0 (6H,m), 3.96 (1H,dd,J=4.0Hz,10.0Hz), 4.35-4.4 (1H,m), 5.06 (1H,d,J=14.3Hz), 5.44 (1H,d,J=14.3Hz), 5.54 (1H,d,J=4.0Hz), 7.45-7.7 (2H,m), 8.15-8.25 (2H,m).

Example 187

Synthesis of p-nitrobenzyl (5R,6R)-6-((S)-1-tert-butyldimethylsilyloxypropyl)-2-(2-indanyl)thiopenem-3-carboxylate

To a solution of p-nitrobenzyl (5R,6R)-6-((S)-1-tert-butyldimethylsilyloxypropyl)-2-mercaptopenem-3-carboxylate (triethylamine complex) (161.6 mg, 0.26 mmol) in dry tetrahydrofuran (1.5 mℓ), triphenylphosphine (100.5 mg, 0.65 mmol) and 2-hydroxyindane (5.06 mg, 0.38 mmol) were added under an argon gas stream at room temperature. The reaction mixture was ice-cooled, followed by the addition of diethyl azodicarboxylate (61 μℓ, 0.39 mmol). Thirty minutes later, the solvent in the reaction mixture was distilled off under reduced pressure and the residue was subjected to column chromatography by using silica gel (10 g). From ethyl acetate-hexane (1:10, V/V), the title compound was obtained as a slightly yellowish oil (65.4 mg, 40% yield).

Examples 188 to 190

Following the procedures of Example 187 except for the use of various hydroxy compounds instead of 2-hydroxyindane, the corresponding penem compounds were obtained.

Structural formulas and physical data of the compounds obtained in Examples 187 to 190 are shown in Table 71.

Table 71

| Chemical Structure | N M R |
|---|---|
| 1 8 7 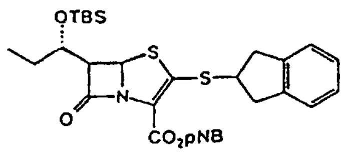 | ($CDCl_3$) 0.14(6H,s),0.89(9H,s),1.01(3H,t,J=7.4Hz),1.75-1.95(2H,m),3.09 (1H,dd,J=5.8Hz,16.6Hz),3.12 (1H,dd,J=5.8Hz,16.6Hz),3.47 (1H,dd,J=7.7Hz,16.6Hz),3.55 (1H,dd,J=7.7Hz,16.6Hz),4.1-4.25 (2H,m),4.35-4.45(1H,m),5.18 (1H,d,J=13.7Hz),5.44(1H,d,J=13.7Hz),5.75(1H,d,J=4.0Hz),7.15-7.25(4H,m),7.58(2H,d,J=8.7Hz),8.18 (2H,d,J=8.7Hz) |
| 1 8 8 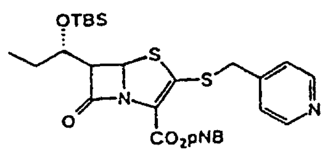 | ($CDCl_3$) 0.02(3H,s),0.07(3H,s),0.86(9H,s),0.97(3H,t,J=7.4Hz),1.7-1.9(2H,m),4.08(1H,dd,J=4.0Hz,9.6Hz),4.13 (1H,d,J=14.4Hz),4.22(1H,d,J=14.4Hz),4.2-4.3(1H,m),5.21(1H,d,J=13.7Hz),5.47(1H,d,J=13.7Hz),5.67(1H,d,J=4.0Hz),7.29(2H,d,J=6.0Hz),7.60 (2H,d,J=8.7Hz),8.21(2H,d,J=8.7Hz),8.58(2H,d,J=6.0Hz) |
| 1 8 9 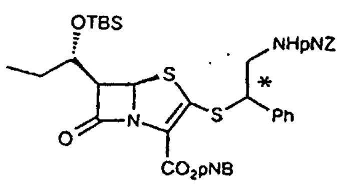 (*:Isomer A) | ($CDCl_3$) 0.11(6H,s),0.86(9H,s),0.97(3H,t,J=7.4Hz),1.7-1.9(2H,m),3.6-3.75 (2H,m),4.04(1H,dd,J=4.0Hz,9.7Hz),4.2-4.3(1H,m),4.55-4.65(1H,m),5.0-5.1(1H,m),5.16(1H,d,J=13.4Hz),5.19(1H,d,J=13.7Hz),5.20(1H,d,J=13.4Hz),5.46(1H,d,J=13.7Hz),5.54(1H,d,J=4.0Hz),7.25-7.4(5H,m),7.46(2H,d,J=8.6Hz),7.60(2H,d,J=8.6Hz),8.20(2H,d,J=8.6Hz),8.21(2H,d,J=8.6Hz) |
| 1 9 0 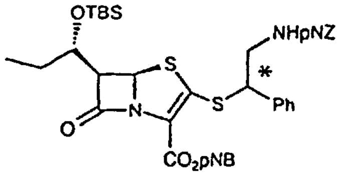 (*:Isomer B) | ($CDCl_3$) -0.12(3H,s),-0.01(3H,s),0.83(9H,s),0.93(3H,t,J=7.4Hz),1.65-1.85(2H,m),3.6-3.7(2H,m),4.04(1H,dd,J=3.8Hz,9.9Hz),4.1-4.2(1H,m),4.55-4.65(1H,m),5.05-5.15(1H,m),5.16(1H,d,J=13.0Hz),5.20(1H,d,J=13.8Hz),5.21(1H,d,J=13.0Hz),5.49(1H,d,J=13.8Hz),5.64 (1H,d,J=3.8Hz),7.25-7.4(5H,m),7.47 (2H,d,J=8.3Hz),7.62(2H,d,J=8.3Hz),8.21(4H,d,J=8.3Hz) |

Example 191

Synthesis of p-nitrobenzyl (5R,6R)-6-((S)-1-tert-butyldimethylsilyloxypropyl)-2-(3,4-dichlorobenzyl)thiopenem-3-carboxylate

To a solution of p-nitrobenzyl (5R,6R)-6-((S)-1-tert-butyldimethylsilyloxypropyl)-2-mercaptopenem-3-carboxylate (triethylamine complex) (248 mg, 0.406 mmol) in dry methylene chloride (1.3 mℓ), triethylamine (85 μℓ, 0.61 mmol) and 3,4-dichlorobenzyl chloride (112 μℓ, 0.81 mmol) were added under an argon gas stream at room temperature. Seventy minutes later, the solvent in the reaction mixture was distilled off under reduced pressure and the residue was subjected to column chromatography by using silica gel (10 g) (ethyl acetate-hexane, 1:5, V/V), the title compound was obtained as a slightly yellowish oil (282 mg, 69% yield).

Examples 192 to 193

Following the procedures of Example 191 except for the use of appropriate halogen compounds instead of 3,4-dichlorobenzyl chloride, the corresponding penem compounds were obtained. Structural formulas and physical data of the compounds obtained in Examples 191 to 193 are shown in Table 72.

Table 72

| Chemical Structure | NMR |
|---|---|
| 191 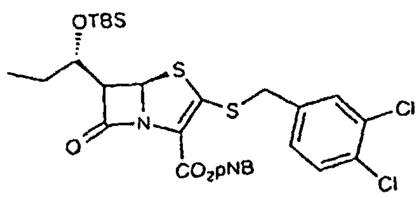 | ($CDCl_3$) 0.03(3H,s),0.08(3H,s),0.86(9H,s), 0.98(3H,t,J=7.4Hz),1.7-1.9(2H,m), 4.08(1H,dd,J=4.0Hz,9.7Hz),4.11 (1H,d,J=14.0Hz),4.18(1H,d,J=14.0Hz), 4.25(1H,dt,J=4.5Hz,9.7Hz),5.20 (1H,d,J=13.7Hz),5.47(1H,d,J=13.7Hz), 5.68(1H,d,J=4.0Hz),7.22(1H,dd, J=1.9Hz,8.3Hz),7.43(1H,d,J=8.3Hz), 7.49(1H,d,J=1.9Hz),7.60(2H,d, J=8.7Hz),8.21(2H,d,J=8.7Hz) |
| 192 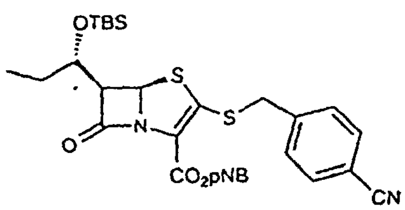 | ($CDCl_3$) 0.02(3H,s),0.08(3H,s),0.86(9H,s), 0.98(3H,t,J=7.4Hz),1.7-1.9(2H,m), 4.08(1H,dd,J=4.0Hz,9.6Hz),4.15- 4.3(1H,m),4.20(1H,d,J=14.0Hz), 4.28(1H,d,J=14.0Hz),5.20(1H,d, J=13.7Hz),5.46(1H,d,J=13.7Hz), 5.67(1H,d,J=4.0Hz),7.48(2H,d, J=8.2Hz),7.60(2H,d,J=8.7Hz), 7.62(2H,d,J=8.2Hz),8.21(2H,d,J=8.7Hz) |
| 193 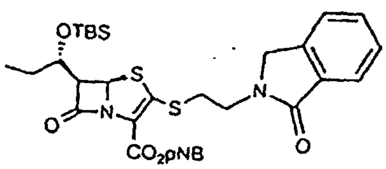 | ($CDCl_3$) 0.12(6H,s),0.89(9H,s),0.98 (3H,t,J=7Hz),1.78-1.82(2H,m), 3.24-3.32(1H,m),3.38-3.41 (1H,m),3.90-4.10(3H,m), 4.49(2H,s),5.19(1H,d,J=14Hz), 5.44(1H,d,J=14Hz), 5.65(1H,d,J=4Hz),7.41-7.49 (2H,m),7.53-7.59(3H,m), 7.84(1H,d,J=8Hz), 8.19(2H,d,J=9Hz) |

Examples 194 to 202

Following the procedures of Example 12 except for the use of various 6-hydroxyl-protected penem derivatives instead of allyl (5R,6R)-6-((S)-1-tert-butyldimethylsilyloxypropyl)-2-methylthiopenem-3-carboxylate, the corresponding 6-hydroxyl-deprotected penem derivatives were obtained.

Physical data of the 6-hydroxyl-deprotected penem derivatives obtained in Examples 194 to 202 are shown in Tables 73 to 75.

Table 73

| Chemical Structure | N M R |
|---|---|
| 194 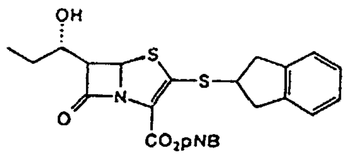 | ($CDCl_3$)<br>1.08(3H,t,J=7.4Hz),1.95-2.1(2H,m),<br>3.08(1H,dd,J=5.8Hz,16.5Hz),<br>3.13(1H,dd,J=5.8Hz,16.5Hz),<br>3.48(1H,dd,J=7.7Hz,16.5Hz),<br>3.55(1H,dd,J=7.7Hz,16.5Hz),<br>3.96(1H,dd,J=4.0Hz,10.5Hz),<br>4.1-4.2(2H,m),<br>5.18(1H,d,J=13.7Hz),<br>5.45(1H,d,J=13.7Hz),<br>5.79(1H,d,J=4.0Hz),7.15-7.25(4H,m),<br>7.59(2H,d,J=8.8Hz),<br>8.19(2H,d,J=8.8Hz) |
| 195 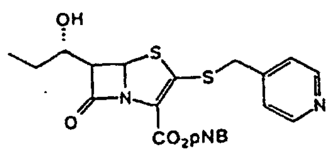 | ($CDCl_3$)<br>1.06(3H,t,J=7.5Hz),<br>1.9-2.05(2H,m),<br>3.94(1H,dd,J=4.0Hz,10.4Hz),<br>4.0-4.1(1H,m),<br>4.13(1H,d,J=14.0Hz),<br>4.25(1H,d,J=14.0Hz),<br>5.21(1H,d,J=13.6Hz),<br>5.47(1H,d,J=13.6Hz),<br>5.72(1H,d,J=4.0Hz),<br>7.30(2H,d,J=5.9Hz),<br>7.60(2H,d,J=8.7Hz),<br>8.22(2H,d,J=8.7Hz),<br>8.59(2H,d,J=5.9Hz) |
| 196 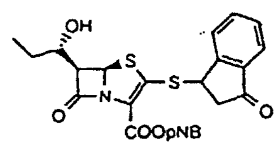 | ($CDCl_3$)<br>1.07(3H,t,J=7Hz),1.58-1.64(1H,m),<br>1.91-2.02(1H,m),3.20-3.29(1H,m),<br>3.70-3.95(2H,m),4.25-4.28(1H,m),<br>5.20-5.32(1H,m),5.45(1H,d,J=14Hz),<br>5.75-5.79(1H,m),7.41-7.79(6H,m),<br>8.18-9.24(2H,m) |
| 197 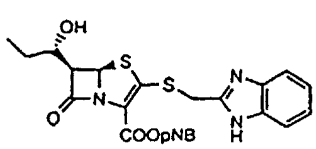 | ($CDCl_3$)<br>1.01(3H,t,J=7Hz),1.47-1.51(1H,m),<br>1.87-1.95(1H,m),<br>3.92(1H,dd,J=4Hz,10Hz),<br>3.97-4.03(1H,m),<br>4.47(1H,d,J=16Hz),4.52(1H,d,J=16Hz),<br>5.22(1H,d,J=14Hz),5.46(1H,d,J=14Hz),<br>5.59(1H,d,J=4Hz),7.26-7.30(2H,m),<br>7.68-7.64(4H,m),8.19-8.23(2H,m) |

Table 74

| Chemical Structure | N M R |
|---|---|
| 198 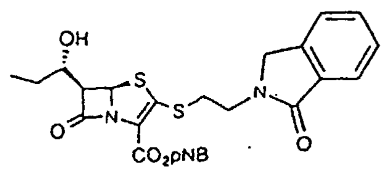 | $(CDCl_3)$ 1.03(3H,t,J=7Hz),1.53-1.60(1H,m), 1.90-1.96(1H,m),2.22(1H,d,J=5Hz), 3.29-3.39(2H,m),3.90-4.00(3H,m), 4.50(2H,s),5.19(1H,d,J=14Hz), 5.44(1H,d,J=14Hz),5.69(1H,d,J=4Hz), 7.42-7.49(2H,m),7.53-7.57(1H,m), 7.59(2H,d,J=9Hz),7.84(1H,d,J=8Hz), 8.19(2H,d,J=9Hz) |
| 199 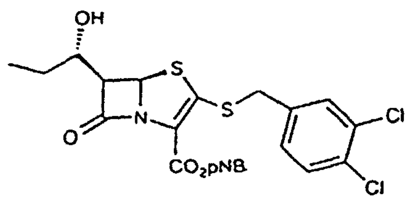 | $(CDCl_3)$ 1.06(3H,t,J=7.4Hz),1.5-1.65(1H,m), 1.65(1H,d,J=5.4Hz),1.9-2.05(1H,m), 3.94(1H,dd,J=4.0Hz,10.4Hz), 4.05-4.15(1H,m),4.11(1H,d,J=13.3Hz), 4.22(1H,d,J=13.3Hz),5.20(1H,d, J=13.7Hz),5.46(1H,d,J=13.7Hz), 5.73(1H,d,J=4.0Hz),7.19(1H,dd, J=2.0Hz,8.2Hz),7.41(1H,d,J=8.2Hz), 7.47(1H,d,J=2.0Hz),7.60(2H,d,J=8. 7Hz),8.21(2H,d,J=8.7Hz) |
| 200 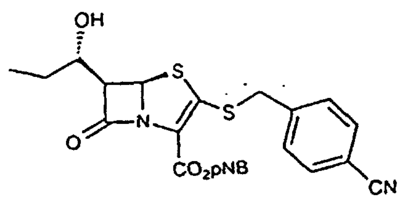 | $(CDCl_3)$ 1.06(3H,t,J=7.4Hz),1.55-1.7(2H,m), 1.9-2.05(1H,m),3.97(1H,dd,J=4.0Hz, 10.4Hz),4.0-4.15(1H,m),4.20(1H,d, J=13.5Hz),4.32(1H,d,J=13.5Hz), 5.20(1H,d,J=13.7Hz),5.46(1H,d, J=13.7Hz),5.73(1H,d,J=4.0Hz), 7.47(2H,d,J=8.2Hz),7.60(2H,d, J=8.7Hz),7.64(2H,d,J=8.2Hz), 8.21(2H,d,J=8.7Hz) |
| 201 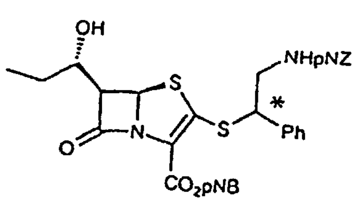 (*:Isomer A) | $(CDCl_3)$ 1.05(3H,t,J=7.4Hz),1.5-1.65(1H,m), 1.9-2.0(2H,m),3.6-3.7(1H,m), 3.7-3.8(1H,m),3.90(1H,dd, J=4.0Hz,10.3Hz),4.05-4.15(1H,m), 4.55-4.65(1H,m),5.0-5.1(1H,m), 5.19(2H,s),5.19(1H,d,J=13.7Hz), 5.45(1H,d,J=13.7Hz),5.60(1H,d,J= 4.0Hz),7.25-7.4(5H,m),7.46(2H,d, J=8.6Hz),7.59(2H,d,J=8.6Hz), 8.20(4H,d,J=8.6Hz) |

Table 75

| Chemical Structure | N M R |
|---|---|
| 202 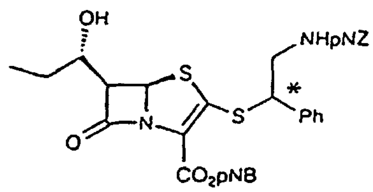 (*:Isomer B) | ($CDCl_3$) 1.02(3H,t,J=7.4Hz),1.5-1.6(1H,m), 1.64(1H,d,J=5.3Hz),1.85-2.0(1H,m), 3.65-3.75(1H,m),3.75-3.9(1H,m), 3.90(1H,dd,J=3.9Hz,10.4Hz), 3.95-4.05(1H,m),4.55-4.65(1H,m), 5.0-5.1(1H,m),5.17(2H,s),5.20 (1H,d,J=13.8Hz),5.47(1H,d,J=13.8Hz), 5.67(1H,d,J=3.9Hz),7.25-7.45(5H,m), 7.45(2H,d,J=7.9Hz),7.61(2H,d, J=8.7Hz),8.20(2H,d,J=7.9Hz), 8.21(2H,d,J=8.7Hz) |

Examples 203 to 243

Following the procedures of Example 48 except for the use of various p-nitrobenzyl penem-3-carboxylate derivatives instead of p-nitrobenzyl (5R,6R)-2-((S)-1-allylpyrrolidin-3-yl)thio-6-((S)-hydroxypropyl)penem-3-carboxylate, the corresponding penem-3-carboxylic acid derivatives were obtained.

Physical data of the compounds obtained in Examples 203 to 243 are shown in Tables 76 to 86.

Table 76

| Chemical Structure | N M R | I R |
|---|---|---|
| 203 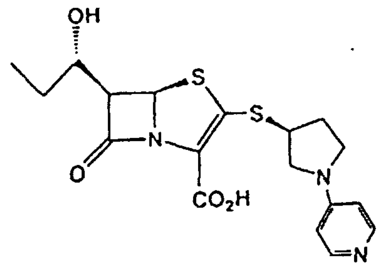 | ($D_2O$-$CD_3OD$) 1.02(t,7Hz,3H),1.50-1.60 (m,1H),1.82-1.92(m,1H), 2.19-2.30(m,1H),2.53-2.65 (m,1H),3.62-3.80(m,3H), 4.00-4.08(m,3H),4.15-4.23 (m,1H),5.79(brs,1H),6.82 (d,J=7Hz,2H),8.08(d,J=7Hz,2H) | 3429 1764 1648 1550 1376 (KBr) |
| 204 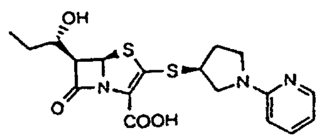 | ($CD_3OD$) 1.04(3H,t,J=7Hz),1.50-1.57 (1H,m),1.88-1.93(1H,m), 2.14-2.20(1H,m),2.52-2.58 (1H,m),3.55-3.58(1H,m),3.62-3.68(2H,m),3.93-4.03(3H,m), 4.10-4.12(1H,m),5.78(1H,d, J=3Hz),6.58(1H,d,J=9Hz), 6.64(1H,dd,J=6Hz),7.57(1H,m), 8.00(1H,d,J=5Hz) | |
| 205 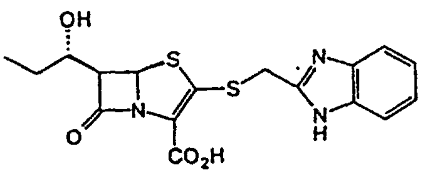 | ($CD_3OD$) 0.96(3H,t,J=7Hz),1.37-1.48 (1H,m),1.76-1.84(1H,m), 2.70-2.94(1H,m),3.82(2H,brs), 4.34(1H,d,J=15Hz),4.48 (1H,d,J=15Hz),5.60(1H,s), 7.21(2H,m),7.51(2H,m) | |
| 206 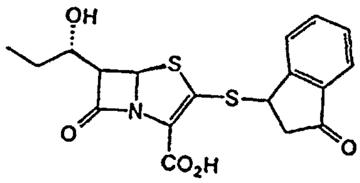 | ($CD_3OD$) 1.02(3H,m),1.45-1.57 (1H,m),1.87-1.96(1H,m), 3.81-3.85(1H,m),3.94-4.01 (1H,m),4.44(2H,brs),5.59 (1H,d,J=4Hz),7.42(1H,m), 7.54(1H,m),7.67-7.73(2H,m) | 1760 1710 1600 (KBr) |

Table 77

| Chemical Structure | N M R | I R |
|---|---|---|
| 2 0 7 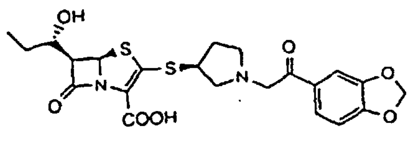 | ($CD_3OD$) 1.03(3H,t,J=7Hz),1.48-1.58 (1H,m),1.83-2.00(2H,m), 2.40-2.55(1H,m),2.61-2.90 (1H,m),3.10-3.20(1H,m), 3.49-3.70(3H,m),3.85-3.99 (3H,m),5.71(1H,d,J=4Hz), 6.07(2H,s),6.93(1H,d,J=8Hz), 7.44(1H,brs),7.64(1H,d,J=8Hz) | 1765 1680 1600 (KBr) |
| 2 0 8 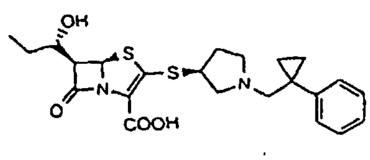 | ($CDCl_3$) 0.92(4H,m),1.01(3H,brs), 1.50-1.62(1H,m),1.78-2.00 (2H,m),2.03-2.40(1H,m), 2.76-2.95(3H,m),2.99 (2H,brs),3.30-3.45(1H,m), 3.65-3.80(1H,m),3.83-3.92 (1H,m),4.00-4.06(1H,m), 5.54(1H,brs),7.16-7.20 (1H,m),7.27-7.30(2H,m), 7.37-7.39(2H,m) | 1765 1590 (KBr) |
| 2 0 9 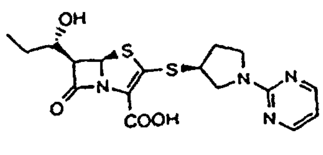 | ($DMSO-d_6$) 0.93(3H,t,J=7Hz),1.37-1.44 (1H,m),1.74-1.79(1H,m), 1.91-1.97(1H,m),3.52-3.61 (3H,m),3.70(1H,dd,J=4Hz,10Hz), 3.81-3.87(2H,m),3.94 (1H,dd,J=7Hz,12Hz),5.61 (1H,d,J=4Hz),6.60(1H,dd, J=5Hz,5Hz),8.33(2H,d,J=5Hz) | 3430 2925 1760 1630 1585 (KBr) |
| 2 1 0 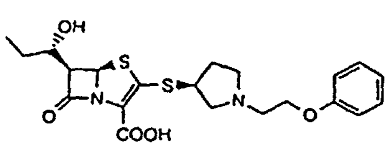 | ($CD_3OD$) 1.02(3H,t,J=7Hz),1.49-1.53 (1H,m),1.88-2.03(2H,m), 2.51-2.58(1H,m),2.74-2.92 (2H,m),3.14-3.20(1H,m),3.35 (2H,m),3.59(1H,m),3.89-3.98 (3H,m),4.25(2H,t,J=5Hz), 5.63(1H,d,J=4Hz),6.97 (3H,m),7.29(2H,m) | 1770 1600 (KBr) |

Table 78

| Chemical Structure | N M R | I R |
|---|---|---|
| 2 1 1 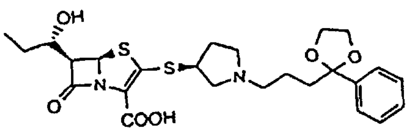 | ($CD_3OD$) 1.03(3H,m),1.51-1.70 (3H,m),1.82-1.95(3H,m), 2.35-2.46(1H,m),2.62-2.95(5H,m),3.12(1H,m), 3.76(2H,m),3.80-3.93 (2H,m),4.01(2H,m),5.67 (1H,m),7.30-7.39(3H,m), 7.42-7.48(2H,m) | 1730 1600 ($CHCl_3$) |
| 2 1 2 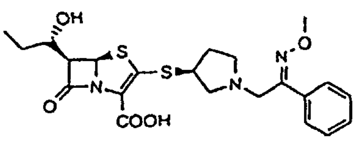 | ($CDCl_3$) 1.02(3H,t,J=7Hz),1.49-1.55 (1H,m),1.79-1.92(2H,m), 2.32-2.37(1H,m),2.70-2.90 (3H,m),3.20-3.30(1H,m), 3.78-3.86(1H,m),3.81-3.95 (3H,m),3.98(3H,s),5.69 (1H,d,J=4Hz),7.37(3H,m), 7.74(2H,m) | |
| 2 1 3 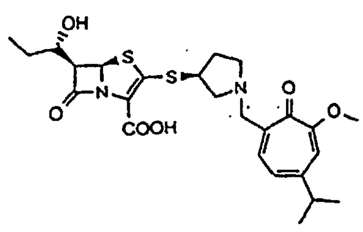 | ($CD_3OD$) 1.02(3H,t,J=7Hz),1.32 (6H,d,J=7Hz),1.58-1.61 (1H,m),1.82-1.95(2H,m), 2.31-2.42(1H,m),2.70-3.02 (5H,m),3.78-3.87(3H,m), 3.97(3H,s),5.65(1H,d,J=4Hz), 6.97(1H,s),7.02(1H,d,J=10Hz), 7.78(1H,d,J=10Hz) | 3420 1765 1630 1595 (KBr) |
| 2 1 4 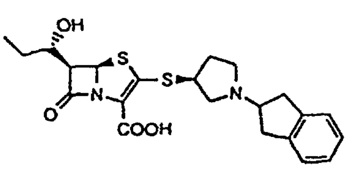 | ($CD_3OD$) 1.02(3H,m),1.48-1.56(1H,m), 1.84-1.97(1H,m),1.98-2.08 (1H,m),2.49-2.60(2H,m), 2.70-2.98(1H,m),3.07-3.18 (2H,m),3.36-3.63(3H,m), 3.70-3.85(1H,m),3.88-4.06 (4H,m),5.70(1H,m),7.19 (2H,m),7.81(2H,m) | 1790 1580 (KBr) |

Table 79

| Chemical Structure | N M R | I R |
|---|---|---|
| 2 1 5 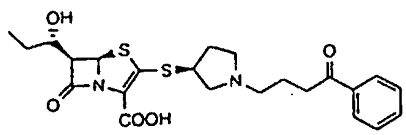 | ($CD_3OD$) 1.03(3H,t,J=7Hz),1.50-1.53 (1H,m),1.87-1.94(1H,m),2.01-2.12(3H,m),2.52-2.57(1H,m), 3.11-3.20(4H,m),3.40-3.54 (3H,m),3.90(1H,dd,J=6Hz,11Hz), 3.96-4.04(2H,m),4.44(1H,m), 5.70(1H,d,J=4Hz),7.49(2H,dd, J=7Hz,8Hz),7.60(1H,dd, J=7Hz,8Hz),8.10(2H,d,J=1Hz) | 1770 1685 (KBr) |
| 2 1 6 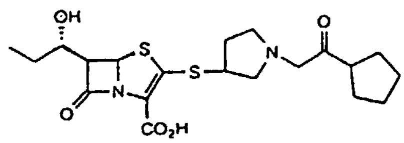 | ($CD_3OD$) 1.02(3H,t,J=7.4Hz),1.44-1.54 (1H,m),1.57-1.96(11H,m),2.29-2.42(1H,m),2.54-2.72(2H,m), 2.84-3.03(2H,m),3.31-3.38(1H,m), 3.57(2H,d,J=3.4Hz),3.84(1H,dd, J=3.9 and 10.6Hz),3.94-4.00 (1H,m),5.67(1H,d,J=4.0Hz) | 3420 2961 1765 1593 1380 (KBr) |
| 2 1 7 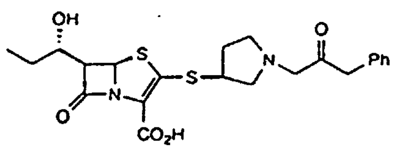 | ($CD_3OD$) 1.02(3H,t,J=7.4Hz),1.39-1.56 (1H,m),1.70-1.94(2H,m), 2.23-2.37(1H,m),2.50-2.67 (2H,m),2.73-2.83(1H,m), 3.22-3.32(1H,m),3.47-3.54 (2H,m),3.71-3.79(1H,m),3.74 (2H,s),3.81-3.86(1H,m),3.92-4.00(1H,m),5.65(1H,d, J=3.9Hz),7.21-7.26(3H,m), 7.28-7.35(2H,m) | 3383 1763 1594 1382 (KBr) |
| 2 1 8 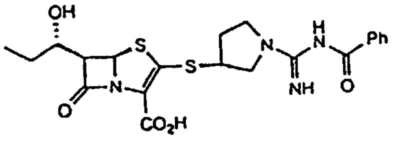 | ($D_2O$) 0.92-1.06(3H,brs),1.48-1.64(1H,brs),1.75-1.84 (1H,brs),2.10-2.16(1H,brs), 2.47-2.65(1H,brs),3.40-4.24(7H,m),5.73-5.87(1H,m), 7.52-7.58(2H,m),7.63-7.70 (1H,m),7.93-7.99(2H,m) | 3313 1774 1676 1561 1372 (KBr) |

Table 80

| Chemical Structure | N M R | IR |
|---|---|---|
| 2 1 9 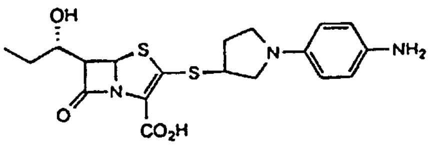 | ($D_2O$, $CD_3OD$) 0.99(3H,t,J=7.4Hz),1.51-1.62 (1H,m),1.79-1.91(1H,m),2.10-2.21(1H,m),2.50-2.60(1H,m),3.32-3.68(4H,m),3.79-3.86 (1H,m),4.04-4.12(1H,m),4.15-4.22(1H,m),5.84 (1H,d,J=3.1Hz),6.78 (2H,d,J=8.9Hz),7.26(2H,d,J=8.9Hz) | 1764 1604 1348 (KBr) |
| 2 2 0 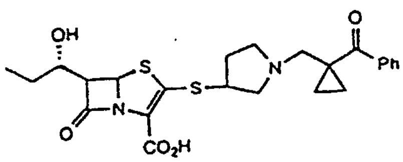 | ($D_2O$) 1.00(3H,t,J=7.4Hz),1.26-1.36 (2H,m),1.49-1.63(3H,m),1.82-1.97(2H,m),2.39-2.51 (1H,m),2.98-3.62(6H,m),3.92-4.00(1H,m),4.05-4.11 (2H,m),5.76(1H,d,J=3.1Hz),7.55-7.62(2H,m),7.68-7.73 (1H,m),7.83-7.88(2H,m) | 3384 1769 1672 1578 1378 (KBr) |
| 2 2 1 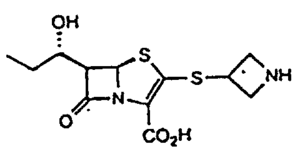 | ($D_2O$) 1.00(3H,t,J=7.4Hz),1.48-1.62 (1H,m),1.78-1.92(1H,m),3.85-4.32(4H,m),4.41-4.66 (3H,m),5.78-5.86(1H,m) | 3346 1764 1586 1382 (KBr) |
| 2 2 2 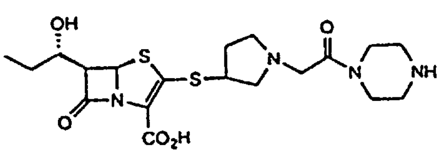 | ($D_2O$) 1.00(3H,t,J=7.4Hz),1.50-1.63(1H,m),1.79-1.97 (2H,m),2.39-2.52(1H,m),2.82-2.95(1H,m),2.99-3.17 (2H,m),3.15-3.37(5H,m),3.63-3.91(6H,m),3.90-4.00 (1H,m),3.98-4.15(2H,m),5.79(1H,d,J=3.6Hz) | 3368 2945 1768 1654 1375 (KBr) |

Table 81

| Chemical Structure | N M R | I R |
|---|---|---|
| 2 2 3 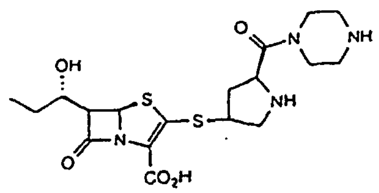 | ($D_2O$) 1.01(3H,t,J=7.4Hz), 1.50-1.63(1H,m),1.72-1.80 (1H,m),1.82-1.95(1H,m), 2.73-2.82(1H,m),2.94-3.18 (3H,m),3.20-3.35(2H,m), 3.58-4.22(9H,m), 5.80(1H,d,J=3.6Hz) | 3407 1764 1631 1377 (KBr) |
| 2 2 4 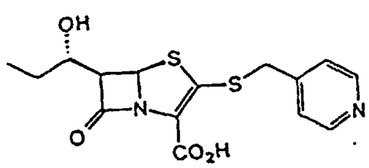 | ($CDCl_3$) 1.01(3H,t,J=7.4Hz),1.4-1.55 (1H,m),1.8-1.95(1H,m),3.8-3.9 (2H,m),4.07(1H,d,J=14.2Hz), 4.31(1H,d,J=14.2Hz),4.48 (1H,brs),5.57(1H,d,J=3.6Hz), 7.48(2H,d,J=6.1Hz), 8.45(2H,d,J=6.1Hz) | 1382 1603 1762 3384 (KBr) |
| 2 2 5 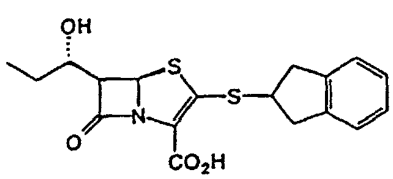 | ($CD_3OD$) 1.04(3H,t,J=7.4Hz),1.45-1.6 (1H,m),1.85-2.0(1H,m), 2.99(1H,dd,J=5.3Hz,16.5Hz), 3.07(1H,dd,J=5.3Hz,16.5Hz), 3.45(1H,dd,J=7.6Hz,16.5Hz), 3.52(1H,dd,J=7.6Hz,16.5Hz), 3.90(1H,dd,J=3.9Hz,10.7Hz), 3.95-4.05(1H,m),4.12(1H,tt, J=5.3Hz,7.6Hz),5.73(1H,d,J= 3.9Hz),7.1-7.25(4H,m) | 1382 1586 1764 3418 (KBr) |
| 2 2 6 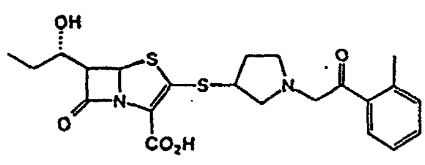 | ($CD_3OD$) 1.02(3H,t,J=7.4Hz),1.45- 1.6(1H,m),1.75-2.0(2H,m), 2.3-2.45(1H,m),2.46(3H,s), 2.6-2.7(1H,m),2.7-2.8 (1H,m),2.9-3.0(1H,m), 3.4-3.55(1H,m),3.75-3.9 (2H,m),3.9-4.05(3H,m), 5.67(1H,d,J=4.0Hz),7.25- 7.3(2H,m),7.35-7.45(1H,m), 7.65-7.75(1H,m) | 1378 1600 1766 3430 (KBr) |

Table 82

| Chemical Structure | N M R | I R |
|---|---|---|
| 2 2 7 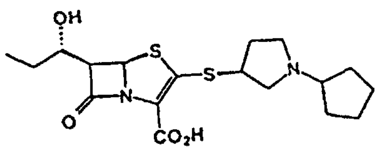 | $(CD_3OD)$ 1.02(3H,t,J=7.4Hz),1.45-1.6(1H,m),1.6-1.75(4H,m),1.75-1.95(3H,m),1.95-2.2(3H,m),2.5-2.65(1H,m),3.2-3.35(1H,m),3.35-3.45(2H,m),3.45-3.55(1H,m),3.55-3.65(1H,m),3.90(1H,dd,J=4.0Hz,10.6Hz),3.95-4.0(1H,m),4.0-4.1(1H,m),5.69(1H,d,J=4.0Hz) | 1378 1586 1770 3421 (KBr) |
| 2 2 8 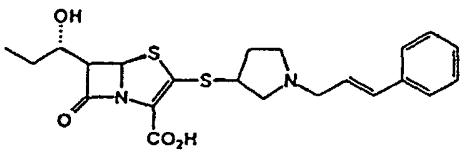 | $(CD_3OD)$ 1.01(3H,t,J=7.4Hz),1.45-1.55(1H,m),1.85-1.95(1H,m),1.95-2.05(1H,m),2.5-2.6(1H,m),3.1-3.45(3H,m),3.55-3.65(1H,m),3.81(2H,d,J=7.3Hz),3.88(1H,dd,J=3.8Hz,10.6Hz),3.9-4.0(1H,m),4.0-4.1(1H,m),5.67(1H,d,J=3.8Hz),6.33(1H,dt,J=7.3Hz,15.7Hz),6.82(1H,d,J=15.7Hz),7.25-7.4(3H,m),7.45-7.5(2H,m) | 1377. 1588 1767 3334 (KBr) |
| 2 2 9 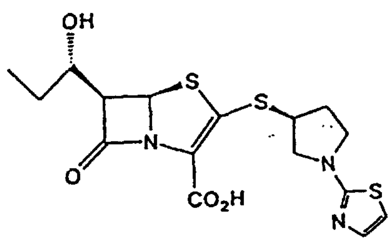 | $(CD_3OD)$ 1.03(t,J=7Hz,3H),1.47-1.60(m,1H),1.85-1.95(m,1H),2.50-2.65(m,1H),3.45-3.65(m,3H),3.90-4.05(m,3H),4.10-4.18(m,1H),5.77(d,J=4Hz,1H),6.63(d,J=4Hz,1H),7.12(d,J=4Hz,1H) | 3418 1774 1540 (KBr) |
| 2 3 0 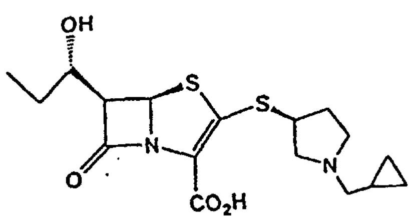 | $(CD_3OD)$ 0.33(2H,m),0.65(2H,m),0.99-1.05(4H,m),1.46-1.56(1H,m),1.87-2.02(2H,m),2.46-2.56(1H,m),2.84(2H,d,J=7Hz),3.13-3.20(1H,m),3.57-3.61(1H,m),3.86-3.90(1H,m),3.94-3.99(2H,m),5.69(1H,d,J=4Hz) | 1765 1610 (KBr) |

Table 83

| Chemical Structure | N M R | I R |
|---|---|---|
| 231<br>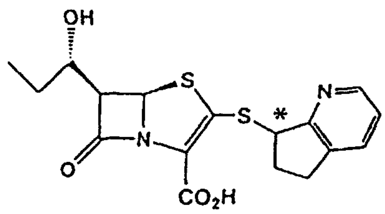<br>(*:Isomer A) | ($CD_3OD$)<br>1.04(t,J=7Hz,3H),1.47-1.60 (m,1H),1.85-1.97(m,1H), 2.40-2.50(m,1H),2.70-2.80 (m,1H),2.90-3.02(m,1H), 3.12-3.23(m,1H),3.92(dd,J=11Hz,4Hz,1H),4.00-4.08(m,1H), 4.91(dd,J=7Hz,4Hz,1H),5.76 (d,J=4Hz,1H),7.26(dd,J=7Hz,5Hz,1H), 7.72(d,J=7Hz,1H),8.33(d,J=5Hz,1H) | 3367<br>1774<br>1380<br>(KBr) |
| 232<br>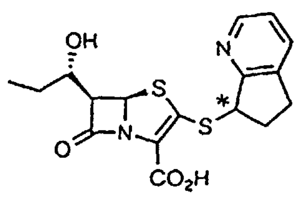<br>(*:Isomer B) | ($CD_3OD$)<br>1.03(t,J=7Hz,3H),1.45-1.55 (m,1H),1.85-1.95(m,1H), 2.30-2.40(m,1H),2.67-2.77 (m,1H),2.93-3.03(m,1H), 3.12-3.22(m,1H),3.88-4.02 (m,2H),5.74(d,J=4Hz,1H),7.25 (dd,J=7Hz,5Hz,1H),7.70(d,J=7Hz,1H), 8.34(d,J=5Hz,1H) | 3369<br>1764<br>1586<br>1380<br>(KBr) |
| 233<br>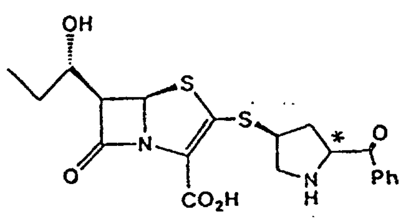<br>(*:Isomer A) | ($D_2O$)<br>1.12(t,J=7Hz,3H),1.60-1.78 (m,1H),1.90-2.05(m,1H), 2.65-2.75(m,1H),1.85-1.95 (m,1H),1.85(m,1H),4.05-4.13 (m,1H),4.22(s,2H),4.30-4.40 (m,1H),5.85(t,1H,J=7Hz), 5.95(s,1H),7.65-7.85(m,2H), 7.90-8.05(m,1H), 8.10-8.30(m,2H) | 1774<br>1376<br>(KBr) |
| 234<br>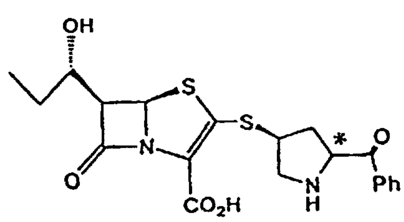<br>(*:Isomer B) | ($D_2O$)<br>1.13(t,J=7Hz,3H),1.63-1.76 (m,1H),1.90-2.00(m,1H), 2.33-2.42(m,1H),3.21-3.32 (m,1H),3.5-3.8(m,2H),4.0-4.4 (m,3H),5.5(m,1H),5.58(d,J=3Hz,1H), 7.7-7.8(m,2H),7.85-7.95(m,1H), 8.08-8.15(m,2H) | 1774<br>1376<br>3390<br>(KBr) |

Table 84

| Chemical Structure | N M R | I R |
|---|---|---|
| 2 3 5 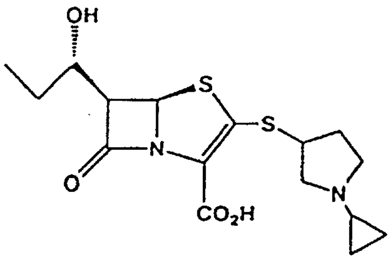 | ($CD_3OD$) 0.58-0.70(m,4H),1.02(t,J=7Hz,3/2H), 1.03(t,J=7Hz,3/2H),1.47-1.57(m,1H), 1.85-1.97(m,2H),2.10-2.20(m,1H), 2.40-2.58(m,1H),3.00-3.23(m,3H), 3.37-3.50(m,1H),3.80-4.00(m,2H), 5.72(d,J=4Hz,1/2H), 5.74(d,J=4Hz,1/2H) | 3380 1766 1590 1378 (KBr) |
| 2 3 6 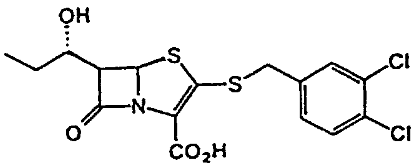 | ($CD_3OD$) 1.01(3H,t,J=7Hz),1.44-1.52 (1H,m),1.85-1.96(1H,m), 3.80-3.91(2H,m), 4.00(1H,d,J=14Hz), 4.27(1H,d,J=14Hz), 5.57(1H,d,J=4Hz), 7.32(1H,d,J=8Hz), 7.42(1H,d,J=8Hz), 7.56(1H,s) | 1760 1630 1595 (KBr) |
| 2 3 7 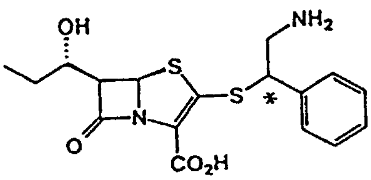 (*:Isomer A) | ($CD_3OD$) 1.01(3H,t,J=7Hz),1.42-1.54 (1H,m),1.83-1.96(1H,m), 3.30-3.37(1H,m), 3.78-3.89(2H,m), 5.48(1H,d,J=4Hz), 7.20-7.38(5H,m) | |
| 2 3 8 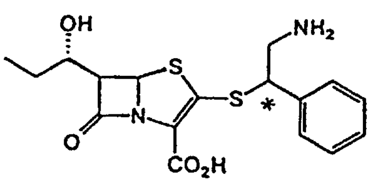 (*:Isomer B) | ($CDCl_3$) 1.01(3H,t,J=7Hz), 1.44-1.52(1H,m),1.84-1.92 (1H,m),3.32-3.40(1H,m), 3.86-3.90(2H,m),5.61(1H,s), 7.26-7.40(5H,m) | 1755 1630 1600 (KBr) |

Table 85

| Chemical Structure | N M R | I R |
|---|---|---|
| 239 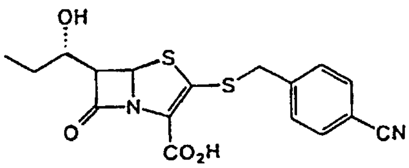 | ($CDCl_3$) 1.02(3H,t,J=7Hz),1.42-1.51 (1H,m),1.84-1.92(1H,m), 3.83(2H,m),4.10(1H,d,J=14Hz), 4.37(1H,d,J=14Hz),5.56(1H,brs), 7.58(2H,d,J=8Hz),7.65(2H,d,J=8Hz) | 1765 1585 (KBr) |
| 240 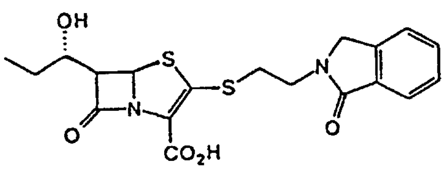 | ($CD_3OD$) 1.00(3H,t,J=7Hz),1.40-1.51 (1H,m),1.81-1.90(1H,m), 3.10-3.18(2H,m), 3.80-3.85(1H,m),3.89-3.96 (3H,m),4.60(2H,s),5.52 (1H,d,J=4Hz),7.47-7.50 (1H,m),7.57-7.59(2H,m), 7.76(1H,d,J=7Hz) | 1760 1670 1590 (KBr) |
| 241 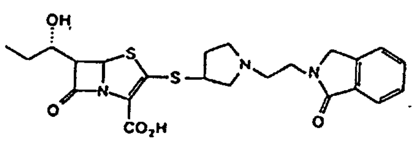 | ($CDCl_3$) 1.02(3H,t,J=7Hz),1.47-1.53 (1H,m),1.80-1.93(2H,m), 2.33-2.48(1H,m),2.80-3.10 (6H,m),3.78-3.97(5H,m), 4.60(2H,s),5.67(1H,d,J=4Hz), 7.48(1H,m),7.58(2H,m), 7.74-7.85(1H,m) | 1765 1670 (KBr) |
| 242 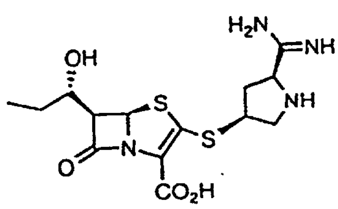 | ($D_2O$) 1.00(3H,t,J=7.3Hz), 1.52-1.63(1H,m),1.81-2.02 (2H,m),2.76-2.90(1H,m), 3.20-3.28(1H,m),3.39-3.67 (2H,m),3.92-4.20(3H,m), 5.80(1H,d,J=3.1Hz) | 3400 1764 1600 1383 (KBr) |

Table 86

| Chemical Structure | N M R | I R |
|---|---|---|
| 2 4 3 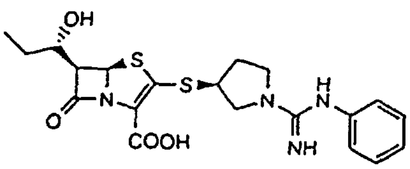 | (DMSO-$d_6$) 0.94(3H,t,J=7Hz),1.42(1H,m), 1.77(1H,m),2.02(1H,m), 2.34-2.45(1H,m), 2.90-3.01(1H,m), 3.52-3.69(3H,m), 3.73-3.84(2H,m), 3.94(1H,m),5.63(1H,m), 7.23(3H,m),7.40(2H,m) | 1765 1625 1590 (KBr) |

Example 244

Synthesis of (5R,6R)-2-[(S)-1-[(3,4-dihydroxy)-phenacyl]pyrrolidin-3-yl]thio-6-((S)-2-hydroxypropyl)penem-3-carboxylic acid

To a solution of p-nitrobenzyl (5R,6R)-6-[(S)-1-(4-pentenoyl)pyrrolidin-3-yl]thio-6-((S)-2-hydroxypropyl)penem-3-carboxylate (100 mg, 0.18 mmol) in THF/water (0.37 mℓ/0.18 mℓ), iodine (114 mg) was added at room temperature. After the resultant mixture was stirred for 10 minutes at the same temperature, a 5% aqueous solution of sodium thiosulfate·$5H_2O$ and a saturated aqueous solution of sodium hydrogencarbonate were added. The thus-obtained mixture was extracted with methylene chloride. The organic layer was dried over anhydrous sodium sulfate and then concentrated under reduced pressure. The residue so obtained was dissolved in DMF (0.5 mℓ), followed by the successive addition of (3,4-dinitrobenzyl)phenacyl bromide (100 mg, 0.2 mmol) and triethylamine (40 mg, 0.4 mmol). The resultant mixture was stirred at room temperature for 30 minutes. After the solvent was distilled off by a pump, the residue was purified by flash column chromatography, whereby a residue containing p-nitrobenzyl (5R,6R)-6-[(S)-1-[(3,4-dinitrobenzyl)-phenacyl]pyrrolidin-3-yl]thio-6-((S)-2-hydroxypropyl)-penem-3-carboxylate was obtained. The residue was dissolved in THF/0.1 M phosphate buffer of pH 7 (6 mℓ/4 mℓ), followed by stirring under a hydrogen gas atmosphere for 4.5 hours in the presence of 10% palladium/carbon (130 mg). After the catalyst was filtered off, the filtrate was lyophilized. The residue so obtained was purified by HPLC, whereby the title compound was obtained (1 mg, 1% yield).

NMR δ($CD_3OD$):

1.02 (t,7Hz,3H), 1.45-1.55 (m,1H), 1.80-1.95 (m,1H), 2.30-2.50 (m,1H), 2.65-3.00 (m,3H), 3.84 (dd,J=11Hz,4Hz, 1H), 3.95-4.05 (m,2H), 5.66 (d,J=4Hz,1H), 6.79 (d,J=8Hz,1H), 7.40-7.50 (m,1H).

IR (KBr):

1766.

Example 245

Synthesis of (5R,6R)-2-[(S)-1-(3-aminophenacyl)-pyrrolidin-3-yl]thio-6-((S)-2-hydroxypropyl)-penem-3-carboxylic acid

To a mixed solution of p-nitrobenzyl (5R,6R)-2-[(S)-1-(4-pentenoyl)pyrrolidin-3-yl]thio-6-((S)-2-hydroxypropyl) penem-3-carboxylate (70 mg, 0.13 mmol) in THF/water (0.26 mℓ/0.13 mℓ), iodine (90 mg) was added at room temperature. After the resultant mixture was stirred for 10 minutes at the same temperature, a 5% aqueous solution of sodium thiosulfate·$5H_2O$ and a saturated aqueous solution of sodium hydrogencarbonate were added. The resultant mixture was extracted with methylene chloride.

The organic layer was dried over anhydrous sodium sulfate and then concentrated under reduced pressure. The residue was purified by flash column chromatography, whereby p-nitrobenzyl (5R,6R)-2-((S)-1-(3-nitrophenacyl) pyrrolidin-3-yl)thio-6-((S)-2-hydroxypropyl)penem-3-carboxylate was obtained.
NMR δ($CD_3OD$):
1.06 (t,7Hz,3H), 1.55-1.65 (m,1H), 1.90-2.05 (m,1H), 2.40-2.50 (m,1H), 2.74-2.95 (m,3H), 3.40-3.45 (m,1H), 3.85-3.90 (m,1H), 3.94 (dd,J=10Hz,4Hz,1H), 4.00 (s,2H), 4.18-4.25 (m,2H), 5.20 (d,J=13Hz,9H), 5.45 (d,J=13Hz,1H), 5.75 (d,J=4Hz,1H), 7.61 (d,J=8Hz,2H), 7.65-7.75 (m,1H), 8.20-8.25 (m,3H), 8.33 (d,J=8Hz,1H), 8.42 (dd,J=8Hz,1Hz, 1H).

The thus-obtained ester was treated in a similar manner as in Example 48, whereby the title compound was obtained (0.8 mg, 7% yield).
NMR ($D_2O$):
1.11 (t,J=7Hz,3H), 4.65-4.7 (m,1H), 1.95-2.05 (m,1H), 2.25-2.35 (m,1H), 2.70-2.85 (m,1H), 3.40-4.30 (m,8H), 5.92 (d,J=4Hz,1H), 7.25-7.30 (m,1H), 7.45-7.55 (m,3H).
IR (KBr):
1766.

Example 246

Synthesis of (5R,6R)-2-(1-methyl-6,7-dihydro-5H-cyclopenta[b]pyridinium-7-yl)thio-6-((S)-2-hydroxypropyl)penem-3-carboxylic acid

To a solution of p-nitrobenzyl (5R,6R)-2-(6,7-dihydro-5H-cyclopenta[b]pyridin-7-yl)thio-6-((S)-2-hydroxypropyl)penem-3-carboxylate (50 mg, 0.1 mmol) in acetone (3 mℓ), methyl iodide (0.3 mℓ) was added at room temperature and 24 hours later, the solvent was distilled off under reduced pressure. The residue so obtained was added successively with THF/water (4 mℓ/4 mℓ) and iron powder (420 mg), followed by stirring at 0°C for 20 minutes. Insoluble matter was then filtered off. After the solvent was distilled off at room temperature under reduced pressure, the residue was purified by HPLC, whereby two types of isomers were obtained as a first eluate fraction (2.4 mg, 7% yield) and a second eluate fraction (1.4 mg, 4% yield), respectively.

(First eluate fraction)

NMR ($D_2O$):
1.08 (t,J=7Hz,3H), 1.57-1.70 (m,1H), 1.87-2.00 (m,1H), 2.80-3.00 (m,2H), 3.20-3.60 (m,2H), 4.10-4.20 (m,2H), 4.49 (s,3H), 5.29 (d,J=7Hz,1H), 5.86 (d,J=3Hz,1H), 7.93 (dd,J=8Hz,6Hz,1H), 8.45 (d,J=8Hz,1H), 8.64 (d,J=6Hz,1H).
IR (KBr):
3440, 1770, 1594.
(Second eluate fraction)
NMR ($D_2O$):
1.06 (t,J=7Hz,3H), 1.55-1.70 (m,1H), 1.85-2.00 (m,1H), 2.65-2.75 (m,1H), 2.90-3.05 (m,1H), 3.20-3.50 (m,1H), 3.35-3.45 (m,1H), 4.10-4.20 (m,1H), 4.46 (s,3H), 5.22 (d,J=8Hz,1H), 5.78 (d,J=3Hz,1H), 7.92 (dd,J=8Hz,6Hz,1H), 8.42 (d,J=8Hz,1H), 8.64 (d,J=6Hz,1H).
IR (KBr):
3440, 1770, 1598, 1372.

Example 247

Synthesis of (5R,6R)-2-[1-(1-acetamido-4-pyridinium)pyrrolidin-3-ylthio]-6-((S)-hydroxypropyl)penem-3-carboxylate

In Example 246, iodoacetamide (25 mg, 0.14 mmol) was added to a solution of p-nitrobenzyl (5R,6R)-2-[1-(4-pyridyl)pyrrolidin-3-ylthio]penem-3-carboxylate (12 mg, 0.022 mmol) in lieu of p-nitrobenzyl (5R,6R)-2-(6,7-dihydro-5H-cyclopenta[b]pyridin-7-yl)thio-6-((S)-2-hydroxypropyl)penem-3-carboxylate in methylene chloride (5 mℓ). The resultant mixture was stirred at room temperature for 14 hours. The reaction mixture was concentrated under reduced pressure, and the residue so obtained was added with methanol-0.1 M phosphate buffer (pH 7.0) (4 mℓ - 3 mℓ), 10% palladium carbon (50 mg). The mixture so obtained was stirred under a hydrogen gas atmosphere for 2 hours. The catalyst was filtered off and the filtrate was purified by HPLC, whereby the title compound was obtained (0.6 mg, 5% yield).
NMR δ($CD_3OD$) :
1.03 (3H,t,J=7Hz), 1.42-1.51 (1H,m), 1.81-1.90 (1H,m), 2.11-2.22 (1H,m), 2.55-2.61 (1H,m), 3.61-3.80 (4H,m), 3.87 (1H,m), 3.92-4.00 (2H,m), 4.51 (2H,s), 5.70 (1H,d,J=4Hz), 6.75 (2H,m), 8.06 (2H,d,J=8Hz).

IR (KBr):
1765, 1645, 1550.

Example 248

Synthesis of (5R,6R)-6-((S)-hydroxypropyl)-2-((3S,5R)-5-pyridylmethylpyrrolidin-3-yl)thiopenem-3-carboxylate

To a solution of (3S,5R)-3-benzoylthio-5-hydroxymethyl-1-p-nitrobenzyloxypyrrolidine (184 mg, 0.44 mmol) in methylene chloride (1.5 mℓ), 2,6-lutidine (67 μℓ, 0.57 mmol), trifluoromethanesulfonic anhydride (97 μℓ, 0.57 mmol) and pyridine (46 μℓ, 0.57 mmol) were added at -78°C. The reaction mixture was gradually heated to room temperature. The solvent was then distilled off under reduced pressure, whereby a crude reaction product containing the title compound was obtained (470 mg).

A portion (138 mg) of the crude reaction product was dissolved in a mixture of methanol (1.5 mℓ) and water (3 mℓ), followed by the addition of 1 N NaOH (0.33 mℓ, 0.33 mmol) at 0°C. The resultant mixture was stirred at room temperature for 1 hour. The solvent was distilled off under reduced pressure and the residue so obtained was purified by HPLC, whereby (3S,5R)-3-mercapto-1-p-nitrobenzyloxy-5-pyridylmethylpyrrolidine (40 mg) was obtained. To a solution of the whole thiol so obtained and p-nitrobenzyl (5R,6R)-6-((S)-hydroxypropyl)-2-methylsulfinylpenem-3-carboxylate (40 mg, 0.07 mmol) in DMF (4 mℓ), diisopropylethylamine (26 μℓ, 0.15 mmol) was added at -20°C. After the thus-obtained mixture was stirred at the same temperature for 30 minutes, the solvent was distilled off under reduced pressure at room temperature. The residue so obtained was dissolved in THF/phosphate buffer (pH 7) (1.5 mℓ/3 mℓ), and the resulting solution was stirred in the presence of palladium-carbon (10%) (120 mg) in a hydrogen gas atmosphere for 2 hours. Insoluble matter was filtered off and the filtrate was purified by HPLC, whereby the title compound was obtained (2 mg, 1% yield).

NMR ($D_2O$):
1.00 (3H,t,J=7.4Hz), 1.48-1.62 (1H,m), 1.71-1.90 (2H,m), 2.69-2.78 (1H,m), 3.29 (1H,dd,J=4.4 and 12.5Hz), 3.57 (1H,dd,J=7.0 and 12.5Hz), 3.97-4.10 (3H,m), 4.81 (1H,dd,J=8.9 and 13.9Hz), 4.96 (1H,dd,J=5.0 and 13.9Hz), 5.79 (1H,d,J=2.7Hz), 8.11-8.18 (2H,m), 8.60-8.67 (1H,m), 8.88-8.94 (2H,m).

IR (KBr):
3401, 1764, 1578, 1376.

Examples 249 to 257

Following the procedures of Example 103 except for the use of methyl benzylimidate tetrafluoroboric acid salt, methyl propaneimidate tetrafluoroboric acid salt, ethyl formimidate hydrochloride, methyl N-methylacetimidate tetrafluoroboric acid salt and ethyl fluoroacetimidate hydrochloride instead of methyl acetimidate tetrafluoroboric acid salt, the corresponding compounds of Examples 249 to 253 were obtained.

Further, following the procedures of Example 103 except for the use of methyl acetimidate tetrafluoroboric acid salt and ethyl formimidate hydrochloride and also the use of p-nitrobenzyl (5R,6R)-6-((S)-1-hydroxypropyl)-2-(1-p-nitrobenzyloxycarbonylazetidin-3-yl)-thiopenem-3-carboxylate, p-nitrobenzyl (5R,6R)-6-((S)-1-hydroxypropyl)-2-[1-(2-(4-p-nitrobenzyloxycarbonylpiperazin-1-yl)-2-oxoethyl]pyrrolidin-3-yl]thiopenem-3-carboxylate and p-nitrobenzyl (5R,6R)-6-((S)-hydroxypropyl)-2-[1-(4-nitrophenyl)pyrrolidin-3-yl]thiopenem-3-carboxylate instead of p-nitrobenzyl (5R,6R)-6-((S)-1-hydroxypropyl)-2-((S)-p-nitrobenzyloxycarbonylpyrrolidin-3-yl)thiopenem-3-carboxylate, the corresponding compounds of Examples 254 to 257 were obtained.

Physical data of the compounds obtained in Examples 249 to 257 are shown in Tables 87 to 89.

Table 87

| Chemical Structure | NMR | IR |
|---|---|---|
| 249 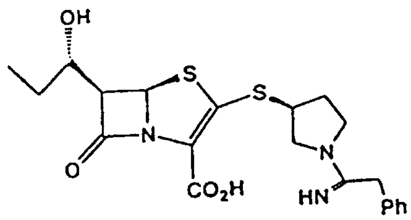 | ($CD_3OD$) 1.01(3H,m),1.48-1.53(1H,m), 1.86-2.00(1H,m),2.03-2.10 (1H,m),2.38-2.50(1H,m), 3.60-3.77(2H,m),3.80-3.96 (5H,m),3.98(2H,s), 5.60(1H,d,J=4Hz), 7.30-7.42(5H,m) | 1770 1585 (KBr) |
| 250 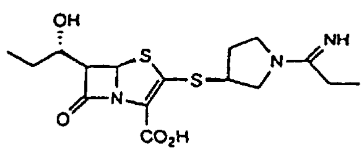 | ($D_2O$) 1.01(3H,t,J=7.4Hz), 1.20-1.29(3H,m), 1.52-1.72(2H,m), 1.80-1.94(1H,m), 2.14-2.26(1H,m), 2.48-2.65(2H,m), 3.48-4.22(7H,m), 5.80-5.85(1H,m) | |
| 251 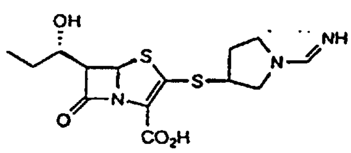 | ($D_2O$) 1.01(3H,t,J=7.4Hz), 1.50-1.64(1H,m), 1.80-1.93(1H,m), 2.00-2.12(1H,m), 3.41-4.18(7H,m), 5.78-5.84(1H,m), 8.12-8.17(1H,brs) | 3414 1765 1653 1384 (KBr) |
| 252 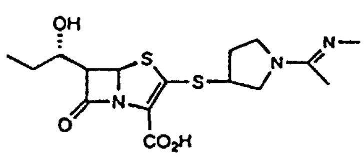 | ($D_2O$) 1.01(3H,t,J=7.4Hz),1.52-1.64(1H,m),1.82-1.94(1H,m), 2.08-2.25(1H,m),2.27-2.32 (3H,m),2.45-2.60(1H,m), 3.01-3.06(3H,m),3.50-3.68 (2H,m),3.78-3.96(2H,m), 4.05-4.20(3H,m), 5.81(1H,d,J=3.4Hz) | 3418 1766 1653 1590 1376 (KBr) |

Table 88

| Chemical Structure | N M R | I R |
|---|---|---|
| 253 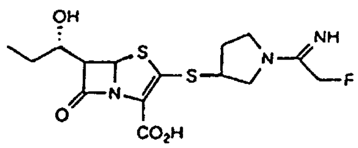 | ($D_2O$) 0.97-1.03(3H,m),1.51-1.63 (1H,m),1.82-1.94(1H,m), 2.17-2.27(1H,m),2.50-2.62 (1H,m),3.35-3.84(4H,m), 4.01-4.23(3H,brs),5.32-5.38(1H,brs),5.44-5.49 (1H,brs),5.81-5.85(1H,brs) | 3418 1764 1589 1377 (KBr) |
| 254 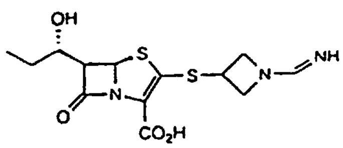 | ($D_2O$) 1.00(3H,t,J=7.2Hz),1.50-1.64(1H,m),1.79-1.93(1H,m), 3.36-4.50(7H,m),5.83 (1H,d,J=3.1Hz),7.80(1H,s) | 3376 1766 1632 1384 (KBr) |
| 255 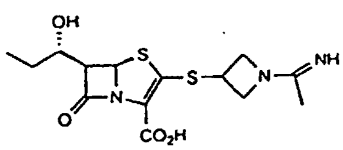 | ($D_2O$) 1.00(3H,t,J=7.4Hz),1.50-1.63(1H,m),1.80-1.92(1H,m), 2.05-2.13(3H,m),3.40-3.62 (1H,m),3.88-4.44(6H,m), 5.78-5.83(1H,m) | 3423 1766 1652 1382 (KBr) |
| 256 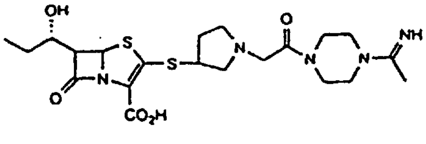 | ($D_2O$) 1.00(3H,t,J=7.4Hz),1.50-1.62(1H,m),1.80-1.94(1H,m), 1.78-2.12(1H,m),2.35(3H,s), 2.48-2.61(1H,m),3.15-3.43 (3H,m),3.54-3.90(9H,m), 3.96-4.18(5H,m), 5.80(1H,d,J=3.6Hz) | 3368 2974 1772 1631 1590 (KBr) |

Table 89

| Chemical Structure | NMR | IR |
|---|---|---|
| 257 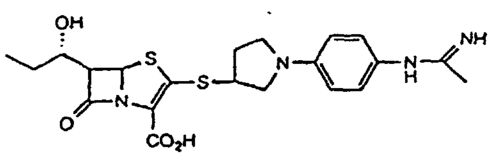 | ($D_2O$) 1.01(3H,t,J=7.3Hz),1.50-1.63(1H,m),1.81-1.94(1H,m),2.10-2.11(1H,m),2.39(3H,s),2.50-2.61(1H,m),3.34-3.60(4H,m),3.77-3.86(1H,m),4.04-4.18(2H,m),5.80-5.85(1H,m),6.77-6.86(2H,m),7.18-7.27(2H,m) | 3384 1767 1520 1377 (KBr) |

Preparation Example 1

Capsules

In accordance with the following compositions and preparation method, capsules were prepared. The active ingredient can be any compound according to the present invention.

| | | Composition 1 | Composition 2 |
|---|---|---|---|
| (1) | Active ingredient | 250 mg | 100 mg |
| (2) | Corn starch | 20 mg | 10 mg |
| (3) | Magnesium stearate | 5 mg | 2 mg |
| | (Total amount) | 275 mg | 112 mg |

(Preparation method)

To give the above amounts per capsule, the ingredient (1) and the ingredient (2) were measured and mixed together in a suitable mixer, followed by the addition of the ingredient (3). The thus-obtained powder was mixed further.

The resulting mixture was filled in capsules by a capsule filler.

Preparation Example 2

Tablets

In accordance with the following compositions and preparation method, tablets were prepared. The active ingredient can be any compound according to the present invention.

| (Composition) | |
|---|---|
| (1) Active ingredient | 250 mg |
| (2) Lactose | 55 mg |
| (3) Corn starch | 40 mg |
| (4) Magnesium stearate | 5 mg |

(continued)

| (Composition) | |
|---|---|
| (Total amount) | 350 mg |

(Preparation method)

To give the above amounts per tablet, the ingredients (1) to (3) were measured and mixed together in a suitable mixer.

The ingredient (4) was added further, followed by further mixing for several minutes. The resulting mixture was compressed into tables of predetermined dimensions and weight by a tablet machine.

Preparation Example 3

Injections

In accordance with the following preparation method, injections were prepared. The active ingredient can be any compound according to the present invention.

(Preparation method)

Injections were obtained by filling a sterilized aqueous solution of the active ingredient in 20-mℓ, 10-mℓ or 5-mℓ ampoules to contain the active ingredient in an amount of 1.0 g, 0.5 g or 0.25 g and then sealing them.

## Claims

**1.** A (5R,6R)-6-((S)-1-hydroxypropyl)-penem derivative represented by the following formula (I):

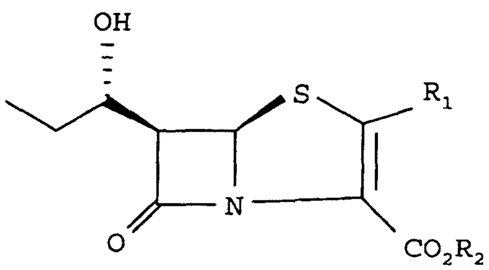

(I)

wherein:

$R_1$ represents an alkyl, alkenyl, $C_{7-14}$ aralkyl, $C_{6-10}$ aryl, alkylthio, alkenylthio, $C_{7-14}$ aralkylthio, $C_{6-10}$ arylthio, heterocyclic, heterocyclic thio, acylthio, or mercapto group or a hydrogen atom, any of said groups being optionally substituted with one or more selected from halogen; carboxyl; thiocarboxyl; formyl; nitro; cyano; hydroxyl; amino; imino; ($C_{1-6}$ alkylene)acetal; $C_{1-6}$ alkyl; monocyclic or polycyclic alkyl (which may be in the form of a fused ring with an aromatic hydrocarbon and may contain one or more carbonyl groups in its chain or ring); $C_{2-6}$ alkenyl; $C_{6-10}$ aryl; $C_{7-24}$ aralkyl; $C_{1-6}$ alkylthio; $C_{2-6}$ alkenylthio; $C_{7-24}$ aralkylthio; $C_{6-10}$ arylthio; $C_{1-6}$ alkyloxy; $C_{2-6}$ alkenyloxy; $C_{7-24}$ aralkyloxy; $C_{6-10}$ aryloxy; $C_{1-6}$ alkylsulfinyl; $C_{1-6}$ alkylsulfonyl; $C_{7-24}$ aralkylsulfinyl; $C_{7-24}$ aralkylsulfonyl; $C_{6-10}$ arylsulfinyl; $C_{6-10}$ arylsulfonyl; aminosulfonyl; carbamoyl; carbamoyloxy; carbamoyl $C_{1-6}$ alkyl; imino($C_{1-6}$ alkyl); imino($C_{1-6}$ alkyl)amino; imino(amino) ($C_{1-6}$ alkyl); acyloxy; acylalkyl; silyloxy; heterocyclic; heterocyclic thio; heterocyclic oxy; acyl; esterified carboxyl; esterified thiocarboxyl, or, only when $R_1$ is heterocyclic or heterocyclic thio, unsaturated $C_{5-7}$ cycloalkyl or $C_{9-11}$ fused rings optionally containing one or more carbonyl groups in their rings; and said heterocyclic group or moiety being selected from 3-8 membered saturated or unsaturated, heteromonocyclic groups containing 1 to 4 nitrogen atoms; 1 or 2 oxygen atoms and 1 to 3 nitrogen atoms; 1 or 2 sulfur atoms and 1 to 3 nitrogen atoms; 1 or 2 oxygen atoms; or 1 sulfur atom; and 7-12 membered, unsaturated, heteropolycyclic groups containing 1 to 5 nitrogen

atoms; 1 or 2 oxygen atoms and 1 to 3 nitrogen atoms; or 1 or 2 sulfur atoms and 1 to 3 nitrogen atoms; wherein alkyl or alkyl as part of an alkylthio, aralkyl or aralkylthio group includes monocyclic or polycyclic alkyl which may be in the form of a fused ring with an aromatic hydrocarbon and may contain one or more carbonyl groups in its chain or ring, and

$R_2$ represents a hydrogen atom or a carboxyl-protecting group which is removable *in vivo* to form the free acid; or a pharmacologically acceptable salt thereof.

**2.** The use in the manufacture of a medicament for the treatment of infection with methicillin-resistant Staphylococcus aureus of a (5R,6R)-6-((S)-1-hydroxypropyl)-penem derivative represented by represented by the following formula (I):

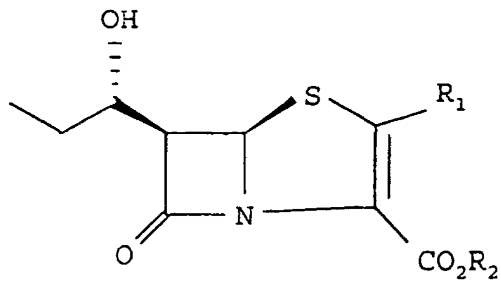

(I)

wherein:

$R_1$ represents an alkyl, alkenyl, $C_{7-14}$ aralkyl, $C_{6-10}$ aryl, alkylthio, alkenylthio, $C_{7-14}$ aralkylthio, $C_{6-10}$ arylthio, heterocyclic, heterocyclic thio, acylthio, or mercapto group or a hydrogen atom, any of said groups being optionally substituted with one or more selected from halogen; carboxyl; thiocarboxyl; formyl; nitro; cyano; hydroxyl; amino; imino; ($C_{1-6}$ alkylene)acetal; $C_{1-6}$ alkyl; monocyclic or polycyclic alkyl (which may be in the form of a fused ring with an aromatic hydrocarbon and may contain one or more carbonyl groups in its chain or ring); $C_{2-6}$ alkenyl; $C_{6-10}$ aryl; $C_{7-24}$ aralkyl; $C_{1-6}$ alkylthio; $C_{2-6}$ alkenylthio; $C_{7-24}$ aralkylthio; $C_{6-10}$ arylthio; $C_{1-6}$ alkyloxy; $C_{2-6}$ alkenyloxy; $C_{7-24}$ aralkyloxy; $C_{6-10}$ aryloxy; $C_{1-6}$ alkylsulfinyl; $C_{1-6}$ alkylsulfonyl; $C_{7-24}$ aralkylsulfinyl; $C_{7-24}$ aralkylsulfonyl; $C_{6-10}$ arylsulfinyl; $C_{6-10}$ arylsulfonyl; aminosulfonyl; carbamoyl; carbamoyloxy; carbamoyl $C_{1-6}$ alkyl; imino($C_{1-6}$ alkyl); imino($C_{1-6}$ alkyl)amino; imino(amino)($C_{1-6}$ alkyl); acyloxy; acylalkyl; silyloxy; heterocyclic; heterocyclic thio; heterocyclic oxy; acyl; esterified carboxyl; esterified thiocarboxyl, or, only when $R_1$ is heterocyclic or heterocyclic thio, unsaturated $C_{5-7}$ cycloalkyl or $C_{9-11}$ fused rings optionally containing one or more carbonyl groups in their rings; and said heterocyclic group or moiety being selected from 3-8 membered saturated or unsaturated, heteromonocyclic groups containing 1 to 4 nitrogen atoms; 1 or 2 oxygen atoms and 1 to 3 nitrogen atoms; 1 or 2 sulfur atoms and 1 to 3 nitrogen atoms; 1 or 2 oxygen atoms; or 1 sulfur atom; and 7-12 membered, unsaturated, heteropolycyclic groups containing 1 to 5 nitrogen atoms; 1 or 2 oxygen atoms and 1 to 3 nitrogen atoms; or 1 or 2 sulfur atoms and 1 to 3 nitrogen atoms; wherein alkyl or alkyl as part of an alkylthio, aralkyl or aralkylthio group includes monocyclic or polycyclic alkyl which may be in the form of a fused ring with an aromatic hydrocarbon and may contain one or more carbonyl groups in its chain or ring, and

$R_2$ represents a hydrogen atom or a carboxyl-protecting group which is removable *in vivo* to form the free acid; or a pharmacologically acceptable salt thereof.

**3.** A penem derivative or a pharmacologically acceptable salt thereof according to Claim 1, wherein in the formula (I), $R_1$ represents a heterocyclic thio group optionally substituted with one or more selected from halogen; carboxyl; thiocarboxyl; formyl; nitro; cyano; hydroxyl; amino; imino; ($C_{1-6}$ alkylene)acetal; $C_{1-6}$ alkyl; monocyclic or polycyclic alkyl (which may be in the form of a fused ring with an aromatic hydrocarbon and may contain one or more carbonyl groups in its chain or ring); $C_{2-6}$ alkenyl; $C_{6-10}$ aryl; $C_{7-24}$ aralkyl; $C_{1-6}$ alkylthio; $C_{2-6}$ alkenylthio; $C_{7-24}$ aralkylthio; $C_{6-10}$ arylthio; $C_{1-6}$ alkyloxy; $C_{2-6}$ alkenyloxy; $C_{7-24}$ aralkyloxy; $C_{6-10}$ aryloxy; $C_{1-6}$ alkylsulfinyl; $C_{1-6}$ alkylsulfonyl; $C_{7-24}$ aralkylsulfinyl; $C_{7-24}$ aralkylsulfonyl; $C_{6-10}$ arylsulfinyl; $C_{6-10}$ arylsulfonyl; aminosulfonyl; carbamoyl; carbamoyloxy; carbamoyl $C_{1-6}$ alkyl; imino($C_{1-6}$ alkyl); imino($C_{1-6}$ alkyl)amino; imino(amino)($C_{1-6}$ alkyl); acyloxy; acylalkyl; silyloxy; heterocyclic; heterocyclic thio; heterocyclic oxy; acyl; esterified carboxyl; esterified thiocarboxyl, or unsaturated $C_{5-7}$ cycloalkyl or $C_{9-11}$ fused rings optionally containing one or more carbonyl groups in their rings; and said heterocyclic moiety being selected from 3-8 membered saturated or unsaturated, heteromonocyclic

groups containing 1 to 4 nitrogen atoms; 1 or 2 oxygen atoms and 1 to 3 nitrogen atoms; 1 or 2 sulfur atoms and 1 to 3 nitrogen atoms; 1 or 2 oxygen atoms; or 1 sulfur atom; and 7-12 membered, unsaturated, heteropolycyclic groups containing 1 to 5 nitrogen atoms; 1 or 2 oxygen atoms and 1 to 3 nitrogen atoms; or 1 or 2 sulfur atoms and 1 to 3 nitrogen atoms.

**4.** A penem derivative or a pharmacologically acceptable salt thereof according to Claim 3, wherein the heterocyclic moiety of $R_1$ is a pyrrolyl, pyrrolinyl, imidazolyl, pyrazolyl, pyridyl, pyrimidyl, pyrazinyl, pyridazinyl, triazolyl, tetrazolyl, dihydrotriazinyl, azetidinyl, pyrrolidinyl, imidazolidinyl, piperidinyl, pyrazolidinyl or piperazinyl group.

**5.** A penem derivative or a pharmacologically acceptable salt thereof according to Claim 4, wherein said heterocyclic moiety is a pyrrolidinyl group.

**6.** A penem derivative or a pharmacologically acceptable salt thereof according to Claim 5, wherein said heterocyclic moiety is an (S)-pyrrolidin-3-yl group.

**7.** A penem derivative or a pharmacologically acceptable salt thereof according to Claim 4, wherein said heterocyclic moiety is a piperidinyl group.

**8.** A penem derivative or a pharmacologically acceptable salt thereof according to Claim 7, wherein said heterocyclic moiety is a piperidin-4-yl group.

**9.** A penem derivative or a pharmacologically acceptable salt thereof according to Claim 7, wherein said heterocyclic moiety is a piperidin-3-yl group.

**10.** A penem derivative or a pharmacologically acceptable salt thereof according to Claim 3, wherein said heterocyclic moiety of $R_1$ is an indolyl, isoindolyl, indolizinyl, benzimidazolyl, quinolyl, isoquinolyl, indazolyl, benzotriazolyl, tetrazolopyridyl, tetrazolopiridazinyl, dihydrotriazolopyridazinyl or 6,7-dihydro-5H-cyclopenta[b]pyridin-7-yl group.

**11.** A penem derivative or a pharmacologically acceptable salt thereof according to Claim 3, wherein said heterocyclic moiety of $R_1$ is an oxazolyl, isooxazolyl, oxadiazolyl or morpholinyl group.

**12.** A penem derivative or a pharmacologically acceptable salt thereof according to Claim 3, wherein said heterocyclic moiety of $R_1$ is a benzoxazolyl or benzoxadiazolyl group.

**13.** A penem derivative or a pharmacologically acceptable salt thereof according to Claim 3, wherein said heterocyclic moiety of $R_1$ is a 1,3-thiazolyl, 1,2-thiazolyl, tiazolinyl, thiadiazolyl or thiazolidinyl group.

**14.** A penem derivative or a pharmacologically acceptable salt thereof according to Claim 3, wherein said heterocyclic moiety of $R_1$ is a benzothiazolyl or benzothiadiazolyl group.

**15.** A penem derivative or a pharmacologically acceptable salt thereof according to Claim 3, wherein said heterocyclic moiety of $R_1$ is a furanyl, pyranyl, tetrahydrofuranyl or tetrahydropyranyl group.

**16.** A penem derivative or a pharmacologically acceptable salt thereof according to Claim 3, wherein said heterocyclic moiety of $R_1$ is a thienyl or tetrahydrothienyl group.

**17.** A penem derivative or a pharmacologically acceptable salt thereof according to Claim 1, wherein $R_1$ represents a said substituted or unsubstituted alkylthio group.

**18.** A penem derivative or a pharmacologically acceptable salt thereof according to Claim 17, wherein the alkyl group of said substituted or unsubstituted alkylthio group is a linear or branched $C_{1-6}$ alkyl group, or a monocyclic or polycyclic alkyl group which may be in the form of a fused ring with an aromatic hydrocarbon.

**19.** A penem derivative or a pharmacologically acceptable salt thereof according to Claim 17, wherein the alkyl group of said substituted or unsubstituted alkylthio group is a methyl, ethyl, n-propyl, isopropyl, n-butyl, tert-butyl or hexyl group.

**20.** A penem derivative or a pharmacologically acceptable salt thereof according to Claim 17, wherein the alkyl group

of said substituted or unsubstituted alkylthio group is a monocyclic or polycyclic alkyl group selected from a cyclopropyl, cyclopentyl, cyclohexyl, menthyl, fenchyl, bornyl or indanyl group.

**21.** A penem derivative or a pharmacologically acceptable salt thereof according to Claim 1, wherein $R_1$ represents a said substituted or unsubstituted alkenylthio group.

**22.** A penem derivative or a pharmacologically acceptable salt thereof according to Claim 21, wherein the alkenyl group of said substituted or unsubstituted alkenylthio group is a linear or branched, $C_{2-6}$ alkenyl group.

**23.** A penem derivative or a pharmacologically acceptable salt thereof according to Claim 21, wherein the alkenyl group of said substituted or unsubstituted alkenylthio group is a vinyl, allyl, 2-chloroallyl, 1-propenyl, 2-butenyl or 2-methyl-2-propenyl group.

**24.** A penem derivative or a pharmacologically acceptable salt thereof according to Claim 1, wherein $R_1$ represents a said substituted or unsubstituted aralkylthio group.

**25.** A penem derivative or a pharmacologically acceptable salt thereof according to Claim 24, wherein the aralkyl group of said substituted or unsubstituted aralkylthio group is an aralkyl group containing 7 to 24 carbon atoms.

**26.** A penem derivative or a pharmacologically acceptable salt thereof according to Claim 24, wherein the aralkyl group of said substituted or unsubstituted aralkylthio group is a benzyl, phenethyl, 3-phenyl-propyl, 2-naphthylmethyl, 2-(1-naphthyl)ethyl, trityl, benzhydryl or 1-phenyl-cyclopropan-1-yl group.

**27.** A penem derivative or a pharmacologically acceptable salt thereof according to Claim 1, wherein $R_1$ represents a said substituted or unsubstituted arylthio group.

**28.** A penem derivative or a pharmacologically acceptable salt thereof according to Claim 27, wherein the aryl group of said substituted or unsubstituted arylthio group is an aryl group containing 6 to 10 carbon atoms.

**29.** A penem derivative or a pharmacologically acceptable salt thereof according to Claim 27, wherein the aryl group of said substituted or unsubstituted arylthio group is a phenyl, tolyl, xylyl, mesityl, cumenyl or naphthyl group.

**30.** A penem derivative or a pharmacologically acceptable salt thereof according to Claim 1, wherein $R_1$ represents a said substituted or unsubstituted aryl group.

**31.** A penem derivative or a pharmacologically acceptable salt thereof according to Claim 1, wherein $R_1$ represents a said substituted or unsubstituted heterocyclic group.

**32.** A penem derivative or a pharmacologically acceptable salt thereof according to Claim 1, wherein $R_1$ is a group represented by the following formula:

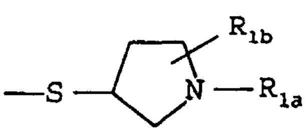

wherein $R_{1a}$ and $R_{1b}$ are the same or different and represent a hydrogen atom or an alkyl, alkenyl, $C_{7-14}$ aralkyl, $C_{6-10}$ aryl, imino $C_{1-6}$ alkyl, imino $C_{1-6}$ alkyl amino, imino(amino) $C_{1-6}$ alkyl, carbamonyl, carbamoyl $C_{1-6}$ alkyl, acyl, acyl $C_{1-6}$ alkyl, carboxyl, heterocyclic or heterocyclic $C_{1-6}$ alkyl; one or more hydrogen atoms of said alkyl, alkenyl, aralkyl, aryl, imino $C_{1-6}$ alkyl , imino $C_{1-6}$ alkyl amino, imino(amino) $C_{1-6}$ alkyl , carbamoyl, carbamoyl $C_{1-6}$ alkyl, heterocyclic or heterocyclic $C_{1-6}$ alkyl group optionally substituted by a halogen atom or a carboxyl, thiocarboxyl, formyl, nitro, cyano, hydroxyl, amino, imino, ($C_{1-6}$ alkylene) acetal, alkyl, alkoxyl, alkenyl, $C_{7-24}$ aralkyl, $C_{6-10}$ aryl, $C_{6-10}$ aryloxy, imino $C_{1-6}$ alkyl, imino $C_{1-6}$ alkyl amino, imino(amino) $C_{1-6}$ alkyl, carbamoyl, carbamoyloxy, carbamoyl $C_{1-6}$ alkyl, heterocyclic, heterocyclic $C_{1-6}$ alkyl, acyl or acylalkyl group; said acyl groups or moietiesrepresenting

an alkylcarbonyl, alkenylcarbonyl, aralkylcarbonyl, arylcarbonyl, heterocyclic carbonyl or heterocyclic ($C_{1-6}$ alkyl) carbonyl group containing said substituted or unsubstituted alkyl, alkenyl, aralkyl, aryl, heterocyclic or heterocyclic $C_{1-6}$ alkyl group; said carboxyl group may be esterified by said substituted or unsubstituted alkyl, alkenyl, aralkyl, aryl, heterocyclic or heterocyclic $C_{1-6}$ alkyl group; said heterocyclic groups or moieties optionally containing one or more carbonyl group in the rings thereof and the tertiary nitrogen atom thereof optionally forming an intramolecular quaternary salt by the introduction of said substituent.

**33.** A penem derivative or a pharmacologically acceptable salt thereof according to Claim 1, wherein $R_1$ is a group represented by the following formula:

$$-S-(CH_2)_n-R_{1c}$$

wherein n is 1 to 3; $R_{1c}$ represents a hydrogen atom or a $C_{6-10}$ aryl, amino, imino $C_{1-6}$ alkyl amino, aminosulfonyl, carbamoyl, acyl, carboxyl or heterocyclic group; one or more hydrogen atoms of said aryl, amino, imino $C_{1-6}$ alkyl - amino, aminosulfonyl, carbamoyl or heterocyclic group optionally substituted by a halogen atomor a carboxyl, thiocarboxyl, formyl, nitro, cyano, hydroxyl, amino, imino, alkyl, alkoxy, alkenyl, $C_{7-24}$ aralkyl, $C_{6-10}$ aryl, $C_{6-10}$ aryloxy, imino($C_{1-6}$ alkyl), imino $C_{1-6}$ alkyl amino, imino(amino) $C_{1-6}$ alkyl, carbamoyl, carbamoyloxy, carbamoyl-$C_{1-6}$ alkyl, heterocyclic, heterocyclic $C_{1-6}$ alkyl, acyl or acylalkyl group; said acyl groupor moiety recited as a substituent representing an alkylcarbonyl, alkenylcarbonyl, aralkylcarbonyl, arylcarbonyl, heterocyclic carbonyl or heterocyclic $C_{1-6}$ alkyl carbonyl group containing one or more alkyl, alkenyl, aralkyl, aryl, heterocyclic or heterocyclic $C_{1-6}$ alkyl groups; one or more hydrogen atoms of these acyl groups or moieties optionally substituted by a halogen atom or a carboxyl, thiocarboxyl, formyl, nitro, cyano, hydroxyl, amino, imino, $C_{1-6}$ alkylene acetal, alkyl, alkoxy, alkenyl, $C_{7-24}$ aralkyl, $C_{6-10}$ aryl, $C_{6-10}$ aryloxy, imino $C_{1-6}$ alkyl, imino $C_{1-6}$ alkyl amino, imino(amino) $C_{1-6}$ alkyl, carbamoyl, carbamoyloxy, carbamoyl $C_{1-6}$ alkyl, heterocyclic, heterocyclic $C_{1-6}$ alkyl, acyl or acylalkyl group; said carboxyl group being optionally esterified by a substituted or unsubstituted alkyl, alkenyl, aralkyl, aryl, heterocyclic or heterocyclic $C_{1-6}$ alkyl group; said heterocyclic group and the heterocyclic group of said heterocyclic $C_{1-6}$ alkyl groups, the latter heterocyclic group being recited as a substituent, optionally containing one or more carbonyl groups in the ring thereof and the tertiary nitrogen atom thereof optionally forming an intramolecular quaternary salt by the introduction of said substituent.

**34.** A penem derivative according to Claim 3 selected from:

(5R,6R)-2-(1-allyl-pyrrolidin-3-yl)thio-6-((S)-1-hydroxypropyl)penem-3-carboxylic acid;
(5R,6R)-2-(1-benzylpyrrolidin-3-yl)thio-6-((S)-1-hydroxypropyl)penem-6-carboxylic acid;
(5R,6R)-2-(pyrrolidin-3-yl)thio-6-((S)-1-hydroxypropyl)-penem-3-carboxylic acid;
(5R, 6R)-2-(1-(1-iminoethyl)pyrrolidin-3-yl)thio-6-((S)-1-hydroxypropyl)penem-3-carboxylic acid;
(5R,6R)-6-((S)-1-hydroxypropyl)-2-(1-phenylpyrrolidin-3-yl)thiopenem-3-carboxylic acid;
(5R,6R)-6-((S)-1-hydroxypropyl)-2-(1-phenethylpyrrolidin-3-yl)thiopenem-3-carboxylic acid;
(5R,6R)-6-((S)-1-hydroxypropyl)-2-(1-(2-hydroxy-2-phenylethyl)pyrrolidin-3-yl)thiopenem-3-carboxylic acid;
(5R,6R)-6-((S)-1-hydroxypropyl)-2-(1-(2-hydroxy-2-phenylethyl)pyrrolidin-3-yl)thiopenem-3-carboxylic acid;
5R,6R)-6-((S)-1-hydroxypropyl)-2-(1-phenacylpyrrolidin-3-yl)thiopenem-3-carboxylic acid;
(5R,6R)-2-(1-(2-p-fluorophenyl-2-oxoethyl)pyrrolidin-3-yl)thio-6-((S)-1-hydroxypropyl)penem-3-carboxylic acid;
(5R,6R)-6-((S)-1-hydroxypropyl)-2-(1-(2-oxo-2-p-tolylethyl)pyrrolidin-3-yl)thiopenem-3-carboxylic acid;
(5R,6R)-6- ((S)-1-hydroxypropyl)-2-(1-(2-p-methoxyphenyl-2-oxoethyl)pyrrolidin-3-yl)thiopenem-3-carboxylic acid;
(5R,6R)-2-(1-(2-benzoylethyl)pyrrolidin-3-yl)thio-6-((S)-1-hydroxypropyl)penem-3-carboxylic acid;
5R,6R)-2-(1-(1-benzoylethyl)pyrrolidin-3-yl)-thio-6-((S)-hydroxypropyl)penem-3-carboxylic acid;
(5R,6R)-6-((S)-1-hydroxypropyl) -2-(1-(1-indanon-2-yl)pyrrolidin-3-yl)thio-penem-3-carboxylic acid;
(5R,6R)-6-((S)-1-hydroxypropyl)-2-(1-(1-indanon-2-yl)pyrrolidin-3-yl)thio-penem-3-carboxylic acid;
(5R,6R)-6-((S)-1-hydroxypropyl)-2-(1-(2-pyridylmethyl)pyrrolidin-3-yl)thiopenem-3-carboxylic acid;
(5R,6R)-6-((S)-1-hydroxypropyl)-2- (1-benzylpiperidin-3-yl)thio-penem-3-carboxylic acid;
(5R,6R)-6-((S)-1-hydroxypropyl)-2- (4-phenylthiazol-2-yl)thiopenem-3-carboxylic acid;
(5R,6R)-6-((S)-1-hydroxypropyl)-2-(1-(4-pyridyl)-pyrrolidin-3-yl)thiopenem-3-carboxylic acid;
(5R,6R)-6-((S)-1-hydroxypropyl)-2-(1-(2-pyridyl)-pyrrolidin-3-yl)thiopenem-3-carboxylic acid;
(5R,6R)-6-((S)-1-hydroxypropyl)-2-(1-iminomethyl)-azetidin-3-yl)thiopenem-3-carboxylic acid;
(5R,6R)-6- ((S)-1-hydroxypropyl)-2-(1-iminoethyl)-azetidin-3-yl)thiopenem-3-carboxylic acid;

(5R,6R)-6-((S)-1-hydroxypropyl)-2-(2,3-cyclopento[b]-pyridin-7-yl)thiopenem-3-carboxylic acid;
(5R,6R)-2-(1-(1-iminopropylpyrrolidin-3-yl)thio-6-((S)-1-hydroxypropyl)penem-3-carboxylic acid;
(5R,6R)-6-((S)-1-hydroxypropyl)-2-(1-(3-benzodioxol-5-yl-acetonyl)pyrrolidin-3-yl)thiopenem-3-carboxylic acid;
(5R,6R)-2-(1-(4-(1-iminoethylamino)ethyl)phenyl)-pyrrolidin-3-yl)thio-6-((S)-1-hydroxypropyl)penem-3-carboxylic acid;
(5R,6R)-6-((S)-1-hydroxypropyl)-2-(1-pyrimidin-2-yl pyrrolidin-3-yl)thiopenem-3-carboxylic acid;
(5R,6R)-6-((S)-1-hydroxypropyl)-2-(1-($\alpha$-iminobenzamidomethyl) pyrrolidin-3-yl)thiopenem-3-carboxylic acid;
5R,6R)-6-((S)-1-hydroxypropyl)-2-(1-($\alpha$-imino)anilinomethyl pyrrolidin-3-yl)thiopenem-3-carboxylic acid;
(5R,6R)-6-((S)-1-hydroxypropyl)-2-(1-cyclopropylmethylpyrrolidin-3-yl)thiopenem-3-carboxylic acid;
(5R,6R)-6-((S)-1-hydroxypropyl)-2-(1-phenoxyethylpyrrolidin-3-yl)thiopenem-3-carboxylic acid;
(5R,6R)-6-((S)-1-hydroxypropyl)-2-(1-phenoxypropylpyrrolidin-3-yl)thiopenem-3-carboxylic acid;
(5R,6R)-6- ((S)-1-hydroxypropyl)-2-(1-o-tolyloxymethylpyrrolidin-3-yl)thiopenem-3-carboxylic acid;
(5R,6R)-6-((S)-1-hydroxypropyl)-2-(1-indan-2-yl-pyrrolidin-3-yl)thiopenem-3-carboxylic acid;
(5R,6R)-6-((S)-1-hydroxypropyl)-2-(1-cyclopentylpyrrolidin-3-yl)thiopenem-3-carboxylic acid;
(5R,6R)-6-((S)-1-hydroxypropyl)-2-(1-(3-phenylallyl)-pyrrolidin-3-yl)thiopenem-3-carboxylic acid;
5R,6R)-2-(1-(1-imino-2-fluoro-ethyl)pyrrolidin-3-yl)-thio-6-((S)-1-hydroxypropyl)penem-3-carboxylic acid;
(5R,6R)-2-(1-cyclopropylpyrrolidin-3-yl)thio-6-((S)-1-hydroxypropyl)penem-3-carboxylic acid; and
(5R,6R)-6-((S)-1-hydroxypropyl)-2-(1-thiazol-2-yl-pyrrolidin-3-yl)thiopenem-3-carboxylic acid

or a pharmacologically acceptable salt thereof.

**35.** A penem derivative according to Claim 1 selected from:

(5R,6R)-6-((S)-1-hydroxypropyl)-2-methylthiopenem-3-carboxylic acid;
(5R,6R)-6-((S)-1-hydroxypropyl)-2-(2-pyridylmethyl)-thiopenem-3-carboxylic acid;
5R,6R)-6-((S)-1-hydroxypropyl)-2-(benzimidazol-2-yl-methyl)thiopenem-3-carboxylic acid;
(5R,6R)-6-((S)-1-hydroxypropyl)-2-((S)-1-(4-pyridylmethyl)thiopenem-3-carboxylic acid;
(5R,6R)-2-(2-(1-iminoethylamino) ethyl) thio-6- ((S)-1-hydroxypropyl)penem-3-carboxylic acid;
(5R,6R)-2-(2-($\alpha$-iminobenzyl) aminoethyl)thio-6-((S)-1-hydroxypropyl)penem-3-carboxylic acid;
(5R, 6R)-6-((S)-1-hydroxypropyl)-2-(2-phenylaminocarbonylethyl)thiopenem-3-carboxylic acid;
(5R,6R)-6-((S)-1-hydroxypropyl)-2-(2-(N-methyl-N-phenacylamino)ethyl)thiopenem-3-carboxylic acid;
(5R,6R)-6-((S)-1-hydroxypropyl)-2-(2-(pyrrolidin-1-yl)ethyl)thiopenem-3-carboxylic acid;
(5R,6R)-6-((S)-1-hydroxypropyl)-2-(2-(1-pyrrolyl)-ethyl)thiopenem-3-carboxylic acid;
(5R,6R)-2-(2-aminoethyl)thio-6-((S)-1-hydroxypropyl)penem-3-carboxylic acid;
(5R,6R)-6-((S)-1-hydroxypropyl)-2-phenylthiopenem-3-carboxylic acid;
(5R,6R)-6-((S)-1-hydroxypropyl)-2-(3,4-dichlorobenzyl)thiopenem-3-carboxylic acid;
(5R,6R)-6-((S)-1-hydroxypropyl)-2-(3-cyanobenzyl)-thiopenem-3-carboxylic acid; and
5R,6R)-6-((S)-1-hydroxypropyl)-2-(2-indanyl)thiopenem-3-carboxylic acid

or a pharmacologically acceptable salt thereof.

**36.** A (5R,6R)-6-((S)-1-hydroxypropyl)-penem derivative represented by the following formula (II):

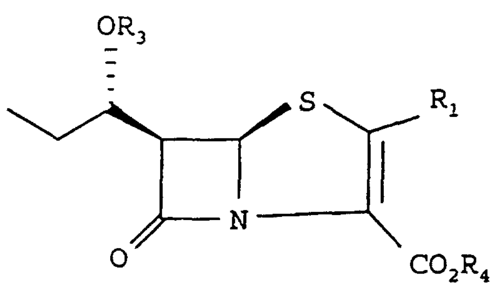

(II)

wherein $R_1$ is as defined in Claim 1, $OR_3$ represents a protected hydroxyl group, and $R_4$ represents a carboxyl-protecting group.

**37.** A penem derivative according to Claim 36, wherein $R_1$ is as defined in any one of Claims 3 to 33.

**38.** A (5R,6R)-6-((S)-1-hydroxypropyl)-penem derivative represented by the following formula (III):

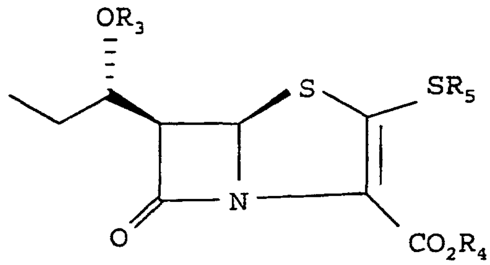

(III)

wherein:

$R_5$ represents an alkyl, alkenyl, $C_{7-14}$ aralkyl, $C_{6-10}$ aryl, heterocyclic or acyl group, any of said groups being optionally substituted with one or more selected from halogen; carboxyl; thiocarboxyl; formyl; nitro; cyano; hydroxyl; amino; imino; ($C_{1-6}$ alkylene)acetal; $C_{1-6}$ alkyl; monocyclic or polycyclic alkyl (which may be in the form of a fused ring with an aromatic hydrocarbon and may contain one or more carbonyl groups in its chain or ring); $C_{2-6}$ alkenyl; $C_{6-10}$ aryl; $C_{7-24}$ aralkyl; $C_{1-6}$ alkylthio; $C_{2-6}$ alkenylthio; $C_{7-24}$ aralkylthio; $C_{6-10}$ arylthio; $C_{1-6}$ alkyloxy; $C_{2-6}$ alkenyloxy; $C_{7-24}$ aralkyloxy; $C_{6-10}$ aryloxy; $C_{1-6}$ alkylsulfinyl; $C_{1-6}$ alkylsulfonyl; $C_{7-24}$ aralkylsulfinyl; $C_{7-24}$ aralkylsulfonyl; $C_{6-10}$ arylsulfinyl; $C_{6-10}$ arylsulfonyl; aminosulfonyl; carbamoyl; carbamoyloxy; carbamoyl $C_{1-6}$ alkyl; imino($C_{1-6}$ alkyl); imino($C_{1-6}$ alkyl)amino; imino(amino) ($C_{1-6}$ alkyl); acyloxy; acylalkyl; silyloxy; heterocyclic; heterocyclic thio; heterocyclic oxy; acyl; esterified carboxyl; esterified thiocarboxyl, or, only when $R_1$ is heterocyclic, unsaturated $C_{5-7}$ cycloalkyl or $C_{9-11}$ fused rings optionally containing one or more carbonyl groups in their rings; and said heterocyclic group being selected from 3-8 membered saturated or unsaturated, heteromonocyclic groups containing 1 to 4 nitrogen atoms; 1 or 2 oxygen atoms and 1 to 3 nitrogen atoms; 1 or 2 sulfur atoms and 1 to 3 nitrogen atoms; 1 or 2 oxygen atoms; or 1 sulfur atom; and 7-12 membered, unsaturated, heteropolycyclic groups containing 1 to 5 nitrogen atoms; 1 or 2 oxygen atoms and 1 to 3 nitrogen atoms; or 1 or 2 sulfur atoms and 1 to 3 nitrogen atoms; wherein alkyl or alkyl as part of an alkylthio, aralkyl or aralkylthio group includes monocyclic or polycyclic alkyl which may be in the form of a fused ring with an aromatic hydrocarbon and may contain one or more carbonyl groups in its chain or ring,
$OR_3$ represents a protected hydroxyl group, and
$R_4$ represents a carboxyl-protecting group.

**39.** A (5R,6R)-6-((S)-1-hydroxypropyl)-penem derivative represented by the following formula (IV):

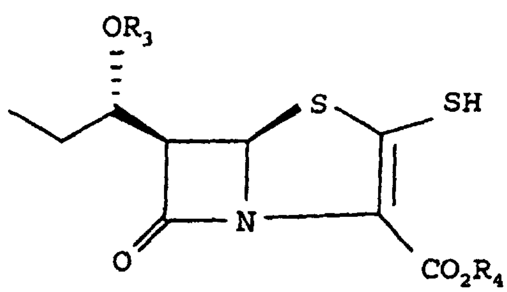

(IV)

wherein $OR_3$ represents a protected hydroxyl group and $R_4$ represents a carboxyl-protecting group.

**40.** A medicament comprising, as an active ingredient, a (5R,6R)-6-((S)-1-hydroxypropyl)-penem derivative represented by the formula (I) as defined in Claim 1 or a pharmacologically acceptable salt thereof.

**41.** A medicament according to Claim 40, wherein the penem derivative is as defined in any one of Claims 3 to 39.

**42.** The use in the manufacture of an antibacterial medicament of a (5R,6R)-6-((S)-1-hydroxypropyl)-penem derivative represented by formula (I) as defined in Claim 1 or a pharmacologically acceptable salt thereof.

**43.** A use according to Claim 42, wherein the penem derivative is as defined in any one of Claims 3 to 39.

**44.** A use according to Claim 2, wherein the penem derivative or pharmacologically acceptable salt thereof is as defined in any one of Claims 3 to 39.

**45.** A penem derivative or a pharmacologically acceptable salt thereof according to Claim 1 for use in therapy.

**46.** A penem derivative or a pharmacologically acceptable salt thereof according to Claim 45, wherein the penem derivative is as defined in any one of Claims 2 to 39.

**Patentansprüche**

**1.** (5R,6R) - 6 - [(S) - 1 - Hydroxypropyl] - penemderivat gemäß der folgenden Formel (I):

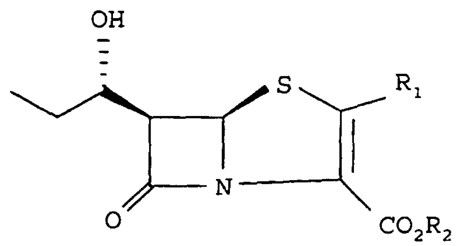

(I)

worin

$\mathbf{R_1}$ jeweils den Rest oder die Gruppe Alkyl, Alkenyl, $C_{7-14}$ - Aralkyl, $C_{6-10}$ - Aryl, Alkylthio (einschließlich monocyclisches oder polycyclisches Alkylthio, welches in Form eines kondensierten Ringes mit einem aromatischen Kohlenwasserstoff vorliegen kann, wobei dieser Rest eine oder mehrere Carbonylgruppen in seiner Kette oder seinem Ring enthalten kann), Alkenylthio, $C_{7-14}$ - Aralkylthio, $C_{6-10}$ - Arylthio, einen heterocyclischen Rest, eine heterocyclische Thiogruppe, Acylthio, oder eine Mercaptogruppe bzw. ein Wasserstoffatom bedeutet, wobei jede(r) beliebige der vorgenannten Gruppen oder Reste gegebenenfalls mit 1 oder mehreren Resten substituiert sein kann, welche ausgewählt sind aus Halogen; Carboxyl; Thiocarboxyl; Formyl; Nitro; Cyano; Hydroxyl; Amino; Imino; ($C_{1-6}$ - Alkylen) - acetal; $C_{1-6}$ - Alkyl; monocyclischem oder polycyclischem Alkyl (welches in Form eines kondensierten Ringes mit einem aromatischen Kohlenwasserstoff vorliegen kann, wobei dieser Rest eine oder mehrere Carbonylgruppen in seiner Kette oder seinem Ring enthalten kann); $C_{2-6}$ - Alkenyl; $C_{6-10}$ - Aryl; $C_{7-24}$ - Aralkyl; $C_{1-6}$ - Alkylthio; $C_{2-6}$ - Alkenylthio; $C_{7-24}$ - Aralkylthio; $C_{6-10}$ - Arylthio; $C_{1-6}$ - Alkyloxy; $C_{2-6}$ - Alkenyloxy; $C_{7-24}$ - Aralkyloxy; $C_{6-10}$ - Aryloxy; $C_{1-6}$ - Alkylsulfinyl; $C_{1-6}$ - Alkylsulfonyl; $C_{7-24}$ - Aralkylsulfinyl; $C_{7-24}$ - Aralkylsulfonyl; $C_{6-10}$ - Arylsulfinyl; $C_{6-10}$ - Arylsulfonyl; Aminosulfonyl; Carbamoyl; Carbamoyloxy; Carbamoyl - $C_{1-6}$ - alkyl; Imino - ($C_{1-6}$ - alkyl); Imino - ($C_{1-6}$ - alkyl) - amino; Imino - (amino)-- ($C_{1-6}$ - alkyl); Acyloxy; Acylalkyl; Silyloxy; einem heterocyclischen Rest; einer heterocyclischen Thiogruppe; einer heterocyclischen Oxygruppe; Acyl; einem veresterten Carboxyl; einem veresterten Thiocarboxyl, oder einzig unter der Bedingung, dass dann, wenn $R_1$ einen heterocyclischen Rest oder eine heterocyclische Thiogruppe bedeutet, die Auswahl ungesättigtes $C_{5-7}$ - Cycloalkyl oder kondensierte $C_{9-11}$ - Ringe darstellt, welche gegebenenfalls eine oder mehrere Carbonylgruppen in den Ringen enthalten, wobei der vorgenannte heterocyclische Rest oder Molekülteil ausgewählt ist aus 3-8--gliedrigen gesättigten oder ungesättigten heteromonocyclischen Resten mit 1 bis 4 Stickstoffatomen; 1 oder 2 Sauerstoffatomen und 1 bis 3 Stickstoffatomen; 1 oder 2 Schwefelatomen und 1 bis 3 Stickstoffatomen; 1 oder 2 Sauerstoffatomen; oder 1 Schwefelatom, sowie ausgewählt ist aus 7-12 - gliedrigen ungesättigten heteropolycyclischen Resten mit 1 bis 5 Stickstoffatomen; 1 oder 2 Sauerstoffatomen und 1 bis 3 Stickstoffatomen; oder 1 bzw. 2 Schwefelatomen und 1 bis 3 Stickstoffatomen, worin Alkyl oder Alkyl jeweils als Teil eines Alkylthio-, Aralkyl- oder Aralkylthiorestes monocyclisches oder polycyclisches Alkyl mit einschließt, das in Form eines kondensierten Ringes mit einem aromatischen Kohlenwasserstoff vorliegen und eine oder mehrere Carbonylgruppen in dessen Kette oder Ring enthalten kann, und

$R_2$ ein Wasserstoffatom oder eine Schutzgruppe für Carboxyl bedeutet, die in vivo zur Bildung der freien Säure entfernt werden kann;
oder ein pharmakologisch annehmbares Salz davon.

2. Verwendung eines Derivats von (5R,6R) - 6 - [(S) - 1 - hydroxypropyl] - penem, das der folgenden Formel (I) entspricht, bei der Herstellung eines Arzneimittels zur Behandlung einer Infektion mit Staphylococcus aureus, der gegenüber Methicillin resistent ist:

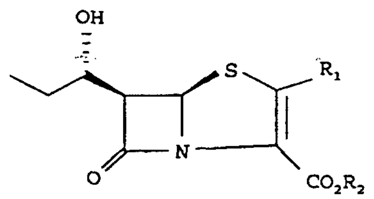

(I)

worin

$R_1$ jeweils den Rest oder die Gruppe Alkyl, Alkenyl, $C_{7-14}$ - Aralkyl, $C_{6-10}$ - Aryl, Alkylthio, Alkenylthio, $C_{7-14}$ - Aralkylthio, $C_{6-10}$ - Arylthio, einen heterocyclischen Rest, eine heterocyclische Thiogruppe, Acylthio, oder eine Mercaptogruppe bzw. ein Wasserstoffatom bedeutet, wobei jede(r) beliebige der vorgenannten Gruppen oder Reste gegebenenfalls mit 1 oder mehreren Resten substituiert sein kann, welche ausgewählt sind aus Halogen; Carboxyl; Thiocarboxyl; Formyl; Nitro; Cyano; Hydroxyl; Amino; Imino; ($C_{1-6}$ - Alkylen) - acetal; $C_{1-6}$ - Alkyl; monocyclischem oder polycyclischem Alkyl (welches in Form eines kondensierten Ringes mit einem aromatischen Kohlenwasserstoff vorliegen kann, wobei dieser Rest eine oder mehrere Carbonylgruppen in seiner Kette oder seinem Ring enthalten kann); $C_{2-6}$ - Alkenyl; $C_{6-10}$ - Aryl; $C_{7-24}$ - Aralkyl; $C_{1-6}$ - Alkylthio; $C_{2-6}$ - Alkenylthio; $C_{7-24}$ - Aralkylthio; $C_{6-10}$ - Arylthio; $C_{1-6}$ - Alkyloxy; $C_{2-6}$ - Alkenyloxy; $C_{7-24}$ - Aralkyloxy; $C_{6-10}$ - Aryloxy; $C_{1-6}$ - Alkylsulfinyl; $C_{1-6}$ - Alkylsulfonyl; $C_{7-24}$ - Aralkylsulfinyl; $C_{7-24}$ - Aralkylsulfonyl; $C_{6-10}$ - Arylsulfinyl; $C_{6-10}$ - Arylsulfonyl; Aminosulfonyl; Carbamoyl; Carbamoyloxy; Carbamoyl - $C_{1-6}$ - alkyl; Imino - ($C_{1-6}$ - alkyl); Imino - ($C_{1-6}$ - alkyl) - amino; Imino - (amino)-- ($C_{1-6}$ - alkyl); Acyloxy; Acylalkyl; Silyloxy; einem heterocyclischen Rest; einer heterocyclischen Thiogruppe; einer heterocyclischen Oxygruppe; Acyl; einem veresterten Carboxyl; einem veresterten Thiocarboxyl, oder einzig unter der Bedingung, dass dann, wenn $R_1$ einen heterocyclischen Rest oder eine heterocyclische Thiogruppe bedeutet, die Auswahl ungesättigtes $C_{5-7}$ - Cycloalkyl oder kondensierte $C_{9-11}$ - Ringe darstellt, welche gegebenenfalls eine oder mehrere Carbonylgruppen in den Ringen enthalten, wobei der vorgenannte heterocyclische Rest oder Molekülteil ausgewählt ist aus 3-8--gliedrigen gesättigten oder ungesättigten heteromonocyclischen Resten mit 1 bis 4 Stickstoffatomen; 1 oder 2 Sauerstoffatomen und 1 bis 3 Stickstoffatomen; 1 oder 2 Schwefelatomen und 1 bis 3 Stickstoffatomen; 1 oder 2 Sauerstoffatomen; oder 1 Schwefelatom, sowie ausgewählt ist aus 7-12 - gliedrigen ungesättigten heteropolycyclischen Resten mit 1 bis 5 Stickstoffatomen; 1 oder 2 Sauerstoffatomen und 1 bis 3 Stickstoffatomen; oder 1 bzw. 2 Schwefelatomen und 1 bis 3 Stickstoffatomen, worin Alkyl oder Alkyl jeweils als Teil eines Alkylthio-, Aralkyl- oder Aralkylthiorestes monocyclisches oder polycyclisches Alkyl mit einschließt, das in Form eines kondensierten Ringes mit einem aromatischen Kohlenwasserstoff vorliegen und eine oder mehrere Carbonylgruppen in dessen Kette oder Ring enthalten kann, und
$R_2$ ein Wasserstoffatom oder eine Schutzgruppe für Carboxyl bedeutet, die in vivo zur Bildung der freien Säure entfernt werden kann;
oder ein pharmakologisch annehmbares Salz davon.

3. Penemderivat oder eines seiner pharmakologisch annehmbaren Salze gemäß Anspruch 1, worin in der Formel (I) der Rest

$R_1$ eine heterocyclische Thiogruppe bedeutet, die gegebenenfalls mit einem oder mehreren Resten substituiert sein kann, welche ausgewählt sind aus Halogen; Carboxyl; Thiocarboxyl; Formyl; Nitro; Cyano; Hydroxyl; Amino; Imino; ($C_{1-6}$ - Alkylen) - acetal; $C_{1-6}$ - Alkyl; monocyclischem oder polycyclischem Alkyl (welches in Form eines kondensierten Ringes mit einem aromatischen Kohlenwasserstoff vorliegen kann, wobei dieser Rest eine oder mehrere Carbonylgruppen in seiner Kette oder seinem Ring enthalten kann); $C_{2-6}$ - Alkenyl; $C_{6-10}$ - Aryl; $C_{7-24}$ - Aralkyl; $C_{1-6}$ - Alkylthio; $C_{2-6}$ - Alk-enylthio; $C_{7-24}$ - Aralkylthio; $C_{6-10}$ - Arylthio; $C_{1-6}$ -

Alkyloxy; $C_{2-6}$ - Alkenyloxy; $C_{7-24}$ - Aralkyl-oxy; $C_{6-10}$ - Aryloxy; $C_{1-6}$ - Alkylsulfinyl; $C_{1-6}$ - Alkylsulfonyl; $C_{7-24}$ - Aralkylsulfinyl; $C_{7-24}$ -Alkyl-sulfonyl; $C_{6-10}$ - Arylsulfinyl; $C_{6-10}$ - Arylsulfonyl; Aminosulfonyl; Carbamoyl; Carbamoyloxy; Carb-amoyl - $C_{1-6}$ - alkyl; Imino - ($C_{1-6}$ - alkyl); Imino - ($C_{1-6}$ - alkyl) - amino; Imino - (amino)--($C_{1-6}$ - alkyl); Acyloxy; Acylalkyl; Silyloxy; einem heterocyclischen Rest; einer heterocyclischen Thiogruppe; einer heterocyclischen Oxygruppe; Acyl; einem veresterten Carboxyl; einem veresterten Thiocarboxyl, oder einzig unter der Bedingung, dass dann, wenn $R_1$ einen heterocyclischen Rest oder eine heterocyclische Thiogruppe bedeutet, die Auswahl ungesättigtes $C_{5-7}$ - Cycloalkyl oder kondensierte $C_{9-11}$ - Ringe darstellt, welche gegebenenfalls eine oder mehrere Carbonylgruppen in den Ringen enthalten, wobei der vorgenannte heterocyclische Rest oder Molekülteil ausgewählt ist aus 3-8 - gliedrigen gesättigten oder ungesättigten heteromonocyclischen Resten mit 1 bis 4 Stickstoffatomen; 1 oder 2 Sauerstoffatomen und 1 bis 3 Stickstoffatomen; 1 oder 2 Schwefelatomen und 1 bis 3 Stickstoffatomen; 1 oder 2 Sauerstoffatomen oder 1 Schwefelatom, sowie ausgewählt ist aus 7-12 - gliedrigen ungesättigten heteropolycyclischen Resten mit 1 bis 5 Stickstoffatomen bzw. mit 1 oder 2 Sauerstoffatomen und 1 bis 3 Stickstoffatomen; oder 1 bzw. 2 Schwefelatomen und 1 bis 3 Stickstoffatomen.

**4.** Penemderivat oder eines seiner pharmakologisch annehmbaren Salze gemäß Anspruch 3, worin der heterocyclische Molekülteil von $\mathbf{R_1}$ eine der folgenden Gruppen darstellt: Pyrrolyl, Pyrrolinyl, Imidazolyl, Pyrazolyl, Pyridyl, Pyrimidyl, Pyrazinyl, Pyridazinyl, Triazolyl, Tetrazolyl, Dihydrotriazinyl, Azetidinyl, Pyrrolidinyl, Imidazolidinyl, Piperidinyl, Pyrazolidinyl oder Piperazinyl.

**5.** Penemderivat oder eines seiner pharmakologisch annehmbaren Salze gemäß Anspruch 4, worin der vorgenannte heterocyclische Molekülteil eine Pyrrolidinylgruppe bedeutet.

**6.** Penemderivat oder eines seiner pharmakologisch annehmbaren Salze gemäß Anspruch 5, worin der vorgenannte heterocyclische Molekülteil eine (S) - Pyrrolidin-3-ylgruppe bedeutet.

**7.** Penemderivat oder eines seiner pharmakologisch annehmbaren Salze gemäß Anspruch 4, worin der vorgenannte heterocyclische Molekülteil eine Piperidinylgruppe bedeutet.

**8.** Penemderivat oder eines seiner pharmakologisch annehmbaren Salze gemäß Anspruch 7, worin der vorgenannte heterocyclische Molekülteil eine Piperidin-4-ylgruppe bedeutet.

**9.** Penemderivat oder eines seiner pharmakologisch annehmbaren Salze gemäß Anspruch 7, worin der vorgenannte heterocyclische Molekülteil eine Piperidin-3-ylgruppe bedeutet.

**10.** Penemderivat oder eines seiner pharmakologisch annehmbaren Salze gemäß Anspruch 3, worin der vorgenannte heterocyclische Molekülteil von $\mathbf{R_1}$ eine der folgenden Gruppen bedeutet: Indolyl, Isoindolyl, Indolizinyl, Benzimidazolyl, Chinolyl, Isochinolyl, Indazolyl, Benzotriazolyl, Tetrazolopyridyl, Tetrazolo-pyridazinyl, Dihydrotriazolopyridazinyl oder 6,7-Dihydro-5H-cyclopenta[b]pyridin-7-yl.

**11.** Penemderivat oder eines seiner pharmakologisch annehmbaren Salze gemäß Anspruch 3, worin der vorgenannte heterocyclische Molekülteil von $\mathbf{R_1}$ eine Oxazolyl-, Isooxazolyl-, Oxadiazolyl- oder Morpholinylgruppe bedeutet.

**12.** Penemderivat oder eines seiner pharmakologisch annehmbaren Salze gemäß Anspruch 3, worin der vorgenannte heterocyclische Molekülteil von $\mathbf{R_1}$ eine Benzoxazolyl- oder Benzoxadiazolylgruppe bedeutet.

**13.** Penemderivat oder eines seiner pharmakologisch annehmbaren Salze gemäß Anspruch 3, worin der vorgenannte heterocyclische Molekülteil von $\mathbf{R_1}$ eine 1,3-Thiazolyl-, 1,2-Thiazolyl-, Thiazolinyl-, Thiadiazolyl- oder Thiazolidinylgruppe bedeutet.

**14.** Penemderivat oder eines seiner pharmakologisch annehmbaren Salze gemäß Anspruch 3, worin der vorgenannte heterocyclische Molekülteil von $\mathbf{R_1}$ eine Benzothiazolyl- oder Benzothiadiazolylgruppe bedeutet.

**15.** Penemderivat oder eines seiner pharmakologisch annehmbaren Salze gemäß Anspruch 3, worin der vorgenannte heterocyclische Molekülteil von $\mathbf{R_1}$ eine Furanyl-, Pyranyl-, Tetrahydrofuranyl- oder Tetrahydropyranylgruppe bedeutet.

**16.** Penemderivat oder eines seiner pharmakologisch annehmbaren Salze gemäß Anspruch 3, worin der vorgenannte

heterocyclische Molekülteil von **$R_1$** eine Thienyl- oder Tetrahydrothienylgruppe bedeutet.

**17.** Penemderivat oder eines seiner pharmakologisch annehmbaren Salze gemäß Anspruch 1, worin **$R_1$** eine vorgenannte substituierte oder nichtsubstitutierte Alkylthiogruppe bedeutet.

**18.** Penemderivat oder eines seiner pharmakologisch annehmbaren Salze gemäß Anspruch 17, worin der Alkylrest der vorgenannten substituierten oder nichtsubstituierten Alkylthiogruppe einen geradoder verzweigtkettigen $C_{1-6}$ - Alkylrest oder einen monocyclischen oder polycyclischen Alkylrest dartellt, der in Form eines kondensierten Ringes mit einem aromatischen Kohlenwasserstoff vorliegen kann.

**19.** Penemderivat oder eines seiner pharmakologisch annehmbaren Salze gemäß Anspruch 17, worin der Alkylrest der vorgenannten substituierten oder nichtsubstituierten Alkylthiogruppe eine Methyl-, Ethyl-, n-Propyl-, Isopropyl-, n-Butyl-, tert.-Butyl- oder Hexylgruppe bedeutet.

**20.** Penemderivat oder eines seiner pharmakologisch annehmbaren Salze gemäß Anspruch 17, worin der Alkylrest der vorgenannten substituierten oder nichtsubstituierten Alkylthiogruppe einen monocyclischen oder polycyclischen Alkylrest darstellt, der aus einer Cyclopropyl-, Cyclopentyl-, Cyclohexyl, Menthyl-, Fenchyl-, Bornyl oder Indanylgruppe ausgewählt ist.

**21.** Penemderivat oder eines seiner pharmakologisch annehmbaren Salze gemäß Anspruch 1, worin **$R_1$** einen vorgenannten substituierten oder nichtsubstituierten Alkenylthiorest bedeutet.

**22.** Penemderivat oder eines seiner pharmakologisch annehmbaren Salze gemäß Anspruch 21, worin die Alkenylgruppe des vorgenannten substitutierten oder nichtsubstitutierten Alkenylthiorests einen gerad- oder verzweigtkettigen $C_{2-6}$ - Alkenylrest darstellt.

**23.** Penemderivat oder eines seiner pharmakologisch annehmbaren Salze gemäß Anspruch 21, worin der Alkenylrest des vorgenannten substitutierten oder nichtsubstitutierten Alkenylthiorests eine Vinyl-, Allyl-, 2-Chlorallyl-, 1-Propenyl-, 2-Butenyl- oder 2-Methyl-2-Propenylgruppe bedeutet.

**24.** Penemderivat oder eines seiner pharmakologisch annehmbaren Salze gemäß Anspruch 1, worin **$R_1$** einen vorgenannten substituierten oder nichtsubstituierten Aralkylthiorest bedeutet.

**25.** Penemderivat oder eines seiner pharmakologisch annehmbaren Salze gemäß Anspruch 24, worin die Aralkylgruppe des vorgenannten substitutierten oder nichtsubstitutierten Aralkylthiorests einen Aralkylrest mit 7 bis 24 Kohlenstoffatomen darstellt.

**26.** Penemderivat oder eines seiner pharmakologisch annehmbaren Salze gemäß Anspruch 24, worin der Aralkylrest des vorgenannten substitutierten oder nichtsubstitutierten Aralkylthiorests eine Benzyl-, Phenethyl-, 3-Phenylpropyl-, 2-Naphthylmethyl-, 2-(1-Naphthyl)-ethyl-, Trityl-, Benzhydryl- oder 1-Phenylcyclopropan-1-ylgruppe darstellt.

**27.** Penemderivat oder eines seiner pharmakologisch annehmbaren Salze gemäß Anspruch 1, worin **$R_1$** einen substituierten oder nichtsubstituierten Arylthiorest bedeutet.

**28.** Penemderivat oder eines seiner pharmakologisch annehmbaren Salze gemäß Anspruch 27, worin der Arylrest des vorgenannten substitutierten oder nichtsubstitutierten Arylthiorests einen Arylrest mit 6 bis 10 Kohlenstoffatomen darstellt.

**29.** Penemderivat oder eines seiner pharmakologisch annehmbaren Salze gemäß Anspruch 27, worin der Arylrest des vorgenannten substitutierten oder nichtsubstitutierten Arylthiorests eine Phenyl-, Tolyl-, Xylyl-, Mesityl-, Cumenyl- oder Naphthylgruppe darstellt.

**30.** Penemderivat oder eines seiner pharmakologisch annehmbaren Salze gemäß Anspruch 1, worin **$R_1$** einen substituierten oder nichtsubstituierten Arylrest bedeutet.

**31.** Penemderivat oder eines seiner pharmakologisch annehmbaren Salze gemäß Anspruch 1, worin **$R_1$** einen substituierten oder nichtsubstituierten heterocyclischen Rest bedeutet.

**32.** Penemderivat oder eines seiner pharmakologisch annehmbaren Salze gemäß Anspruch 1, worin $\mathbf{R_1}$ einen Rest bedeutet, welcher der folgenden Formel entspricht:

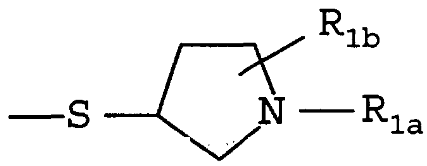

worin $\mathbf{R_{1a}}$ and $\mathbf{R_{1b}}$ gleich oder verschieden sind und ein Wasserstoffatom oder einen der folgenden Reste bzw. Gruppen bedeuten: Alkyl, Alkenyl, $C_{7-14}$ - Aralkyl, $C_{6-10}$ - Aryl, Imino - $C_{1-6}$ - alkyl, Imino - $C_{1-6}$ - alkylamino, Imino-(amino)-$C_{1-6}$-alkyl, Carbamoyl, Carbamoyl-$C_{1-6}$-alkyl, Acyl, Acyl--$C_{1-6}$ - alkyl, Carboxyl, heterocyclischer Rest oder heterocyclisches $C_{1-6}$ - Alkyl, wobei 1 oder mehrere Wasserstoffatome von vorgenanntem Alkyl, Alkenyl, Aralkyl, Aryl, Imino - $C_{1-6}$ - alkyl, Imino - $C_{1-6}$ - alkylamino, Imino-(amino)-$C_{1-6}$-alkyl, Carbamoyl, Carbamoyl-$C_{1-6}$-alkyl, einem heterocyclischen Rest oder einem heterocyclischen $C_{1-6}$ - Alkylrest gegebenenfalls durch ein Halogenatom oder Carboxyl, Thiocarboxyl, Formyl, Nitro, Cyano, Hydroxyl, Amino, Imino, ($C_{1-6}$ - Alkylen)-acetal, Alkyl, Alkoxyl, Alkenyl, $C_{7-24}$ - Aralkyl, $C_{6-10}$ - Aryl, $C_{6-10}$ - Aryloxy, Imino-$C_{1-6}$ - alkyl, Imino - $C_{1-6}$-- alkylamino, Imino-(amino)-$C_{1-6}$-alkyl, Carbamoyl, Carbamoyloxy, Carbamoyl - $C_{1-6}$ - alkyl, einen heterocyclischen Rest, einen heterocyclischen $C_{1-6}$ - Alkylrest, Acyl oder einen Acylalkylrest substituiert sind, und wobei die genannten Acylreste oder Molekülteile ein Alkylcarbonyl, Alkenylcarbonyl, Aralkylcarbonyl, Arylcarbonyl, heterocyclisches Carbonyl oder einen heterocyclischen ($C_{1-6}$ - Alkyl)-- carbonylrest darstellen, welche das vorgenannte substitutierte oder nichtsubstitutierte Alkyl, Alkenyl, Aralkyl, Aryl, einen heterocyclischen Rest oder heterocyclischen $C_{1-6}$ - Alkylrest enthalten, und wobei die genannte Carboxylgruppe durch substitutiertes oder nichtsubstitutiertes Alkyl, Alkenyl, Aralkyl, Aryl, einen heterocyclischen Rest oder einen heterocyclischen $C_{1-6}$ - Alkylrest verestert sein kann, und die genannten heterocyclischen Reste oder Molekülteile gegebenenfalls in ihrem Ring 1 oder mehrere Carbonylgruppen enthalten und das tertiäre Stickstoffatom darin gegebenenfalls ein intramolekulares quaternäres Salz durch die Einführung des genannten Substituenten bildet.

**33.** Penemderivat oder eines seiner pharmakologisch annehmbaren Salze gemäß Anspruch 1, worin $\mathbf{R_1}$ einen Rest bedeutet, welcher der folgenden Formel entspricht:

$$\mathbf{-S\text{-}(CH_2)_n\text{-}R_{1c}}$$

worin n die Zahl 1 bis 3 bedeutet und $\mathbf{R_{1c}}$ ein Wasserstoffatom oder den folgenden Rest bzw. die folgende Gruppe bedeutet: $C_{6-10}$ - Aryl, Amino, Imino-$C_{1-6}$ - alkylamino, Aminosulfonyl, Carbamoyl, Acyl, Carboxyl oder einen heterocyclischen Rest, wobei 1 oder mehrere Wasserstoffatome von genanntem Aryl, Amino, Imino-$C_{1-6}$ - alkylamino, Aminosulfonyl, Carbamoyl oder einem heterocyclischen Rest gegebenenfalls durch ein Halogenatom oder Carboxyl, Thiocarboxyl, Formyl, Nitro, Cyano, Hydroxyl, Amino, Imino, Alkyl, Alkoxy, Alkenyl, $C_{7-24}$ - Aralkyl, $C_{6-10}$ - Aryl, $C_{6-10}$ - Aryloxy, Imino-($C_{1-6}$ - alkyl), Imino-C $_{1-6}$ - alkylamino, Imino-(amino)-$C_{1-6}$ - alkyl, Carbamoyl, Carbamoyloxy, Carbamoyl-$C_{1-6}$ - alkyl, einen heterocyclischen Rest, heterocyclisches $C_{1-6}$ - Alkyl, Acyl oder einen Acylalkyl substituiert ist und der genannte Acylrest oder Molekülteil als ein Substituent angeführt ist, der ein Alkylcarbonyl, Alkenylcarbonyl, Aralkylcarbonyl, Arylcarbonyl, ein heterocyclisches Carbonyl oder einen heterocyclischen $C_{1-6}$ - Alkylcarbonylrest mit 1 oder mehreren Alkyl-, Alkenyl-, Aralkyl-, Aryl-, heterocyclischen Resten oder heterocyclischen $C_{1-6}$ - Alkylresten darstellt, und wobei 1 oder mehrere Wasserstoffatome dieser Acylreste oder -molekülteile gegebenenfalls durch ein Halogenatom oder Carboxyl, Thiocarboxyl, Formyl, Nitro, Cyano, Hydroxyl, Amino, Imino, $C_{1-6}$ - Alkylenacetal, Alkyl, Alkoxy, Alkenyl, $C_{7-24}$ - Aralkyl, $C_{6-10}$ - Aryl, $C_{6-10}$ - Aryloxy, Imino-$C_{1-6}$ - alkyl, Imino--$C_{1-6}$ - alkylamino, Imino-(amino)-$C_{1-6}$ - alkyl, Carbamoyl, Carbamoyloxy, Carbamoyl-$C_{1-6}$ - alkyl, einen heterocyclischen Rest, ein heterocyclisches $C_{1-6}$ - Alkyl, Acyl oder einen Acylalkylrest substituiert sind, und die genannte Carboxylgruppe gegebenenfalls durch substitutiertes oder nichtsubstituiertes Alkyl, Alkenyl, Aralkyl, Aryl, einen heterocyclischen Rest oder heterocyclischen $C_{1-6}$ - Alkylrest verestert ist, und wobei der vorgenannte heterocyclische Rest und die heterocyclische Gruppe der angeführten heterocyclischen $C_{1-6}$ - Alkylreste und der letztgenannte heterocyclische Rest als Substituent aufgeführt sind, der gegebenenfalls 1 oder mehrere Carbonylgruppen in seinem Ring aufweist und gegebenenfalls das tertiäre Stickstoffatom darin durch die Einführung des genannten Substituenten ein intramolekulares quaternäres Salz bildet.

**34.** Penemderivat gemäß Anspruch 3, das aus den folgenden Verbindungen ausgewählt ist:

(5R,6R)- 2-(1-Allylpyrrolidin-3-yl)-thio-6-[(S)-1-hydroxypropyl]-penem-3-carbonsäure;
(5R,6R)-2-(1-Benzylpyrrolidin-3-yl)-thio-6-[(S)-1-hydroxypropyl]-penem-6-carbonsäure;
(5R,6R)-2-(Pyrrolidin-3-yl)-thio-6-[(S)-1-hydroxypropyl]-penem-3-carbonsäure;
(5R,6R) - 2 -[1 - (1 - Iminoethyl) - pyrrolidin - 3 - yl] - thio - 6 - [(S) - 1 - hydroxypropyl]-- penem - 3 - carbonsäure;
(5R,6R)-6-[(S)-1-Hydroxypropyl]-2-(1-phenylpyrrolidin-3-yl)-thiopenem-3-carbonsäure;
(5R,6R)-6-[(S)-1-Hydroxypropyl]-2-(1-phenethylpyrrolidin-3-yl)-thiopenem-3-carbonsäure;
(5R,6R) - 6 - [(S)-1-Hydroxypropyl]- 2 - [1 - (2 - hydroxy - 2 - phenylethyl) - pyrrolidin--3 - yl] - thiopenem - 3 - carbonsäure;
(5R,6R) - 6 - [(S) - 1 - Hydroxypropyl] - 2 - [1 - (2 - hydroxy - 2 - phenylethyl) - pyrrolidin-- 3 - yl] - thiopenem - 3 - carbonsäure;
(5R,6R)-6-[(S)-1-Hydroxypropyl]-2-(1-phenacylpyrrolidin-3-yl)-thiopenem-3-carbonsäure;
(5R,6R) - 2 - [1 - (2 - p-Fluorphenyl - 2 - oxoethyl) - pyrrolidin - 3 - yl] - thio - 6 - [(S)-- 1 - hydroxypropyl] - penem - 3 - carbonsäure;
(5R,6R) - 6 - [(S) - 1 - Hydroxypropyl] - 2 - [1 - (2 - oxo - 2 - p-tolylethyl) - pyrrolidin-- 3 - yl] - thiopenem - 3 - carbonsäure;
(5R,6R) - 6 - [(S) - 1 - Hydroxypropyl] - 2 - [1 - (2 - p-methoxyphenyl - 2 - oxoethyl)-- pyrrolidin - 3 - yl] - thiopenem - 3 - carbonsäure;
(5R,6R) - 2 - [1 - (2 - Benzoylethyl) - pyrrolidin - 3 - yl] - thio - 6 - [(S) - 1 - hydroxypropyl]-- penem - 3 - carbonsäure;
(5R,6R)-2-[1-(1-Benzoylethyl)-pyrrolidin-3-yl]-thio-6-[(S)-hydroxypropyl]-penem-3-carbonsäure;
(5R,6R) - 6 - [(S) - 1 - Hydroxypropyl] - 2 - [1 - (1-indanon-2-yl) - pyrrolidin - 3 - yl] - thiopenem - 3 - carbonsäure;
(5R,6R) - 6 - [(S) - 1 - Hydroxypropyl] - 2 - [1 - (1 - indanon - 2 - yl) - pyrrolidin - 3 - yl]-- thiopenem - 3 - carbonsäure;
(5R,6R) - 6 - [(S) - 1 - Hydroxypropyl] - 2 - [1 - (2 - pyridylmethyl) - pyrrolidin - 3 - yl]-- thiopenem - 3 - carbonsäure;
(5R,6R)-6-[(S)-1-Hydroxypropyl]-2-(1-benzylpiperidin-3-yl)-thiopenem-3-carbonsäure;
(5R,6R)-6-[(S)-1-Hydroxypropyl]-2-(4-phenylthiazol-2-yl)-thiopenem-3-carbonsäure;
(5R,6R)-6-[(S)-1-Hydroxypropyl]-2-[1-(4-pyridyl)-pyrrolidin-3-yl]-thiopenem-3-carbonsäure;
(5R,6R)-6-[(S)-1-Hydroxypropyl]-2-[1-(2-pyridyl)-pyrrolidin-3-yl)]-thiopenem-3-carbonsäure;
(5R,6R)-6-[(S)-1-Hydroxypropyl]-2-(1-iminomethyl)-azetidin-3-yl)-thiopenem-3-carbonsäure;
(5R,6R)-6-[(S)-1-Hydroxypropyl]-2-(1-iminoethyl)-azetidin-3-yl)-thiopenem-3-carbonsäure;
(5R,6R)-6-[(S)-1-Hydroxypropyl]-2-(2,3-cyclopento[b]pyridin-7-yl)-thiopenem-3-carbonsäure;
(5R,6R) - 2 - [1 - (1 - Iminopropyl) - pyrrolidin - 3 - yl] - thio - 6 - [(S) - 1- hydroxypropyl]-- penem - 3 - carbonsäure;
(5R,6R) - 6 - [(S) - 1 - Hydroxypropyl] - 2 - [1 - (3 - benzodioxol - 5 - yl - acetonyl) - pyrrolidin - 3 - yl] - thiopenem - 3 - carbonsäure;
(5R,6R)-2-{1-[4-(1-Iminoethylamino)-ethyl)-phenyl]-pyrrolidin-3-yl}-thio-6-[(S)-1-hydroxypropyl)-penem-3-carbonsäure;
(5R,6R) - 6 - [(S) - 1 - Hydroxypropyl] - 2 - (1 - pyrimidin - 2 - yl - pyrrolidin - 3 - yl) - thiopenem - 3 - carbonsäure;
(5R,6R) - 6 - [(S) - 1 - Hydroxypropyl] - 2 - [1 - ($\alpha$ - imino - benzamidomethyl) - pyrrolidin-- 3 - yl] - thiopenem - 3 - carbonsäure;
(5R,6R) - 6 - [(S) - 1 - Hydroxypropyl) - 2 - [1 - ($\alpha$-imino) - anilinomethylpyrrolidin - 3 - yl]-- thiopenem - 3 - carbonsäure ;
(5R,6R) - 6 - [(S) - 1 - Hydroxypropyl] - 2 - (1 - cyclopropylmethylpyrrolidin - 3 - yl) - thiopenem - 3 - carbonsäure;
(5R,6R) - 6 - [(S)-1-Hydroxypropyl] - 2 - (1 - phenoxyethylpyrrolidin - 3 -yl) - thiopenem - 3 -- carbonsäure;
(5R,6R)-6-[(S)-1-Hydroxypropyl]-2-(1-phenoxypropylpyrrolidin-3-yl)-thiopenem-3-carbonsäure;
(5R,6R) - 6 - [(S) - 1 - Hydroxypropyl] - 2 - (1 - o-tolyloxymethylpyrrolidin - 3 - yl) - thiopenem-3-carbonsäure;
(5R,6R) - 6 - [(S) - 1 - Hydroxypropyl] - 2 - (1 - indan - 2 - ylpyrrolidin-3-yl) - thiopenem-- 3 - carbonsäure;
(5R,6R)-6-[(S)-1-Hydroxypropyl]-2-(1-cyclopentylpyrrolidin-3-yl)-thiopenem-3-carbonsäure;
(5R,6R)-6-[(S)-1-Hydroxypropyl]-2-[1-(3-phenylallyl)-pyrrolidin-3-yl]-thiopenem-3-carbonsäure;
(5R,6R) - 2 - [1 - (1 - Imino - 2 - fluorethyl) - pyrrolidin-3-yl] - thio - 6 - [(S - 1 - hydroxypropyl] - penem - 3 - carbonsäure;
(5R,6R)-2-(1-Cyclopropylpyrrolidin-3-yl)-thio-6-[(S)-1-hydroxypropyl]-penem-3-carbonsäure und
(5R,6R)-6-[(S)-1-Hydroxypropyl]-2-(1-thiazol-2-ylpyrrolidin-3-yl)-thiopenem-3-carbonsäure oder

ein pharmakologisch annehmbares Salz davon.

**35.** Penemderivat gemäß Anspruch 1, welches ausgewählt ist aus:

(5R,6R) - 6 - [(S) - 1 - Hydroxypropyl] - 2 - methylthiopenem - 3 - carbonsäure;
(5R,6R) - 6 - [(S) - 1 - Hydroxypropyl] - 2 - (2 - pyridylmethyl) - thiopenem - 3 - carbonsäure;
(5R,6R)-6-[(S)-1-Hydroxypropyl]-2-(benzimidazol-2-ylmethyl)-thiopenem-3-carbonsäure;
(5R,6R)-6-[(S)-1-Hydroxypropyl]-2-[(S)-1-(4-pyridylmethyl)]-thiopenem-3-carbonsäure;
(5R,6R)-2-[2-(1-Iminoethylamino)-ethyl]-thio-6-[(S)-1-hydroxypropyl]-penem-3-carbonsäure;
(5R,6R)-2-[2-(a-Iminobenzyl)-aminoethyl]-thio-6-[(S)-1-hydroxypropyl]-penem-3-carbonsäure;
(5R,6R)-6-[(S)-1-Hydroxypropyl]-2-(2-phenylaminocarbonylethyl)-thiopenem-3-carbonsäure;
(5R,6R) - 6 - [(S) - 1 - Hydroxypropyl] - 2 - [2 - (N-methyl-N-phenacylamino) - ethyl] - thiopenem - 3 - carbonsäure;
(5R,6R)-6-[(S)-1-Hydroxypropyl]-2-[2-(pyrrolidin-1-yl)-ethyl]-thiopenem-3-carbonsäure;
(5R,6R)-6-[(S)-1-Hydroxypropyl]-2-[2-(1-pyrrolyl)-ethyl]-thiopenem-3-carbonsäure;
(5R,6R)-2-(2-Aminoethyl)-thio-6-[(S)-1-hydroxypropyl]-penem-3-carbonsäure;
(5R,6R) - 6 - [(S) - 1 - Hydroxypropyl] - 2 - phenylthiopenem - 3 - carbonsäure;
(5R,6R)-6-[(S)-1-Hydroxypropyl]-2-(3,4-dichlorbenzyl)-thiopenem-3-carbonsäure;
(5R,6R)-6-[(S)-1-Hydroxypropyl]-2-(3-cyanobenzyl)-thiopenem-3-carbonsäure und
(5R,6R)-6-[(S)-1-Hydroxypropyl)-2-(2-Indanyl)-thiopenem-3-carbonsäure

oder ein pharmakologisch annehmbares Salz davon.

**36.** (5R,6R) - 6 - [(S) - 1 - Hydroxypropyl] - penemderivat, welches der folgenden Formel (II) entspricht:

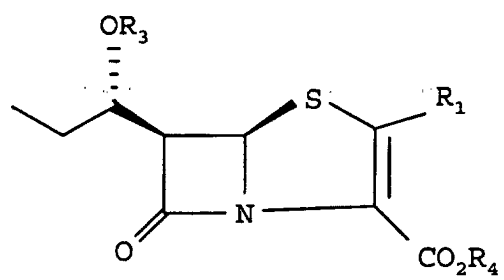

(II)

worin
**R$_1$** die im Anspruch 1 angegebenen Bedeutungen aufweist, wobei **OR$_3$** eine geschützte Hydroxylgruppe und **R$_4$** eine Schutzgruppe für Carboxyl bedeuten.

**37.** Penemderivat gemäß dem Anspruch 36, worin **R$_1$** die in einem der Ansprüche 3 bis 33 angegebenen Bedeutungen aufweist.

**38.** (5R,6R) - 6 - [(S) - 1 - Hydroxypropyl] - penemderivat, das der folgenden Formel (III) entspricht:

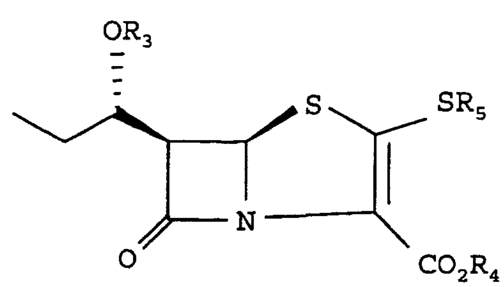

(III)

worin

**R$_5$** Alkyl, Alkenyl, $C_{7-14}$ - Aralkyl, $C_{6-10}$ - Aryl, einen heterocyclischen Rest oder eine Acylgruppe bedeutet,

wobei ein beliebiger der vorgenannten Reste gegebenenfalls substituiert sein kann mit 1 oder mehreren Resten, die ausgewählt sind aus Halogen; Carboxyl; Thiocarboxyl; Formyl; Nitro; Cyano; Hydroxyl; Amino; Imino; ($C_{1-6}$ - Alkylen)-acetal; $C_{1-6}$ - Alkyl; monocyclischem oder polycyclischem Alkyl (das in Form eines kondensierten Ringes mit einem aromatischen Kohlenwasserstoff vorliegen und 1 oder mehrere Carbonylgruppen in seiner Kette oder seinem Ring enthalten kann); $C_{2-6}$ - Alkenyl; $C_{6-10}$ - Aryl; $C_{7-24}$ - Aralkyl; $C_{1-6}$ - Alkylthio; $C_{2-6}$ - Alkenylthio; $C_{7-24}$ - Aralkylthio; $C_{6-10}$ - Arylthio; $C_{1-6}$ - Alkyloxy; $C_{6-10}$- Alkenyloxy; $C_{7-24}$ - Aralkyloxy; $C_{6-10}$ - Aryloxy; $C_{1-6}$ -Alkylsulfinyl; $C_{1-6}$ - Alkylsulfonyl; $C_{7-24}$ - Aralkylsulfinyl; $C_{7-24}$ - Aralkylsulfonyl; $C_{6-10}$ - Arylsulfinyl; $C_{6-10}$ - Arylsulfonyl; Aminosulfonyl; Carbamoyl; Carbamoyloxy; Carbamoyl-$C_{1-6}$ -alkyl; Imino-($C_{1-6}$ - alkyl); Imino - ($C_{1-6}$ - alkylamino; Imino - (amino) - ($C_{1-6}$ - alkyl); Acyloxy; Acylalkyl; Silyloxy; einem heterocyclischen Rest; einer heterocyclischen Thiogruppe; einer heterocyclischen Oxygruppe; Acyl; einem veresterten Carboxyl oder veresterten Thiocarboxyl, oder dann einzig unter der Bedingung, dass **$R_1$** einen heterocyclischen Rest bedeutet, die Auswahl ungesättigtes $C_{5-7}$ - Cycloalkyl oder $C_{9-11}$-kondensierte Ringe darstellt, die gegebenenfalls 1 oder mehrere Carbonylgruppen in ihren Ringen enthalten, wobei der genannte heterocyclische Rest ausgewählt ist aus 3-8 - gliedrigen gesättigten oder ungesättigten, heteromonocyclischen Resten mit 1 bis 4 Stickstoffatomen; 1 oder 2 Sauerstoffatomen und 1 bis 3 Stickstoffatomen; 1 oder 2 Schwefelatomen und 1 bis 3 Stickstoffatomen; 1 oder 2 Sauerstoffatomen; oder 1 Schwefelatom; sowie aus 7-12 - gliedrigen, ungesättigten, heteropolycyclischen Resten mit 1 bis 5 Stickstoffatomen; 1 oder 2 Sauerstoffatomen und 1 bis 3 Stickstoffatomen; oder 1 oder 2 Schwefelatomen und 1 bis 3 Stickstoffatomen, worin Alkyl oder Alkyl als Teil eines Alkylthio-, Aralkyl- oder Aralkylthiorestes ein monocyclisches oder polycyclisches Alkyl mit einschließt, das in Form eines kondensierten Ringes mit einem aromatischen Kohlenwasserstoff vorliegen und 1 oder mehrere Carbonylgruppen in seiner Kette oder Ring enthalten kann, wobei **$OR_3$** eine geschützte Hydroxylgruppe darstellt, und wobei
**$R_4$** eine Schutzgruppe für Carboxyl bedeutet.

**39.** (5R,6R) - 6 - [(S) - 1 - Hydroxypropyl) - penemderivat, das der folgenden Formel (IV)

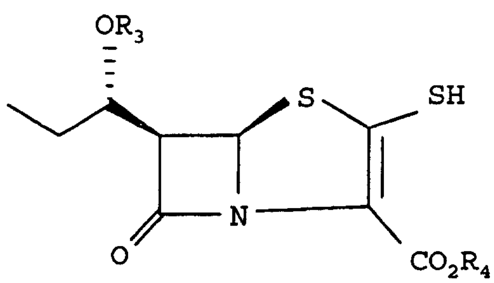

(IV)

entspricht:
worin
**$OR_3$** eine geschützte Hydroxylgruppe und **$R_4$** eine Schutzgruppe für Carboxyl darstellen.

**40.** Arzneimittel, welches als Wirkstoff ein (5R,6R) - 6 - [(S) - 1 - Hydroxypropyl] - penemderivat enthält, das der Formel (I) gemäß dem Anspruch 1 entspricht, oder welches eines seiner pharmakologisch annehmbaren Salze umfasst.

**41.** Arzneimittel gemäß Anspruch 40, worin das Penemderivat die in einem der Ansprüche 3 bis 39 angegebenen Bedeutungen aufweist.

**42.** Verwendung eines (5R,6R) - 6 - [(S) - 1 - Hydroxypropyl) - penemderivats, das der gemäß Anspruch 1 definierten Formel (I) entspricht, oder eines pharmakologisch annehmbaren Salzes bei der Herstellung eines antibakteriellen Arzneimittels.

**43.** Verwendung gemäß Anspruch 42, bei der das Penemderivat die in einem der Ansprüche 3 bis 39 angegebenen Bedeutungen aufweist.

**44.** Verwendung gemäß Anspruch 2, bei welcher das Penemderivat oder eines seiner pharmakologisch annehmbaren Salze die in einem der Ansprüche 3 bis 39 angegebenen Bedeutungen aufweist.

**45.** Penemderivat oder eines seiner pharmakologisch annehmbaren Salze gemäß Anspruch 1 zur Verwendung bei der therapeutischen Behandlung.

**46.** Penemderivat oder eines seiner pharmakologisch annehmbaren Salze gemäß Anspruch 45, wobei das Penemderivat die in einem der Ansprüche 2 bis 39 angegebenen Bedeutungen aufweist.

**Revendications**

**1.** Dérivé de (5R,6R)-6-((S)-1-hydroxypropyl)-pénème représenté par la formule suivante (I) :

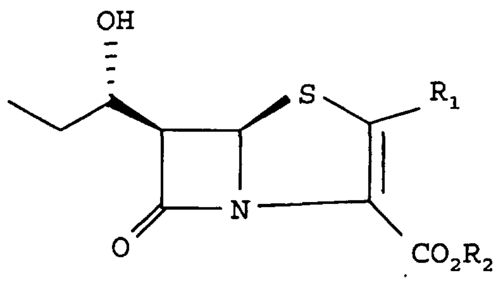

(I)

dans laquelle :

$R_1$ représente un radical alkylalcényle, aralkyle en $C_7$ à $C_{14}$, aryle en $C_6$ à $C_{10}$, alkylthio, alcénylthio, (aralkyle en $C_7$ à $C_{14}$)-thio, (aryle en $C_6$ à $C_{10}$)-thio, hétérocyclique, thio-hétérocyclique, acylthio ou mercapto ou un atome d'hydrogène, l'un quelconque desdits radicaux étant éventuellement substitué par un ou plusieurs éléments choisis parmi les radicaux halogéno ; carboxyle ; thiocarboxyle ; formyle ; nitro ; cyano ; hydroxyle ; amino ; imino ; (alkylène en $C_1$ à $C_6$)acétal ; alkyle en $C_1$ à $C_6$ ; alkyle monocyclique ou polycyclique (qui peut être sous forme d'un cycle condensé avec un hydrocarbure aromatique et peut contenir un ou plusieurs radicaux carbonyle dans sa chaîne ou dans son cycle) ; alcényle en $C_2$ à $C_6$ ; aryle en $C_6$ à $C_{10}$ ; aralkyle en $C_7$ à $C_{24}$ ; (alkyle en $C_1$ à $C_6$)-thio ; (alcényle en $C_2$ à $C_6$)-thio ; (aralkyle en $C_7$ à $C_{24}$)-thio ; (aryle en $C_6$ à $C_{10}$)-thio ; alkyloxy en $C_1$ à $C_6$ ; alcényloxy en $C_2$ à $C_6$ ; aralkyloxy en $C_7$ à $C_{24}$ ; aryloxy en $C_6$ à $C_{10}$ ; (alkyle en $C_1$ à $C_6$)-sulfinyle ; (alkyle en $C_1$ à $C_6$)- sulfonyle ; (aralkyle en $C_7$ à $C_{24}$)-sulfinyle ; (aralkyle en $C_7$ à $C_{24}$)-sulfonyle ; (aryle en $C_6$ à $C_{10}$)-sulfinyle ; (aryle en $C_6$ à $C_{10}$)-sulfonyle ; aminosulfonyle ; carbamoyle ; carbamoyloxy ; carbamoyl-(alkyle en $C_1$ à $C_6$) ; imino-(alkyle en $C_1$ à $C_6$) ; imino-(alkyle en $C_1$ à $C_6$)-amino ; imino-amino- (alkyle en $C_1$ à $C_6$) ; acyloxy ; acylalkyle ; silyloxy ; hétérocyclique ; thio-hétérocyclique ; oxyhétérocyclique ; acyle ; carboxyle estérifié ; thiocarboxyle estérifié, ou, seulement quand $R_1$ sont des cycles condensés en $C_9$ à $C_{11}$, cyclo-(alkyle en $C_5$ à $C_7$) insaturé, hétérocyclique ou thio-hétérocyclique, contenant éventuellement un ou plusieurs radicaux carbonyle dans leurs cycles ; et ledit radical ou fraction hétérocyclique étant choisi parmi des radicaux hétéromonocycliques, saturés ou insaturés, ayant 3 à 8 éléments contenant 1 à 4 atomes d'azote ; 1 ou 2 atomes d'oxygène et 1 à 3 atomes d'azote ; 1 ou 2 atomes de soufre et 1 à 3 atomes d'azote ; 1 ou 2 atomes d'oxygène ; ou 1 atome de soufre ; et des radicaux hétéropolycycliques, insaturés, ayant 7 à 12 éléments contenant 1 à 5 atomes d'azote ; 1 ou 2 atomes d'oxygène et 1 à 3 atomes d'azote ; ou 1 ou 2 atomes de soufre et 1 à 3 atomes d'azote ; dans laquelle le radical alkyle ou alkyle en tant que partie d'un radical alkylthio, aralkyle ou aralkylthio inclut un radical alkyle monocyclique ou polycyclique qui peut être sous forme d'un cycle condensé avec un hydrocarbure aromatique et peut contenir un ou plusieurs radicaux carbonyle dans sa chaîne ou dans son cycle, et

$R_2$ représente un atome d'hydrogène ou un radical de protection d'un groupe carboxyle qui peut être éliminé *in vivo* pour former l'acide libre ;

ou un de ses sels acceptables sur le plan pharmacologique.

**2.** Utilisation dans la fabrication d'un médicament pour le traitement de l'infection par *Staphylococcus aureus* résistant à la méthicilline d'un dérivé de (5R,6R)-6-((S)-1-hydroxypropyl)-pénème représenté par la formule suivante (I) :

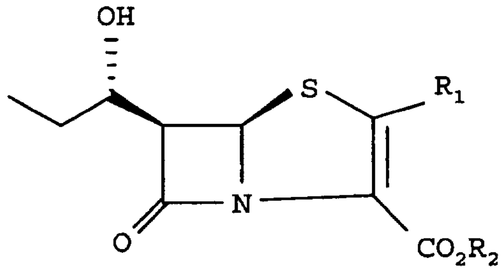

(I)

dans laquelle :

$R_1$ représente un radical alkyle, alcényle, aralkyle en $C_7$ à $C_{14}$, aryle en $C_6$ à $C_{10}$, alkylthio, alcénylthio, (aralkyle en $C_7$ à $C_{14}$)-thio, (aryle en $C_6$ à $C_{10}$)-thio, hétérocyclique, thio-hétérocyclique, acylthio ou mercapto ou un atome d'hydrogène, l'un quelconque desdits radicaux étant éventuellement substitué par un ou plusieurs éléments choisis parmi les radicaux halogéno ; carboxyle ; thiocarboxyle ; formyle ; nitro ; cyano ; hydroxyle ; amino ; imino ; (alkylène en $C_1$ à $C_6$)acétal ; alkyle en $C_1$ à $C_6$ ; alkyle monocyclique ou polycyclique (qui peut être sous forme d'un cycle condensé avec un hydrocarbure aromatique et peut contenir un ou plusieurs radicaux carbonyle dans sa chaîne ou dans son cycle) ; alcényle en $C_2$ à $C_6$ ; aryle en $C_6$ à $C_{10}$ ; aralkyle en $C_7$ à $C_{24}$ ; (alkyle en $C_1$ à $C_6$)-thio ; (alcényle en $C_2$ à $C_6$)-thio ; (aralkyle en $C_7$ à $C_{24}$)-thio ; (aryle en $C_6$ à $C_{10}$)-thio ; alkyloxy en $C_1$ à $C_6$ ; alcényloxy en $C_2$ à $C_6$ ; aralkyloxy en $C_7$ à $C_{24}$ ; aryloxy en $C_6$ à $C_{10}$ ; (alkyle en $C_1$ à $C_6$)-sulfinyle ; (alkyle en $C_1$ à $C_6$)- sulfonyle ; (aralkyle en $C_7$ à $C_{24}$)-sulfinyle ; (aralkyle en $C_7$ à $C_{24}$)-sulfonyle ; (aryle en $C_6$ à $C_{10}$)-sulfinyle ; (aryle en $C_6$ à $C_{10}$)-sulfonyle ; aminosulfonyle ; carbamoyle ; carbamoyloxy ; carbamoyl-(alkyle en $C_1$ à $C_6$) ; imino-(alkyle en $C_1$ à $C_6$) ; imino-(alkyle en $C_1$ à $C_6$)amino ; imino-amino-(alkyle en $C_1$ à $C_6$) ; acyloxy ; acylalkyle ; silyloxy ; hétérocyclique ; thio-hétérocyclique ; oxyhétérocyclique ; acyle ; carboxyle estérifié ; thiocarboxyle estérifié, ou, seulement quand $R_1$ sont des cycles condensés en $C_9$ à $C_{11}$, cyclo-(alkyle en C5 à $C_7$) insaturé, hétérocyclique ou thio-hétérocyclique contenant éventuellement un ou plusieurs radicaux carbonyle dans leurs cycles ; et ledit radical ou fraction hétérocyclique étant choisi parmi des radicaux hétéromonocycliques, saturés ou insaturés, ayant 3 à 8 éléments contenant 1 à 4 atomes d'azote ; 1 ou 2 atomes d'oxygène et 1 à 3 atomes d'azote ; 1 ou 2 atomes de soufre et 1 à 3 atomes d'azote ; 1 ou 2 atomes d'oxygène ; ou 1 atome de soufre ; et des radicaux hétéropolycycliques, insaturés, ayant 7 à 12 éléments contenant 1 à 5 atomes d'azote ; 1 ou 2 atomes d'oxygène et 1 à 3 atomes d'azote ; ou 1 ou 2 atomes de soufre et 1 à 3 atomes d'azote ; dans laquelle le radical alkyle ou alkyle en tant que partie d'un radical alkylthio, aralkyle ou aralkylthio inclut un radical alkyle monocyclique ou polycyclique qui peut être sous forme d'un cycle condensé avec un hydrocarbure aromatique et peut contenir un ou plusieurs radicaux carbonyle dans sa chaîne ou dans son cycle, et

$R_2$ représente un atome d'hydrogène ou un radical de protection d'un groupe carboxyle qui peut être éliminé in vivo pour former l'acide libre ;

ou un de ses sels acceptables sur le plan pharmacologique.

3. Dérivé de pénème ou un de ses sels acceptables sur le plan pharmacologique selon la revendication 1, dans lequel dans la formule (I), $R_1$ représente un radical thio-hétérocyclique éventuellement substitué par un ou plusieurs éléments choisis parmi les radicaux halogéno ; carboxyle ; thiocarboxyle ; formyle ; nitro ; cyano ; hydroxyle ; amino ; imino ; (alkylène en $C_1$ à $C_6$)acétal ; alkyle en $C_1$ à $C_6$ ; alkyle monocyclique ou polycyclique (qui peut être sous forme d'un cycle condensé avec un hydrocarbure aromatique et peut contenir un ou plusieurs radicaux carbonyle dans sa chaîne ou dans son cycle) ; alcényle en $C_2$ à $C_6$ ; aryle en $C_6$ à $C_{10}$ ; aralkyle en $C_7$ à $C_{24}$ ; (alkyle en $C_1$ à $C_6$)-thio ; (alcényle en $C_2$ à $C_6$)-thio ; aralkyle en $C_7$ à $C_{24}$)-thio ; (aryle en $C_6$ à $C_{10}$)-thio ; alkyloxy en $C_1$ à $C_6$ ; alcényloxy en $C_2$ à $C_6$ ; aralkyloxy en $C_7$ à $C_{24}$; aryloxy en $C_6$ à $C_{10}$ ; (alkyle en $C_1$ à $C_6$)-sulfinyle ; (alkyle en $C_1$ à $C_6$)-sulfonyle ; (aralkyle en $C_7$ à $C_{24}$)-sulfinyle ; (aralkyle en $C_7$ à $C_{24}$)-sulfonyle ; (aryle en $C_6$ à $C_{10}$)-sulfinyle ; (aryle en $C_6$ à $C_{10}$)-sulfonyle ; aminosulfonyle ; carbamoyle ; carbamoyloxy ; carbamoyl-(alkyle en $C_1$ à $C_6$) ; imino-(alkyle en $C_1$ à $C_6$) ; imino-(alkyle en $C_1$ à $C_6$)amino ; imino-amino-(alkyle en $C_1$ à $C_6$) ; acyloxy ; acylalkyle ; silyloxy ; hétérocyclique ; thio-hétérocyclique ; oxy-hétérocyclique ; acyle ; carboxyle estérifié ; thiocarboxyle estérifié, ou cyclo-(alkyle en $C_5$ à $C_7$) insaturé ou des cycles condensés en $C_9$ à $C_{11}$ contenant éventuellement un ou plusieurs groupes carbonyle dans leurs cycles ; et ladite fraction hétérocyclique étant choisie parmi des radicaux hétéromonocycliques, saturés ou insaturés, ayant 3 à 8 éléments contenant 1 à 4 atomes d'azote ;

1 ou 2 atomes d'oxygène et 1 à 3 atomes d'azote ; 1 ou 2 atomes de soufre et 1 à 3 atomes d'azote ; 1 ou 2 atomes d'oxygène ; ou 1 atome de soufre ; et des radicaux hétéropolycycliques, insaturés, ayant 7 à 12 éléments contenant 1 à 5 atomes d'azote ; 1 ou 2 atomes d'oxygène et 1 à 3 atomes d'azote ; ou 1 ou 2 atomes de soufre et 1 à 3 atomes d'azote.

**4.** Dérivé de pénème ou un de ses sels acceptables sur le plan pharmacologique selon la revendication 3, dans lequel la fraction hétérocyclique de $R_1$ est un radical pyrrolyle, pyrrolinyle, imidazolyle, pyrazolyle, pyridyle, pyrimidyle, pyrazinyle, pyridazinyle, triazolyle, tétrazolyle, dihydrotriazinyle, azétidinyle, pyrrolidinyle, imidazolidinyle, pipéridinyle, pyrazolidinyle ou pipérazinyle.

**5.** Dérivé de pénème ou un de ses sels acceptables sur le plan pharmacologique selon la revendication 4, dans lequel ladite fraction hétérocyclique est un radical pyrrolidinyle.

**6.** Dérivé de pénème ou un de ses sels acceptables sur le plan pharmacologique selon la revendication 5, dans lequel ladite fraction hétérocyclique est un radical (S)-pyrrolidin-3-yle.

**7.** Dérivé de pénème ou un de ses sels acceptables sur le plan pharmacologique selon la revendication 4, dans lequel ladite fraction hétérocyclique est un radical pipéridinyle.

**8.** Dérivé de pénème ou un de ses sels acceptables sur le plan pharmacologique selon la revendication 7, dans lequel ladite fraction hétérocyclique est un radical pipéridin-4-yle.

**9.** Dérivé de pénème ou un de ses sels acceptables sur le plan pharmacologique selon la revendication 7, dans lequel ladite fraction hétérocyclique est un radical pipéridin-3-yle.

**10.** Dérivé de pénème ou un de ses sels acceptables sur le plan pharmacologique selon la revendication 3, dans lequel ladite fraction hétérocyclique de $R_1$ est un radical indolyle, isoindolyle, indolizinyle, benzimidazolyle, quinolyle, isoquinolyle, indazolyle, benzotriazolyle, tétrazolopyridyle, tétrazolopiridazinyle, dihydrotriazolopyridazinyle ou 6,7-dihydro-5H-cyclopenta[b]pyridin-7-yle.

**11.** Dérivé de pénème ou un de ses sels acceptables sur le plan pharmacologique selon la revendication 3, dans lequel ladite fraction hétérocyclique de $R_1$ est un radical oxazolyle, isooxazolyle, oxadiazolyle ou morpholinyle.

**12.** Dérivé de pénème ou un de ses sels acceptables sur le plan pharmacologique selon la revendication 3, dans lequel ladite fraction hétérocyclique de $R_1$ est un radical benzoxazolyle ou benzoxadiazolyle.

**13.** Dérivé de pénème ou un de ses sels acceptables sur le plan pharmacologique selon la revendication 3, dans lequel ladite fraction hétérocyclique de $R_1$ est un radical 1,3-thiazolyle, 1,2-thiazolyle, thiazolinyle, thiadiazolyle ou thiazolidinyle.

**14.** Dérivé de pénème ou un de ses sels acceptables sur le plan pharmacologique selon la revendication 3, dans lequel ladite fraction hétérocyclique de $R_1$ est un radical benzothiazolyle ou benzothiadiazolyle.

**15.** Dérivé de pénème ou un de ses sels acceptables sur le plan pharmacologique selon la revendication 3, dans lequel ladite fraction hétérocyclique de $R_1$ est un radical furanyle, pyranyle, tétrahydrofuranyle ou tétrahydropyranyle.

**16.** Dérivé de pénème ou un de ses sels acceptables sur le plan pharmacologique selon la revendication 3, dans lequel ladite fraction hétérocyclique de $R_1$ est un radical thiényle ou tétrahydrothiényle.

**17.** Dérivé de pénème ou un de ses sels acceptables sur le plan pharmacologique selon la revendication 1, dans lequel $R_1$ représente un dit radical alkylthio substitué ou non substitué.

**18.** Dérivé de pénème ou un de ses sels acceptables sur le plan pharmacologique selon la revendication 17, dans lequel le radical alkyle dudit radical alkylthio substitué ou non substitué est un radical alkyle en $C_1$ à $C_6$ linéaire ou ramifié, ou un radical alkyle monocyclique ou polycyclique qui peut être sous forme d'un cycle condensé avec un hydrocarbure aromatique.

**19.** Dérivé de pénème ou un de ses sels acceptables sur le plan pharmacologique selon la revendication 17, dans lequel le radical alkyle dudit radical alkylthio substitué ou non substitué est un radical méthyle, éthyle, n-propyle, isopropyle, n-butyle, tert-butyle ou hexyle.

**20.** Dérivé de pénème ou un de ses sels acceptables sur le plan pharmacologique selon la revendication 17, dans lequel le radical alkyle dudit radical alkylthio substitué ou non substitué est un radical alkyle monocyclique ou polycyclique choisi parmi un radical cyclopropyle, cyclopentyle, cyclohexyle, menthyle, fenchyle, bornyle ou indanyle.

**21.** Dérivé de pénème ou un de ses sels acceptables sur le plan pharmacologique selon la revendication 1, dans lequel $R_1$ représente un dit radical alcénylthio substitué ou non substitué.

**22.** Dérivé de pénème ou un de ses sels acceptables sur le plan pharmacologique selon la revendication 21, dans lequel le radical alcényle dudit radical alcénylthio substitué ou non substitué est un radical alcényle en $C_2$ à $C_6$ linéaire ou ramifié.

**23.** Dérivé de pénème ou un de ses sels acceptables sur le plan pharmacologique selon la revendication 21, dans lequel le radical alcényle dudit radical alcénylthio substitué ou non substitué est un radical vinyle, allyle, 2-chloroallyle, 1-propényle, 2-butényl ou 2-méthyl-2-propényle.

**24.** Dérivé de pénème ou un de ses sels acceptables sur le plan pharmacologique selon la revendication 1, dans lequel $R_1$ représente un dit radical aralkylthio substitué ou non substitué.

**25.** Dérivé de pénème ou un de ses sels acceptables sur le plan pharmacologique selon la revendication 24, dans lequel le radical aralkyle dudit radical aralkylthio substitué ou non substitué est un radical aralkyle contenant 7 à 24 atomes de carbone.

**26.** Dérivé de pénème ou un de ses sels acceptables sur le plan pharmacologique selon la revendication 24, dans lequel le radical aralkyle dudit radical aralkylthio substitué ou non substitué est un radical benzyle, phénéthyle, 3-phényl-propyle, 2-naphtylméthyle, 2-(1-naphtyl)-éthyle, trityle, benzhydryle ou 1-phényl-cyclopropan-1-yle.

**27.** Dérivé de pénème ou un de ses sels acceptables sur le plan pharmacologique selon la revendication 1, dans lequel $R_1$ représente un dit radical arylthio substitué ou non substitué.

**28.** Dérivé de pénème ou un de ses sels acceptables sur le plan pharmacologique selon la revendication 27, dans lequel le radical aryle dudit radical arylthio substitué ou non substitué est un radical aryle contenant 6 à 10 atomes de carbone.

**29.** Dérivé de pénème ou un de ses sels acceptables sur le plan pharmacologique selon la revendication 27, dans lequel le radical aryle dudit radical arylthio substitué ou non substitué est un radical phényle, tolyle, xylyle, mésityle, cuményle ou naphtyle.

**30.** Dérivé de pénème ou un de ses sels acceptables sur le plan pharmacologique selon la revendication 1, dans lequel $R_1$ représente un dit radical aryle substitué ou non substitué.

**31.** Dérivé de pénème ou un de ses sels acceptables sur le plan pharmacologique selon la revendication 1, dans lequel $R_1$ représente un dit radical hétérocyclique substitué ou non substitué.

**32.** Dérivé de pénème ou un de ses sels acceptables sur le plan pharmacologique selon la revendication 1, dans lequel $R_1$ est un radical représenté par la formule suivante :

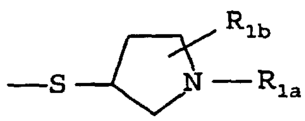

dans laquelle $R_{1a}$ et $R_{1b}$ sont identiques ou différents et représentent un atome d'hydrogène ou un radical alkyle, alcényle, aralkyle en $C_7$ à $C_{14}$, aryle en $C_6$ à $C_{10}$, imino-(alkyle en $C_1$ à $C_6$), imino-(alkyle en $C_1$ à $C_6$)-amino, imino-amino-(alkyle en $C_1$ à $C_6$), carbamonyle, carbamoyl-(alkyle en $C_1$ à $C_6$), acyle, acyl-(alkyle en $C_1$ à $C_6$), carboxyle, hétérocyclique ou alkyle en $C_1$ à $C_6$ hétérocyclique ; un ou plusieurs atomes d'hydrogène dudit radical alkyle, alcényle, aralkyle, aryle, imino- (alkyle en $C_1$ à $C_6$), imino-(alkyle en $C_1$ à $C_6$)-amino, imino-amino-(alkyle en $C_1$ à $C_6$), carbamoyle, carbamoyl-(alkyle en $C_1$ à $C_6$), hétérocyclique ou alkyle en $C_1$ à $C_6$ hétérocyclique éventuellement substitué par un atome d'halogène ou un radical carboxyle, thiocarboxyle, formyle, nitro, cyano, hydroxyle, amino, imino, (alkylène en $C_1$ à $C_6$)acétal, alkyle, alcoxyle, alcényle, aralkyle en $C_7$ à $C_{24}$, aryle en $C_6$ à $C_{10}$, aryloxy en $C_6$ à $C_{10}$, imino-(alkyle en $C_1$ à $C_6$), imino-(alkyle en $C_1$ à $C_6$)-amino, imino-amino-(alkyle en $C_1$ à $C_6$), carbamoyle, carbamoyloxy, carbamoyl-(alkyle en $C_1$ à $C_6$), hétérocyclique, alkyle en $C_1$ à $C_6$ hétérocyclique, acyle ou acylalkyle ; lesdits radicaux ou fractions acyle représentant un radical alkylcarbonyle, alcénylcarbonyle, aralkylcarbonyle, arylcarbonyle, carbonyle hétérocyclique ou (alkyle en $C_1$ à $C_6$)-carbonyle hétérocyclique contenant ledit radical alkyle substitué ou non substitué, alcényle, aralkyle, aryle, hétérocyclique ou alkyle en $C_1$ à $C_6$ hétérocyclique ; ledit radical carboxyle peut être estérifié par ledit radical alkyle substitué ou non substitué, alcényle, aralkyle, aryle, hétérocyclique ou alkyle en $C_1$ à $C_6$ hétérocyclique ; lesdits radicaux ou fractions hétérocycliques contenant éventuellement un ou plusieurs radicaux carbonyle dans leurs cycles et leur atome d'azote tertiaire formant éventuellement un sel quaternaire intramoléculaire par l'introduction dudit substituant.

**33.** Dérivé de pénème ou un de ses sels acceptables sur le plan pharmacologique selon la revendication 1, dans lequel $R_1$ est un radical représenté par la formule suivante :

$$-S-(CH_2)_n-R_{1c}$$

dans laquelle n vaut 1 à 3 ; $R_{1c}$ représente un atome d'hydrogène ou un radical aryle en $C_6$ à $C_{10}$, amino, imino-(alkkyle en $C_1$ à $C_6$)-amino, aminosulfonyle, carbamoyle, acyle, carboxyle ou hétérocyclique ; un ou plusieurs atomes d'hydrogène dudit radical aryle, amino, imino-(alkyle en $C_1$ à $C_6$)-amino, aminosulfonyle, carbamoyle ou hétérocyclique éventuellement substitué par un atome d'halogène ou un radical carboxyle, thiocarboxyle, formyle, nitro, cyano, hydroxyle, amino, imino, alkyle, alcoxy, alcényle, aralkyle en $C_7$ à $C_{24}$, aryle en $C_6$ à $C_{10}$, aryloxy en $C_6$ à $C_{10}$, imino-(alkyle en $C_1$ à $C_6$), imino-(alkyle en $C_1$ à $C_6$)-amino, imino-amino-(alkyle en $C_1$ à $C_6$), carbamoyle, carbamoyloxy, carbamoyl-(alkyle en $C_1$ à $C_6$), hétérocyclique, alkyle en $C_1$ à $C_6$ hétérocyclique, acyle ou acylalkyle ; ledit radical ou fraction acyle désigné en tant que substituant représentant un radical alkylcarbonyle, alcénylcarbonyle, aralkylcarbonyle, arylcarbonyle, carbonyle hétérocyclique ou (alkyle en $C_1$ à $C_6$)-carbonyle hétérocyclique contenant un ou plusieurs radicaux alkyle, alcényle, aralkyle, aryle, hétérocyclique ou alkyle en $C_1$ à $C_6$ hétérocyclique ; un ou plusieurs atomes d'hydrogène de ces radicaux ou fractions acyle éventuellement substituées par un atome d'halogène ou un radical carboxyle, thiocarboxyle, formyle, nitro, cyano, hydroxyle, amino, imino, (alkylène en $C_1$ à $C_6$)-acétal, alkyle, alcoxy, alcényle, aralkyle en $C_7$ à $C_{24}$, aryle en $C_6$ à $C_{10}$, aryloxy en $C_6$ à $C_{10}$, imino-(alkyle en $C_1$ à $C_6$), imino-(alkyle en $C_1$ à $C_6$)-amino, imino-amino-(alkyle en $C_1$ à $C_6$), carbamoyle, carbamoyloxy, carbamoyl-(alkyle en $C_1$ à $C_6$), hétérocyclique, alkyle en $C_1$ à $C_6$ hétérocyclique, acyle ou acylalkyle ; ledit radical carboxyle étant éventuellement estérifié par un radical alkyle substitué ou non substitué, alcényle, aralkyle, aryle, hétérocyclique ou alkyle en $C_1$ à $C_6$ hétérocyclique ; ledit radical hétérocyclique et le radical hétérocyclique desdits radicaux alkyle en $C_1$ à $C_6$ hétérocycliques, le dernier radical hétérocyclique étant désigné tant que substituant, contenant éventuellement un ou plusieurs groupes carbonyle dans son cycle et son atome d'azote tertiaire formant éventuellement un sel quaternaire intramoléculaire par l'introduction dudit substituant.

**34.** Dérivé de pénème selon la revendication 3, choisi parmi :

l'acide (5R,6R)-2-(1-allyl-pyrrolidin-3-yl)thio-6-((S)-1-hydroxypropyl)-pénème-3-carboxylique ;
l'acide (5R,6R)-2-(1-benzylpyrrolidin-3-yl)thio-6-((S)-1-hydroxypropyl)-pénème-6-carboxylique ;
l'acide (5R, 6R)-2-(pyrrolidin-3-yl)thio-6-((S)-1-hydroxypropyl)-pénème-3-carboxylique ;
l'acide (5R,6R)-2-(1-(1-iminoéthyl)pyrrolidin-3-yl)thio-6-((S)-1-hydroxypropyl)-pénème-3-carboxylique ;
l'acide (5R,6R)-6-((S)-1-hydroxypropyl)-2-(1-phénylpyrrolidin-3-yl)thio-pénème-3-carboxylique ;
l'acide (5R,6R)-6-((S)-1-hydroxypropyl)-2-(1-phénéthylpyrrolidin-3-yl)-thiopénème-3-carboxylique ;
l'acide (5R,6R)-6-((S)-1-hydroxypropyl)-2-(1-(2-hydroxy-2-phényléthyl)pyrrolidin-3-yl)thiopénème-3-carboxylique ;
l'acide (5R,6R)-6-((S)-1-hydroxypropyl)-2-(1-(2-hydroxy-2-phényléthyl)pyrrolidin-3-yl)thiopénème-3-carboxylique ;

l'acide (5R,6R)-6-((S)-1-hydroxypropyl)-2-(1-phénacylpyrrolidin-3-yl)-thio-pénème-3-carboxylique ;
l'acide (5R,6R)-2-(1-(2-p-fluorophényl-2-oxoéthyl)pyrrolidin-3-yl)thio-6-((S)-1-hydroxypropyl)pénème-3-carboxylique ;
l'acide (5R,6R)-6-((S)-1-hydroxypropyl)-2-(1-(2-oxo-2-p-tolyl-éthyl)-pyrrolidin-3-yl)thiopénème-3-carboxylique ;
l'acide (5R,6R)-6-((S)-1-hydroxypropyl)-2-(1-(2-p-méthoxyphényl-2-oxo-éthyl)pyrrolidin-3-yl)thiopénème-3-carboxylique ;
l'acide (5R,6R)-2-(1-(2-benzoyléthyl)pyrrolidin-3-yl)thio-6-((S)-1-hydroxypropyl)pénème-3-carboxylique ;
l'acide (5R,6R)-2-(1-(1-benzoyléthyl)pyrrolidin-3-yl)thio-6-((S)-hydroxy-propyl)pénème-3-carboxylique ;
l'acide (5R,6R)-6-((S)-1-hydroxypropyl)-2-(1-(1-indanon-2-yl)pyrrolidin-3-yl)thio-pénème-3-carboxylique ;
l'acide (5R,6R)-6-((S)-1-hydroxypropyl)-2-(1-(1-indanon-2-yl)pyrrolidin-3-yl)thio-pénème-3-carboxylique ;
l'acide (5R,6R)-6-((S)-1-hydroxypropyl)-2-(1- (2-pyridylméthyl)-pyrrolidin-3-yl)-thiopénème-3-carboxylique ;
l'acide (5R,6R)-6-((S)-1-hydroxypropyl)-2-(1-benzylpipéridin-3-yl)thio-pénème-3-carboxylique ;
l'acide (5R,6R)-6-((S)-1-hydroxypropyl)-2-(4-phénylthiazol-2-yl)-thio-pénème-3-carboxylique ;
l'acide (5R,6R)-6-((S)-1-hydroxypropyl)-2-(1-(4-pyridyl)-pyrrolidin-3-yl)thiopénème-3-carboxylique ;
l'acide (5R,6R)-6-((S)-1-hydroxypropyl)-2-(1-(2-pyridyl)-pyrrolidin-3-yl)thiopénème-3-carboxylique ;
l'acide (5R,6R)-6-((S)-1-hydroxypropyl)-2-(1-iminométhyl)-azétidin-3-yl)thiopénème-3-carboxylique ;
l'acide (5R,6R)-6-((S)-1-hydroxypropyl)-2-(1-iminoéthyl)-azétidin-3-yl)thiopénème-3-carboxylique ;
l'acide (5R,6R)-6-((S)-1-hydroxypropyl)-2-(2,3-cyclopento[b]pyridin-7-yl)-thiopénème-3-carboxylique ;
l'acide (5R,6R)-2-(1-(1-iminopropylpyrrolidin-3-yl)thio-6-((S)-1-hydroxypropyl)pénème-3-carboxylique ;
l'acide (5R,6R)-6-((S)-1-hydroxypropyl)-2-(1-(3-benzodioxol-5-yl-acétonyl)pyrrolidin-3-yl)thiopénème-3-carboxylique ;
l'acide (5R,6R)-2-(1-(4-(1-iminoéthylamino)éthyl)-phényl)-pyrrolidin-3-yl)thio-6-((S)-1-hydroxypropyl)pénème-3-carboxylique ;
l'acide (5R,6R)-6-((S)-1-hydroxypropyl)-2-(1-pyrimidin-2-yl-pyrrolidin-3-yl)thiopénème-3-carboxylique ;
l'acide (5R,6R)-6-((S)-1-hydroxypropyl)-2-(1-($\alpha$-iminobenzamidométhyl)-pyrrolidin-3-yl)thiopénème-3-carboxylique ;
l'acide (5R,6R)-6-((S)-1-hydroxypropyl)-2-(1-($\alpha$-imino)anilinométhyl-pyrrolidin-3-yl) thiopénème-3-carboxylique ;
l'acide (5R,6R)-6-((S)-1-hydroxypropyl)-2-(1-cyclo-propylméthyl-pyrrolidin-3-yl)-thiopénème-3-carboxylique ;
l'acide (5R,6R)-6-((S)-1-hydroxypropyl)-2-(1-phénoxyéthylpyrrolidin-3-yl)thiopénème-3-carboxylique ;
l'acide (5R,6R)-6-((S)-1-hydroxypropyl)-2-(1-phénoxypropyl-pyrrolidin-3-yl)thiopénème-3-carboxylique ;
l'acide (5R,6R)-6-((S)-1-hydroxypropyl)-2-(1-o-tolyloxyméthylpyrrolidin-3-yl)thiopénème-3-carboxylique ;
l'acide (5R,6R)-6-((S)-1-hydroxypropyl)-2-(1-indan-2-yl-pyrrolidin-3-yl)thiopénème-3-carboxylique ;
l'acide (5R,6R)-6-((S)-1-hydroxypropyl)-2-(1-cyclo-pentylpyrrolidin-3-yl)thiopénème-3-carboxylique ;
l'acide (5R,6R)-6-((S)-1-hydroxypropyl)-2-(1-(3-phénylallyl)-pyrrolidin-3-yl)thiopénème-3-carboxylique ;
l'acide (5R,6R)-2-(1-(1-imino-2-fluoro-éthyl)pyrrolidin-3-yl)-thio-6-((S)-1-hydroxypropyl)pénème-3-carboxylique ;
l'acide (5R,6R)-2-(1-cyclopropylpyrrolidin-3-yl)thio-6-((S)-1-hydroxypropyl)pénème-3-carboxylique ; et
l'acide (5R,6R)-6-((S)-1-hydroxypropyl)-2-(1-thiazol-2-yl-pyrrolidin-3-yl)thiopénème-3-carboxylique ;

ou un de ses sels acceptables sur le plan pharmacologique.

**35.** Dérivé de pénème selon la revendication 1, choisi parmi :

l'acide (5R,6R)-6-((S)-1-hydroxypropyl)-2-méthylthiopénème-3-carboxylique ;
l'acide (5R,6R)-6-((S)-1-hydroxypropyl)-2-(2-pyridylméthyl)-thiopénème-3-carboxylique ;
l'acide (5R,6R)-6-((S)-1-hydroxypropyl)-2-(benzimidazol-2-yl-méthyl)-thiopénème-3-carboxylique ;
l'acide (5R,6R)-6-((S)-1-hydroxypropyl)-2-((S)-1-(4-pyridyl-méthyl)-thiopénème-3-carboxylique ;
l'acide (5R,6R) -2- (2-(1-iminoéthylamino)éthyl)thio-6- ((S)-1-hydroxypropyl)pénème-3-carboxylique ;
l'acide (5R,6R)-2-(2-($\alpha$-iminobenzyl)aminoéthyl)thio-6-((S)-1-hydroxypropyl)pénème-3-carboxylique ;
l'acide (5R,6R)-6-((S)-1-hydroxypropyl)-2-(2-phényl-aminocarbonyl-éthyl)-thiopénème-3-carboxylique ;
l'acide (5R,6R)-6-((S)-1-hydroxypropyl)-2-(2-(N-méthyl-N-phénacylamino)-éthyl)thiopénème-3-carboxylique ;
l'acide (5R,6R)-6-((S)-1-hydroxypropyl)-2-(2-(pyrrolidin-1-yl)-éthyl)-thiopénème-3-carboxylique ;
l'acide (5R,6R)-6-((S)-1-hydroxypropyl)-2-(2-(1-pyrrolyl)-éthyl)-thiopénème-3-carboxylique ;
l'acide (5R,6R)-2-(2-aminoéthyl)thio-6-((S)-1-hydroxypropyl)pénème-3-carboxylique ;
l'acide (5R,6R)-6-((S)-1-hydroxypropyl)-2-phénylthiopénème-3-carboxylique ;
l'acide (5R,6R)-6-((S)-1-hydroxypropyl)-2-(3,4-dichlorobenzyl)-thiopénème-3-carboxylique ;

l'acide (5R,6R)-6-((S)-1-hydroxypropyl)-2-(3-cyano-benzyl)-thiopénème-3-carboxylique ; et
l'acide (5R,6R)-6-((S)-1-hydroxypropyl)-2-(2-indanyl)-thiopénème-3-carboxylique ; ou

un de ses sels acceptables sur le plan pharmacologique.

**36.** Dérivé de (5R,6R)-6-((S)-1-hydroxypropyl)-pénème représenté par la formule suivante (II) :

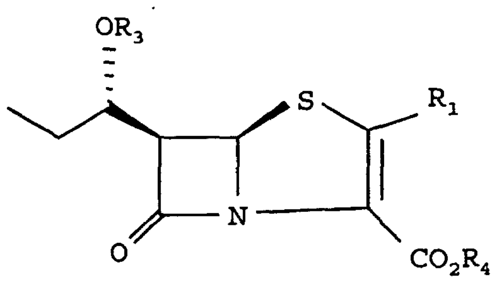

(II)

dans laquelle $R_1$ est tel que défini selon la revendication 1, $OR_3$ représente un radical hydroxyle protégé, et $R_4$ représente un radical de protection d'un groupe carboxyle.

**37.** Dérivé de pénème selon la revendication 36, dans lequel $R_1$ est tel que défini selon l'une quelconque des revendications 3 à 33.

**38.** Dérivé de (5R,6R)-6-((S)-1-hydroxypropyl)-pénème représenté par la formule suivante (III) :

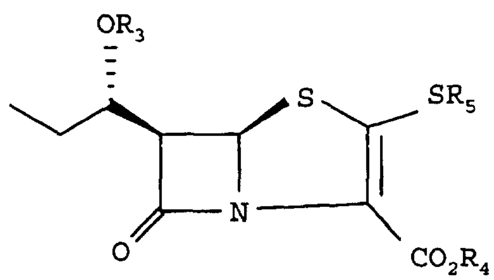

(III)

dans laquelle :

$R_5$ représente un radical alkyle, alcényle, aralkyle en $C_7$ à $C_{14}$, aryle en $C_6$ à $C_{10}$, hétérocyclique ou acyle, l'un quelconque desdits radicaux étant éventuellement substitué par un ou plusieurs éléments choisis parmi les radicaux halogéno ; carboxyle ; thiocarboxyle ; formyle ; nitro ; cyano ; hydroxyle ; amino ; imino ; (alkylène en $C_1$ à $C_6$)acétal ; alkyle en $C_1$ à $C_6$ ; alkyle monocyclique ou polycyclique (qui peut être sous forme d'un cycle condensé avec un hydrocarbure aromatique et peut contenir un ou plusieurs radicaux carbonyle dans sa chaîne ou dans son cycle) ; alcényle en $C_2$ à $C_6$ ; aryle en $C_6$ à $C_{10}$ ; aralkyle en $C_7$ à $C_{24}$ ; (alkyle en $C_1$ à $C_6$)-thio ; (alcényle en $C_2$ à $C_6$)-thio ; (aralkyle en $C_7$ à $C_{24}$)-thio ; (aryle en $C_6$ à $C_{10}$)-thio ; alkyloxy en $C_1$ à $C_6$ ; alcényloxy en $C_2$ à $C_6$ ; aralkyloxy en $C_7$ à $C_{24}$ ; aryloxy en $C_6$ à $C_{10}$ ; (alkyle en $C_1$ à $C_6$)-sulfinyle ; (alkyle en $C_1$ à $C_6$)- sulfonyle ; (aralkyle en $C_7$ à $C_{24}$)-sulfinyle ; (aralkyle en $C_7$ à $C_{24}$)-sulfonyle ; (aryle en $C_6$ à $C_{10}$)-sulfinyle ; (aryle en $C_6$ à $C_{10}$)-sulfonyle ; aminosulfonyle ; carbamoyle ; carbamoyloxy ; carbamoyl-(alkyle en $C_1$ à $C_6$) ; imino-(alkyle en $C_1$ à $C_6$) ; imino-(alkyle en $C_1$ à $C_6$)amino ; imino-amino(alkyle en $C_1$ à $C_6$) ; acyloxy ; acylalkyle ; silyloxy ; hétérocyclique ; thio-hétérocyclique ; oxyhétérocyclique ; acyle ; carboxyle estérifié ; thiocarboxyle estérifié, ou, seulement quand $R_1$ sont des cycles condensés en $C_9$ à $C_{11}$, cycloalkyle en $C_5$ à $C_7$ insaturé ou hétérocyclique contenant éventuellement un ou plusieurs groupes carbonyle dans leurs cycles ; et ledit radical hétérocyclique étant choisi parmi des radicaux hétéromonocycliques, saturés ou insaturés, ayant 3 à 8 éléments contenant 1 à 4 atomes d'azote ; 1 ou 2 atomes d'oxygène et 1 à 3 atomes d'azote ; 1 ou 2 atomes de soufre et 1 à 3 atomes d'azote ; 1 ou 2 atomes d'oxygène ; ou 1 atome de soufre ; et des radicaux hétéropolycycliques, insaturés, ayant 7 à 12 éléments contenant 1 à 5 atomes d'azote ; 1 ou 2 atomes d'oxygène et 1 à 3 atomes d'azote ; ou 1 ou 2 atomes de soufre et 1 à 3 atomes d'azote ; dans laquelle le radical alkyle ou alkyle en tant que partie d'un radical alkylthio, aralkyle ou aralkylthio inclut un radical alkyle monocyclique ou polycyclique qui peut être sous forme d'un cycle condensé avec un hydrocarbure aromatique et peut contenir un ou plusieurs radicaux carbonyle dans sa chaîne ou dans son cycle, $OR_3$ représente un radical hydroxyle protégé, et

$R_4$ représente un radical de protection d'un groupe carboxyle.

**39.** Dérivé de (5R,6R)-6-((S)-1-hydroxypropyl)-pénème représenté par la formule suivante (IV) :

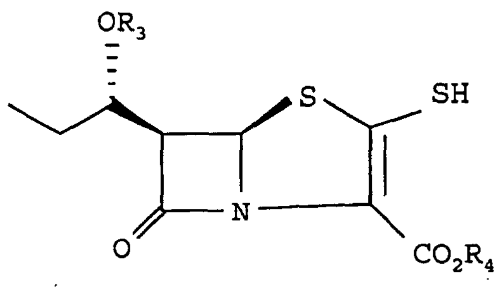

(IV)

dans laquelle $OR_3$ représente un radical hydroxyle protégé et $R_4$ représente un radical de protection d'un groupe carboxyle.

**40.** Médicament comprenant, en tant que principe actif, un dérivé de (5R,6R)-6-((S)-1-hydroxypropyl)-pénème représenté par la formule (I) tel que défini selon la revendication 1 ou un de ses sels acceptables sur le plan pharmacologique.

**41.** Médicament selon la revendication 40, dans lequel le dérivé de pénème est tel que défini selon l'une quelconque des revendications 3 à 39.

**42.** Utilisation dans la fabrication d'un médicament antibactérien d'un dérivé de (5R,6R)-6-((S)-1-hydroxypropyl)-pénème représenté par la formule (I) tel que défini selon la revendication 1 ou un de ses sels acceptables sur le plan pharmacologique.

**43.** Utilisation selon la revendication 42, dans laquelle le dérivé de pénème est tel que défini selon l'une quelconque des revendications 3 à 39.

**44.** Utilisation selon la revendication 2, dans lequel le Dérivé de pénème ou un de ses sels acceptables sur le plan pharmacologique est tel que défini selon l'une quelconque des revendications 3 à 39.

**45.** Dérivé de pénème ou un de ses sels acceptables sur le plan pharmacologique selon la revendication 1 en vue d'une utilisation thérapeutique.

**46.** Dérivé de pénème ou un de ses sels acceptables sur le plan pharmacologique selon la revendication 45, dans lequel le dérivé de pénème est tel que défini selon l'une quelconque des revendications 2 à 39.